(19) Europäisches Patentamt
European Patent Office
Office européen des brevets

(11) **EP 4 551 562 B1**

(12) **EUROPEAN PATENT SPECIFICATION**

(45) Date of publication and mention of the grant of the patent:
**24.06.2026 Bulletin 2026/26**

(21) Application number: **23739207.1**

(22) Date of filing: **05.07.2023**

(51) International Patent Classification (IPC):
***C07D 281/10*** (2006.01) ***C07D 285/36*** (2006.01)
***A61P 1/16*** (2006.01) ***A61P 3/06*** (2006.01)
***A61P 5/00*** (2006.01) ***A61P 9/00*** (2006.01)
***A61K 31/554*** (2006.01)

(52) Cooperative Patent Classification (CPC):
**C07D 281/10; A61P 1/16; A61P 3/06; A61P 5/00; A61P 9/00; C07D 285/36**

(86) International application number:
**PCT/EP2023/068476**

(87) International publication number:
**WO 2024/008766 (11.01.2024 Gazette 2024/02)**

(54) **BENZOTHIA(DI)AZEPINE COMPOUNDS AND THEIR USE AS BILE ACID MODULATORS**

BENZOTHIA(DI)AZEPIN-VERBINDUNGEN UND IHRE VERWENDUNG ALS GALLENSÄUREMODULATOREN

COMPOSÉS DE BENZOTHIA (DI) AZÉPINE ET LEUR UTILISATION EN TANT QUE MODULATEURS D'ACIDE BILIAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.07.2022 IN 202211038517**

(43) Date of publication of application:
**14.05.2025 Bulletin 2025/20**

(73) Proprietor: **Albireo AB**
**413 46 Göteborg (SE)**

(72) Inventors:
- **STARKE, Ingemar**
  **413 01 Göteborg (SE)**
- **GILLBERG, Per-Göran**
  **694 96 Åsbro (SE)**
- **STRÄNGBERG, Ellen**
  **437 31 Lindome (SE)**
- **KULKARNI, Santosh S.**
  **Bangalore 560078 (IN)**

(74) Representative: **Novitas Patent AB**
**P.O. Box 55557**
**102 04 Stockholm (SE)**

(56) References cited:
**EP-A2- 3 210 977**
**WO-A1-01/66533**
**WO-A1-2022/029101**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 551 562 B1

## Description

### TECHNICAL FIELD

The invention relates to 1,5-benzothiazepine and 1,2,5-benzothiadiazepine derivatives of formula (I). These compounds are bile acid modulators having apical sodium-dependent bile acid transporter (ASBT) and/or liver bile acid transport (LBAT) inhibitory activity. The invention also relates to pharmaceutical compositions comprising these compounds and to the use of these compounds in the treatment of cardiovascular diseases, fatty acid metabolism and glucose utilization disorders, gastrointestinal diseases and liver diseases.

### BACKGROUND

Bile acids are physiological detergents that play an important role in the intestinal absorption and transport of lipids, nutrients and vitamins. They are also signaling molecules that activate nuclear receptors and cell signaling pathways that regulate lipid, glucose and energy metabolism. Bile acids are steroid acids that are synthesized from cholesterol in the liver and stored in the gallbladder as mixed micelles. During digestion, the duodenum triggers the release of hormones that cause the gallbladder to contract, thereby releasing bile acids in the small intestine where they enable absorption of fat-soluble vitamins and cholesterol. When they reach the ileum, bile acids are reabsorbed from the intestine and secreted into portal blood to return to the liver via the portal venous circulation. Over 90% of the bile acids are thus recycled and returned to the liver. These bile acids are then transported across the sinusoidal membrane of hepatocytes and re-secreted across the canalicular membrane into bile. In this first pass, 75-90% of bile acids are taken up by hepatocytes, completing one round of enterohepatic circulation. The fraction of bile acids that escapes being cleared in the liver enters the systemic circulation where the free bile acids are filtered by the renal glomerulus, efficiently reclaimed in the proximal tubules and exported back into the systemic circulation. Interestingly, most of the bile acids secreted across the canalicular membrane into bile are derived from the recirculating pool with less than 10% coming from new *de novo* hepatic synthesis. The small fraction of bile acids that is not reabsorbed in the ileum reaches the colon. Within the intestinal lumen, the primary bile acids are transformed into secondary bile acids under the action of intestinal bacteria, mainly by single or dual dehydroxylation reactions of the steroid nucleus. The bile acids that escape intestinal absorption are thereafter excreted into the faeces.

Overall, the efficient transport system helps maintain a constant bile acid pool, ensuring sufficiently high levels of conjugated bile acids in the intestine to promote lipid absorption as well as reduce the small intestinal bacterial load. The system also minimizes fecal and urinary bile acid loss and protects the intestinal and hepatobiliary compartments by eliminating potentially cytotoxic detergents (as reviewed by Kosters and Karpen (Xenobiotica 2008, vol. 38, p. 1043-1071); by Chiang (J. Lipid Res. 2009, vol. 50, p. 1955-1966); and by Dawson (Handb. Exp. Pharmacol. 2011, vol. 201, p. 169-203)).

The regulation of the bile acid pool size has been found to play a key role in cholesterol homeostasis by hepatic conversion of cholesterol to bile acid, which represents a major route for elimination of cholesterol from the body. The liver plays an essential role in removing endogenous and xenobiotic compounds from the body. The normal hepatobiliary secretion and enterohepatic circulation are required for the elimination of endogenous compounds such as cholesterol and bilirubin and their metabolites from the body, thereby maintaining lipid and bile acid homeostasis. (Kosters and Karpen, Xenobiotica 2008, vol. 38, p. 1043-1071).

The reabsorption of bile acids in the ileum may be inhibited by apical sodium-dependent bile acid transporter (ASBT) inhibitor compounds. Inhibition of bile acid reabsorption has been reported useful in the treatment of several diseases, including dyslipidemia, diabetes, obesity, constipation, cholestatic liver diseases, non-alcoholic steatohepatitis and other hepatic diseases. A number of ASBT inhibitor compounds has been disclosed over the past decades, see e.g. WO 93/16055, WO 94/18183, WO 94/18184, WO 96/05188, WO 96/08484, WO 96/16051, WO 97/33882, WO 98/03818, WO 98/07449, WO 98/40375, WO 99/35135, WO 99/64409, WO 99/64410, WO 00/47568, WO 00/61568, WO 00/38725, WO 00/38726, WO 00/38727, WO 00/38728, WO 00/38729, WO 01/66533, WO 01/68096, WO 02/32428, WO 02/50051, WO 03/020710, WO 03/022286, WO 03/022825, WO 03/022830, WO 03/061663, WO 03/091232, WO 03/106482, WO 2004/006899, WO 2004/076430, WO 2007/009655, WO 2007/009656, WO 2011/137135, WO 2019/234077, WO 2020/161216, WO 2020/161217, WO 2021/110883, WO 2021/110884, WO 2021/110885, WO 2021/110886, WO 2021/110887, WO 2022/029101, DE 19825804, EP 864582, EP 489423, EP 549967, EP 573848, EP 624593, EP 624594, EP 624595, EP 624596, EP 0864582, EP 1173205, EP 1535913 and EP 3210977.

Despite the number of ASBT inhibitor compounds that have been previously reported, there is a need for additional bile acid modulating compounds that have an optimized profile with respect to potency, selectivity and bioavailability.

DETAILED DESCRIPTION OF THE INVENTION

It has been discovered that certain 1,5-benzothiazepine and 1,2,5-benzothiadiazepine derivates are potent inhibitors of apical sodium-dependent bile acid transporter (ASBT) and/or liver bile acid transporter (LBAT), and may be useful for treating diseases wherein inhibition of bile acid circulation is desirable.

Any references in the description to methods of treatment refer to the compounds, pharmaceutical compositions and medicaments of the present invention for use in a method for treatment of the human (or animal) body by therapy (or for diagnosis).

In a first aspect, therefore, the invention relates to a compound of formula (I)

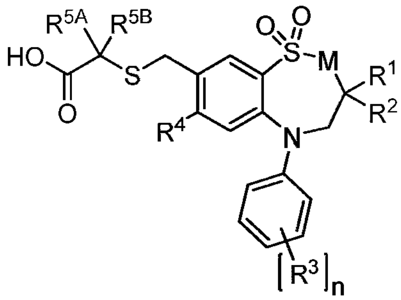

(I)

wherein

- M is $-CH_2-$ or $-NR^6-$;
- $R^1$ is $C_{1-4}$ alkyl;
- $R^2$ is selected from the group consisting of hydrogen and $C_{1-4}$ alkyl;
- $R^3$ is independently selected from the group consisting of hydrogen, halogen, hydroxy, $C_{1-4}$ alkyl, $C_{1-4}$ haloalkyl, $C_{1-4}$ alkoxy, $C_{1-4}$ haloalkoxy, cyano, nitro, amino, *N*-($C_{1-4}$ alkyl)amino and *N,N*-di($C_{1-4}$ alkyl)amino;
- n is an integer 1, 2 or 3;
- $R^4$ is selected from the group consisting of hydrogen, halogen, cyano, $C_{1-4}$ alkyl, $C_{3-6}$ cycloalkyl, $C_{1-4}$ alkoxy, $C_{3-6}$ cycloalkyloxy, $C_{1-4}$ alkylthio, $C_{3-6}$ cycloalkylthio, $C_{1-4}$ alkylsulfonyl, $C_{3-6}$ cycloalkylsulfonyl, amino, *N*-($C_{1-4}$ alkyl)amino and *N,N*-di($C_{1-4}$ alkyl)amino;
- $R^{5A}$ and $R^{5B}$ are each independently selected from the group consisting of hydrogen, halogen, hydroxy, $C_{1-4}$ alkyl and $C_{1-4}$ alkoxy; or $R^{5A}$ and $R^{5B}$, together with the carbon atom to which they are attached, form a 3- to 5-membered saturated carbocyclic ring; and
- $R^6$ is selected from the group consisting of hydrogen and $C_{1-4}$ alkyl;

or a pharmaceutically acceptable salt thereof.

In some embodiments, $R^1$ is $C_{2-4}$ alkyl. In a preferred embodiment, $R^1$ is *n*-butyl.

In some embodiments, $R^2$ is hydrogen. In some embodiments, $R^2$ is methyl, ethyl or *n*-butyl.

In some embodiments, $R^3$ is independently selected from the group consisting of hydrogen, fluoro, chloro, bromo, hydroxy, methoxy, amino, methylamino and dimethylamino. In a preferred embodiment, n is 1, i.e. the phenyl-ring is substituted with only one substituent $R^3$. In another preferred embodiment, $R^3$ is in the para-position.

In some embodiments, $R^4$ is selected from the group consisting of fluoro, chloro, bromo, methyl, ethyl, cyclopropyl, methoxy, ethoxy, methylthio, ethylthio, methylsulfonyl, amino, methylamino and dimethylamino. In some embodiments, $R^4$ is methoxy, methylthio, methylsulfonyl or dimethylamino. In some embodiments, $R^4$ is methoxy or methylthio.

In some embodiments, $R^{5A}$ and $R^{5B}$ are each independently hydrogen or $C_{1-4}$ alkyl, or together with the carbon atom to which they are attached form a cyclopropyl ring. In some embodiments, $R^{5A}$ and $R^{5B}$ are each independently hydrogen or methyl.

In some embodiments, $R^6$ is hydrogen. In some embodiments, $R^6$ is methyl.

In a preferred embodiment, the compound of formula (I) is a compound of formula (I-a):

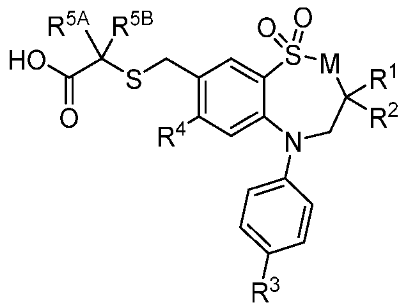

(I-a)

wherein

M is selected from the group consisting of $-CH_2-$, -NH- and $-NCH_3-$;
$R^1$ is $C_{2-4}$ alkyl;
$R^2$ is selected from the group consisting of hydrogen and $C_{1-4}$ alkyl;
$R^3$ is selected from the group consisting of hydrogen, fluoro, chloro, bromo, hydroxy, methoxy, amino, methylamino, dimethylamino;
$R^4$ is selected from the group consisting of fluoro, chloro, bromo, methyl, cyclopropyl, methoxy, ethoxy, methylthio, ethylthio, methylsulfonyl, amino, methylamino and dimethylamino;
$R^{5A}$ and $R^{5B}$ are each independently hydrogen or $C_{1-4}$ alkyl, or together with the carbon atom to which they are attached, form a cyclopropyl ring;

or a pharmaceutically acceptable salt thereof.

In another preferred embodiment, the compound of formula (I) is a compound of formula (I-a), wherein

M is selected from the group consisting of $-CH_2-$, -NH- and $-N(CH_3)-$;
$R^1$ is n-butyl;
$R^2$ is selected from the group consisting of hydrogen and $C_{1-4}$ alkyl;
$R^3$ is selected from the group consisting of hydrogen and fluoro;
$R^4$ is methoxy, methylthio, methylsulfonyl or dimethylamino;
$R^{5A}$ and $R^{5B}$ are each independently hydrogen or methyl;

or a pharmaceutically acceptable salt thereof.

In a particular embodiment, the compound of formula (I) is selected from the group consisting of:

2-(((3-butyl-3-ethyl-7-(methylthio)-1,1-dioxido-5-phenyl-2,3,4,5-tetrahydro-1,5-benzothiazepin-8-yl)methyl)thio) acetic acid;
(S)-2-(((3-butyl-3-ethyl-7-(methylthio)-1,1-dioxido-5-phenyl-2,3,4,5-tetrahydro-1,5-benzothiazepin-8-yl)methyl) thio)acetic acid;
(R)-2-(((3-butyl-3-ethyl-7-(methylthio)-1,1-dioxido-5-phenyl-2,3,4,5-tetrahydro-1,5-benzothiazepin-8-yl)methyl) thio)acetic acid;
2-(((3,3-dibutyl-7-(methylthio)-1,1-dioxido-5-phenyl-2,3,4,5-tetrahydro-1,5-benzothiazepin-8-yl)methyl)thio)acetic acid;
2-(((3-butyl-3-methyl-7-(methylthio)-1,1-dioxido-5-phenyl-2,3,4,5-tetrahydro-1,5-benzothiazepin-8-yl)methyl)thio) acetic acid;
(S)-2-(((3-butyl-3-methyl-7-(methylthio)-1,1-dioxido-5-phenyl-2,3,4,5-tetrahydro-1,5-benzothiazepin-8-yl)methyl) thio)acetic acid;
(R)-2-(((3-butyl-3-methyl-7-(methylthio)-1,1-dioxido-5-phenyl-2,3,4,5-tetrahydro-1,5-benzothiazepin-8-yl)methyl) thio)acetic acid;
2-(((3-butyl-3-ethyl-5-(4-fluorophenyl)-7-(methylthio)-1,1-dioxido-2,3,4,5-tetrahydro-1,5-benzothiazepin-8-yl) methyl)thio)acetic acid;
(S)-2-(((3-butyl-3-ethyl-5-(4-fluorophenyl)-7-(methylthio)-1,1-dioxido-2,3,4,5-tetrahydro-1,5-benzothiazepin-8-yl) methyl)thio)acetic acid;
(R)-2-(((3-butyl-3-ethyl-5-(4-fluorophenyl)-7-(methylthio)-1,1-dioxido-2,3,4,5-tetrahydro-1,5-benzothiazepin-8-yl) methyl)thio)acetic acid;
2-(((3,3-dibutyl-7-(methylthio)-1,1-dioxido-5-phenyl-2,3,4,5-tetrahydro-1,2,5-benzothiadiazepin-8-yl)methyl)thio) acetic acid;

2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid;
(S)-2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid;
(R)-2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid;
2-(((3-butyl-2-methyl-7-(methylthio)-1,1-dioxido-5-phenyl-2,3,4,5-tetrahydro-1,2,5-benzothiadiazepin-8-yl)methyl)thio)acetic acid;
(S)-2-(((3-butyl-2-methyl-7-(methylthio)-1,1-dioxido-5-phenyl-2,3,4,5-tetrahydro-1,2,5-benzothiadiazepin-8-yl)methyl)thio)acetic acid;
(R)-2-(((3-butyl-2-methyl-7-(methylthio)-1,1-dioxido-5-phenyl-2,3,4,5-tetrahydro-1,2,5-benzothiadiazepin-8-yl)methyl)thio)acetic acid;
2-(((3-Butyl-3-ethyl-7-methoxy-1,1-dioxido-5-phenyl-2,3,4,5-tetrahydro-1,5-benzothiazepin-8-yl)methyl)thio)acetic acid;
(S)-2-(((3-butyl-3-ethyl-7-methoxy-1,1-dioxido-5-phenyl-2,3,4,5-tetrahydro-1,5-benzothiazepin-8-yl)methyl)thio)acetic acid;
(R)-2-(((3-butyl-3-ethyl-7-methoxy-1,1-dioxido-5-phenyl-2,3,4,5-tetrahydro-1,5-benzothiazepin-8-yl)methyl)thio)acetic acid;
2-(((3-Butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid;
(S)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid;
(R)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid;
2-(((3,3-Dibutyl-7-(methylthio)-1,1-dioxido-5-phenyl-2,3,4,5-tetrahydro-1,5-benzothiazepin-8-yl)methyl)thio)-2-methylpropanoic acid;
2-(((3-Butyl-3-methyl-7-(methylthio)-1,1-dioxido-5-phenyl-2,3,4,5-tetrahydro-1,5-benzothiazepin-8-yl)methyl)thio)-2-methylpropanoic acid;
(S)-2-(((3-butyl-3-methyl-7-(methylthio)-1,1-dioxido-5-phenyl-2,3,4,5-tetrahydro-1,5-benzothiazepin-8-yl)methyl)thio)-2-methylpropanoic acid;
(R)-2-(((3-butyl-3-methyl-7-(methylthio)-1,1-dioxido-5-phenyl-2,3,4,5-tetrahydro-1,5-benzothiazepin-8-yl)methyl)thio)-2-methylpropanoic acid;
2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
(S)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
(R)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
2-(((3,3-dibutyl-2-methyl-7-(methylthio)-1,1-dioxido-5-phenyl-2,3,4,5-tetrahydro-1,2,5-benzothiadiazepin-8-yl)methyl)thio)acetic acid;
2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoic acid;
2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoic acid;
2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
(S)-2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
(R)-2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
2-(((3-butyl-7-methoxy-3-methyl-1,1-dioxido-5-phenyl-2,3,4,5-tetrahydro-1,5-benzothiazepin-8-yl)methyl)thio)acetic acid;
(S)-2-(((3-butyl-7-methoxy-3-methyl-1,1-dioxido-5-phenyl-2,3,4,5-tetrahydro-1,5-benzothiazepin-8-yl)methyl)thio)acetic acid;
(R)-2-(((3-butyl-7-methoxy-3-methyl-1,1-dioxido-5-phenyl-2,3,4,5-tetrahydro-1,5-benzothiazepin-8-yl)methyl)thio)acetic acid;
2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;

(S)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;
(R)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;
2-(((3,3-dibutyl-7-methoxy-1,1-dioxido-5-phenyl-2,3,4,5-tetrahydro-1,5-benzothiazepin-8-yl)methyl)thio)acetic acid;
2-(((3,3-dibutyl-5-(4-fluorophenyl)-7-methoxy-1,1-dioxido-2,3,4,5-tetrahydro-1,5-benzothiazepin-8-yl)methyl)thio) acetic acid;
2-(((3-butyl-3-ethyl-5-(4-fluorophenyl)-7-methoxy-1,1-dioxido-2,3,4,5-tetrahydro-1,5-benzothiazepin-8-yl)methyl) thio)-2-methylpropanoic acid;
(S)-2-(((3-butyl-3-ethyl-5-(4-fluorophenyl)-7-methoxy-1,1-dioxido-2,3,4,5-tetrahydro-1,5-benzothiazepin-8-yl) methyl)thio)-2-methylpropanoic acid;
(R)-2-(((3-butyl-3-ethyl-5-(4-fluorophenyl)-7-methoxy-1,1-dioxido-2,3,4,5-tetrahydro-1,5-benzothiazepin-8-yl) methyl)thio)-2-methylpropanoic acid;
2-(((3-butyl-7-methoxy-2-methyl-1,1-dioxido-5-phenyl-2,3,4,5-tetrahydro-1,2,5-benzothiadiazepin-8-yl)methyl) thio)acetic acid;
(S)-2-(((3-butyl-7-methoxy-2-methyl-1,1-dioxido-5-phenyl-2,3,4,5-tetrahydro-1,2,5-benzothiadiazepin-8-yl)methyl) thio)acetic acid;
(R)-2-(((3-butyl-7-methoxy-2-methyl-1,1-dioxido-5-phenyl-2,3,4,5-tetrahydro-1,2,5-benzothiadiazepin-8-yl)methyl) thio)acetic acid;
2-(((3-butyl-7-methoxy-2-methyl-1,1-dioxido-5-phenyl-2,3,4,5-tetrahydro-1,2,5-benzothiadiazepin-8-yl)methyl) thio)-2-methylpropanoic acid;
(S)-2-(((3-butyl-7-methoxy-2-methyl-1,1-dioxido-5-phenyl-2,3,4,5-tetrahydro-1,2,5-benzothiadiazepin-8-yl)methyl) thio)-2-methylpropanoic acid;
(R)-2-(((3-butyl-7-methoxy-2-methyl-1,1-dioxido-5-phenyl-2,3,4,5-tetrahydro-1,2,5-benzothiadiazepin-8-yl)methyl) thio)-2-methylpropanoic acid;
2-(((3-butyl-3-ethyl-5-(4-fluorophenyl)-7-methoxy-1,1-dioxido-2,3,4,5-tetrahydro-1,5-benzothiazepin-8-yl)methyl) thio)acetic acid;
(S)-2-(((3-butyl-3-ethyl-5-(4-fluorophenyl)-7-methoxy-1,1-dioxido-2,3,4,5-tetrahydro-1,5-benzothiazepin-8-yl) methyl)thio)acetic acid;
(R)-2-(((3-butyl-3-ethyl-5-(4-fluorophenyl)-7-methoxy-1,1-dioxido-2,3,4,5-tetrahydro-1,5-benzothiazepin-8-yl) methyl)thio)acetic acid;
2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid;
(S)-2-(((3-butyl-7-methoxy-3-methyl-1,1-dioxido-5-phenyl-2,3,4,5-tetrahydro-1,5-benzothiazepin-8-yl)methyl) thio)-2-methylpropanoic acid;
(R)-2-(((3-butyl-7-methoxy-3-methyl-1,1-dioxido-5-phenyl-2,3,4,5-tetrahydro-1,5-benzothiazepin-8-yl)methyl) thio)-2-methylpropanoic acid;
2-(((3,3-dibutyl-7-methoxy-1,1-dioxido-5-phenyl-2,3,4,5-tetrahydro-1,5-benzothiazepin-8-yl)methyl)thio)-2-methylpropanoic acid;
2-(((3-butyl-3-ethyl-7-methoxy-1,1-dioxido-5-phenyl-2,3,4,5-tetrahydro-1,2,5-benzothiadiazepin-8-yl)methyl)thio) acetic acid;
(R)-2-(((3-butyl-3-ethyl-7-methoxy-1,1-dioxido-5-phenyl-2,3,4,5-tetrahydro-1,2,5-benzothiadiazepin-8-yl)methyl) thio)acetic acid;
(S)-2-(((3-butyl-3-ethyl-7-methoxy-1,1-dioxido-5-phenyl-2,3,4,5-tetrahydro-1,2,5-benzothiadiazepin-8-yl)methyl) thio)acetic acid;
2-(((3-butyl-7-(dimethylamino)-3-ethyl-1,1-dioxido-5-phenyl-2,3,4,5-tetrahydro-1,2,5-benzothiadiazepin-8-yl) methyl)thio)acetic acid;
2-(((3-butyl-3-ethyl-7-(methylsulfonyl)-1,1-dioxido-5-phenyl-2,3,4,5-tetrahydro-1,2,5-benzothiadiazepin-8-yl) methyl)thio)acetic acid;
(S)-2-(((3-Butyl-3-ethyl-7-(methylsulfonyl)-1,1-dioxido-5-phenyl-2,3,4,5-tetrahydro-1,2,5-benzothiadiazepin-8-yl) methyl)thio)acetic acid;
(R)-2-(((3-butyl-3-ethyl-7-(methylsulfonyl)-1,1-dioxido-5-phenyl-2,3,4,5-tetrahydro-1,2,5-benzothiadiazepin-8-yl) methyl)thio)acetic acid;
(R)-2-(((3-butyl-7-(dimethylamino)-1,1-dioxido-5-phenyl-2,3,4,5-tetrahydro-1,2,5-benzothiadiazepin-8-yl)methyl) thio)acetic acid;
(R)-2-(((3-butyl-7-(methylthio)-1,1-dioxido-5-phenyl-2,3,4,5-tetrahydro-1,2,5-benzothiadiazepin-8-yl)methyl)thio) acetic acid;

(R)-2-(((3-butyl-7-methoxy-1,1-dioxido-5-phenyl-2,3,4,5-tetrahydro-1,2,5-benzothiadiazepin-8-yl)methyl)thio) acetic acid;
2-(((3-Butyl-7-(dimethylamino)-3-methyl-1,1-dioxido-5-phenyl-2,3,4,5-tetrahydro-1,2,5-benzothiadiazepin-8-yl) methyl)thio)acetic acid;
2-(((3-butyl-7-methoxy-3-methyl-1,1-dioxido-5-phenyl-2,3,4,5-tetrahydro-1,2,5-benzothiadiazepin-8-yl)methyl) thio)acetic acid; and
2-(((3-Butyl-3-methyl-7-(methylthio)-1,1-dioxido-5-phenyl-2,3,4,5-tetrahydro-1,2,5-benzothiadiazepin-8-yl)methyl) thio)acetic acid;

or a pharmaceutically acceptable salt thereof.

As used herein, the term "halo" refers to fluoro, chloro, bromo and iodo.

As used herein, the term "$C_{1-6}$ alkyl" refers to a straight or branched alkyl group having from 1 to 6 carbon atoms, and the term "$C_{1-4}$ alkyl" refers to a straight or branched alkyl group having from 1 to 4 carbon atoms. Examples of $C_{1-4}$ alkyl include methyl, ethyl, n-propyl, isopropyl, n-butyl, isobutyl, sec-butyl and tert-butyl.

As used herein, the term "$C_{3-6}$ cycloalkyl" refers to a monocyclic saturated hydrocarbon ring having from 3 to 6 carbon atoms. Examples of $C_{3-6}$ cycloalkyl include cyclopropyl, cyclobutyl, cyclopentyl and cyclohexyl.

As used herein, the term "$C_{1-4}$ haloalkyl" refers to a straight or branched $C_{1-4}$ alkyl group, as defined herein, wherein one or more hydrogen atoms have been replaced with halogen. Examples of $C_{1-4}$ haloalkyl include chloromethyl, fluoroethyl and trifluoromethyl.

As used herein, the terms "$C_{1-4}$ alkoxy" and "$C_{1-4}$ alkylthio" refer to a straight or branched $C_{1-4}$ alkyl group attached to the remainder of the molecule through an oxygen or sulphur atom, respectively.

As used herein, the term "$C_{1-4}$ alkylsulfonyl" refers to a straight or branched $C_{1-4}$ alkyl group attached to the remainder of the molecule through a sulfonyl ($-S(O)_2-$) group.

The term "amino" refers to an $-NH_2$ group. As used herein, the terms "*N*-($C_{1-4}$ alkyl)amino" and "N,N-di($C_{1-4}$ alkyl) amino" refer to an amino group wherein one or both hydrogen atom(s), respectively, are replaced with a straight or branched $C_{1-4}$ alkyl group. Examples of *N*-($C_{1-4}$ alkyl)amino include methylamino, ethylamino and tert-butylamino, and examples of *N,N*-di-($C_{1-4}$ alkyl)amino include dimethylamino and diethylamino.

As used herein, the term "pharmaceutically acceptable" refers to those compounds, materials, compositions and/or dosage forms that are suitable for human pharmaceutical use and that are generally safe, non-toxic and neither biologically nor otherwise undesirable.

As used herein, the term "about" refers to a value or parameter herein that includes (and describes) embodiments that are directed to that value or parameter per se. For example, description referring to "about 20" includes description of "20." Numeric ranges are inclusive of the numbers defining the range. Generally speaking, the term "about" refers to the indicated value of the variable and to all values of the variable that are within the experimental error of the indicated value (e.g., within the 95% confidence interval for the mean) or within 10 percent of the indicated value, whichever is greater.

The 1,5-benzothiazepine and 1,2,5-benzothiadiazepine compounds of formula (I), or pharmaceutically acceptable salts thereof, are inhibitors of the apical sodium-dependent bile acid transporter (ASBT inhibitors), of the liver bile acid transporter (LBAT inhibitors), or of both the apical sodium-dependent bile acid and liver bile acid transporters (dual ASBT/LBAT inhibitors). They are therefore useful in the treatment or prevention of conditions, disorders and diseases wherein inhibition of bile acid circulation is desirable, such as cardiovascular diseases, fatty acid metabolism and glucose utilization disorders, gastrointestinal diseases and liver diseases.

Cardiovascular diseases and disorders of fatty acid metabolism and glucose utilization include, but are not limited to, hypercholesterolemia; disorders of fatty acid metabolism; type 1 and type 2 diabetes mellitus; complications of diabetes, including cataracts, micro- and macrovascular diseases, retinopathy, neuropathy, nephropathy and delayed wound healing, tissue ischaemia, diabetic foot, arteriosclerosis, myocardial infarction, acute coronary syndrome, unstable angina pectoris, stable angina pectoris, stroke, peripheral arterial occlusive disease, cardiomyopathy, heart failure, heart rhythm disorders and vascular restenosis; diabetes-related diseases such as insulin resistance (impaired glucose homeostasis), hyperglycemia, hyperinsulinemia, elevated blood levels of fatty acids or glycerol, obesity, dyslipidemia, hyperlipidemia including hypertriglyceridemia, metabolic syndrome (syndrome X), atherosclerosis and hypertension; and for increasing high density lipoprotein levels.

Gastrointestinal diseases and disorders include constipation (including chronic constipation, functional constipation, chronic idiopathic constipation (CIC), intermittent/sporadic constipation, constipation secondary to diabetes mellitus, constipation secondary to stroke, constipation secondary to chronic kidney disease, constipation secondary to multiple sclerosis, constipation secondary to Parkinson's disease, constipation secondary to systemic sclerosis, drug induced constipation, irritable bowel syndrome with constipation (IBS-C), irritable bowel syndrome mixed (IBS-M), pediatric functional constipation and opioid induced constipation); Crohn's disease; primary bile acid malabsorption; irritable bowel syndrome (IBS); inflammatory bowel disease (IBD); ileal inflammation; and reflux disease and complications thereof, such as Barrett's esophagus, bile reflux esophagitis and bile reflux gastritis.

A liver disease as defined herein is any disease in the liver and in organs connected therewith, such as the pancreas, portal vein, the liver parenchyma, the intrahepatic biliary tree, the extrahepatic biliary tree, and the gall bladder. In some cases, a liver disease a bile acid-dependent liver disease. Liver diseases and disorders include, but are not limited to, an inherited metabolic disorder of the liver; inborn errors of bile acid synthesis; congenital bile duct anomalies; biliary atresia; post-Kasai biliary atresia; post-liver transplantation biliary atresia; neonatal hepatitis; neonatal cholestasis; hereditary forms of cholestasis; cerebrotendinous xanthomatosis; a secondary defect of BA synthesis; Zellweger's syndrome; cystic fibrosis-associated liver disease; alpha1-antitrypsin deficiency; Alagilles syndrome (ALGS); Byler syndrome; a primary defect of bile acid (BA) synthesis; progressive familial intrahepatic cholestasis (PFIC) including PFIC-1, PFIC-2, PFIC-3 and non-specified PFIC, post-biliary diversion PFIC and post-liver transplant PFIC; benign recurrent intrahepatic cholestasis (BRIC) including BRIC1, BRIC2 and non-specified BRIC, post-biliary diversion BRIC and post-liver transplant BRIC; autoimmune hepatitis; primary biliary cirrhosis (PBC); liver fibrosis; non-alcoholic fatty liver disease (NAFLD); non-alcoholic steatohepatitis (NASH); portal hypertension; cholestasis; Down syndrome cholestasis; drug-induced cholestasis; intrahepatic cholestasis of pregnancy (jaundice during pregnancy); intrahepatic cholestasis; extrahepatic cholestasis; parenteral nutrition associated cholestasis (PNAC); low phospholipid-associated cholestasis; lymphedema cholestasis syndrome 1 (LCS1); primary sclerosing cholangitis (PSC); immunoglobulin G4 associated cholangitis; primary biliary cholangitis; cholelithiasis (gallstones); biliary lithiasis; choledocholithiasis; gallstone pancreatitis; Caroli disease; malignancy of bile ducts; malignancy causing obstruction of the biliary tree; biliary strictures; AIDS cholangiopathy; ischemic cholangiopathy; pruritus due to cholestasis or jaundice; pancreatitis; chronic autoimmune liver disease leading to progressive cholestasis; hepatic steatosis; alcoholic hepatitis; acute fatty liver; fatty liver of pregnancy; drug-induced hepatitis; iron overload disorders; congenital bile acid synthesis defect type 1 (BAS defect type 1); drug-induced liver injury (DILI); hepatic fibrosis; congenital hepatic fibrosis; hepatic cirrhosis; Langerhans cell histiocytosis (LCH); neonatal ichthyosis sclerosing cholangitis (NISCH); erythropoietic protoporphyria (EPP); idiopathic adulthood ductopenia (IAD); idiopathic neonatal hepatitis (INH); non syndromic paucity of interlobular bile ducts (NS PILBD); North American Indian childhood cirrhosis (NAIC); hepatic sarcoidosis; amyloidosis; necrotizing enterocolitis; serum bile acid-caused toxicities, including cardiac rhythm disturbances (e.g., atrial fibrillation) in setting of abnormal serum bile acid profile, cardiomyopathy associated with liver cirrhosis ("cholecardia"), and skeletal muscle wasting associated with cholestatic liver disease; polycystic liver disease; viral hepatitis (including hepatitis A, hepatitis B, hepatitis C, hepatitis D and hepatitis E); hepatocellular carcinoma (hepatoma); cholangiocarcinoma; bile acid-related gastrointestinal cancers; and cholestasis caused by tumours and neoplasms of the liver, of the biliary tract and of the pancreas. Compounds of formula (I), or pharmaceutically acceptable salts thereof, are also useful in the enhancement of corticosteroid therapy in liver disease.

Other diseases that may be treated or prevented by the compounds of formula (I), or pharmaceutically acceptable salts thereof, include hyperabsorption syndromes (including abetalipoproteinemia, familial hypobetalipoproteinemia (FHBL), chylomicron retention disease (CRD) and sitosterolemia); hypervitaminosis and osteopetrosis; hypertension; glomerular hyperfiltration; polycystic kidney disease (PKD), including autosomal dominant polycystic kidney disease (ADPKD) and autosomal recessive polycystic kidney disease (ARPKD); and pruritus of renal failure. The compounds are also useful in the protection against liver- or metabolic disease-associated kidney injury.

The transport of bile acids in the human body is controlled by the action of the members of the SLC10 family of solute carrier proteins, in particular by the $Na^+$-taurocholate cotransporting polypeptide (NTCP, also called liver bile acid transporter (LBAT); gene symbol *SLC10A1),* which is expressed in the sinusoidal membrane of hepatocytes, and by the apical sodium dependent bile acid transporter (ASBT, also called ileal bile acid transporter (IBAT), ISBT, ABAT or NTCP2; gene symbol *SLC10A2),* which is expressed in the apical membrane of ileal enterocytes, proximal renal tubule cells, biliary epithelium, large cholangiocytes and gallbladder epithelial cells. In the liver, bile acids are efficiently extracted from portal blood by the liver bile acid transporter (LBAT) and re-secreted across the canalicular membrane by the bile salt export pump (BSEP; gene symbol *ABCB11).* The reabsorption of bile acids in the ileum is handled by the apical sodium-dependent bile acid transporter (ASBT), where it is commonly referred to as ileal bile acid transporter (IBAT). Both LBAT and ASBT function as electrogenic sodium-solute cotransporters that move two or more $Na^+$ ions per molecule of solute.

Xenobiotics and endobiotics, including bile acids, are taken up by the liver from portal blood and secreted into bile by distinct transport proteins with individualized substrate specificities. Glycine-and taurine-conjugated bile acids exist in anionic form and are unable to cross membranes by diffusion, and thus, are completely dependent on membrane transport proteins to enter or exit the hepatocyte (Kosters and Karpen, Xenobiotica 2008, vol. 38, p. 1043-1071). ASBT and LBAT prefer glycine- and taurine-conjugated bile salts over their unconjugated counterparts and demonstrate a higher affinity for dihydroxy bile salts than for trihydroxy bile salts. No non-bile acid substrates have been identified for ASBT yet, however, LBAT was also found to transport a variety of steroid sulfates, hormones and xenobiotics.

LBAT is not as thoroughly characterized as ASBT in terms of drug inhibition requirements. Dong et al. have identified FDA approved drugs that inhibit human LBAT and compared LBAT and ASBT inhibition requirements. A series of LBAT inhibition studies were performed using FDA approved drugs, in concert with iterative computational model development. Screening studies identified 27 drugs as novel LBAT inhibitors, including irbesartan (Ki =11.9 $\mu$M) and

ezetimibe (Ki = 25.0 μM). The common feature pharmacophore indicated that two hydrophobes and one hydrogen bond acceptor were important for inhibition of LBAT. From 72 drugs screened in vitro, a total of 31 drugs inhibited LBAT, while 51 drugs (i.e. more than half) inhibited ASBT. Hence, while there was inhibitor overlap, ASBT unexpectedly was more permissive to drug inhibition than was LBAT, and this may be related to LBAT's possessing fewer pharmacophore features (Dong et al., Mol. Pharm. 2013, vol. 10, p. 1008-1019).

Vaz et al. describe the identification of LBAT deficiency as a new inborn error of metabolism with a relatively mild clinical phenotype. The identification of LBAT deficiency confirms that this transporter is the main import system for conjugated bile salts into the liver, but also indicates that auxiliary transporters are able to sustain the enterohepatic cycle in its absence (Vaz et al., Hepatology 2015, vol. 61, p. 260-267). These findings support the hypothesis that LBAT inhibition is a safe mechanism of action, as the hepatocytes still have the possibility to take up the necessary amount of bile acids.

Liu et al. describe the identification of a new type of hypercholanemia that is associated with homozygosity for the p.Ser267Phe mutation in *SLC10A1* (LBAT). The allele frequency of this mutation in gene *SLC10A1* varies in different populations, with the highest incidence occurring in Southern China (8% and 12% in Chinese Han and Dai respectively) and in Vietnam (11%). This "hidden" hypercholanemia was believed to affect 0.64% of the Southern Han, 1.44% of the Dai Chinese population, and 1.21% of the Vietnamese population. An increase in conjugated and unconjugated serum BA levels in the homozygous individuals was also observed. Liu et al. suggest that this finding is most likely due to reduced BA transport from the portal circulation into hepatocytes. This supports the hypothesis that the physiological function of the enterohepatic circulation is not only to recycle bile acids but also to clear bile acids from the circulation to achieve homeostasis (Karpen and Dawson, Hepatology 2015, vol. 61, p. 24-27). Alternatively, the liver may synthesize increased levels of bile acids to compensate for the reduced enterohepatic recirculation in the homozygous carriers. As LBAT also transports unconjugated bile acids, the increase of the unconjugated bile acids in this study was not surprising (Liu et al., Scientific Reports 2017, 7: 9214, p. 1-7).

LBAT has been found to be downregulated in several forms of cholestatic liver injury and cholestasis, whereas ASBT has been found to be downregulated in a variety of gastrointestinal disorders such as Crohn's disease, primary bile acid malabsorption, inflammatory bowel disease, and ileal inflammation but upregulated in cholestasis. LBAT also functions as a cellular receptor for viral entry of the hepatitis B virus (HBV) and hepatitis D virus (HDV), which in turn is the major cause of liver disease and hepatocellular carcinoma.

ASBT inhibition has been investigated for decreasing plasma cholesterol levels and improving insulin resistance, as well as to relieving the hepatic bile acid burden in cholestatic liver disease. In addition, ASBT inhibition has been found to restore insulin levels and normoglycemia, thus establishing ASBT inhibition as a promising treatment for type 2 diabetes mellitus. ASBT inhibitors are also used for treatment of functional constipation.

As ASBT is predominantly expressed in the ileum (where it is often referred to as IBAT), ASBT inhibitors need not be systemically available. On the other hand, ASBT is also expressed in the proximal tubule cells of the kidneys. ASBT inhibitors that are systemically available may therefore also inhibit the reuptake of bile acids in the kidneys. It is believed that this would lead to increased levels of bile acids in urine, and to an increased removal of bile acids from the body via the urine. Systemically available ASBT inhibitors that exert their effect not only in the ileum but also in the kidneys are therefore expected to lead to a greater reduction of bile acid levels than non-systemically available ASBT inhibitors that only exert their effect in the ileum.

Compounds having a high ASBT inhibiting potency are particularly suitable for the treatment of liver diseases that cause cholestasis, such as progressive familial intrahepatic cholestasis (PFIC), Alagilles syndrome, biliary atresia and non-alcoholic steatohepatitis (NASH).

Biliary atresia is a rare pediatric liver disease that involves a partial or total blockage (or even absence) of large bile ducts. This blockage or absence causes cholestasis that leads to the accumulation of bile acids that damages the liver. In some embodiments, the accumulation of bile acids occurs in the extrahepatic biliary tree. In some embodiments, the accumulation of bile acids occurs in the intrahepatic biliary tree. The current standard of care is the Kasai procedure, which is a surgery that removes the blocked bile ducts and directly connects a portion of the small intestine to the liver. There are currently no approved drug therapies for this disorder.

Provided herein is a compound of formula (I), or a pharmaceutically acceptable salt thereof, for use in the treatment of biliary atresia. In some embodiments, the subject has undergone the Kasai procedure prior to administration of a compound of formula (I), or a pharmaceutically acceptable salt thereof. In some embodiments, the subject is administered a compound of formula (I), or a pharmaceutically acceptable salt thereof, prior to undergoing the Kasai procedure. In some embodiments, the treatment of biliary atresia decreases the level of serum bile acids in the subject. In some embodiments, the level of serum bile acids is determined by, for example, an ELISA enzymatic assay or the assays for the measurement of total bile acids as described in Danese et al., PLoS One. 2017, vol. 12(6): e0179200. In some embodiments, the level of serum bile acids can decrease by, for example, 10% to 40%, 20% to 50%, 30% to 60%, 40% to 70%, 50% to 80%, or by more than 90% of the level of serum bile acids prior to administration of a compound of formula (I), or a pharmaceutically acceptable salt thereof. In some embodiments, the treatment of bilary atresia includes treatment of pruritus.

PFIC is a rare genetic disorder that is estimated to affect between one in every 50,000 to 100,000 children born worldwide and causes progressive, life-threatening liver disease.

One manifestation of PFIC is pruritus, which often results in a severely diminished quality of life. In some cases, PFIC leads to cirrhosis and liver failure. Current therapies include Partial External Biliary Diversion (PEBD) and liver transplantation, however, these options can carry substantial risk of post-surgical complications, as well as psychological and social issues.

Three alternative gene defects have been identified that correlate to three separate PFIC subtypes known as types 1, 2 and 3:

- PFIC, type 1, which is sometimes referred to as "Byler disease," is caused by impaired bile secretion due to mutations in the ATP8B1 gene, which codes for a protein that helps to maintain an appropriate balance of fats known as phospholipids in cell membranes in the bile ducts. An imbalance in these phospholipids is associated with cholestasis and elevated bile acids in the liver. Subjects affected by PFIC, type 1 usually develop cholestasis in the first months of life and, in the absence of surgical treatment, progress to cirrhosis and end-stage liver disease before the end of the first decade of life.

- PFIC, type 2, which is sometimes referred to as "Byler syndrome," is caused by impaired bile salt secretion due to mutations in the ABCB11 gene, which codes for a protein, known as the bile salt export pump, that moves bile acids out of the liver. Subjects with PFIC, type 2 often develop liver failure within the first few years of life and are at increased risk of developing a type of liver cancer known as hepatocellular carcinoma.

- PFIC, type 3, which typically presents in the first years of childhood with progressive cholestasis, is caused by mutations in the ABCB4 gene, which codes for a transporter that moves phospholipids across cell membranes.

In addition, TJP2 gene, NR1H4 gene or Myo5b gene mutations have been proposed to be causes of PFIC. In addition, some subjects with PFIC do not have a mutation in any of the ATP8B1, ABCB11, ABCB4, TJP2, NR1H4 or Myo5b genes. In these cases, the cause of the condition is unknown.

Exemplary mutations of the ATP8B1 gene or the resulting protein are listed in Tables 2 and 3, with numbering based on the human wild type ATP8B1 protein (e.g., SEQ ID NO: 1) or gene (e.g., SEQ ID NO: 2). Exemplary mutations of the ABCB11 gene or the resulting protein are listed in Tables 4 and 5, with numbering based on the human wild type ABCB11 protein (e.g., SEQ ID NO: 3) or gene (e.g., SEQ ID NO: 4).

As can be appreciated by those skilled in the art, an amino acid position in a reference protein sequence that corresponds to a specific amino acid position in SEQ ID NO: 1 or 3 can be determined by aligning the reference protein sequence with SEQ ID NO: 1 or 3 (e.g., using a software program, such as ClustalW2). Changes to these residues (referred to herein as "mutations") may include single or multiple amino acid substitutions, insertions within or flanking the sequences, and deletions within or flanking the sequences. As can be appreciated by those skilled in the art, an nucleotide position in a reference gene sequence that corresponds to a specific nucleotide position in SEQ ID NO: 2 or 4 can be determined by aligning the reference gene sequence with SEQ ID NO: 2 or 4 (e.g., using a software program, such as ClustalW2). Changes to these residues (referred to herein as "mutations") may include single or multiple nucleotide substitutions, insertions within or flanking the sequences, and deletions within or flanking the sequences. See also Kooistra, et al., "KLIFS: A structural kinase-ligand interaction database," Nucleic Acids Res. 2016, vol. 44, no. D1, pp. D365-D371.

Canonical protein sequence of ATP8B1 (SEQ ID NO: 1) - Uniprot ID O43520

MSTERDSETT FDEDSQPNDE VVPYSDDETE DELDDQGSAV EPEQNRVNRE AEENREPFRK
ECTWQVKAND RKYHEQPHFM NTKFLCIKES KYANNAIKTY KYNAFTFIPM NLFEQFKRAA
NLYFLALLIL QAVPQISTLA WYTTLVPLLV VLGVTAIKDL VDDVARHKMD KEINNRTCEV
IKDGRFKVAK WKEIQVGDVI RLKKNDFVPA DILLLSSSEP NSLCYVETAE LDGETNLKFK
MSLEITDQYL QREDTLATFD GFIECEEPNN RLDKFTGTLF WRNTSFPLDA DKILLRGCVI
RNTDFCHGLV IFAGADTKIM KNSGKTRFKR TKIDYLMNYM VYTIFVVLIL LSAGLAIGHA
YWEAQVGNSS WYLYDGEDDT PSYRGFLIFW GYIIVLNTMV PISLYVSVEV IRLGQSHFIN
WDLQMYYAEK DTPAKARTTT LNEQLGQIHY IFSDKTGTLT QNIMTFKKCC INGQIYGDHR
DASQHNHNKI EQVDFSWNTY ADGKLAFYDH YLIEQIQSGK EPEVRQFFFL LAVCHTVMVD
RTDGQLNYQA ASPDEGALVN AARNFGFAFL ARTQNTITIS ELGTERTYNV LAILDFNSDR
KRMSIIVRTP EGNIKLYCKG ADTVIYERLH RMNPTKQETQ DALDIFANET LRTLCLCYKE
IEEKEFTEWN KKFMAASVAS TNRDEALDKV YEEIEKDLIL LGATAIEDKL QDGVPETISK
LAKADIKIWV LTGDKKETAE NIGFACELLT EDTTICYGED INSLLHARME NQRNRGGVYA
KFAPPVQESF FPPGGNRALI ITGSWLNEIL LEKKTKRNKI LKLKFPRTEE ERRMRTQSKR
RLEAKKEQRQ KNFVDLACEC SAVICCRVTP KQKAMVVDLV KRYKKAITLA IGDGANDVNM
IKTAHIGVGI SGQEGMQAVM SSDYSFAQFR YLQRLLLVHG RWSYIRMCKF LRYFFYKNFA
FTLVHFWYSF FNGYSAQTAY EDWFITLYNV LYTSLPVLLM GLLDQDVSDK LSLRFPGLYI
VGQRDLLFNY KRFFVSLLHG VLTSMILFFI PLGAYLQTVG QDGEAPSDYQ SFAVTIASAL
VITVNFQIGL DTSYWTFVNA FSIFGSIALY FGIMFDFHSA GIHVLFPSAF QFTGTASNAL
RQPYIWLTII LAVAVCLLPV VAIRFLSMTI WPSESDKIQK HRKRLKAEEQ WQRRQQVFRR
GVSTRRSAYA FSHQRGYADL ISSGRSIRKK RSPLDAIVAD GTAEYRRTGD S

Canonical DNA Sequence for ATP8B1 (SEQ ID NO: 2)

ATG AGT ACA GAA AGA GAC TCA GAA ACG ACA TTT GAC GAG GAT TCT CAG CCT
AAT GAC GAA GTG GTT CCC TAC AGT GAT GAT GAA ACA GAA GAT GAA CTT GAT
GAC CAG GGG TCT GCT GTT GAA CCA GAA CAA AAC CGA GTC AAC AGG GAA GCA
GAG GAG AAC CGG GAG CCA TTC AGA AAA GAA TGT ACA TGG CAA GTC AAA GCA
AAC GAT CGC AAG TAC CAC GAA CAA CCT CAC TTT ATG AAC ACA AAA TTC TTG
TGT ATT AAG GAG AGT AAA TAT GCG AAT AAT GCA ATT AAA ACA TAC AAG TAC
AAC GCA TTT ACC TTT ATA CCA ATG AAT CTG TTT GAG CAG TTT AAG AGA GCA
GCC AAT TTA TAT TTC CTG GCT CTT CTT ATC TTA CAG GCA GTT CCT CAA ATC
TCT ACC CTG GCT TGG TAC ACC ACA CTA GTG CCC CTG CTT GTG GTG CTG GGC
GTC ACT GCA ATC AAA GAC CTG GTG GAC GAT GTG GCT CGC CAT AAA ATG GAT
AAG GAA ATC AAC AAT AGG ACG TGT GAA GTC ATT AAG GAT GGC AGG TTC AAA
GTT GCT AAG TGG AAA GAA ATT CAA GTT GGA GAC GTC ATT CGT CTG AAA AAA
AAT GAT TTT GTT CCA GCT GAC ATT CTC CTG CTG TCT AGC TCT GAG CCT AAC
AGC CTC TGC TAT GTG GAA ACA GCA GAA CTG GAT GGA GAA ACC AAT TTA AAA
TTT AAG ATG TCA CTT GAA ATC ACA GAC CAG TAC CTC CAA AGA GAA GAT ACA
TTG GCT ACA TTT GAT GGT TTT ATT GAA TGT GAA GAA CCC AAT AAC AGA CTA
GAT AAG TTT ACA GGA ACA CTA TTT TGG AGA AAC ACA AGT TTT CCT TTG GAT
GCT GAT AAA ATT TTG TTA CGT GGC TGT GTA ATT AGG AAC ACC GAT TTC TGC
CAC GGC TTA GTC ATT TTT GCA GGT GCT GAC ACT AAA ATA ATG AAG AAT AGT
GGG AAA ACC AGA TTT AAA AGA ACT AAA ATT GAT TAC TTG ATG AAC TAC ATG
GTT TAC ACG ATC TTT GTT GTT CTT ATT CTG CTT TCT GCT GGT CTT GCC ATC
GGC CAT GCT TAT TGG GAA GCA CAG GTG GGC AAT TCC TCT TGG TAC CTC TAT
GAT GGA GAA GAC GAT ACA CCC TCC TAC CGT GGA TTC CTC ATT TTC TGG GGC
TAT ATC ATT GTT CTC AAC ACC ATG GTA CCC ATC TCT CTC TAT GTC AGC GTG
GAA GTG ATT CGT CTT GGA CAG AGT CAC TTC ATC AAC TGG GAC CTG CAA ATG
TAC TAT GCT GAG AAG GAC ACA CCC GCA AAA GCT AGA ACC ACC ACA CTC AAT

GAA CAG CTC GGG CAG ATC CAT TAT ATC TTC TCT GAT AAG ACG GGG ACA CTC
ACA CAA AAT ATC ATG ACC TTT AAA AAG TGC TGT ATC AAC GGG CAG ATA TAT
GGG GAC CAT CGG GAT GCC TCT CAA CAC AAC CAC AAC AAA ATA GAG CAA GTT
GAT TTT AGC TGG AAT ACA TAT GCT GAT GGG AAG CTT GCA TTT TAT GAC CAC
TAT CTT ATT GAG CAA ATC CAG TCA GGG AAA GAG CCA GAA GTA CGA CAG TTC
TTC TTC TTG CTC GCA GTT TGC CAC ACA GTC ATG GTG GAT AGG ACT GAT GGT
CAG CTC AAC TAC CAG GCA GCC TCT CCC GAT GAA GGT GCC CTG GTA AAC GCT
GCC AGG AAC TTT GGC TTT GCC TTC CTC GCC AGG ACC CAG AAC ACC ATC ACC
ATC AGT GAA CTG GGC ACT GAA AGG ACT TAC AAT GTT CTT GCC ATT TTG GAC
TTC AAC AGT GAC CGG AAG CGA ATG TCT ATC ATT GTA AGA ACC CCA GAA GGC
AAT ATC AAG CTT TAC TGT AAA GGT GCT GAC ACT GTT ATT TAT GAA CGG TTA
CAT CGA ATG AAT CCT ACT AAG CAA GAA ACA CAG GAT GCC CTG GAT ATC TTT
GCA AAT GAA ACT CTT AGA ACC CTA TGC CTT TGC TAC AAG GAA ATT GAA GAA
AAA GAA TTT ACA GAA TGG AAT AAA AAG TTT ATG GCT GCC AGT GTG GCC TCC
ACC AAC CGG GAC GAA GCT CTG GAT AAA GTA TAT GAG GAG ATT GAA AAA GAC
TTA ATT CTC CTG GGA GCT ACA GCT ATT GAA GAC AAG CTA CAG GAT GGA GTT
CCA GAA ACC ATT TCA AAA CTT GCA AAA GCT GAC ATT AAG ATC TGG GTG CTT
ACT GGA GAC AAA AAG GAA ACT GCT GAA AAT ATA GGA TTT GCT TGT GAA CTT
CTG ACT GAA GAC ACC ACC ATC TGC TAT GGG GAG GAT ATT AAT TCT CTT CTT
CAT GCA AGG ATG GAA AAC CAG AGG AAT AGA GGT GGC GTC TAC GCA AAG TTT
GCA CCT CCT GTG CAG GAA TCT TTT TTT CCA CCC GGT GGA AAC CGT GCC TTA
ATC ATC ACT GGT TCT TGG TTG AAT GAA ATT CTT CTC GAG AAA AAG ACC AAG
AGA AAT AAG ATT CTG AAG CTG AAG TTC CCA AGA ACA GAA GAA GAA AGA CGG
ATG CGG ACC CAA AGT AAA AGG AGG CTA GAA GCT AAG AAA GAG CAG CGG CAG
AAA AAC TTT GTG GAC CTG GCC TGC GAG TGC AGC GCA GTC ATC TGC TGC CGC
GTC ACC CCC AAG CAG AAG GCC ATG GTG GTG GAC CTG GTG AAG AGG TAC AAG
AAA GCC ATC ACG CTG GCC ATC GGA GAT GGG GCC AAT GAC GTG AAC ATG ATC
AAA ACT GCC CAC ATT GGC GTT GGA ATA AGT GGA CAA GAA GGA ATG CAA GCT
GTC ATG TCG AGT GAC TAT TCC TTT GCT CAG TTC CGA TAT CTG CAG AGG CTA
CTG CTG GTG CAT GGC CGA TGG TCT TAC ATA AGG ATG TGC AAG TTC CTA CGA
TAC TTC TTT TAC AAA AAC TTT GCC TTT ACT TTG GTT CAT TTC TGG TAC TCC
TTC TTC AAT GGC TAC TCT GCG CAG ACT GCA TAC GAG GAT TGG TTC ATC ACC
CTC TAC AAC GTG CTG TAC ACC AGC CTG CCC GTG CTC CTC ATG GGG CTG CTC
GAC CAG GAT GTG AGT GAC AAA CTG AGC CTC CGA TTC CCT GGG TTA TAC ATA
GTG GGA CAA AGA GAC TTA CTA TTC AAC TAT AAG AGA TTC TTT GTA AGC TTG
TTG CAT GGG GTC CTA ACA TCG ATG ATC CTC TTC TTC ATA CCT CTT GGA GCT
TAT CTG CAA ACC GTA GGG CAG GAT GGA GAG GCA CCT TCC GAC TAC CAG TCT
TTT GCC GTC ACC ATT GCC TCT GCT CTT GTA ATA ACA GTC AAT TTC CAG ATT
GGC TTG GAT ACT TCT TAT TGG ACT TTT GTG AAT GCT TTT TCA ATT TTT GGA
AGC ATT GCA CTT TAT TTT GGC ATC ATG TTT GAC TTT CAT AGT GCT GGA ATA
CAT GTT CTC TTT CCA TCT GCA TTT CAA TTT ACA GGC ACA GCT TCA AAC GCT
CTG AGA CAG CCA TAC ATT TGG TTA ACT ATC ATC CTG GCT GTT GCT GTG TGC
TTA CTA CCC GTC GTT GCC ATT CGA TTC CTG TCA ATG ACC ATC TGG CCA TCA
GAA AGT GAT AAG ATC CAG AAG CAT CGC AAG CGG TTG AAG GCG GAG GAG CAG
TGG CAG CGA CGG CAG CAG GTG TTC CGC CGG GGC GTG TCA ACG CGG CGC TCG
GCC TAC GCC TTC TCG CAC CAG CGG GGC TAC GCG GAC CTC ATC TCC TCC GGG
CGC AGC ATC CGC AAG AAG CGC TCG CCG CTT GAT GCC ATC GTG GCG GAT GGC
ACC GCG GAG TAC AGG CGC ACC GGG GAC AGC TGA

**Table 2. Exemplary ATP8B1 Mutations**

| |
|---|
| Amino acid position 3 (e.g., T3K)[27] |
| Amino acid position 23 (e.g., P23L)[5] |
| Amino acid position 45 (e.g., N45T)[5,8,9] |
| Amino acid position 46 (e.g., R46X)[A,25] |
| Amino acid position 62 (e.g., C62R)[28] |
| Amino acid position 63 (e.g., T63T)[41] |
| Amino acid position 70 (e.g., D70N)[1,6] |
| Amino acid position 71 (e.g., R71H)[43] |
| Amino acid position 78 (e.g., H78Q)[19] |
| Amino acid position 82 (e.g., T82T)[41] |
| Amino acid position 92 (e.g., Y92Y)[41] |
| Amino acid position 93 (e.g., A93A)[6] |
| Amino acid position 96 (e.g., A96G)[27] |
| Amino acid position 114 (e.g., E114Q)[8] |
| Amino acid position 127 (e.g., L127P[6], L127V[36]) |
| Amino acid position 177 (e.g., T177T)[6] |
| Amino acid position 179 (e.g., E179X)[29] |
| Δ Amino acid positions 185-282[44] |
| Amino acid position 197 (e.g., G197Lfs*10)[22] |
| Amino acid position 201 (e.g., R201S[27], R201H[35]) |
| Amino acid position 203 (e.g., K203E[5,8], K203R[9], K203fs[25]) |
| Amino acid position 205 (e.g., N205fs[6], N205Kfs*2[35]) |
| Amino acid position 209 (e.g., P209T)[4] |
| Amino acid position 217 (e.g., S217N)[43] |
| Amino acid position 232 (e.g., D232D)[30] |
| Amino acid position 233 (e.g., G233R)[38] |
| Amino acid position 243 (e.g., L243fs*28)[33] |
| Amino acid position 265 (e.g., C265R)[25] |
| Amino acid position 271 (e.g., R271X[13], R271R[30]) |
| Amino acid position 288 (e.g., L288S)[6] |
| Amino acid position 294 (e.g., L294S)[43] |
| Amino acid position 296 (e.g., R296C)[11] |
| Amino acid position 305 (e.g., F305I)[23] |
| Amino acid position 306 (e.g., C306R)[23] |
| Amino acid position 307 (e.g., H307L)[35] |
| Amino acid position 308 (e.g., G308V[1], G308D[6], G308S[35]) |
| Amino acid position 314 (e.g., G314S)[13] |
| Amino acid position 320 (e.g., M320Vfs*13)[11] |
| Amino acid position 337 (e.g., M337R)[18] |
| Amino acid position 338 (e.g., N338K)[18] |

(continued)

| |
|---|
| Amino acid position 340 (e.g., M340V)[18] |
| Amino acid position 344 (e.g., I344F)[6,20] |
| Amino acid position 349 (e.g., I349T)[41] |
| Amino acid position 358 (e.g., G358R)[28] |
| Amino acid position 367 (e.g., G367G)[41] |
| Amino acid position 368 (e.g., N368D)[41] |
| Amino acid position 393 (e.g., I393V)[27] |
| Amino acid position 403 (e.g., S403Y)[6] |
| Amino acid position 407 (e.g., S407N)[40] |
| Amino acid position 412 (e.g., R412P)[6] |
| Amino acid position 415 (e.g., Q415R)[27] |
| Amino acid position 422 (e.g., D422H)[35] |
| Amino acid position 429 (e.g., E429A)[6] |
| Amino acid position 446 (e.g., G446R)[4,11] |
| Amino acid position 453 (e.g., S453Y)[6] |
| Amino acid position 454 (e.g., D454G)[6] |
| Amino acid position 455 (e.g., K455N)[43] |
| Amino acid position 456 (e.g., T456M[3,6], T456K[35]) |
| Amino acid position 457 (e.g., G457G[6], G457fs*6[33]) |
| Amino acid position 469 (e.g., C469G)[41] |
| Amino acid position 478 (e.g., H478H)[41] |
| Amino acid position 500 (e.g., Y500H)[6] |
| Amino acid position 525 (e.g., R525X)[4] |
| Δ Amino acid position 529[6] |
| Amino acid position 535 (e.g., H535L[6], H535N[41]) |
| Amino acid position 553 (e.g., P553P)[43] |
| Amino acid position 554 (e.g., D554N[1,6], D554A[35]) |
| Δ Amino acid positions 556-628[44] |
| Δ Amino acid positions 559-563[35] |
| Amino acid position 570 (e.g., L570L)[41] |
| Amino acid position 577 (e.g., I577V)[19] |
| Amino acid position 581 (e.g., E581K)[35] |
| Amino acid positions 554 and 581 (e.g., D554A+E581K)[35] |
| Amino acid position 585 (e.g., E585X)[21] |
| Amino acid position 600 (e.g., R600W[2,4], R600Q[6]) |
| Amino acid position 602 (e.g., R602X)[3,6] |
| Amino acid position 628 (e.g., R628W)[6] |
| Amino acid position 631 (e.g., R631Q)[28] |
| Δ Amino acid positions 645-699[4] |
| Amino acid position 661 (e.g., I661T)[1,4,6] |

(continued)

| |
|---|
| Amino acid position 665 (e.g., E665X)[4,6] |
| Amino acid position 672 (e.g., K672fs[6], K672Vfs*1[35]) |
| Amino acid position 674 (e.g., M674T)[19] |
| Amino acid positions 78 and 674 (e.g., H78Q/M674T)[19] |
| Amino acid position 684 (e.g., D684D)[41] |
| Amino acid position 688 (e.g., D688G)[6] |
| Amino acid position 694 (e.g., I694T[6], I694N[17]) |
| Amino acid position 695 (e.g., E695K)[27] |
| Amino acid position 709 (e.g., K709fs[6], K709Qfs*41[13]) |
| Amino acid position 717 (e.g., T717N)[4] |
| Amino acid position 733 (e.g., G733R)[6] |
| Amino acid position 757 (e.g., Y757X)[4] |
| Amino acid position 749 (e.g., L749P)[21] |
| Amino acid position 792 (e.g., P792fs)[6] |
| Δ Amino acid position 795-797[6] |
| Amino acid position 809 (e.g., I809L)[27] |
| Amino acid position 814 (e.g., K814N)[28] |
| Amino acid position 833 (e.g., R833Q[27], R833W[41]) |
| Amino acid position 835 (e.g., K835Rfs*36)[35] |
| Amino acid position 845 (e.g., K845fs)[25] |
| Amino acid position 849 (e.g., R849Q)[24] |
| Amino acid position 853 (e.g., F853S, F853fs)[6] |
| Amino acid position 867 (e.g., R867C[1], R867fs[6], R867H[23]) |
| Amino acid position 885 (e.g., K885T)[41] |
| Amino acid position 888 (e.g., T888T)[41] |
| Amino acid position 892 (e.g., G892R)[6] |
| Amino acid position 912 (e.g., G912R)[35] |
| Amino acid position 921 (e.g., S921S)[41] |
| Amino acid position 924 (e.g., Y924C)[28] |
| Amino acid position 930 (e.g., R930X[6], R930Q[28]) |
| Amino acid position 941 (e.g., R941X)[35] |
| Amino acid position 946 (e.g., R946T)[41] |
| Amino acid position 952 (e.g., R952Q[5,9,15] , R952X[6]) |
| Amino acid position 958 (e.g., N958fs)[6] |
| Amino acid position 960 (e.g., A960A)[41] |
| Δ Amino acid position 971[43] |
| Amino acid position 976 (e.g., A976E[41], A976A[43]) |
| Amino acid position 981 (e.g., E981K)[20] |
| Amino acid position 994 (e.g., S994R)[4] |
| Amino acid position 1011 (e.g., L1011fs*18)[33] |

(continued)

| |
|---|
| Amino acid position 1012 (e.g., S10121)[10] |
| Amino acid position 1014 (e.g., R1014X)[6,11] |
| Amino acid position 1015 (e.g., F1015L)[27] |
| Amino acid position 1023 (e.g., Q1023fs)[6] |
| Amino acid position 1040 (e.g., G1040R)[1,6] |
| Amino acid position 1044 (e.g., S0144L)[34] |
| Amino acid position 1047 (e.g., L1047fs)[6] |
| Amino acid position 1050 (e.g., I1050K)[31] |
| Amino acid position 1052 (e.g., L1052R)[28] |
| Amino acid position 1095 (e.g., W1095X)[11] |
| Amino acid position 1098 (e.g., V1098X)[35] |
| Amino acid position 1131 (e.g., Q1131X)[44] |
| Amino acid position 1142 (e.g., A1142Tfs*35)[43] |
| Amino acid position 1144 (e.g., Y1144Y)[43] |
| Amino acid position 1150 (e.g., I1150T)[41] |
| Amino acid position 1152 (e.g., A1152T)[36] |
| Amino acid position 1159 (e.g., P1159P)[25,43] |
| Amino acid position 1164 (e.g., R1164X)[6] |
| Amino acid position 1193 (e.g., R1193fs*39)[33] |
| Amino acid position 1197 (e.g., V1197L)[41] |
| Amino acid position 1208 (e.g., A1208fs)[6] |
| Amino acid position 1209 (e.g., Y1209Lfs*28)[4] |
| Amino acid position 1211 (e.g., F1211L)[27] |
| Amino acid position 1219 (e.g., D1219H[5], D1219G[27]) |
| Amino acid position 1223 (e.g., S1223S)[41] |
| Amino acid position 1233 (e.g., P1233P)[41] |
| Amino acid position 1241 (e.g., G1241fs)[6] |
| Amino acid position 1248 (e.g., T1248T)[43] |
| Splice site mutation IVS3+1_+3delGTG[6] |
| Splice site mutation IVS3-2A>G[6] |
| IVS6+5T>G[17,25] |
| Splice site mutation IVS8+1G>T[6] |
| IVS9-G>A[26] |
| IVS12+1G>A[25] |
| Splice site mutation IVS17-1G>A[6] |
| Splice site mutation IVS18+2T>C[6] |
| Splice site mutation IVS20-4CT>AA |
| Splice site mutation IVS21+5G>A[6] |
| Splice site mutation IVS23-3C>A[6] |
| Splice site mutation IVS26+2T>A[6] |

(continued)

| |
|---|
| g.24774-42062del[4] |
| c.-4C>G[41] |
| c.145C>T[12] |
| c.181-72G>A[9] |
| c.182-5T>A[41] |
| c.182-72G>A[41] |
| c.246A>G[9] |
| c.239G>A[39] |
| c.279+1_279+3delGTG[46] |
| c.280-2A>G[46] |
| c.625_62715delinsACAGTAAT[46] |
| c.554+122C>T[9] |
| c.555-3T>C[27] |
| c.625+5 G>T[4] |
| Amino acid position 209 (e.g., P209T) and c.625+5 G>T[4] |
| c.628-30G>A[41] |
| c.628-31C>T[41] |
| c.698+1G>T[46] |
| c.698+20C>T[41] |
| c.782-1G>A[46] |
| c.782-34G>A[41] |
| Δ795-797[14] |
| c.782 -1G>A[4] |
| c.852A>C[27] |
| c.941-1G>A[46] |
| c.1014C>T[9] |
| c.1029+35G>A[9] |
| c.1221-8C.G[41] |
| 1226delA[16] |
| c.1429+1G>A[46] |
| c.1429+2T>G[13] |
| c.1429+49G>A[41] |
| c.1430-42A>G[41] |
| c.1493T>C[12] |
| c.1587_1589delCTT[46] |
| c.1630+2T>G[27] |
| c.1631-10T>A[41] |
| c.1637-37T>C[41] |
| 1660 G>A[14] |
| 1798 C>T[14] |

(continued)

| |
|---|
| 1799 G>A[14] |
| c.1819-39_41delAA[9] |
| c.1819+1G>A[31] |
| c.1820-27G>A[41] |
| c.1918+8C>T[27] |
| c.1933-1G>AK46 |
| c.2097+2T>C[32] |
| c.2097+60T>G[41] |
| c.2097+89T>C[41] |
| c.2097+97T>G[41] |
| c.2210-114T>C[9] |
| 2210delA[16] |
| c.2210-45_50dupATAAAA[9] |
| c.2285+29C.T[41] |
| c.2285+32A>G[41] |
| c.2286-4_2286-3delinsAA[46] |
| c.2418+5G>A[46] |
| c.2707+3G>C[27] |
| c.2707+9T>G[41] |
| c.2707+43A>G[41] |
| c.2709-59T>C[41] |
| c.2931+9A>G[41] |
| c.2931+59T>A[41] |
| c.2932-3C>A[46] |
| c.2932+59T>A[9] |
| c.2937A>C[27] |
| c.3016-9C>A[31] |
| c.3033-3034del[19] |
| 3122delTCCTA/ |
| insACATCGATGTTGATGTTAGG[45] |
| 3318 G>A[14] |
| c.3400+2T>A[46] |
| c.3401-175C>T[9] |
| c.3401-167C>T[9] |
| c.3401-108C>T[9] |
| c.3531+8G>T[9,15] |
| c.3532-15C>T[9] |
| Δ Phe ex 15[4] |
| Ex1_Ex13del[6] |
| Ex2_Ex6del[33] |

(continued)

| |
|---|
| Ex12_Ex14del[27] |
| Skipped Exon 24[45] |
| del5'UTR-ex18[11] |
| c.*11C>T[41] |
| c.*1101 + 366G > A[7] |
| g.92918del565[31] |
| GC preceding exon 16 (e.g., resulting in a 4 bp deletion)[42] |
| Frameshift from the 5' end of exon 16[42] |
| 5' 1.4 kb deletion[46] |

**Table 3. Selected ATP8B1 Mutations Associated with PFIC-1**

| |
|---|
| Amino acid position 23 (e.g., P23L)[5] |
| Amino acid position 78 (e.g., H78Q)[19] |
| Amino acid position 93 (e.g., A93A)[6] |
| Amino acid position 96 (e.g., A96G)[27] |
| Amino acid position 127 (e.g., L127P)[6] |
| Amino acid position 197 (e.g., G197Lfs*10)[22] |
| Amino acid position 205 (e.g., N205fs)[6] |
| Amino acid position 209 (e.g., P209T)[4] |
| Amino acid position 233 (e.g., G233R)[38] |
| Amino acid position 243 (e.g., L243fs*28)[33] |
| Amino acid position 288 (e.g., L288S)[6] |
| Amino acid position 296 (e.g., R296C)[11] |
| Amino acid position 308 (e.g., G308V[1,6]) |
| Amino acid position 320 (e.g., M320Vfs*13)[11] |
| Amino acid position 403 (e.g., S403Y)[6] |
| Amino acid position 407 (e.g., S407N)[40] |
| Amino acid position 412 (e.g., R412P)[6] |
| Amino acid position 415 (e.g., Q415R)[27] |
| Amino acid position 429 (e.g., E429A)[6] |
| Amino acid position 446 (e.g., G446R)[4] |
| Amino acid position 456 (e.g., T456M)[3,6] |
| Amino acid position 457 (e.g., G457G[6], G457fs*6[33]) |
| Amino acid position 500 (e.g., Y500H)[6] |
| Amino acid position 525 (e.g., R525X)[4] |
| Δ Amino acid position 529[6] |
| Amino acid position 535 (e.g., H535L)[6] |
| Amino acid position 554 (e.g., D554N)[1,6] |
| Amino acid position 577 (e.g., I577V)[19] |
| Amino acid position 585 (e.g., E585X)[21] |

(continued)

| |
|---|
| Amino acid position 600 (e.g., R600W)[4] |
| Amino acid position 602 (e.g., R602X)[3,6] |
| Amino acid position 661 (e.g., I661T)[4,6] |
| Amino acid position 665 (e.g., E665X)[4,6] |
| Δ Amino acid positions 645-699[4] |
| Amino acid position 672 (e.g., K672fs)[6] |
| Amino acid position 674 (e.g., M674T)[19] |
| Amino acid positions 78 and 674 (e.g., H78Q/M674T)[19] |
| Amino acid position 688 (e.g., D688G)[6] |
| Amino acid position 694 (e.g., I694N)[17] |
| Amino acid position 695 (e.g., E695K)[27] |
| Amino acid position 709 (e.g., K709fs)[6] |
| Amino acid position 717 (e.g., T717N)[4] |
| Amino acid position 733 (e.g., G733R)[6] |
| Amino acid position 749 (e.g., L749P)[21] |
| Amino acid position 757 (e.g., Y757X)[4] |
| Amino acid position 792 (e.g., P792fs)[6] |
| Amino acid position 809 (e.g., I809L)[21] |
| Amino acid position 853 (e.g., F853S, F853fs)[6] |
| Amino acid position 867 (e.g., R867fs)[6] |
| Amino acid position 892 (e.g., G892R)[6] |
| Amino acid position 930 (e.g., R930X[6], R952Q[15]) |
| Amino acid position 952 (e.g., R952X)[6] |
| Amino acid position 958 (e.g., N958fs)[6] |
| Amino acid position 981 (e.g., E981K)[20] |
| Amino acid position 994 (e.g., S994R)[4] |
| Amino acid position 1014 (e.g., R1014X)[6,11] |
| Amino acid position 1015 (e.g., F1015L)[27] |
| Amino acid position 1023 (e.g., Q1023fs)[6] |
| Amino acid position 1040 (e.g., G1040R)[1,6] |
| Amino acid position 1047 (e.g., L1047fs)[6] |
| Amino acid position 1095 (e.g., W1095X)[11] |
| Amino acid position 1208 (e.g., A1208fs)[6] |
| Amino acid position 1209 (e.g., Y1209Lfs*28)[4] |
| Amino acid position 1211 (e.g., F1211L)[27] |
| Amino acid position 1219 (e.g., D1219H[5], D1219G[27]) |
| Splice site mutation IVS3+1_+3delGTG[6] |
| Splice site mutation IVS3-2A>G[6] |
| IVS6+5T>G[17] |
| Splice site mutation IVS8+1G>T[6] |

(continued)

| |
|---|
| IVS9-G>A[26] |
| Splice site mutation IVS17-1G>A[6] |
| Splice site mutation IVS18+2T>C[6] |
| Splice site mutation IVS21+5G>A[6] |
| g.24774-42062del[4] |
| c.145C>T[12] |
| c.239G>A[39] |
| c.625+5 G>T[4] |
| Amino acid position 209 (e.g., P209T) and c.625+5 G>T[4] |
| c.782 -1G>A[4] |
| c.1493T>C[12] |
| c.1630+2T>G[27] |
| 1660 G>A[14] |
| c.2707+3G>C[27] |
| c.2097+2T>C[32] |
| c.3033-3034del[19] |
| 3318 G>A[14] |
| c.3158+8G>T[15] |
| Δ Phe ex 15[4] |
| Ex1_Ex13del[6] |
| Ex2_Ex6del[33] |
| Ex12_Ex14del[27] |
| del5'UTR-ex18[11] |
| c.*1101 + 366G > A[7] |
| GC preceding exon 16 (e.g., resulting in a 4 bp deletion)[42] |
| Frameshift from the 5' end of exon 16[42] |
| [A] A mutation to 'X' denotes an early stop codon |

*References for Tables 2 and 3*

[1] Folmer et al., Hepatology. 2009, vol. 50(5), p. 1597-1605.
[2] Hsu et al., Hepatol Res. 2009, vol. 39(6), p. 625-631.
[3] Alvarez et al., Hum Mol Genet. 2004, vol. 13(20), p. 2451-2460.
[4] Davit-Spraul et al., Hepatology 2010, vol. 51(5), p. 1645-1655.
[5] Vitale et al., J Gastroenterol. 2018, vol. 53(8), p. 945-958.
[6] Klomp et al., Hepatology 2004, vol. 40(1), p. 27-38.
[7] Zarenezhad et al., Hepatitis Monthly: 2017, vol. 17(2); e43500.
[8] Dixon et al., Scientific Reports 2017, vol. 7, 11823.
[9] Painter et al., Eur J Hum Genet. 2005, vol. 13(4), p. 435-439.
[10] Deng et al., World J Gastroenterol. 2012, vol. 18(44), p. 6504-6509.
[11] Giovannoni et al., PLoS One. 2015, vol. 10(12): e0145021.
[12] Li et al., Hepatology International 2017, vol. 11, No. 1, Supp. Supplement 1, pp. S180. Abstract Number: OP284.
[13] Togawa et al., Journal of Pediatric Gastroenterology and Nutrition 2018, vol. 67, Supp. Supplement 1, pp. S363.

Abstract Number: 615.

[14] Miloh et al., Gastroenterology 2006, vol. 130, No. 4, Suppl. 2, pp. A759-A760. Meeting Info.: Digestive Disease Week Meeting/107th Annual Meeting of the American-Gastroenterological-Association. Los Angeles, CA, USA. May 19.

[15] Dröge et al., Zeitschrift fur Gastroenterologie 2015, vol. 53, No. 12. Abstract Number: A3-27. Meeting Info: 32. Jahrestagung der Deutschen Arbeitsgemeinschaft zum Studium der Leber. Dusseldorf, Germany. 22 Jan 2016-23 Jan 2016

[16] Mizuochi et al., Clin Chim Acta. 2012, vol. 413(15-16), p. 1301-1304.

[17] Liu et al., Hepatology International 2009, vol. 3, No. 1, p. 184-185. Abstract Number: PE405. Meeting Info: 19th Conference of the Asian Pacific Association for the Study of the Liver. Hong Kong, China. 13 Feb 2009-16 Feb 2009

[18] McKay et al., Version 2. F1000Res. 2013; 2: 32. DOI: 10.12688/f1000research.2-32.v2

[19] Hasegawa et al., Orphanet J Rare Dis. 2014, vol. 9:89.

[20] Stone et al., J Biol Chem. 2012, vol. 287(49), p. 41139-51.

[21] Kang et al., J Pathol Transl Med. 2019 May 16. doi: 10.4132/jptm.2019.05.03. [Epub ahead of print]

[22] Sharma et al., BMC Gastroenterol. 2018, vol. 18(1), p. 107.

[23] Uegaki et al., Intern Med. 2008, vol. 47(7), p. 599-602.

[24] Goldschmidt et al., Hepatol Res. 2016, vol. 46(4), p. 306-311.

[25] Liu et al., J Pediatr Gastroenterol Nutr. 2010, vol. 50(2), p. 179-183.

[26] Jung et al., J Pediatr Gastroenterol Nutr. 2007, vol. 44(4), p. 453-458.

[27] Bounford. University of Birmingham. Dissertation Abstracts International, (2016) Vol. 75, No. 1C. Order No.: AAI10588329. ProQuest Dissertations & Theses.

[28] Stolz et al., Aliment Pharmacol Ther. 2019, vol. 49(9), p. 1195-1204.

[29] Ivashkin et al., Hepatology International 2016, vol. 10, No. 1, Supp. SUPPL. 1, pp. S461. Abstract Number: LBO-38. Meeting Info: 25th Annual Conference of the Asian Pacific Association for the Study of the Liver, APASL 2016. Tokyo, Japan. 20 Feb 2016-24 Feb 2016

[30] Blackmore et al., J Clin Exp Hepatol. 2013, vol. 3(2), p. 159-161.

[31] Matte et al., J Pediatr Gastroenterol Nutr. 2010, vol. 51(4), p. 488-493.

[32] Squires et al., J Pediatr Gastroenterol Nutr. 2017, vol. 64(3), p. 425-430.

[33] Hayshi et al., EBioMedicine. 2018, vol. 27, p. 187-199.

[34] Nagasaka et al., J Pediatr Gastroenterol Nutr. 2007, vol. 45(1), p. 96-105.

[35] Wang et al., PLoS One. 2016; vol. 11(4): e0153114.

[36] Narchi et al., Saudi J Gastroenterol. 2017, vol. 23(5), p. 303-305.

[37] Alashkar et al., Blood 2015, vol. 126, No. 23. Meeting Info.: 57th Annual Meeting of the American-Society-of-Hematology. Orlando, FL, USA. December 05 -08, 2015. Amer Soc Hematol.

[38] Ferreira et al., Pediatric Transplantation 2013, vol. 17, Supp. SUPPL. 1, pp. 99. Abstract Number: 239. Meeting Info: IPTA 7th Congress on Pediatric Transplantation. Warsaw, Poland. 13 Jul 2013-16 Jul 2013.

[39] Pauli-Magnus et al., J Hepatol. 2005, vol. 43(2), p. 342-357.

[40] Jericho et al., Journal of Pediatric Gastroenterology and Nutrition 2015, vol. 60(3), p. 368-374.

[41] van der Woerd et al., PLoS One. 2013, vol. 8(11): e80553.

[42] Copeland et al., J Gastroenterol Hepatol. 2013, vol. 28(3), p. 560-564.

[43] Dröge et al., J Hepatol. 2017, vol. 67(6), p. 1253-1264.

[44] Chen et al., Journal of Pediatrics 2002, vol. 140(1), p. 119-124.

[45] Jirsa et al., Hepatol Res. 2004, vol. 30(1), p. 1-3.

[46] van der Woerd et al., Hepatology 2015, vol. 61(4), p. 1382-1391.

In some embodiments, the mutation in ATP8B1 is selected from L127P, G308V, T456M, D554N, F529del, I661T, E665X, R930X, R952X, R1014X, and G1040R.

Canonical Protein Sequence of ABCB11 (SEQ ID NO: 3) - Uniprot ID O95342

MSDSVILRSI KKFGEENDGF ESDKSYNNDK KSRLQDEKKG DGVRVGFFQL FRFSSSTDIW
LMFVGSLCAF LHGIAQPGVL LIFGTMTDVF IDYDVELQEL QIPGKACVNN TIVWTNSSLN
QNMTNGTRCG LLNIESEMIK FASYYAGIAV AVLITGYIQI CFWVIAAARQ IQKMRKFYFR
RIMRMEIGWF DCNSVGELNT RFSDDINKIN DAIADQMALF IQRMTSTICG FLLGFFRGWK
LTLVIISVSP LIGIGAATIG LSVSKFTDYE LKAYAKAGVV ADEVISSMRT VAAFGGEKRE
VERYEKNLVF AQRWGIRKGI VMGFFTGFVW CLIFLCYALA FWYGSTLVLD EGEYTPGTLV
QIFLSVIVGA LNLGNASPCL EAFATGRAAA TSIFETIDRK PIIDCMSEDG YKLDRIKGEI
EFHNVTFHYP SRPEVKILND LNMVIKPGEM TALVGPSGAG KSTALQLIQR FYDPCEGMVT
VDGHDIRSLN IQWLRDQIGI VEQEPVLFST TIAENIRYGR EDATMEDIVQ AAKEANAYNF
IMDLPQQFDT LVGEGGGQMS GGQKQRVAIA RALIRNPKIL LLDMATSALD NESEAMVQEV
LSKIQHGHTI ISVAHRLSTV RAADTIIGFE HGTAVERGTH EELLERKGVY FTLVTLQSQG
NQALNEEDIK DATEDDMLAR TFSRGSYQDS LRASIRQRSK SQLSYLVHEP PLAVVDHKST
YEEDRKDKDI PVQEEVEPAP VRRILKFSAP EWPYMLVGSV GAAVNGTVTP LYAFLFSQIL
GTFSIPDKEE QRSQINGVCL LFVAMGCVSL FTQFLQGYAF AKSGELLTKR LRKFGFRAML
GQDIAWFDDL RNSPGALTTR LATDASQVQG AAGSQIGMIV NSFTNVTVAM IIAFSFSWKL
SLVILCFFPF LALSGATQTR MLTGFASRDK QALEMVGQIT NEALSNIRTV AGIGKERRFI
EALETELEKP FKTAIQKANI YGFCFAFAQC IMFIANSASY RYGGYLISNE GLHFSYVFRV
ISAVVLSATA LGRAFSYTPS YAKAKISAAR FFQLLDRQPP ISVYNTAGEK WDNFQGKIDF
VDCKFTYPSR PDSQVLNGLS VSISPGQTLA FVGSSGCGKS TSIQLLERFY DPDQGKVMID
GHDSKKVNVQ FLRSNIGIVS QEPVLFACSI MDNIKYGDNT KEIPMERVIA AAKQAQLHDF
VMSLPEKYET NVGSQGSQLS RGEKQRIAIA RAIVRDPKIL LLDEATSALD TESEKTVQVA
LDKAREGRTC IVIAHRLSTI QNADIIAVMA QGVVIEKGTH EELMAQKGAY YKLVTTGSPI S

Canonical DNA Sequence of ABCB11 (SEQ ID NO: 4)

ATG TCT GAC TCA GTA ATT CTT CGA AGT ATA AAG AAA TTT GGA GAG GAG AAT
GAT GGT TTT GAG TCA GAT AAA TCA TAT AAT AAT GAT AAG AAA TCA AGG TTA
CAA GAT GAG AAG AAA GGT GAT GGC GTT AGA GTT GGC TTC TTT CAA TTG TTT
CGG TTT TCT TCA TCA ACT GAC ATT TGG CTG ATG TTT GTG GGA AGT TTG TGT
GCA TTT CTC CAT GGA ATA GCC CAG CCA GGC GTG CTA CTC ATT TTT GGC ACA
ATG ACA GAT GTT TTT ATT GAC TAC GAC GTT GAG TTA CAA GAA CTC CAG ATT
CCA GGA AAA GCA TGT GTG AAT AAC ACC ATT GTA TGG ACT AAC AGT TCC CTC
AAC CAG AAC ATG ACA AAT GGA ACA CGT TGT GGG TTG CTG AAC ATC GAG AGC
GAA ATG ATC AAA TTT GCC AGT TAC TAT GCT GGA ATT GCT GTC GCA GTA CTT
ATC ACA GGA TAT ATT CAA ATA TGC TTT TGG GTC ATT GCC GCA GCT CGT CAG
ATA CAG AAA ATG AGA AAA TTT TAC TTT AGG AGA ATA ATG AGA ATG GAA ATA
GGG TGG TTT GAC TGC AAT TCA GTG GGG GAG CTG AAT ACA AGA TTC TCT GAT
GAT ATT AAT AAA ATC AAT GAT GCC ATA GCT GAC CAA ATG GCC CTT TTC ATT
CAG CGC ATG ACC TCG ACC ATC TGT GGT TTC CTG TTG GGA TTT TTC AGG GGT
TGG AAA CTG ACC TTG GTT ATT ATT TCT GTC AGC CCT CTC ATT GGG ATT GGA
GCA GCC ACC ATT GGT CTG AGT GTG TCC AAG TTT ACG GAC TAT GAG CTG AAG
GCC TAT GCC AAA GCA GGG GTG GTG GCT GAT GAA GTC ATT TCA TCA ATG AGA
ACA GTG GCT GCT TTT GGT GGT GAG AAA AGA GAG GTT GAA AGG TAT GAG AAA
AAT CTT GTG TTC GCC CAG CGT TGG GGA ATT AGA AAA GGA ATA GTG ATG GGA
TTC TTT ACT GGA TTC GTG TGG TGT CTC ATC TTT TTG TGT TAT GCA CTG GCC
TTC TGG TAC GGC TCC ACA CTT GTC CTG GAT GAA GGA GAA TAT ACA CCA GGA
ACC CTT GTC CAG ATT TTC CTC AGT GTC ATA GTA GGA GCT TTA AAT CTT GGC
AAT GCC TCT CCT TGT TTG GAA GCC TTT GCA ACT GGA CGT GCA GCA GCC ACC
AGC ATT TTT GAG ACA ATA GAC AGG AAA CCC ATC ATT GAC TGC ATG TCA GAA
GAT GGT TAC AAG TTG GAT CGA ATC AAG GGT GAA ATT GAA TTC CAT AAT GTG
ACC TTC CAT TAT CCT TCC AGA CCA GAG GTG AAG ATT CTA AAT GAC CTC AAC
ATG GTC ATT AAA CCA GGG GAA ATG ACA GCT CTG GTA GGA CCC AGT GGA GCT
GGA AAA AGT ACA GCA CTG CAA CTC ATT CAG CGA TTC TAT GAC CCC TGT GAA
GGA ATG GTG ACC GTG GAT GGC CAT GAC ATT CGC TCT CTT AAC ATT CAG TGG
CTT AGA GAT CAG ATT GGG ATA GTG GAG CAA GAG CCA GTT CTG TTC TCT ACC
ACC ATT GCA GAA AAT ATT CGC TAT GGC AGA GAA GAT GCA ACA ATG GAA GAC
ATA GTC CAA GCT GCC AAG GAG GCC AAT GCC TAC AAC TTC ATC ATG GAC CTG
CCA CAG CAA TTT GAC ACC CTT GTT GGA GAA GGA GGA GGC CAG ATG AGT GGT
GGC CAG AAA CAA AGG GTA GCT ATC GCC AGA GCC CTC ATC CGA AAT CCC AAG
ATT CTG CTT TTG GAC ATG GCC ACC TCA GCT CTG GAC AAT GAG AGT GAA GCC
ATG GTG CAA GAA GTG CTG AGT AAG ATT CAG CAT GGG CAC ACA ATC ATT TCA
GTT GCT CAT CGC TTG TCT ACG GTC AGA GCT GCA GAT ACC ATC ATT GGT TTT
GAA CAT GGC ACT GCA GTG GAA AGA GGG ACC CAT GAA GAA TTA CTG GAA AGG
AAA GGT GTT TAC TTC ACT CTA GTG ACT TTG CAA AGC CAG GGA AAT CAA GCT
CTT AAT GAA GAG GAC ATA AAG GAT GCA ACT GAA GAT GAC ATG CTT GCG AGG
ACC TTT AGC AGA GGG AGC TAC CAG GAT AGT TTA AGG GCT TCC ATC CGG CAA
CGC TCC AAG TCT CAG CTT TCT TAC CTG GTG CAC GAA CCT CCA TTA GCT GTT
GTA GAT CAT AAG TCT ACC TAT GAA GAA GAT AGA AAG GAC AAG GAC ATT CCT
GTG CAG GAA GAA GTT GAA CCT GCC CCA GTT AGG AGG ATT CTG AAA TTC AGT
GCT CCA GAA TGG CCC TAC ATG CTG GTA GGG TCT GTG GGT GCA GCT GTG AAC
GGG ACA GTC ACA CCC TTG TAT GCC TTT TTA TTC AGC CAG ATT CTT GGG ACT
TTT TCA ATT CCT GAT AAA GAG GAA CAA AGG TCA CAG ATC AAT GGT GTG TGC
CTA CTT TTT GTA GCA ATG GGC TGT GTA TCT CTT TTC ACC CAA TTT CTA CAG
GGA TAT GCC TTT GCT AAA TCT GGG GAG CTC CTA ACA AAA AGG CTA CGT AAA
TTT GGT TTC AGG GCA ATG CTG GGG CAA GAT ATT GCC TGG TTT GAT GAC CTC

AGA AAT AGC CCT GGA GCA TTG ACA ACA AGA CTT GCT ACA GAT GCT TCC CAA GTT CAA GGG GCT GCC GGC TCT CAG ATC GGG ATG ATA GTC AAT TCC TTC ACT AAC GTC ACT GTG GCC ATG ATC ATT GCC TTC TCC TTT AGC TGG AAG CTG AGC CTG GTC ATC TTG TGC TTC TTC CCC TTC TTG GCT TTA TCA GGA GCC ACA CAG ACC AGG ATG TTG ACA GGA TTT GCC TCT CGA GAT AAG CAG GCC CTG GAG ATG GTG GGA CAG ATT ACA AAT GAA GCC CTC AGT AAC ATC CGC ACT GTT GCT GGA ATT GGA AAG GAG AGG CGG TTC ATT GAA GCA CTT GAG ACT GAG CTG GAG AAG CCC TTC AAG ACA GCC ATT CAG AAA GCC AAT ATT TAC GGA TTC TGC TTT GCC TTT GCC CAG TGC ATC ATG TTT ATT GCG AAT TCT GCT TCC TAC AGA TAT GGA GGT TAC TTA ATC TCC AAT GAG GGG CTC CAT TTC AGC TAT GTG TTC AGG GTG ATC TCT GCA GTT GTA CTG AGT GCA ACA GCT CTT GGA AGA GCC TTC TCT TAC ACC CCA AGT TAT GCA AAA GCT AAA ATA TCA GCT GCA CGC TTT TTT CAA CTG CTG GAC CGA CAA CCC CCA ATC AGT GTA TAC AAT ACT GCA GGT GAA AAA TGG GAC AAC TTC CAG GGG AAG ATT GAT TTT GTT GAT TGT AAA TTT ACA TAT CCT TCT CGA CCT GAC TCG CAA GTT CTG AAT GGT CTC TCA GTG TCG ATT AGT CCA GGG CAG ACA CTG GCG TTT GTT GGG AGC AGT GGA TGT GGC AAA AGC ACT AGC ATT CAG CTG TTG GAA CGT TTC TAT GAT CCT GAT CAA GGG AAG GTG ATG ATA GAT GGT CAT GAC AGC AAA AAA GTA AAT GTC CAG TTC CTC CGC TCA AAC ATT GGA ATT GTT TCC CAG GAA CCA GTG TTG TTT GCC TGT AGC ATA ATG GAC AAT ATC AAG TAT GGA GAC AAC ACC AAA GAA ATT CCC ATG GAA AGA GTC ATA GCA GCT GCA AAA CAG GCT CAG CTG CAT GAT TTT GTC ATG TCA CTC CCA GAG AAA TAT GAA ACT AAC GTT GGG TCC CAG GGG TCT CAA CTC TCT AGA GGG GAG AAA CAA CGC ATT GCT ATT GCT CGG GCC ATT GTA CGA GAT CCT AAA ATC TTG CTA CTA GAT GAA GCC ACT TCT GCC TTA GAC ACA GAA AGT GAA AAG ACG GTG CAG GTT GCT CTA GAC AAA GCC AGA GAG GGT CGG ACC TGC ATT GTC ATT GCC CAT CGC TTG TCC ACC ATC CAG AAC GCG GAT ATC ATT GCT GTC ATG GCA CAG GGG GTG GTG ATT GAA AAG GGG ACC CAT GAA GAA CTG ATG GCC CAA AAA GGA GCC TAC TAC AAA CTA GTC ACC ACT GGA TCC CCC ATC AGT TGA

**Table 4. Exemplary ABCB11 Mutations**

| |
|---|
| Amino acid position 1 (e.g., M1V)[9] |
| Amino acid position 4 (e.g., S4X)[A,64] |
| Amino acid position 8 (e.g., R8X)[88] |
| Amino acid position 19 (e.g., G19R)[56] |
| Amino acid position 24 (e.g., K24X)[35] |
| Amino acid position 25 (e.g., S25X)[5,14] |
| Amino acid position 26 (e.g., Y26Ifs*7)[38] |
| Amino acid position 36 (e.g., D36D)[27] |
| Amino acid position 38 (e.g., K38Rfs*24)[73] |
| Amino acid position 43 (e.g., V43I)[57] |
| Amino acid position 49 (e.g., Q49X)[73] |
| Amino acid position 50 (e.g., L50S, L50W)[57] |
| Amino acid position 52 (e.g., R52W[26], R52R[28]) |
| Amino acid position 56 (e.g., S56L)[58] |
| Amino acid position 58 (e.g., D58N)[62] |
| Amino acid position 62 (e.g., M62K)[9] |

(continued)

| |
|---|
| Amino acid position 66 (e.g., S66N)[17] |
| Amino acid position 68 (e.g., C68Y)[41] |
| Amino acid position 50 (e.g., L50S)[5,7] |
| Amino acid position 71 (e.g., L71H)[73] |
| Amino acid position 74 (e.g., I74R)[71] |
| Amino acid position 77 (e.g., P77A)[73] |
| Amino acid position 87 (e.g., T87R)[67] |
| Amino acid position 90 (e.g., F90F)[7,27] |
| Amino acid position 93 (e.g., Y93S[13], Y93X[88]) |
| Amino acid position 96 (e.g., E96X)[88] |
| Amino acid position 97 (e.g., L97X)[39] |
| Amino acid position 101 (e.g., Q101Dfs*8)[9] |
| Amino acid position 107 (e.g., C107R)[36] |
| Amino acid position 112 (e.g., I112T)[9] |
| Amino acid position 114 (e.g., W114R)[29] |
| Amino acid position 123 (e.g. M123T)[67] |
| Amino acid position 127 (e.g., T127Hfs*6)[5] |
| Amino acid position 129 (e.g., C129Y)[25] |
| Amino acid position 130 (e.g., G130G)[77] |
| Amino acid position 134 (e.g., I134I)[28] |
| Amino acid position 135 (e.g., E135K[7,13], E135L[17]) |
| Amino acid position 137 (e.g., E137K)[7] |
| Amino acid position 157 (e.g., Y157C)[5] |
| Amino acid position 161 (e.g., C161X)[39] |
| Amino acid position 164 (e.g., V164Gfs*7[30], V164I[85]) |
| Amino acid position 167 (e.g., A167S[4], A167V[7], A167T[9,17]) |
| Amino acid position 181 (e.g., R181I)[35] |
| Amino acid position 182 (e.g., I182K)[9] |
| Amino acid position 183 (e.g., M183V[8], M183T[9]) |
| Amino acid position 185 (e.g., M185I)[73] |
| Amino acid position 186 (e.g., E186G)[2,7,22] |
| Amino acid position 188 (e.g., G188W)[73] |
| Amino acid position 194 (e.g., S194P)[7] |
| Amino acid position 198 (e.g., L198P)[7] |
| Amino acid position 199 (e.g., N199Ifs*15X)[88] |
| Amino acid position 206 (e.g., I206V)[28] |
| Amino acid position 212 (e.g., A212T)[73] |
| Amino acid position 217 (e.g., M217R)[88] |
| Amino acid position 225 (e.g., T22SP)[57] |
| Amino acid position 226 (e.g., S226L)[9] |

(continued)

| |
|---|
| Amino acid position 232 (e.g., L232Cfs*9)[9] |
| Amino acid position 233 (e.g., L233S)[86] |
| Amino acid position 238 (e.g., G238V)[2,7] |
| Amino acid position 242 (e.g., I242I)[5,7] |
| Amino acid position 245 (e.g., I24STfs*26)[57] |
| Amino acid position 256 (e.g., A256G)[9] |
| Amino acid position 260 (e.g., G260D)[7] |
| Amino acid position 269 (e.g., Y269Y)[27] |
| Amino acid position 277 (e.g., A277E)[77] |
| Amino acid position 283 (e.g., E283D)[73] |
| Amino acid positions 212 and 283 (e.g., A212T+E283D)[73] |
| Amino acid position 284 (e.g., V284L[7,39], V284A[7], V284D[23]) |
| Amino acid position 297 (e.g., E297G[1,2,5,7], E297K[7]) |
| Amino acid position 299 (e.g., R299K)[28] |
| Amino acid position 303 (e.g., R303K[8], R303M[63] R303fsX321[83]) |
| Amino acid position 304 (e.g., Y304X)[26] |
| Amino acid position 312 (e.g., Q312H)[7] |
| Amino acid position 313 (e.g., R313S)[5,7] |
| Amino acid position 314 (e.g., W314X)[57] |
| Amino acid position 318 (e.g., K318Rfs*26)[29] |
| Amino acid position 319 (e.g., G319G)[7] |
| Amino acid position 327 (e.g., G327E)[5,7] |
| Amino acid position 330 (e.g., W330X)[24] |
| Amino acid position 336 (e.g., C336S)[2,7] |
| Amino acid position 337 (e.g., Y337H)[21,27] |
| Amino acid position 342 (e.g., W342G)[50] |
| Amino acid position 354 (e.g., R354X)[9] |
| Amino acid position 361 (e.g., Q361X[57], Q361R[74]) |
| Amino acid position 366 (e.g., V366V[28], V366D[57]) |
| Amino acid position 368 (e.g., V368Rfs*27)[5] |
| Amino acid position 374 (e.g., G374S)[3] |
| Amino acid position 380 (e.g., L380Wfs*18)[5] |
| Amino acid position 382 (e.g., A382G)[88] |
| Δ Amino acid positions 382-388[5] |
| Δ Amino acid positions 383-389[57] |
| Amino acid position 387 (e.g., R387H)[9] |
| Amino acid position 390 (e.g., A390P)[5,7] |
| Amino acid position 395 (e.g., E395E)[28] |
| Amino acid position 404 (e.g., D404G)[9] |
| Amino acid position 410 (e.g., G410D)[5,7] |

(continued)

| |
|---|
| Amino acid position 413 (e.g., L413W)[5,7] |
| Amino acid position 415 (e.g., R415X)[42] |
| Amino acid position 416 (e.g., I416I)[27] |
| Amino acid position 420 (e.g., I420T)[9] |
| Amino acid position 423 (e.g., H423R)[13] |
| Amino acid position 432 (e.g., R432T)[1,2,7] |
| Amino acid position 436 (e.g., K436N)[40] |
| Amino acid position 440 (e.g., D440E)[88] |
| Amino acid position 444 (e.g., V444A)[2] |
| Amino acid position 454 (e.g., V454X)[49] |
| Amino acid position 455 (e.g., G455E)[9] |
| Amino acid position 457 (e.g., S457Vfs*23)[88] |
| Amino acid position 461 (e.g., K461E)[2,7] |
| Amino acid position 462 (e.g., S462R)[88] |
| Amino acid position 463 (e.g., T463I)[5,7] |
| Amino acid position 466 (e.g., Q466K)[5,7] |
| Amino acid position 470 (e.g., R470Q[5,7], R470X[9]) |
| Amino acid position 471 (e.g., Y472X)[5] |
| Amino acid position 472 (e.g., Y472C[5,27], Y472X[14]) |
| Amino acid position 473 (e.g., D473Q[35], D473V[88]) |
| Amino acid position 475 (e.g., C475X)[29] |
| Amino acid position 481 (e.g., V481E)[5,7] |
| Amino acid position 482 (e.g., D482G)[2,5,7] |
| Amino acid position 484 (e.g., H484Rfs*5)[9] |
| Amino acid position 487 (e.g., R487H[2], R487P[5]) |
| Amino acid position 490 (e.g., N490D)[5,7] |
| Amino acid position 493 (e.g., W493X)[8] |
| Amino acid position 496 (e.g., D496V)[88] |
| Amino acid position 498 (e.g., I498T)[2,7] |
| Amino acid position 499 (e.g., G499E)[73] |
| Amino acid position 501 (e.g., V501G)[68] |
| Amino acid position 504 (e.g., E504K)[79] |
| Amino acid position 510 (e.g., T510T)[7] |
| Amino acid position 512 (e.g., I512T)[5,7] |
| Amino acid position 515 (e.g., N515T[5,7], N515D[64]) |
| Amino acid position 516 (e.g., I516M)[17] |
| Amino acid position 517 (e.g., R517H)[5,7] |
| Amino acid position 520 (e.g., R520X)[5] |
| Amino acid position 523 (e.g., A523G)[13] |
| Amino acid position 528 (e.g., I528Sfs*21[5], I528X[9], I528T[73]) |

(continued)

| |
|---|
| Amino acid position 535 (e.g., A535A[7], A535X[89]) |
| Amino acid position 540 (e.g., F540L)[46] |
| Amino acid position 541 (e.g., I541L[5,7], I541T[5,17]) |
| Amino acid position 546 (e.g., Q546K[39], Q546H[73]) |
| Amino acid position 548 (e.g., F548Y)[5,7] |
| Amino acid position 549 (e.g., D549V)[9] |
| Amino acid position 554 (e.g., E554K)[21] |
| Amino acid position 556 (e.g., G556R)[67] |
| Amino acid position 558 (e.g., Q558H)[23] |
| Amino acid position 559 (e.g., M559T)[57] |
| Amino acid position 562 (e.g., G562D[5,7], G562S[73]) |
| Amino acid position 570 (e.g., A570T[2,5,7], A570V[26]) |
| Amino acid position 575 (e.g., R575X[2,5], R575Q[21]) |
| Amino acid position 580 (e.g., L580P)[57] |
| Amino acid position 586 (e.g., T586I)[7] |
| Amino acid position 587 (e.g., S587X)[73] |
| Amino acid position 588 (e.g., A588V[5,7], A588P[73]) |
| Amino acid position 591 (e.g., N591S)[2,7] |
| Amino acid position 593 (e.g., S593R)[2,7] |
| Amino acid position 597 (e.g., V597V[9], V597L[13]) |
| Amino acid position 603 (e.g., K603K)[55] |
| Amino acid position 609 (e.g., H609Hfs*46)[26] |
| Amino acid position 610 (e.g., I610Gfs*45[9], I610T[57])[9] |
| Amino acid position 615 (e.g., H615R)[26] |
| Amino acid position 616 (e.g., R616G[28], R616H[73]) |
| Amino acid position 619 (e.g., T619A)[28] |
| Amino acid position 623 (e.g., A623A)[28] |
| Amino acid position 625 (e.g., T625Nfs*5)[26] |
| Amino acid position 627 (e.g., I627T)[7] |
| Amino acid position 628 (e.g., G628Wfs*3)[70] |
| Amino acid position 636 (e.g., E636G)[2] |
| Amino acid position 648 (e.g., G648Vfs*6[5], G648V[50]) |
| Amino acid position 655 (e.g., T655I)[7] |
| Amino acid position 669 (e.g., I669V)[26] |
| Amino acid position 676 (e.g., D676Y)[11] |
| Amino acid position 677 (e.g., M677V)[7,13] |
| Amino acid position 679 (e.g., A679V)[58] |
| Amino acid position 685 (e.g., G685W)[60] |
| Amino acid position 696 (e.g., R696W[27], R696Q[58]) |
| Amino acid position 698 (e.g., R698H[7,9], R698K[61], R698C[88]) |

(continued)

| |
|---|
| Amino acid position 699 (e.g., S699P)[9] |
| Amino acid position 701 (e.g., S701P)[58] |
| Amino acid position 702 (e.g., Q702X)[89] |
| Amino acid position 709 (e.g., E709K)[7] |
| Amino acid position 710 (e.g., P710P)[7] |
| Amino acid position 712 (e.g., L712L)[28] |
| Amino acid position 721 (e.g., Y721C)[88] |
| Amino acid position 729 (e.g., D724N)[39] |
| Amino acid position 731 (e.g., P731S)[23] |
| Amino acid position 740 (e.g., P740Qfs*6)[73] |
| Amino acid position 758 (e.g., G758R)[5] |
| Amino acid position 766 (e.g., G766R)[5,24] |
| Amino acid position 772 (e.g., Y772X)[5] |
| Amino acid position 804 (e.g., A804A)[7] |
| Amino acid position 806 (e.g., G806D[44], G806G[55]) |
| Amino acid position 809 (e.g., S809F)[81] |
| Amino acid position 817 (e.g., G817G)[88] |
| Amino acid position 818 (e.g., Y818F)[7] |
| Amino acid position 824 (e.g., G824E)[42] |
| Amino acid position 825 (e.g., G825G)[73] |
| Amino acid position 830 (e.g., R830Gfs*28)[73] |
| Amino acid position 832 (e.g., R832C[7,26], R832H[41]) |
| Amino acid position 842 (e.g., D842G)[2] |
| Amino acid position 848 (e.g., D848N)[73] |
| Amino acid position 855 (e.g., G855R)[11] |
| Amino acid position 859 (e.g., T859R)[5,7] |
| Amino acid position 865 (e.g., A865V)[27] |
| Amino acid position 866 (e.g., S866A)[57] |
| Amino acid position 868 (e.g., V868D)[73] |
| Amino acid position 869 (e.g., Q869P)[73] |
| Amino acid position 875 (e.g., Q875X)[73] |
| Amino acid position 877 (e.g., G877R)[56] |
| Amino acid position 879 (e.g., I879R)[88] |
| Amino acid position 893 (e.g., A893V)[57] |
| Amino acid position 901 (e.g., S901R[17], S901I[73]) |
| Amino acid position 903 (e.g., V903G)[57] |
| Δ Amino acid position 919[12] |
| Amino acid position 923 (e.g., T923P)[2,7] |
| Amino acid position 926 (e.g., A926P)[2,7] |
| Amino acid position 928 (e.g., R928X[15], R928Q[40]) |

(continued)

| |
|---|
| Amino acid position 930 (e.g., K930X[5], K930Efs*79[5,10], K930Efs*49[26]) |
| Amino acid position 931 (e.g., Q931P)[27] |
| Amino acid position 945 (e.g., S945N)[57] |
| Amino acid position 948 (e.g., R948C)[5,7,26] |
| Amino acid position 958 (e.g., R958Q)[28] |
| Amino acid position 969 (e.g., K969K)[88] |
| Δ Amino acid positions 969-972[5] |
| Amino acid position 973 (e.g., T973I)[57] |
| Amino acid position 976 (e.g., Q976R[58], Q976X[88]) |
| Amino acid position 979 (e.g., N979D)[5,7] |
| Amino acid position 981 (e.g., Y981Y)[28] |
| Amino acid position 982 (e.g., G982R)[2,5,7] |
| Amino acid positions 444 and 982 (e.g., V444A+G982R)[38] |
| Amino acid position 995 (e.g., A995A)[28] |
| Amino acid position 1001 (e.g., R1001R)[9] |
| Amino acid position 1003 (e.g., G1003R)[24] |
| Amino acid position 1004 (e.g., G1004D)[2,7] |
| Amino acid position 1027 (e.g., S1027R)[26] |
| Amino acid position 1028 (e.g., A1028A[7,10,88], A1028E[88]) |
| Amino acid position 1029 (e.g., T1029K)[5] |
| Amino acid position 1032 (e.g., G1032R)[12] |
| Amino acid position 1041 (e.g., Y1041X)[9] |
| Amino acid position 1044 (e.g., A1044P)[88] |
| Amino acid position 1050 (e.g., R1050C)[2,7,57] |
| Amino acid position 1053 (e.g., Q1053X)[57] |
| Amino acid position 1055 (e.g., L10SSP)[36] |
| Amino acid position 1057 (e.g., R1057X[2], R1057Q[58]) |
| Amino acid position 1058 (e.g., Q1058Hfs*38[9], Q1058fs*38[17], Q1058X[73]) |
| Amino acid position 1061 (e.g., I1061Vfs*34)[9] |
| Amino acid position 1083 (e.g., C1083Y)[47] |
| Amino acid position 1086 (e.g., T1086T)[28] |
| Amino acid position 1090 (e.g., R1090X)[2,5] |
| Amino acid position 1099 (e.g., L1099Lfs*38)[26] |
| Amino acid position 1100 (e.g., S1100Qfs*38)[13] |
| Amino acid position 1110 (e.g., A1110E)[5,7] |
| Amino acid position 1112 (e.g., V1112F)[70] |
| Amino acid position 1116 (e.g., G1116R[7], G1116F[9,17], G1116E[36]) |
| Amino acid position 1120 (e.g., S1120N)[88] |
| Amino acid position 1128 (e.g., R1128H[2,7], R1128C[5,7,13]) |
| Amino acid position 1131 (e.g., D1131V)[27] |

(continued)

| |
|---|
| Amino acid position 1144 (e.g., S1144R)' |
| Amino acid position 1147 (e.g., V1147X)[5] |
| Amino acid position 1153 (e.g., R1153C[2,5,7], R1153H[5]) |
| Amino acid position 1154 (e.g., S1154P)[5,7] |
| Amino acid position 1162 (e.g., E1162X)[39] |
| Δ Amino acid position 1165[88] |
| Amino acid position 1164 (e.g., V1164Gfs*7) |
| Amino acid position 1173 (e.g., N1173D)[57] |
| Amino acid position 1175 (e.g., K117ST)[58] |
| Amino acid position 1186 (e.g., E1186K)[7] |
| Amino acid position 1192 (e.g., A1192Efs*50)[9] |
| Amino acid position 1196 (e.g., Q1196X)[88] |
| Amino acid position 1197 (e.g., L1197G)[7] |
| Amino acid position 1198 (e.g., H1198R)[27] |
| Amino acid position 1204 (e.g., L1204P)[88] |
| Amino acid position 1208 (e.g. Y1208C)[73] |
| Amino acid position 1210 (e.g., T1210P[5,7], T1210F[57]) |
| Amino acid position 1211 (e.g., N1211D)[7] |
| Amino acid position 1212 (e.g., V1212F)[36] |
| Amino acid position 1215 (e.g., Q1215X)[5] |
| Amino acid position 1221 (e.g., R1221K)[53] |
| Amino acid position 1223 (e.g., E1223D)[7] |
| Amino acid position 1226 (e.g., R1226P)[73] |
| Amino acid position 1228 (e.g., A1228V)[7] |
| Amino acid position 1231 (e.g., R1231W[5,7], R1231Q[5,7]) |
| Amino acid position 1232 (e.g., A1232D)[17] |
| Amino acid position 1235 (e.g., R1235X)[5,12] |
| Amino acid position 1242 (e.g., L1242I)[5,7] |
| Amino acid position 1243 (e.g., D1243G)[67] |
| Amino acid position 1249 (e.g., L1249X)[73] |
| Amino acid position 1256 (e.g., T1256fs*1296)[83] |
| Amino acid position 1268 (e.g., R1268Q)[2,7] |
| Amino acid position 1276 (e.g., R1276H)[30] |
| Amino acid position 1283 (e.g., A1283A[28], A1283V[88]) |
| Amino acid position 1292 (e.g., G1292V)[73] |
| Amino acid position 1298 (e.g., G1298R)[5] |
| Amino acid position 1302 (e.g., E1302X)[5] |
| Amino acid position 1311 (e.g., Y1311X)[57] |
| Amino acid position 1316 (e.g., T1316Lfs*64)[15] |
| Amino acid position 1321 (e.g., S1321N)[57] |

(continued)

| |
|---|
| Intron 4 ((+3)A>C)[1] |
| IVS4-74A>T[89] |
| Splice site mutation 3' Intron 5 c.3901G>A[5] |
| Splice site mutation 5; Intron 7 c.6111G>A[5] |
| Splice site mutation IVS7+1G>A[14] |
| IVS7+5G>A[40] |
| IVS8+1G>C[76] |
| Splice site mutation 5' Intron 9 c.9081delG[5] |
| Splice site mutation 5' Intron 9 c.9081G>T[5] |
| Splice site mutation 5' Intron 9 c.9081G>A[5] |
| Splice site mutation IVS9+1G>T[14] |
| Splice site mutation 3' Intron 13 c.143513_1435-8del[3] |
| Splice site mutation IVS13del-13^-8[14] |
| Splice site mutation 3' Intron 16 c.20128T>G[5] |
| Splice site mutation IVS16-8T>G[14] |
| Splice site mutation 5' Intron 18 c.21781G>T[5] |
| Splice site mutation 5' Intron 18 c.21781G>A[5] |
| Splice site mutation 5' Intron 18 c.21781G>C[5] |
| Splice site mutation 3' Intron 18 c.21792A>G[5] |
| Splice site mutation IVS18+1G>A[14] |
| Splice site mutation 5' Intron 19 c.2343+1G>T[5] |
| Splice site mutation 5' Intron 19 c.2343+2T>C[5] |
| Splice site mutation IVS19+2T>C[14] |
| Splice site mutation IVS19+1G>A[22] |
| Splice site mutation 3' Intron 21 c.26112A>T[5] |
| IVS22+3A>G[89] |
| IVS 23-8 G-A[36] |
| IVS24+5G>A[51] |
| Splice site mutation 5' Intron 24 c.32131delG[5] |
| IVS35-6C>G[89] |
| Putative splice mutation 1198-1G>C[17] |
| Putative splice mutation 1810-3C>G[17] |
| Putative splice mutation 2178+1G>A[17] |
| Putative splice mutation 2344-1G>T[17] |
| Putative splice mutation c.2611-2A>T[39] |
| Putative splice mutation 3213+1_3213+2delinsA[17] |
| c.-24C>A[44,78] |
| c.76 13 G>T[9] |
| c.77-19T>A[52] |
| c.90_93delGAAA[18] |

(continued)

| |
|---|
| c.124G>A[69] |
| c.150 +3 A>C[16] |
| 174C>T[54] |
| c.24ST>C[87] |
| c.249_250insT[18] |
| 270T>C[54] |
| 402C>T[54] |
| 585G>C[54] |
| c.611+1G>A[70] |
| c.611+4A>G[36] |
| c.612-15_-6del10bp[55] |
| c.625A>C[31] |
| c.627+5G>T[31] |
| c.625A>C/ c.627+5G>T[31] |
| 696G>T[54] |
| c. 784+1G>C[49] |
| 807T>C[54] |
| c.886C>T[31] |
| c.890A>G[59] |
| c.908+1G>A[57] |
| c.908+5G>A[55] |
| c.908delG[59] |
| c.909-15A>G[66] |
| 957A>G[54] |
| c.1084-2A>G[57] |
| 1145 1bp deletion[90] |
| 1281C>T[54,57] |
| c.1309-165C > T[19] |
| c.1434 + 174G > A[19] |
| c.1434 + 70C > T[19] |
| c.1530C>A[57] |
| c.1587-1589delCTT[31] |
| c.1621A>C[33,59] |
| c.1638+32T>C[66] |
| c.1638+80C>T[66] |
| 1671C>T[54] |
| 1791G>T[54] |
| 1939delA[14] |
| c.2075+3A>G[53] |
| c.2081T>A[31] |

(continued)

| |
|---|
| c.2093G>A[65] |
| 2098delA[16] |
| c.2138-8T>G[67] |
| 2142A>G[54] |
| c.2178+1G>T[36,39] |
| c.2179-17C>A[66] |
| c.2344-157T>G[66] |
| c.2344-17T>C[66] |
| c.2417G>A[78] |
| c.2541delG[87] |
| c.2620C>T[32,33] |
| c.2815-8A>G[55] |
| c.3003A>G[37] |
| c.3084A>G[48,54] |
| c.3213 +4 A>G[9,37] |
| c.3213 +5 G>A[9] |
| c.3268C>T[75] |
| 3285A>G[54] |
| c.3382C>T[75] |
| 3435A>G[54] |
| c.3491delT[72] |
| c.3589C>T[57] |
| c.3765(+1 +5)del5[42] |
| c.3766-34A>G[66] |
| c.3767-3768insC[6] |
| c.3770delA[67] |
| c.3826C>T[72] |
| c.3846C>T[57] |
| c.3929delG[67] |
| c.*236A>G[66] |
| 1145delC[8] |
| Ex13_Ex17del[82] |

**Table 5. Selected ABCB11 Mutations Associated with PFIC-2**

| |
|---|
| Amino acid position 1 (e.g., M1V)[9] |
| Amino acid position 4 (e.g., S4X)[64] |
| Amino acid position 19 (e.g., G19R)[56] |
| Amino acid position 25 (e.g., S25X)[14] |
| Amino acid position 26 (e.g., Y26Ifs*7)[38] |
| Amino acid position 50 (e.g., L50S)[7,57] |

(continued)

| |
|---|
| Amino acid position 52 (e.g., R52W)[26] |
| Amino acid position 58 (e.g., D58N)[62] |
| Amino acid position 62 (e.g., M62K)[9] |
| Amino acid position 66 (e.g., S66N)[17] |
| Amino acid position 68 (e.g., C68Y)[41] |
| Amino acid position 93 (e.g., Y93S)[13] |
| Amino acid position 101 (e.g., Q101Dfs*8)[9] |
| Amino acid position 107 (e.g., C107R)[36] |
| Amino acid position 112 (e.g., I112T)[9] |
| Amino acid position 114 (e.g., W114R)[29] |
| Amino acid position 129 (e.g., C129Y)[25] |
| Amino acid position 135 (e.g., E135K[13], E135L[17]) |
| Amino acid position 167 (e.g., A167V[7], A167T[9,17]) |
| Amino acid position 182 (e.g., I182K)[9] |
| Amino acid position 183 (e.g., M183V[8], M183T[9]) |
| Amino acid position 225 (e.g., T225P)[57] |
| Amino acid position 226 (e.g., S226L)[9] |
| Amino acid position 232 (e.g., L232Cfs*9)[9] |
| Amino acid position 233 (e.g., L233S)[86] |
| Amino acid position 238 (e.g., G238V)[2,7] |
| Amino acid position 242 (e.g., T242I)[7] |
| Amino acid position 245 (e.g., I245Tfs*26)[57] |
| Amino acid position 256 (e.g., A256G)[9] |
| Amino acid position 260 (e.g., G260D)[57] |
| Amino acid position 284 (e.g., V284L)[7] |
| Amino acid position 297 (e.g., E297G)[2,7] |
| Amino acid position 303 (e.g., R303K[8], R303M[63], R303fsX321[83]) |
| Amino acid position 304 (e.g., Y304X)[26] |
| Amino acid position 312 (e.g., Q312H)[7] |
| Amino acid position 313 (e.g., R313S)[7] |
| Amino acid position 314 (e.g., W314X)[57] |
| Amino acid position 318 (e.g., K318Rfs*26)[29] |
| Amino acid position 327 (e.g., G327E)[7] |
| Amino acid position 330 (e.g., V330X)[24] |
| Amino acid position 336 (e.g., C336S)[2,7] |
| Amino acid position 337 (e.g., Y337H)[21] |
| Amino acid position 342 (e.g., W342G)[50] |
| Amino acid position 354 (e.g., R354X)[9] |
| Amino acid position 361 (e.g., Q361X)[57] |
| Amino acid position 366 (e.g., V366D)[57] |

(continued)

| |
|---|
| Amino acid position 386 (e.g., G386X)[34] |
| Δ Amino acid positions 383-389[57] |
| Amino acid position 387 (e.g., R387H)[9] |
| Amino acid position 390 (e.g., A390P)[7] |
| Amino acid position 410 (e.g., G410D)[7] |
| Amino acid position 413 (e.g., L413W)[7] |
| Amino acid position 415 (e.g., R415X)[42] |
| Amino acid position 420 (e.g., I420T)[9] |
| Amino acid position 454 (e.g., V454X)[49] |
| Amino acid position 455 (e.g., G455E)[9] |
| Amino acid position 461 (e.g., K461E)[2,7] |
| Amino acid position 463 (e.g., T463I)[7] |
| Amino acid position 466 (e.g., Q466K)[7] |
| Amino acid position 470 (e.g., R470Q[7], R470X[9]) |
| Amino acid position 472 (e.g., Y472X[14], Y472C[27]) |
| Amino acid position 475 (e.g., C475X)[29] |
| Amino acid position 481 (e.g., V481E)[7] |
| Amino acid position 482 (e.g., D482G)[2,7] |
| Amino acid position 484 (e.g., H484Rfs*5)[9] |
| Amino acid position 487 (e.g., R487H[2], R487P[84]) |
| Amino acid position 490 (e.g., N490D)[7] |
| Amino acid position 493 (e.g., W493X)[8] |
| Amino acid position 498 (e.g., I498T)[7] |
| Amino acid position 501 (e.g., V501G)[68] |
| Amino acid position 512 (e.g., I512T)[7] |
| Amino acid position 515 (e.g., N515T[7], N515D[64]) |
| Amino acid position 516 (e.g., I516M)[17] |
| Amino acid position 517 (e.g., R517H)[7] |
| Amino acid position 520 (e.g., R520X)[57] |
| Amino acid position 523 (e.g., A523G)[13] |
| Amino acid position 528 (e.g., I528X)[9] |
| Amino acid position 540 (e.g., F540L)[46] |
| Amino acid position 541 (e.g., I541L[7], I541T[17]) |
| Amino acid position 548 (e.g., F548Y)[7] |
| Amino acid position 549 (e.g., D549V)[9] |
| Amino acid position 554 (e.g., E554K)[21] |
| Amino acid position 559 (e.g., M559T)[57] |
| Amino acid position 562 (e.g., G562D)[7] |
| Amino acid position 570 (e.g., A570T[7], A570V[26]) |
| Amino acid position 575 (e.g., R575X[2], R575Q[21]) |

(continued)

| |
|---|
| Amino acid position 588 (e.g., A588V)[7] |
| Amino acid position 591 (e.g., N591S)[9,17] |
| Amino acid position 593 (e.g., S593R)[2,7] |
| Amino acid position 597 (e.g., V597V[9], V597L[13]) |
| Amino acid positions 591 and 597 (e.g., N591S+V597V)[9] |
| Amino acid position 603 (e.g., K603K)[55] |
| Amino acid position 609 (e.g., H609Hfs*46)[26] |
| Amino acid position 610 (e.g., I610Gfs*45)[9] |
| Amino acid position 615 (e.g., H615R)[26] |
| Amino acid position 625 (e.g., T625Nfs*5)[26] |
| Amino acid position 627 (e.g., I627T)[7] |
| Amino acid position 636 (e.g., E636G)[2] |
| Amino acid position 669 (e.g., I669V)[26] |
| Amino acid position 698 (e.g., R609H)[9] |
| Amino acid positions 112 and 698 (e.g., I112T+R698H)[9] |
| Amino acid position 699 (e.g., S699P)[9] |
| Amino acid position 766 (e.g., G766R)[24] |
| Amino acid position 806 (e.g., G806G)[55] |
| Amino acid position 824 (e.g., G824E)[42] |
| Amino acid position 832 (e.g., R832C[7,26], R832H[41]) |
| Amino acid position 842 (e.g., D842G)[2] |
| Amino acid position 859 (e.g., T859R)[7] |
| Amino acid position 865 (e.g., A865V)[45] |
| Amino acid position 877 (e.g., G877R)[56] |
| Amino acid position 893 (e.g., A893V)[57] |
| Amino acid position 901 (e.g., S901R)[17] |
| Amino acid position 903 (e.g., V903G)[57] |
| Δ Amino acid position 919[12] |
| Amino acid position 928 (e.g., R928X)[15,21] |
| Amino acid position 930 (e.g., K930Efs*79[10], K930Efs*49[26]) |
| Amino acid position 948 (e.g., R948C)[7,26] |
| Amino acid position 979 (e.g., N979D)[7] |
| Amino acid position 982 (e.g., G982R)[2,7] |
| Amino acid positions 444 and 982 (e.g., V444A+G982R)[38] |
| Amino acid position 1001 (e.g., R1001R)[9] |
| Amino acid position 1003 (e.g., G1003R)[24] |
| Amino acid position 1004 (e.g., G1004D)[2,7] |
| Amino acid position 1027 (e.g., S1027R)[26] |
| Amino acid position 1028 (e.g., A1028A)[10] |
| Amino acid position 1032 (e.g., G1032R)[12] |

(continued)

| |
|---|
| Amino acid position 1041 (e.g., Y1041X)[9] |
| Amino acid position 1050 (e.g., R1050C)[57] |
| Amino acid position 1053 (e.g., Q1053X)[57] |
| Amino acid position 1055 (e.g., L1055P)[36] |
| Amino acid position 1057 (e.g., R1057X)[2] |
| Amino acid position 1058 (e.g., Q1058Hfs*38[9], Q1058fs*38[17]) |
| Amino acid position 1061 (e.g., I1061Vfs*34)[9] |
| Amino acid position 1083 (e.g., C1083Y)[47] |
| Amino acid position 1090 (e.g., R1090X)[2] |
| Amino acid position 1099 (e.g., L1099Lfs*38)[26] |
| Amino acid position 1100 (e.g., S1100Qfs*38)[13] |
| Amino acid position 1110 (e.g., A1110E)[7] |
| Amino acid position 1116 (e.g., G1116R[7], G1116F[9,17], G1116E[36]) |
| Amino acid position 1128 (e.g., R1128C)[7,13] |
| Amino acid position 1131 (e.g., D1131V)[27] |
| Amino acid position 1144 (e.g., S1144R)' |
| Amino acid position 1153 (e.g., R1153C[2,7], R1153H[7,26]) |
| Amino acid position 1154 (e.g., S1154P)' |
| Amino acid position 1173 (e.g., N1173D)[57] |
| Amino acid position 1192 (e.g., A1192Efs*50)[9] |
| Amino acid position 1198 (e.g., H1198R)[27] |
| Amino acid position 1210 (e.g., T1210P[7], T1210F[57]) |
| Amino acid position 1211 (e.g., N1211D)[7] |
| Amino acid position 1212 (e.g., V1212F)[36] |
| Amino acid position 1231 (e.g., R1231W[7], R1223Q[7]) |
| Amino acid position 1232 (e.g., A1232D)[17] |
| Amino acid position 1235 (e.g., R1235X)[12] |
| Amino acid position 1242 (e.g., L1242I)[7] |
| Amino acid position 1256 (e.g., T1256fs*1296)[83] |
| Amino acid position 1268 (e.g., R1268Q)[2,7] |
| Amino acid position 1302 (e.g. E1302X)[57] |
| Amino acid position 1311 (e.g., Y1311X)[57] |
| Amino acid position 1316 (e.g., T1316Lfs*64)[15] |
| Intron 4 ((+3)A>C)[1] |
| Splice site mutation IVS7+1G>A[14] |
| IVS8+1G>C[76] |
| Splice site mutation IVS9+1G>T[14] |
| Splice site mutation IVS13del-13^-8[14] |
| Splice site mutation IVS16-8T>G[14] |
| Splice site mutation IVS18+1G>A[14] |

(continued)

| |
|---|
| Splice site mutation IVS19+2T>C[14] |
| IVS 23-8 G-A[36] |
| IVS24+5G>A[51] |
| Putative splice mutation 1198-1G>C[17] |
| Putative splice mutation 1810-3C>G[17] |
| Putative splice mutation 2178+1G>A[17] |
| Putative splice mutation 2344-1G>T[17] |
| Putative splice mutation 3213+1_3213+2delinsA[17] |
| c.-24C>A[78] |
| c.76 13 G>T[9] |
| c.77-19T>A[52] |
| c.90_93delGAAA[18] |
| c.124G>A[69] |
| c.150 +3 A>C[10] |
| c.249_250insT[18] |
| c.611+1G>A[84] |
| c.611+4A>G[36] |
| c.612-15_-6del10bp[55] |
| c.625A>C[31] |
| c.627+5G>T[31] |
| c.625A>C/ c.627+5G>T[31] |
| c.886C>T[31] |
| c.890A>G[59] |
| c.908+1G>A[57] |
| c.908+5G>A[55] |
| c.908delG[59] |
| 1273 1bp deletion[91] |
| c.1084-2A>G[57] |
| c.1445A>G[59] |
| c.1587-1589delCTT[31] |
| c.1621A>C[59] |
| 1939delA[14] |
| c.2081T>A[31] |
| 2098delA[16] |
| c.2343+1 G>T[80] |
| c.2178+1G>T[36] |
| c.2417G>A[78] |
| c.2620C>T[32] |
| c.2815-8A>G[55] |
| c.3003A>G[37] |

(continued)

| |
|---|
| c.3213 +4 A>G[9,37] |
| c.3213 +5 G>A[9] |
| c.3268C>T[75] |
| c.3382C>T[75] |
| c.3765(+1 +5)del5[42] |
| c.3767-3768insC[6] |
| 1145delC[8] |
| Ex13_Ex17del[82] |
| [A] A mutation to 'X' denotes an early stop codon |

*References for Tables 4 and 5*

[1] Noe et al., J Hepatol. 2005, vol. 43(3), p. 536-543.
[2] Lam et al., Am J Physiol Cell Physiol. 2007, vol. 293(5), p. C1709-16.
[3] Stindt et al., Liver Int. 2013, vol. 33(10), p. 1527-1735.
[4] Gao et al., Shandong Yiyao 2012, vol. 52(10), p. 14-16.
[5] Strautnieks et al., Gastroenterology. 2008, vol. 134(4), p. 1203-1214.
[6] Kagawa et al., Am J Physiol Gastrointest Liver Physiol. 2008, vol. 294(1), p. G58-67.
[7] Byrne et al., Hepatology. 2009, vol. 49(2), p. 553-567.
[8] Chen et al., J Pediatr. 2008, vol. 153(6), p. 825-832.
[9] Davit-Spraul et al., Hepatology 2010, vol. 51(5), p. 1645-1655.
[10] Dröge et al., Sci Rep. 2016, vol. 6: 24827.
[11] Lang et al., Pharmacogenet Genomics. 2007, vol. 17(1), p. 47-60.
[12] Ellinger et al., World J Gastroenterol. 2017, vol. 23(29), p. :5295-5303.
[13] Vitale et al., J Gastroenterol. 2018, vol. 53(8), p. 945-958.
[14] Knisely et al., Hepatology. 2006, vol. 44(2), p. 478-86.
[15] Ellis et al., Hepatology. 2018, vol. 67(4), p. 1531-1545.
[16] Lam et al., J Hepatol. 2006, vol. 44(1), p. 240-242.
[17] Varma et al., Hepatology 2015, vol. 62(1), p. 198-206.
[18] Treepongkaruna et al., World J Gastroenterol. 2009, vol. 15(34), p. 4339-4342.
[19] Zarenezhad et al., Hepatitis Monthly: 2017, vol. 17(2); e43500.
[20] Hayashi et al., Hepatol Res. 2016, vol. 46(2), p. 192-200.
[21] Guorui et al., Linchuang Erke Zazhi 2013, vol. 31(10), 905-909.
[22] van Mil et al., Gastroenterology. 2004, vol. 127(2), p. 379-384.
[23] Anzivino et al., Dig Liver Dis. 2013, vol. 45(3), p. 226-232.
[24] Park et al., World J Gastroenterol. 2016, vol. 22(20), p. 4901-4907.
[25] Imagawa et al., J Hum Genet. 2018, vol. 63(5), p. 569-577.
[26] Giovannoni et al., PLoS One. 2015, vol. 10(12): e0145021.
[27] Hu et al., Mol Med Rep. 2014, vol. 10(3), p. 1264-1274.
[28] Lang et al,. Drug Metab Dispos. 2006, vol. 34(9), p. 1582-1599.
[29] Masahata et al., Transplant Proc. 2016, vol. 48(9), p. 3156-3162.
[30] Holz et al., Hepatol Commun. 2018, vol. 2(2), p. 152-154.
[31] Li et al., Hepatology International 2017, vol. 11, No. 1, Supp. Supplement 1, pp. S180. Abstract Number: OP284.
[32] Francalanci et al., Laboratory Investigation 2011, vol. 91, Supp. SUPPL. 1, pp. 360A. Abstract Number: 1526.
[33] Francalanci et al., Digestive and Liver Disease 2010, vol. 42, Supp. SUPPL. 1, pp. S16. Abstract Number: T.N.5.
[34] Shah et al., J Pediatr Genet. 2017, vol. 6(2), p. 126-127.
[35] Gao et al., Hepatitis Monthly 2017, vol. 17(10), e55087/1-e55087/6.
[36] Evason et al., Am J Surg Pathol. 2011, vol. 35(5), p. 687-696.
[37] Davit-Spraul et al., Mol Genet Metab. 2014, vol. 113(3), p. 225-229.
[38] Maggiore et al., J Hepatol. 2010, vol. 53(5), p. 981-6.
[39] McKay et al., Version 2. F1000Res. 2013; 2: 32. DOI: 10.12688/f1000research.2-32.v2

[40] Liu et al., Pediatr Int. 2013, vol. 55(2), p. 138-144.
[41] Waisbourd-Zinman et al., Ann Hepatol. 2017, vol. 16(3), p. 465-468.
[42] Griffin, et al., Canadian Journal of Gastroenterology and Hepatology 2016, vol. 2016. Abstract Number: A200. Meeting Info: 2016 Canadian Digestive Diseases Week, CDDW 2016. Montreal, QC, United States. 26 Feb 2016-29 Feb 2016
[43] Qiu et al., Hepatology 2017, vol. 65(5), p. 1655-1669.
[44] Imagawa et al., Sci Rep. 2017, 7:41806.
[45] Kang et al., J Pathol Transl Med. 2019 May 16. doi: 10.4132/jptm.2019.05.03. [Epub ahead of print]
[46] Takahashi et al., Eur J Gastroenterol Hepatol. 2007, vol. 19(11), p. 942-6.
[47] Shimizu et al., Am J Transplant. 2011, vol. 11(2), p. 394-398.
[48] Krawczyk et al., Ann Hepatol. 2012, vol. 11(5), p. 710-744.
[49] Sharma et al., BMC Gastroenterol. 2018, vol. 18(1), p. 107.
[50] Sattler et al., Journal of Hepatology 2017, vol. 66, No. 1, Suppl. S, pp. S177. Meeting Info.: International Liver Congress / 52nd Annual Meeting of the European-Association-for-the-Study-of-the-Liver. Amsterdam, NETHERLANDS. April 19 -23, 2017. European Assoc Study Liver.
[51] Jung et al., J Pediatr Gastroenterol Nutr. 2007, vol. 44(4), p. 453-458.
[52] Sciveres. Digestive and Liver Disease 2010, vol. 42, Supp. SUPPL. 5, pp. S329. Abstract Number: CO18. Meeting Info: 17th National Congress SIGENP. Pescara, Italy. 07 Oct 2010-09 Oct 2010
[53] Sohn et al., Pediatr Gastroenterol Hepatol Nutr. 2019, vol. 22(2), p. 201-206.
[54] Ho et al., Pharmacogenet Genomics. 2010, vol. 20(1), p. 45-57.
[55] Wang et al., Hepatol Res. 2018, vol. 48(7), p. 574-584.
[56] Shaprio et al., J Hum Genet. 2010, vol. 55(5), p. 308-313.
[57] Bounford. University of Birmingham. Dissertation Abstracts International, (2016) Vol. 75, No. 1C. Order No.: AAI10588329. ProQuest Dissertations & Theses.
[58] Stolz et al., Aliment Pharmacol Ther. 2019, vol. 49(9), p. 1195-1204.
[59] Jankowska et al., J Pediatr Gastroenterol Nutr. 2014, vol. 58(1), p. 92-95.
[60] Kim. Journal of Pediatric Gastroenterology and Nutrition 2016, vol. 62, Supp. SUPPL. 1, pp. 620. Abstract Number: H-P-045. Meeting Info: 49th Annual Meeting of the European Society for Paediatric Gastroenterology, Hepatology and Nutrition, ESPGHAN 2016. Athens, Greece. 25 May 2016-28 May 2016.
[61] Pauli-Magnus et al., Hepatology 2003, vol. 38, No. 4 Suppl. 1, pp. 518A. print. Meeting Info.: 54th Annual Meeting of the American Association for the Study of Liver Diseases. Boston, MA, USA. October 24-28, 2003. American Association for the Study of Liver Diseases.
[62] Li et al., Hepatology International 2017, vol. 11, No. 1, Supp. Supplement 1, pp. S362. Abstract Number: PP0347. Meeting Info: 26th Annual Conference of the Asian Pacific Association for the Study of the Liver, APASL 2017. Shanghai, China. 15 Feb 2017-19 Feb 2017.
[63] Rumbo et al., Transplantation 2018, vol. 102, No. 7, Supp. Supplement 1, pp. S848. Abstract Number: P.752. Meeting Info: 27th International Congress of The Transplantation Society, TTS 2018. Madrid, Spain. 30 Jun 2018-05 Jul 2018.
[64] Lee et al., Pediatr Gastroenterol Hepatol Nutr. 2017, vol. 20(2), p. 114-123.
[65] Sherrif et al., Liver international: official journal of the International Association for the Study of the Liver 2013, vol. 33, No. 8, pp. 1266-1270.
[66] Blackmore et al., J Clin Exp Hepatol. 2013, vol. 3(2), p. 159-161.
[67] Matte et al., J Pediatr Gastroenterol Nutr. 2010, vol. 51(4), p. 488-493.
[68] Lin et al., Zhongguo Dang Dai Er Ke Za Zhi. 2018, vol. 20(9), p. 758-764.
[69] Harmanci et al., Experimental and Clinical Transplantation 2015, vol. 13, Supp. SUPPL. 2, pp. 76. Abstract Number: P62. Meeting Info: 1st Congress of the Turkic World Transplantation Society. Astana, Kazakhstan. 20 May 2015-22 May 2015.
[70] Herbst et al., Mol Cell Probes. 2015, vol. 29(5), p. 291-298.
[71] Moghadamrad et al., Hepatology. 2013, vol. 57(6), p. 2539-2541.
[72] Holz et al., Zeitschrift fur Gastroenterologie 2016, vol. 54, No. 8. Abstract Number: KV275. Meeting Info: Viszeralmedizin 2016, 71. Jahrestagung der Deutschen Gesellschaft fur Gastroenterologie, Verdauungs- und Stoffwechselkrankheiten mit Sektion Endoskopie - 10. Herbsttagung der Deutschen Gesellschaft fur Allgemein- und Viszeralchirurgie. Hamburg, Germany. 21 Sep 2016-24 Sep 2016.
[73] Wang et al., PLoS One. 2016; vol. 11(4): e0153114.
[74] Hao et al., International Journal of Clinical and Experimental Pathology 2017, vol. 10(3), p. 3480-3487.
[75] Arnell et al., J Pediatr Gastroenterol Nutr. 2010, vol. 51(4), p. 494-499.
[76] Sharma et al., Indian Journal of Gastroenterology 2017, vol. 36, No. 1, Supp. Supplement 1, pp. A99. Abstract Number: M-20. Meeting Info: 58th Annual Conference of the Indian Society of Gastroenterology, ISGCON 2017.

Bhubaneswar, India. 14 Dec 2017-17 Dec 2017.
[77] Beauséjour et al., Can J Gastroenterol. 2011, vol. 25(6), p. 311-314.
[78] Imagawa et al., Journal of Pediatric Gastroenterology and Nutrition 2016, vol. 63, Supp. Supplement 2, pp. S51. Abstract Number: 166. Meeting Info: World Congress of Pediatric Gastroenterology, Hepatology and Nutrition 2016. Montreal, QC, Canada. 05 Oct 2016-08 Oct 2016.
[79] Peng et al., Zhonghua er ke za zhi (Chinese journal of pediatrics) 2018, vol. 56, No. 6, pp. 440-444.
[80] Tibesar et al., Case Rep Pediatr. 2014, vol. 2014: 185923.
[81] Ng et al., Journal of Pediatric Gastroenterology and Nutrition 2018, vol. 66, Supp. Supplement 2, pp. 860. Abstract Number: H-P-127. Meeting Info: 51st Annual Meeting European Society for Paediatric Gastroenterology, Hepatology and Nutrition, ESPGHAN 2018. Geneva, Switzerland. 09 May 2018-12 May 2018.
[82] Wong et al., Clin Chem. 2008, vol. 54(7), p. 1141-1148.
[83] Pauli-Magnus et al., J Hepatol. 2005, vol. 43(2), p. 342-357.
[84] Jericho et al., Journal of Pediatric Gastroenterology and Nutrition. 60, vol. 3, p. 368-374.
[85] Scheimann et al., Gastroenterology 2007, vol. 132, No. 4, Suppl. 2, pp. A452. Meeting Info.: Digestive Disease Week Meeting/108th Annual Meeting of the American-Gastroenterological-Association. Washington, DC, USA. May 19 -24, 2007. Amer Gastroenterol Assoc; Amer Assoc Study Liver Dis; Amer Soc Gastrointestinal Endoscopy; Soc Surg Alimentary Tract.
[86] Jaquotot-Haerranz et al., Rev Esp Enferm Dig. 2013, vol. 105(1), p. 52-54.
[87] Khosla et al., American Journal of Gastroenterology 2015, vol. 110, No. Suppl. 1, pp. S397. Meeting Info.: 80th Annual Scientific Meeting of the American-College-of-Gastroenterology. Honolulu, HI, USA. October 16 -21, 2015.
[88] Dröge et al., J Hepatol. 2017, vol. 67(6), p. 1253-1264.
[89] Liu et al., Liver International 2010, vol. 30(6), p. 809-815.
[90] Chen et al., Journal of Pediatrics 2002, vol. 140(1), p. 119-124.
[91] U.S. Patent No. 9,295,677

In some embodiments, the mutation in ABCB11 is selected from A167T, G238V, V284L, E297G, R470Q, R470X, D482G, R487H, A570T, N591S, A865V, G982R, R1153C, and R1268Q.

Disclosed herein are methods of treating PFIC (e.g., PFIC-1 and PFIC-2) in a subject that includes performing an assay on a sample obtained from the subject to determine whether the subject has a mutation associated with PFIC (e.g., a ATP8B1, ABCB11, ABCB4, TJP2, NR1H4 or Myo5b mutation), and administering (e.g., specifically or selectively administering) a therapeutically effective amount of a compound of formula (I), or a pharmaceutically acceptable salt thereof, to the subject determined to have a mutation associated with PFIC. In some embodiments, the mutation is a ATP8B1 or ABCB11 mutation. For example, a mutation as provided in any one of Tables 1-4. In some embodiments, the mutation in ATP8B1 is selected from L127P, G308V, T456M, D554N, F529del, I661T, E665X, R930X, R952X, R1014X, and G1040R. In some embodiments, the mutation in ABCB11 is selected from A167T, G238V, V284L, E297G, R470Q, R470X, D482G, R487H, A570T, N591S, A865V, G982R, R1153C, and R1268Q.

Also disclosed are methods for treating PFIC (e.g., PFIC-1 and PFIC-2) in a subject in need thereof, the method comprising: (a) detecting a mutation associated with PFIC (e.g., a ATP8B1, ABCB11, ABCB4, TJP2, NR1H4 or Myo5b mutation) in the subject; and (b) administering to the subject a therapeutically effective amount of a compound of formula (I), or a pharmaceutically acceptable salt thereof. In some embodiments, methods for treating PFIC can include administering a therapeutically effective amount of a compound of formula (I), or a pharmaceutically acceptable salt thereof, to a subject having a mutation associated with PFIC (e.g., a ATP8B1, ABCB11, ABCB4, TJP2, NR1H4 or Myo5b mutation). In some embodiments, the mutation is a ATP8B1 or ABCB11 mutation. For example, a mutation as provided in any one of Tables 1-4. In some embodiments, the mutation in ATP8B1 is selected from L127P, G308V, T456M, D554N, F529del, I661T, E665X, R930X, R952X, R1014X, and G1040R. In some embodiments, the mutation in ABCB11 is selected from A167T, G238V, V284L, E297G, R470Q, R470X, D482G, R487H, A570T, N591S, A865V, G982R, R1153C, and R1268Q.

In some embodiments, the subject is determined to have a mutation associated with PFIC in a subject or a biopsy sample from the subject through the use of any art recognized tests, including next generation sequencsing (NGS). In some embodiments, the subject is determined to have a mutation associated with PFIC using a regulatory agency-approved, e.g., FDA-approved test or assay for identifying a mutation associated with PFIC in a subject or a biopsy sample from the subject or by performing any of the non-limiting examples of assays described herein. Additional methods of diagnosing PFIC are described in Gunaydin, M. et al., Hepat Med. 2018, vol. 10, p. 95-104.

In some embodiments, the treatment of PFIC (e.g., PFIC-1 or PFIC-2) decreases the level of serum bile acids in the subject. In some embodiments, the level of serum bile acids is determined by, for example, an ELISA enzymatic assay or the assays for the measurement of total bile acids as described in Danese et al., PLoS One. 2017, vol. 12(6): e0179200. In some embodiments, the level of serum bile acids can decrease by, for example, 10% to 40%, 20% to 50%, 30% to 60%, 40% to 70%, 50% to 80%, or by more than 90% of the level of serum bile acids prior to administration of a compound of

formula (I), or a pharmaceutically acceptable salt thereof. In some embodiments, the treatment of PFIC includes treatment of pruritus.

Since LBAT is expressed on hepatocytes, LBAT and dual ASBT/LBAT inhibitor substances need to have at least some bioavailability and free fraction in blood. Because LBAT inhibitor compounds only need to survive from the intestine to the liver, it is expected that a relatively low systemic exposure of such compounds will be sufficient, thereby minimizing the potential risk for any side effects in the rest of the body. It is expected that inhibition of LBAT and ASBT will have at least additive effects in decreasing the intrahepatic bile acid concentration. It is also expected that a dual ASBT/LBAT inhibitor may be able to reduce bile acid levels without inducing diarrhoea, as is sometimes observed with ASBT inhibitors.

Compounds having a high LBAT inhibiting potency and sufficient bioavailability are expected to be particularly suitable for the treatment of hepatitis. Compounds having a dual ASBT/LBAT inhibiting potency and sufficient bioavailability are expected to be particularly suitable for the treatment of non-alcoholic steatohepatitis (NASH).

NASH is a common and serious chronic liver disease that resembles alcoholic liver disease, but that occurs in people who drink little or no alcohol. In NASH patients, fat accumulation in the liver, known as nonalcoholic fatty liver disease (NAFLD) or steatosis, and other factors such as high LDL cholesterol and insulin resistance induce chronic inflammation in the liver and may lead to progressive scarring of tissue, known as fibrosis, and cirrhosis, followed eventually by liver failure and death. Patients with NASH have been found to have significantly higher total serum bile acid concentrations than healthy subjects under fasting conditions (2.2- to 2.4-fold increase in NASH) and at all post-prandial time points (1.7- to 2.2-fold increase in NASH). These are driven by increased taurine- and glycine-conjugated primary and secondary bile acids. Patients with NASH exhibited greater variability in their fasting and post-prandial bile acid profile. These results indicate that patients with NASH have higher fasting and post-prandial exposure to bile acids, including the more hydrophobic and cytotoxic secondary species. Increased bile acid exposure may be involved in liver injury and the pathogenesis of NAFLD and NASH (Ferslew et al., Dig Dis Sci. 2015, vol. 60, p. 3318-3328). It is therefore likely that ASBT and/or LBAT inhibition will be beneficial for the treatment of NASH.

NAFLD is characterized by hepatic steatosis with no secondary causes of hepatic steatosis including excessive alcohol consumption, other known liver diseases, or long-term use of a steatogenic medication (Chalasani et al., Hepatology 2018, vol. 67(1), p. 328-357). NAFLD can be categorized into non-alcoholic fatty liver (NAFL) and non-alcoholic steatohepatitis (NASH). According to Chalasani et al., NAFL is defined as the presence of ≥ 5% hepatic steatosis without evidence of hepatocellular injury in the form of hepatocyte ballooning. NASH is defined as the presence of ≥ 5% hepatic steatosis and inflammation with hepatocyte injury (e.g., ballooning), with or without any liver fibrosis. NASH is also commonly associated with hepatic inflammation and liver fibrosis, which can progress to cirrhosis, end-stage liver disease, and hepatocellular carcinoma. While liver fibrosis is not always present in NASH, the severity of the fibrosis, when present, can be linked to long-term outcomes.

There are many approaches used to assess and evaluate whether a subject has NAFLD and if so, the severity of the disease, including differentiating whether the NAFLD is NAFL or NASH. In some embodiments, the severity of NAFLD can be assessed using the NAS. In some embodiments, treatment of NAFLD can be assessed using the NAS. In some embodiments, the NAS can be determined as described in Kleiner et al., Hepatology. 2005, 41(6):1313-1321. See, for example, Table 6 for a simplified NAS scheme adapted from Kleiner.

**Table 6. Example of the NAFLD Activity Score (NAS) with Fibrosis Stage**

| Feature | Degree | Score |
|---|---|---|
| Steatosis | <5% | 0 |
| | 5-33% | 1 |
| | >33-66% | 2 |
| | >66% | 3 |
| Lobular Inflammation | No foci | 0 |
| | <2 foci/200x | 1 |
| | 2-4 foci/200x | 2 |
| | >4 foci/200x | 3 |
| Ballooning degeneration | None | 0 |
| | Few | 1 |
| | Many cells/Prominent ballooning | 2 |

(continued)

| Feature | Degree | Score |
|---|---|---|
| Fibrosis | None | 0 |
| | Perisinusoidal or periportal | 1 |
| | Perisinusoidal & portal/periportal | 2 |
| | Bridging fibrosis | 3 |
| | Cirrhosis | 4 |

In some embodiments, the NAS is determined non-invasively, for example, as described in U.S. Application Publication No. 2018/0140219. In some embodiments, the NAS is determined for a sample from the subject prior to administration of a compound of formula (I), or a pharmaceutically acceptable salt thereof. In some embodiments, the NAS is determined during the period of time or after the period of time of administration of a compound of formula (I), or a pharmaceutically acceptable salt thereof. In some embodiments, a lower NAS score during the period of time or after the period of time of administration of a compound of formula (I), or a pharmaceutically acceptable salt thereof compared to prior to administration of the compound of formula (I), or a pharmaceutically acceptable salt thereof indicates treatment of NAFLD (e.g., NASH). For example, a decrease in the NAS by 1, by 2, by 3, by 4, by 5, by 6, or by 7 indicates treatment of NAFLD (e.g., NASH). In some embodiments, the NAS following administration of a compound of formula (I), or a pharmaceutically acceptable salt thereof, is 7 or less. In some embodiments, the NAS during the period of time of administration of a compound of formula (I), or a pharmaceutically acceptable salt thereof, is 5 or less, 4 or less, 3 or less, or 2 or less. In some embodiments, the NAS during the period of time of administration of a compound of formula (I), or a pharmaceutically acceptable salt thereof, is 7 or less. In some embodiments, the NAS during the period of time of administration of a compound of formula (I), or a pharmaceutically acceptable salt thereof, is 5 or less, 4 or less, 3 or less, or 2 or less. In some embodiments, the NAS after the period of time of administration of a compound of formula (I), or a pharmaceutically acceptable salt thereof, is 7 or less. In some embodiments, the NAS after the period of time of administration of a compound of formula (I), or a pharmaceutically acceptable salt thereof, is 5 or less, 4 or less, 3 or less, or 2 or less.

Additional approaches of assessing and evaluating NASH in a subject include determining one or more of hepatic steatosis (e.g., accumulation of fat in the liver); hepatic inflammation; biomarkers indicative of one or more of liver damage, hepatic inflammation, liver fibrosis, and/or liver cirrhosis (e.g., serum markers and panels). Further examples of physiological indicators of NASH can include liver morphology, liver stiffness, and the size or weight of the subject's liver.

In some embodiments, NASH in the subject is evidenced by an accumulation of hepatic fat and detection of a biomarker indicative of liver damage. For example, elevated serum ferritin and low titers of serum autoantibodies can be common features of NASH.

In some embodiments, methods to assess NASH include magnetic resonance imaging, either by spectroscopy or by proton density fat fraction (MRI-PDFF) to quantify steatosis, transient elastography (FIBROSCAN®), hepatic venous pressure gradient (HPVG), hepatic stiffness measurement with MRE for diagnosing significant liver fibrosis and/or cirrhosis, and assessing histological features of liver biopsy. In some embodiments, magnetic resonance imaging is used to detect one or more of steatohepatitis (NASH-MRI), liver fibrosis (Fibro-MRI), and steatosis. See, for example, U.S. Application Publication Nos. 2016/146715 and 2005/0215882.

In some embodiments, treatment of NASH can include a decrease of one or more symptoms associated with NASH; reduction in the amount of hepatic steatosis; a decrease in the NAS; a decrease in hepatic inflammation; a decrease in the level of biomarkers indicative of one or more of liver damage, inflammation, liver fibrosis, and/or liver cirrhosis; and a reduction in fibrosis and/or cirrhosis, a lack of further progression of fibrosis and/or cirrhosis, or a slowing of the progression of fibrosis and/or cirrhosis in the subject following administration of one or more doses of a compound of formula (I), or a pharmaceutically acceptable salt thereof.

In some embodiments, treatment of NASH comprises a decrease of one or more symptoms associated with NASH in the subject. Exemplary symptoms can include one or more of an enlarged liver, fatigue, pain in the upper right abdomen, abdominal swelling, enlarged blood vessels just beneath the skin's surface, enlarged breasts in men, enlarged spleen, red palms, jaundice, and pruritus. In some embodiments, the subject is asymptomatic. In some embodiments, the total body weight of the subject does not increase. In some embodiments, the total body weight of the subject decreases. In some embodiments, the body mass index (BMI) of the subject does not increase. In some embodiments, the body mass index (BMI) of the subject decreases. In some embodiments, the waist and hip (WTH) ratio of the subject does not increase. In some embodiments, the waist and hip (WTH) ratio of the subject decreases.

In some embodiments, treatment of NASH can be assessed by measuring hepatic steatosis. In some embodiments, treatment of NASH comprises a reduction in hepatic steatosis following administration of a compound of formula (I),

or a pharmaceutically acceptable salt thereof, as described herein. In some embodiments, hepatic steatosis is determined by one or more methods selected from the group consisting of ultrasonography, computed tomography (CT), magnetic resonance imaging, magnetic resonance spectroscopy (MRS), magnetic resonance elastography (MRE), transient elastography (TE) (e.g., FIBROSCAN®), measurement of liver size or weight, or by liver biopsy (see, e.g., Di Lascio et al., Ultrasound Med Biol. 2018, vol. 44(8), p. 1585-1596; Lv et al., J Clin Transl Hepatol. 2018, vol. 6(2), p. 217-221; Reeder et al., J Magn Reson Imaging. 2011, vol. 34(4), spcone; and de Lédinghen V, et al., J Gastroenterol Hepatol. 2016, vol. 31(4), p. 848-855). A subject diagnosed with NASH can have greater than about 5% hepatic steatosis, for example, greater than about 5% to about 25%, about 25% to about 45%, about 45% to about 65%, or greater than about 65% hepatic steatosis. In some embodiments, a subject with greater than about 5% to about 33% hepatic steatosis has stage 1 hepatic steatosis, a subject with about 33% to about 66% hepatic steatosis has stage 2 hepatic steatosis, and a subject with greater than about 66% hepatic steatosis has stage 3 hepatic steatosis.

In some embodiments, the amount of hepatic steatosis is determined prior to administration of a compound of formula (I), or a pharmaceutically acceptable salt thereof. In some embodiments, the amount of hepatic steatosis is determined during the period of time or after the period of time of administration of the compound of formula (I), or a pharmaceutically acceptable salt thereof. In some embodiments, a reduction in the amount of hepatic steatosis during the period of time or after the period of time of administration of the compound of formula (I), or a pharmaceutically acceptable salt thereof, compared to prior to administration of the compound of formula (I), or a pharmaceutically acceptable salt thereof, indicates treatment of NASH. For example, a reduction in the amount of hepatic steatosis by about 1% to about 50%, about 25% to about 75%, or about 50% to about 100% indicates treatment of NASH. In some embodiments, a reduction in the amount of hepatic steatosis by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% indicates treatment of NASH.

In some embodiments, the presence of hepatic inflammation is determined by one or more methods selected from the group consisting of biomarkers indicative of hepatic inflammation and a liver biopsy sample(s) from the subject. In some embodiments, the severity of hepatic inflammation is determined from a liver biopsy sample(s) from the subject. For example, hepatic inflammation in a liver biopsy sample can be assessed as described in Kleiner et al., Hepatology 2005, vol. 41(6), p. 1313-1321 and Brunt et al., Am J Gastroenterol 1999, vol. 94, p. 2467-2474. In some embodiments, the severity of hepatic inflammation is determined prior to administration of a compound of formula (I), or a pharmaceutically acceptable salt thereof. In some embodiments, the severity of hepatic inflammation is determined during the period of time or after the period of time of administration of a compound of formula (I), or a pharmaceutically acceptable salt thereof. In some embodiments, a decrease in the severity of hepatic inflammation during the period of time or after the period of time of administration of a compound of formula (I), or a pharmaceutically acceptable salt thereof, compared to prior to administration of the compound of formula (I), or a pharmaceutically acceptable salt thereof, indicates treatment of NASH. For example, a decrease in the severity of hepatic inflammation by about 1% to about 50%, about 25% to about 75%, or about 50% to about 100% indicates treatment of NASH. In some embodiments, a decrease in the severity of hepatic inflammation by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% indicates treatment of NASH.

In some embodiments, treatment of NASH comprises treatment of fibrosis and/or cirrhosis, e.g., a decrease in the severity of fibrosis, a lack of further progression of fibrosis and/or cirrhosis, or a slowing of the progression of fibrosis and/or cirrhosis. In some embodiments, the presence of fibrosis and/or cirrhosis is determined by one or more methods selected from the group consisting of transient elastography (e.g., FIBROSCAN®), non-invasive markers of hepatic fibrosis, and histological features of a liver biopsy. In some embodiments, the severity (e.g., stage) of fibrosis is determined by one or more methods selected from the group consisting of transient elastography (e.g., FIBROSCAN®), a fibrosis-scoring system, biomarkers of hepatic fibrosis (e.g., non-invasive biomarkers), and hepatic venous pressure gradient (HVPG). Non-limiting examples of fibrosis scoring systems include the NAFLD fibrosis scoring system (see, e.g., Angulo et al., Hepatology 2007, vol. 45(4), p. 846-54), the fibrosis scoring system in Brunt et al., Am. J. Gastroenterol. 1999, vol. 94, p. 2467-2474, the fibrosis scoring system in Kleiner et al., Hepatology 2005, vol. 41(6), p. 1313-1321, and the ISHAK fibrosis scoring system (see Ishak et al., J. Hepatol. 1995, vol. 22, p. 696-699).

In some embodiments, the severity of fibrosis is determined prior to administration of a compound of formula (I), or a pharmaceutically acceptable salt thereof. In some embodiments, the severity of fibrosis is determined during the period of time or after the period of time of administration of a compound of formula (I), or a pharmaceutically acceptable salt thereof. In some embodiments, a decrease in the severity of fibrosis during the period of time or after the period of time of administration of a compound of formula (I), or a pharmaceutically acceptable salt thereof, compared to prior to administration of the compound of formula (I), or a pharmaceutically acceptable salt thereof, indicates treatment of NASH. In some embodiments, a decrease in the severity of fibrosis, a lack of further progression of fibrosis and/or cirrhosis, or a slowing of the progression of fibrosis and/or cirrhosis indicates treatment of NASH. In some embodiments, the severity of fibrosis is determined using a scoring system such as any of the fibrosis scoring systems described herein, for example, the

score can indicate the stage of fibrosis, e.g., stage 0 (no fibrosis), stage 1, stage 2, stage 3, and stage 4 (cirrhosis) (see, e.g., Kleiner et al). In some embodiments, a decrease in the stage of the fibrosis is a decrease in the severity of the fibrosis. For example, a decrease by 1, 2, 3, or 4 stages is a decrease in the severity of the fibrosis. In some embodiments, a decrease in the stage, e.g., from stage 4 to stage 3, from stage 4 to stage 2, from stage 4 to stage 1, from stage 4 to stage 0, from stage 3 to stage 2, from stage 3 to stage 1, from stage 3 to stage 0, from stage 2 to stage 1, from stage 2 to stage 0, or from stage 1 to stage 0 indicates treatment of NASH. In some embodiments, the stage of fibrosis decreases from stage 4 to stage 3, from stage 4 to stage 2, from stage 4 to stage 1, from stage 4 to stage 0, from stage 3 to stage 2, from stage 3 to stage 1, from stage 3 to stage 0, from stage 2 to stage 1, from stage 2 to stage 0, or from stage 1 to stage 0 following administration of a compound of formula (I), or a pharmaceutically acceptable salt thereof, compared to prior to administration of the compound of formula (I), or a pharmaceutically acceptable salt thereof. In some embodiments, the stage of fibrosis decreases from stage 4 to stage 3, from stage 4 to stage 2, from stage 4 to stage 1, from stage 4 to stage 0, from stage 3 to stage 2, from stage 3 to stage 1, from stage 3 to stage 0, from stage 2 to stage 1, from stage 2 to stage 0, or from stage 1 to stage 0 during the period of time of administration of a compound of formula (I), or a pharmaceutically acceptable salt thereof, compared to prior to administration of the compound of formula (I), or a pharmaceutically acceptable salt thereof. In some embodiments, the stage of fibrosis decreases from stage 4 to stage 3, from stage 4 to stage 2, from stage 4 to stage 1, from stage 4 to stage 0, from stage 3 to stage 2, from stage 3 to stage 1, from stage 3 to stage 0, from stage 2 to stage 1, from stage 2 to stage 0, or from stage 1 to stage 0 after the period of time of administration of a compound of formula (I), or a pharmaceutically acceptable salt thereof, compared to prior to administration of the compound of formula (I), or a pharmaceutically acceptable salt thereof.

In some embodiments, the presence of NASH is determined by one or more biomarkers indicative of one or more of liver damage, inflammation, liver fibrosis, and/or liver cirrhosis or scoring systems thereof. In some embodiments, the severity of NASH is determined by one or more biomarkers indicative of one or more of liver damage, inflammation, liver fibrosis, and/or liver cirrhosis or scoring systems thereof. The level of the biomarker can be determined by, for example, measuring, quantifying, and monitoring the expression level of the gene or mRNA encoding the biomarker and/or the peptide or protein of the biomarker. Non-limiting examples of biomarkers indicative of one or more of liver damage, inflammation, liver fibrosis, and/or liver cirrhosis and/or scoring systems thereof include the aspartate aminotransferase (AST) to platelet ratio index (APRI); the aspartate aminotransferase (AST) and alanine aminotransferase (ALT) ratio (AAR); the FIB-4 score, which is based on the APRI, alanine aminotransferase (ALT) levels, and age of the subject (see, e.g., McPherson et al., Gut 2010, vol. 59(9), p. 1265-9); hyaluronic acid; pro-inflammatory cytokines; a panel of biomarkers consisting of α2-macroglobulin, haptoglobin, apolipoprotein A1, bilirubin, gamma glutamyl transpeptidase (GGT) combined with a subject's age and gender to generate a measure of fibrosis and necroinflammatory activity in the liver (e.g., FIBROTEST®, FIBROSURE®), a panel of biomarkers consisting of bilirubin, gamma-glutamyltransferase, hyaluronic acid, α2-macroglobulin combined with the subject's age and sex (e.g., HEPASCORE®; see, e.g., Adams et al., Clin. Chem. 2005, vol. 51(10), p. 1867-1873), and a panel of biomarkers consisting of tissue inhibitor of metalloproteinase-1, hyaluronic acid, and α2-macroglobulin (e.g., FIBROSPECT®); a panel of biomarkers consisting of tissue inhibitor of metalloproteinases 1 (TIMP-1), amino-terminal propeptide of type III procollagen (PIIINP) and hyaluronic acid (HA) (e.g., the Enhanced Liver Fibrosis (ELF) score, see, e.g., Lichtinghagen R, et al., J Hepatol. 2013 Aug;59(2):236-42). In some embodiments, the presence of fibrosis is determined by one or more of the FIB-4 score, a panel of biomarkers consisting of α2-macroglobulin, haptoglobin, apolipoprotein A1, bilirubin, gamma glutamyl transpeptidase (GGT) combined with a subject's age and gender to generate a measure of fibrosis and necroinflammatory activity in the liver (e.g., FIBROTEST®, FIBROSURE®), a panel of biomarkers consisting of bilirubin, gamma-glutamyltransferase, hyaluronic acid, α2-macroglobulin combined with the subject's age and sex (e.g., HEPASCORE®; see, e.g., Adams et al., Clin. Chem. 2005, vol. 51(10), p. 1867-1873), and a panel of biomarkers consisting of tissue inhibitor of metalloproteinase-1, hyaluronic acid, and α2-macroglobulin (e.g., FIBROSPECT®); and a panel of biomarkers consisting of tissue inhibitor of metalloproteinases 1 (TIMP-1), amino-terminal propeptide of type III procollagen (PIIINP) and hyaluronic acid (HA) (e.g., the Enhanced Liver Fibrosis (ELF) score).

In some embodiments, the level of aspartate aminotransferase (AST) does not increase. In some embodiments, the level of aspartate aminotransferase (AST) decreases. In some embodiments, the level of alanine aminotransferase (ALT) does not increase. In some embodiments, the level of alanine aminotransferase (ALT) decreases. In some embodiments, the "level" of an enzyme refers to the concentration of the enzyme, e.g., within blood. For example, the level of AST or ALT can be expressed as Units/L.

In some embodiments, the severity of fibrosis is determined by one or more of the FIB-4 score, a panel of biomarkers consisting of α2-macroglobulin, haptoglobin, apolipoprotein A1, bilirubin, gamma glutamyl transpeptidase (GGT) combined with a subject's age and gender to generate a measure of fibrosis and necroinflammatory activity in the liver (e.g., FIBROTEST®, FIBROSURE®), a panel of biomarkers consisting of bilirubin, gamma-glutamyltransferase, hyaluronic acid, α2-macroglobulin combined with the subject's age and sex (e.g., HEPASCORE®; see, e.g., Adams et al., Clin. Chem. 2005, vol. 51(10), p. 1867-1873), and a panel of biomarkers consisting of tissue inhibitor of metalloproteinase-1, hyaluronic acid, and α2-macroglobulin (e.g., FIBROSPECT®); and a panel of biomarkers consisting of tissue

inhibitor of metalloproteinases 1 (TIMP-1), amino-terminal propeptide of type III procollagen (PIIINP) and hyaluronic acid (HA) (e.g., the Enhanced Liver Fibrosis (ELF) score).

In some embodiments, hepatic inflammation is determined by the level of liver inflammation biomarkers, e.g., pro-inflammatory cytokines. Non-limiting examples of biomarkers indicative of liver inflammation include interleukin-(IL) 6, interleukin-(IL) 1β, tumor necrosis factor (TNF)-α, transforming growth factor (TGF)-β, monocyte chemotactic protein (MCP)-1, C-reactive protein (CRP), PAI-1, and collagen isoforms such as Col1a1, Col1a2, and Col4a1 (see, e.g., Neuman, et al., Can. J. Gastroenterol. Hepatol. 2014, vol. 28(11), p. 607-618 and U.S. Patent No. 9,872,844). Liver inflammation can also be assessed by change of macrophage infiltration, e.g., measuring a change of CD68 expression level. In some embodiments, liver inflammation can be determined by measuring or monitoring serum levels or circulating levels of one or more of interleukin-(IL) 6, interleukin-(IL) 1β, tumor necrosis factor (TNF)-α, transforming growth factor (TGF)-β, monocyte chemotactic protein (MCP)-1, and C-reactive protein (CRP).

In some embodiments, the level of one or more biomarkers indicative of one or more of liver damage, inflammation, liver fibrosis, and/or liver cirrhosis is determined for a sample from the subject prior to administration of a compound of formula (I), or a pharmaceutically acceptable salt thereof. In some embodiments, the level of one or more biomarkers indicative of one or more of liver damage, inflammation, liver fibrosis, and/or liver cirrhosis is determined during the period of time or after the period of time of administration of a compound of formula (I), or a pharmaceutically acceptable salt thereof. In some embodiments, a decrease in the level of one or more biomarkers indicative of one or more of liver damage, inflammation, liver fibrosis, and/or liver cirrhosis during the period of time or after the period of time of administration of a compound of formula (I), or a pharmaceutically acceptable salt thereof, compared to prior to administration of the compound of formula (I), or a pharmaceutically acceptable salt thereof, indicates treatment of NASH. For example, a decrease in the level of one or more biomarkers indicative of one or more of liver damage, inflammation, liver fibrosis, and/or liver cirrhosis by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99% indicates treatment of NASH. In some embodiments, the decrease in the level of one or more biomarkers indicative of one or more of liver damage, inflammation, liver fibrosis, and/or liver cirrhosis following administration of the compound of formula (I), or a pharmaceutically acceptable salt thereof, is by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99%. In some embodiments, the level of one or more biomarkers indicative of one or more of liver damage, inflammation, liver fibrosis, and/or liver cirrhosis during the period of time of administration of a compound of formula (I), or a pharmaceutically acceptable salt thereof, is by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99%. In some embodiments, the level of one or more biomarkers indicative of one or more of liver damage, inflammation, liver fibrosis, and/or liver cirrhosis after the period of time of administration of a compound of formula (I), or a pharmaceutically acceptable salt thereof, is by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99%.

In some embodiments, the treatment of NASH decreases the level of serum bile acids in the subject. In some embodiments, the level of serum bile acids is determined by, for example, an ELISA enzymatic assay or the assays for the measurement of total bile acids as described in Danese et al., PLoS One. 2017, vol. 12(6): e0179200. In some embodiments, the level of serum bile acids can decrease by, for example, 10% to 40%, 20% to 50%, 30% to 60%, 40% to 70%, 50% to 80%, or by more than 90% of the level of serum bile acids prior to administration of a compound of formula (I), or a pharmaceutically acceptable salt thereof. In some embodiments, the NASH is NASH with attendant cholestasis. In cholestasis, the release of bile, including bile acids, from the liver is blocked. Bile acids can cause hepatocyte damage (see, e.g., Perez MJ, Briz O. World J. Gastroenterol. 2009, vol. 15(14), p. 1677-1689) likely leading to or increasing the progression of fibrosis (e.g., cirrhosis) and increasing the risk of hepatocellular carcinoma (see, e.g., Sorrentino P et al., Dig. Dis. Sci. 2005, vol. 50(6), p. 1130-1135 and Satapathy SK and Sanyal AJ. Semin. Liver Dis. 2015, vol. 35(3), p. 221-235). In some embodiments, the treatment of NASH includes treatment of pruritus. In some embodiments, the treatment of NASH with attendant cholestasis includes treatment of pruritus. In some embodiments, a subject with NASH with attendant cholestasis has pruritus.

Exemplary biomarkers for NASH are provided in Table 7.

**Table 7. Exemplary NASH biomarkers**

| |
|---|
| **Liver Fibrosis Biomarkers** |
| Aspartate aminotransferase (AST) to platelet ratio index (APRI) |
| Aspartate aminotransferase (AST) and alanine aminotransferase (ALT) ratio (AAR) |
| FIB-4 score[1] |
| Hyaluronic acid |
| Pro-inflammatory cytokines |
| A panel including α2-macroglobulin, haptoglobin, apolipoprotein A1, bilirubin, gamma glutamyl transpeptidase (GGT) combined with a subject's age and gender |
| to generate a measure of fibrosis and necroinflammatory activity in the liver (e.g., FIBROTEST®, FIBROSURE®) |
| A panel including bilirubin, gamma-glutamyltransferase, hyaluronic acid, α2-macroglobulin combined with the subject's age and sex (e.g., HEPASCORE®[2]) |
| A panel including tissue inhibitor of metalloproteinase-1, hyaluronic acid, and α2-macroglobulin (e.g., FIBROSPECT®) |
| A panel including tissue inhibitor of metalloproteinases 1 (TIMP-1), amino-terminal propeptide of type III procollagen (PIIINP) and hyaluronic acid (HA) (e.g., the Enhanced Liver Fibrosis (ELF) score[3]) |
| **Liver inflammation biomarkers[4,5]** |
| Interleukin-(IL) 6 |
| Interleukin-(IL) 1β |
| Tumor necrosis factor (TNF)-α |
| Transforming growth factor (TGF)-β |
| Monocyte chemotactic protein (MCP)-1 |
| C-reactive protein (CRP) |
| PAI-1 |
| Collagen isoforms (e.g., Col1a1, Col1a2, and Col4a1) |
| Change of macrophage infiltration (e.g., a change of CD68 expression level) |

*References for Table 7*

[1] McPherson et al., Gut. 2010, vol. 59(9), p. 1265-1269.
[2] Adams, et al. Clin Chem. 2005, vol. 51(10), p. 1867-1873.
[3] Lichtinghagen, et al. J Hepatol. 2013, vol. 59(2), p. 236-242.
[4] Neuman, et al. Can J Gastroenterol Hepatol. 2014, vol. 28(11), p. 607-618.
[5] U.S. Patent No. 9,872,844

Some compounds of formula (I), or pharmaceutically acceptable salts thereof, may show a higher free fraction in plasma. In some embodiments, the free fraction is greater than about 0.2%, such as greater than about 0.4%, such as greater than about 0.6%, such as greater than about 0.8%, such as greater than about 1.0%, such as greater than about 1.25%, such as greater than about 1.5%, such as greater than about 1.75%, such as greater than about 2.0%, such as greater than about 2.5%, such as greater than about 3%, such as greater than about 4%, such as greater than about 5%, such as greater than about 7.5%, such as greater than about 10%, or such as greater than about 20%.

Some compounds of formula (I), or pharmaceutically acceptable salts thereof, may be excreted in urine. In some embodiments, the fraction of the compound that is excreted in urine is greater than about 0.2%, such as greater than about 0.4%, such as greater than about 0.6%, such as greater than about 0.8%, such as greater than about 1.0%, such as greater than about 2%, such as greater than about 3%, such as greater than about 5%, such as greater than about 7.5%, such as greater than about 10%, such as greater than about 15%, such as greater than about 20%, such as greater than about 30%, or such as greater than about 50%.

Following absorption from the intestine, some compounds of formula (I), or pharmaceutically acceptable salts thereof, may be circulated via the enterohepatic circulation. In some embodiments, the fraction of the compound that is circulated via the enterohepatic circulation is greater than about 0.1%, such as greater than about 0.2%, such as greater than about 0.3%, such as greater than about 0.5%, such as greater than about 1.0%, such as greater than about 1.5%, such as greater than about 2%, such as greater than about 3%, such as greater than about 5%, such as greater than about 7%, such as greater than about 10%, such as greater than about 15%, such as greater than about 20%, such as greater

than about 30% or such as greater than about 50%.

Some compounds of formula (I), or pharmaceutically acceptable salts thereof, may cause renal excretion of bile salts. In some embodiments, the fraction of circulating bile acids that is excreted by the renal route is greater than about 1 %, such as greater than about 2%, such as greater than about 5%, such as greater than about 7%, such as greater than about 10%, such as greater than about 15%, such as greater than about 20%, or such as greater than about 25%.

Some compounds of formula (I), or pharmaceutically acceptable salts thereof, may show improved or optimal permeability. The permeability may be measured in Caco2 cells, and values are given as Papp (apparent permeability) values in cm/s. In some embodiments, the permeability is greater than at least about 0.1 x $10^{-6}$ cm/s, such as greater than about 0.2 x $10^{-6}$ cm/s, such as greater than about 0.4 x $10^{-6}$ cm/s, such as greater than about 0.7 x $10^{-6}$ cm/s, such as greater than about 1.0 x $10^{-6}$ cm/s, such as greater than about 2 x $10^{-6}$ cm/s, such as greater than about 3 x $10^{-6}$ cm/s, such as greater than about 5 x $10^{-6}$ cm/s, such as greater than about 7 x $10^{-6}$ cm/s, such as greater than about 10 x $10^{-6}$ cm/s, such as greater than about 15 x $10^{-6}$ cm/s.

Some compounds of formula (I), or pharmaceutically acceptable salts thereof, may show an improved or optimal bioavailability. In some embodiments, the oral bioavailability is greater than about 5%, such as greater than about 7%, such as greater than about 10%, such as greater than about 15%, such as greater than about 20%, such as greater than about 30%, such as greater than about 40%, such as greater than about 50 %, such as greater than about 60 %, such as greater than about 70% or such as greater than about 80%. In other embodiments, the oral bioavailability is between about 10 and about 90%, such as between about 20 and about 80%, such as between about 30 and about 70% or such as between about 40 and about 60%.

Some compounds of formula (I), or pharmaceutically acceptable salts thereof, may be a substrate to relevant transporters in the kidney.

Some compounds of formula (I), or pharmaceutically acceptable salts thereof, may give rise to concentrations of bile acids in the intestine, the liver and in serum that do not cause adverse gastrointestinal effects.

Some compounds of formula (I), or pharmaceutically acceptable salts thereof, may decrease the concentration of bile acids in the liver without causing gastrointestinal disorders such as diarrhoea.

As used herein, the terms "treatment", "treat" and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

A suitable pharmaceutically acceptable salt of a compound of the invention is, for example, a base-addition salt of a compound of the invention which is sufficiently acidic, such as an alkali metal salt (e.g., a sodium or potassium salt), an alkaline earth metal salt (e.g., a calcium or magnesium salt), an ammonium salt, or a salt with an organic base which affords a physiologically acceptable cation, for example a salt with methylamine, dimethylamine, trimethylamine, piperidine, morpholine or tris-(2-hydroxyethyl)amine.

Some compounds of formula (I), or pharmaceutically acceptable salts thereof, may have chiral centres and/or geometric isomeric centres (E- and Z-isomers). It is to be understood that the invention encompasses all such optical isomers, diastereoisomers and geometric isomers that possess ASBT and/or LBAT inhibitory activity. The invention also encompasses any and all tautomeric forms of compounds of formula (I), or pharmaceutically acceptable salts thereof, that possess ASBT and/or LBAT inhibitory activity. Certain compounds of formula (I), or pharmaceutically acceptable salts thereof, may exist in unsolvated as well as solvated forms, such as, for example, hydrated forms. It is to be understood that the invention encompasses all such solvated forms that possess ASBT and/or LBAT inhibitory activity.

In another aspect, the invention relates to a pharmaceutical composition comprising a therapeutically effective amount of a compound of formula (I), or a pharmaceutically acceptable salt thereof, and one or more pharmaceutically acceptable excipients. The excipients may e.g. include fillers, binders, disintegrants, glidants and lubricants. In general, pharmaceutical compositions may be prepared in a conventional manner using conventional excipients.

Examples of suitable fillers include, but are not limited to, dicalcium phosphate dihydrate, calcium sulfate, lactose (such as lactose monohydrate), sucrose, mannitol, sorbitol, cellulose, microcrystalline cellulose, dry starch, hydrolyzed starches and pregelatinized starch.

Examples of suitable binders include, but are not limited to, starch, pregelatinized starch, gelatin, sugars (such as sucrose, glucose, dextrose, lactose and sorbitol), polyethylene glycol, waxes, natural and synthetic gums (such as acacia gum and tragacanth gum), sodium alginate, cellulose derivatives (such as hydroxypropylmethylcellulose (or hypromellose), hydroxypropylcellulose and ethylcellulose) and synthetic polymers (such as acrylic acid and methacrylic acid copolymers, methacrylic acid copolymers, methyl methacrylate copolymers, aminoalkyl methacrylate copolymers, polyacrylic acid/polymethacrylic acid copolymers and polyvinylpyrrolidone (povidone)).

Examples of suitable disintegrants include, but are not limited to, dry starch, modified starch (such as (partially)

pregelatinized starch, sodium starch glycolate and sodium carboxymethyl starch), alginic acid, cellulose derivatives (such as sodium carboxymethylcellulose, hydroxypropyl cellulose, and low substituted hydroxypropyl cellulose (L-HPC)) and cross-linked polymers (such as carmellose, croscarmellose sodium, carmellose calcium and cross-linked PVP (crospovidone)).

Examples of suitable glidants and lubricants include, but are not limited to, talc, magnesium stearate, calcium stearate, stearic acid, glyceryl behenate, colloidal silica, aqueous silicon dioxide, synthetic magnesium silicate, fine granulated silicon oxide, starch, sodium lauryl sulfate, boric acid, magnesium oxide, waxes (such as carnauba wax), hydrogenated oil, polyethylene glycol, sodium benzoate, polyethylene glycol, and mineral oil.

The pharmaceutical composition may be conventionally coated with one or more coating layers. Enteric coating layers or coating layers for delayed or targeted release of the compound of formula (I), or pharmaceutically acceptable salts thereof, are also contemplated. The coating layers may comprise one or more coating agents, and may optionally comprise plasticizers and/or pigments (or colorants).

Example of suitable coating agents include, but are not limited to, cellulose-based polymers (such as ethylcellulose, hydroxypropylmethylcellulose (or hypromellose), hydroxypropylcellulose, cellulose acetate phthalate, cellulose acetate succinate, hydroxypropyl methylcellulose acetate succinate and hydroxypropyl methylcellulose phthalate), vinyl-based polymers (such as polyvinyl alcohol) and polymers based on acrylic acid and derivatives thereof (such as acrylic acid and methacrylic acid copolymers, methacrylic acid copolymers, methyl methacrylate copolymers, aminoalkyl methacrylate copolymers, polyacrylic acid/polymethacrylic acid copolymers).

Examples of suitable plasticizers include, but are not limited to, triethyl citrate, glyceryl triacetate, tributyl citrate, diethyl phthalate, acetyl tributyl citrate, dibutyl phthalate, dibutyl sebacate and polyethylene glycol.

Examples of suitable pigments include, but are not limited to, titanium dioxide, iron oxides (such as yellow, brown, red or black iron oxides) and barium sulfate.

The pharmaceutical composition may be in a form that is suitable for oral administration, for parenteral injection (including intravenous, subcutaneous, intramuscular and intravascular injection), for topical administration of for rectal administration. In a preferred embodiment, the pharmaceutical composition is in a form that is suitable for oral administration, such as a tablet or a capsule.

The dosage required for the therapeutic or prophylactic treatment will depend on the route of administration, the severity of the disease, the age and weight of the patient and other factors normally considered by the attending physician, when determining the appropriate regimen and dosage level for a particular patient.

The amount of the compound to be administered will vary for the patient being treated, and may vary from about 1 μg/kg of body weight to about 50 mg/kg of body weight per day. A unit dose form, such as a tablet or capsule, will usually contain about 1 to about 250 mg of active ingredient, such as about 1 to about 100 mg, or such as about 1 to about 50 mg, or such as about 1 to about 20 mg, e.g. about 2.5 mg, or about 5 mg, or about 10 mg, or about 15 mg. The daily dose can be administered as a single dose or divided into one, two, three or more unit doses. An orally administered daily dose of a bile acid modulator is preferably within about 0.1 to about 250 mg, more preferably within about 1 to about 100 mg, such as within about 1 to about 5 mg, such as within about 1 to about 10 mg, such as within about 1 to about 15 mg, or such as within about 1 to about 20 mg.

In another aspect, the invention relates to a compound of formula (I), or a pharmaceutically acceptable salt thereof, for use as a medicament. The invention also relates to the use of a compound of formula (I), or a pharmaceutically acceptable salt thereof, as a medicament.

In another aspect, the invention relates to a compound of formula (I), or a pharmaceutically acceptable salt thereof, for use in the treatment or prevention of any of the diseases recited herein.

*Combination therapy*

In one aspect of the invention, the compounds of formula (I), or pharmaceutically acceptable salts thereof, are administered in combination with at least one other therapeutically active agent, such as with one, two, three or more other therapeutically active agents. The compound of formula (I), or a pharmaceutically acceptable salt thereof, and the at least one other therapeutically active agent may be administered simultaneously, sequentially or separately. Therapeutically active agents that are suitable for combination with the compounds of formula (I) include, but are not limited to, known active agents that are useful in the treatment of any of the aforementioned conditions, disorders and diseases.

In one embodiment, compounds of formula (I), or pharmaceutically acceptable salts thereof, are administered in combination with another ASBT inhibitor. Suitable ASBT inhibitors are disclosed in WO 93/16055, WO 94/18183, WO 94/18184, WO 96/05188, WO 96/08484, WO 96/16051, WO 97/33882, WO 98/03818, WO 98/07449, WO 98/40375, WO 99/35135, WO 99/64409, WO 99/64410, WO 00/47568, WO 00/61568, WO 00/38725, WO 00/38726, WO 00/38727, WO 00/38728, WO 00/38729, WO 01/66533, WO 01/68096, WO 02/32428, WO 02/50051, WO 03/020710, WO 03/022286, WO 03/022825, WO 03/022830, WO 03/061663, WO 03/091232, WO 03/106482, WO 2004/006899, WO 2004/076430, WO 2007/009655, WO 2007/009656, WO 2011/137135, WO 2019/234077, WO 2020/161216, WO 2020/161217, WO

2021/110883, WO 2021/110884, WO 2021/110885, WO 2021/110886, WO 2021/110887, WO 2022/029101, DE 19825804, EP 864582, EP 489423, EP 549967, EP 573848, EP 624593, EP 624594, EP 624595, EP 624596, EP 0864582, EP 1173205, EP 1535913 and EP 3210977. Particular examples of suitable ASBT inhibitors include 1,1-dioxo-3,3-dibutyl-5-phenyl-7-methylthio-8-(*N*-{(*R*)-1'-phenyl-1'-[*N*'-(carboxymethyl)carbamoyl]methyl}carbamoylmethoxy)-2,3,4,5-tetrahydro-1,5-benzothiazepine (elobixibat) and 1,1-dioxo-3,3-dibutyl-5-phenyl-7-methylthio-8-(*N*-{(*R*)-$\alpha$-[*N*-((*S*)-1-carboxypropyl) carbamoyl]-4-hydroxybenzyl}carbamoylmethoxy)-2,3,4,5-tetrahydro-1,2,5-benzothiadiazepine (odevixibat).

In another embodiment, compounds of formula (I), or pharmaceutically acceptable salts thereof, are administered in combination with a bile acid binder (also referred to as a bile acid sequestrant, or a resin), such as colesevelam, cholestyramine or cholestipol. In a preferred embodiment of such a combination, the bile acid binder is formulated for colon release. Examples of such formulations are disclosed in e.g. WO 2017/138877, WO 2017/138878, WO 2019/032026 and WO 2019/032027.

In another embodiment, compounds of formula (I), or pharmaceutically acceptable salts thereof, are administered in combination with a DPP-IV inhibitor, including gliptins such as sitagliptin, vildagliptin, saxagliptin, linagliptin, gemigliptin, anagliptin, teneligliptin, alogliptin, trelagliptin, omarigliptin, evogliptin, gosogliptin and dutogliptin, or a pharmaceutically acceptable salt thereof.

In another embodiment, compounds of formula (I), or pharmaceutically acceptable salts thereof, are administered in combination with an HMG CoA reductase inhibitor, such as fluvastatin, lovastatin, pravastatin, simvastatin, atorvastatin, pitavastatin cerivastatin, mevastatin, rosuvastatin, bervastatin or dalvastatin, or a pharmaceutically acceptable salt thereof.

In another embodiment, compounds of formula (I), or pharmaceutically acceptable salts thereof, are administered in combination with a cholesterol absorption inhibitor such as ezetimibe, or a pharmaceutically acceptable salt thereof.

In another embodiment, compounds of formula (I), or pharmaceutically acceptable salts thereof, are administered in combination with a PPAR alpha agonist, including fibrates such as clofibrate, bezafibrate, ciprofibrate, clinofribrate, clofibride, fenofibrate, gemfibrozil, ronifibrate and simfribrate, or a pharmaceutically acceptable salt thereof.

In another embodiment, compounds of formula (I), or pharmaceutically acceptable salts thereof, are administered in combination with a PPAR gamma agonist, including thiazolidinediones such as pioglitazone, rosiglitazone and lobeglitazone, or a pharmaceutically acceptable salt thereof.

In another embodiment, compounds of formula (I), or pharmaceutically acceptable salts thereof, are administered in combination with a dual PPAR alpha/gamma agonist, including glitazars such as saroglitazar, aleglitazar, muraglitazar or tesaglitazar, or a pharmaceutically acceptable salt thereof.

In another embodiment, compounds of formula (I), or pharmaceutically acceptable salts thereof, are administered in combination with a dual PPAR alpha/delta agonist, such as elafibranor.

In yet another embodiment, compounds of formula (I), or pharmaceutically acceptable salts thereof, are administered in combination with a pan PPAR agonist (i.e. a PPAR agonist that has activity across all subtypes: $\alpha$, $\gamma$ and $\delta$), such as IVA337.

In another embodiment, compounds of formula (I), or pharmaceutically acceptable salts thereof, are administered in combination with a farnesoid X receptor (FXR) modulators, including FXR agonists such as cafestol, chenodeoxycholic acid, 6$\alpha$-ethyl-chenodeoxycholic acid (obeticholic acid; INT-747), fexaramine, tropifexor, cilofexor and MET409.

In another embodiment, compounds of formula (I), or pharmaceutically acceptable salts thereof, are administered in combination with a TGR5 receptor modulator, including TGR5 agonists such as 6$\alpha$-ethyl-23(S)-methylcholic acid (INT-777).

In another embodiment, compounds of formula (I), or pharmaceutically acceptable salts thereof, are administered in combination with a dual FXR/TGR5 agonist such as INT-767.

In another embodiment, compounds of formula (I), or pharmaceutically acceptable salts thereof, are administered in combination with ursodeoxycholic acid (UDCA). In yet another embodiment, compounds of formula (I), or pharmaceutically acceptable salts thereof, are administered in combination with nor-ursodeoxycholic acid (nor-UDCA).

In another embodiment, compounds of formula (I), or pharmaceutically acceptable salts thereof, are administered in combination with an FGF19 modulator, such as NGM282.

In another embodiment, compounds of formula (I), or pharmaceutically acceptable salts thereof, are administered in combination with an FGF21 agonist, such as BMS-986036.

In another embodiment, compounds of formula (I), or pharmaceutically acceptable salts thereof, are administered in combination with an integrin inhibitor, such as PLN-74809 and PLN-1474.

In another embodiment, compounds of formula (I), or pharmaceutically acceptable salts thereof, are administered in combination with a CCR2/CCR5 inhibitor, such as cenicriviroc.

In another embodiment, compounds of formula (I), or pharmaceutically acceptable salts thereof, are administered in combination with a caspase protease inhibitor, such as emricasan.

In another embodiment, compounds of formula (I), or pharmaceutically acceptable salts thereof, are administered in combination with a galectin-3 inhibitor, such as GR-MD-02.

In another embodiment, compounds of formula (I), or pharmaceutically acceptable salts thereof, are administered in combination with a stearoyl-CoA desaturase (SCD) Inhibitor, such as aramchol (arachidyl amido cholanoic acid).

In another embodiment, compounds of formula (I), or pharmaceutically acceptable salts thereof, are administered in combination with an apoptosis signal-regulating kinase 1 (ASK1) inhibitor, such as selonsertib.

In another embodiment, compounds of formula (I), or pharmaceutically acceptable salts thereof, are administered in combination with an LOXL2 inhibitor, such as simtuzumab.

In another embodiment, compounds of formula (I), or pharmaceutically acceptable salts thereof, are administered in combination with an ACC inhibitor, such as GS-0976.

In another embodiment, compounds of formula (I), or pharmaceutically acceptable salts thereof, are administered in combination with a thyroid hormone receptor-β agonist, such as MGL3196.

In another embodiment, compounds of formula (I), or pharmaceutically acceptable salts thereof, are administered in combination with a GLP-1 agonist such as liraglutide.

In another embodiment, compounds of formula (I), or pharmaceutically acceptable salts thereof, are administered in combination with a dual glucagon-like peptide and glucagon receptor agonists, such as SAR425899.

In another embodiment, compounds of formula (I), or pharmaceutically acceptable salts thereof, are administered in combination with a mitochondrial pyruvate carrier inhibitor, such as MSDC-0602K.

In another embodiment, compounds of formula (I), or pharmaceutically acceptable salts thereof, are administered in combination with an anti-oxidant agent, such as vitamin E.

In another embodiment, compounds of formula (I), or pharmaceutically acceptable salts thereof, are administered in combination with an SGLT1 inhibitor, an SGLT2 inhibitor or a dual SGLT1 and SGLT2 inhibitor. Examples of such compounds are dapagliflozin, sotagliflozin, canagliflozin, empagliflozin, LIK066 and SGL5213.

In another embodiment, compounds of formula (I), or pharmaceutically acceptable salts thereof, are administered in combination with a diacylglycerol O-Acyltransferase 2 (DGAT2) inhibitor, such as DGAT2RX and PF-06865571.

In another embodiment, compounds of formula (I), or pharmaceutically acceptable salts thereof, are administered in combination with a fatty acid synthase (FASN) Inhibitor, such as TVB-2640.

In another embodiment, compounds of formula (I), or pharmaceutically acceptable salts thereof, are administered in combination with an AMP-activated protein kinase (AMPK) activator, such as PXL-770.

In another embodiment, compounds of formula (I), or pharmaceutically acceptable salts thereof, are administered in combination with a glucocorticoid receptor antagonist (GR), a mineralocorticoid receptor antagonist (MR), or a dual GR/MR antagonist. Examples of such compounds are MT-3995 and CORT-118335.

In another embodiment, compounds of formula (I), or pharmaceutically acceptable salts thereof, are administered in combination with a cannabinoid receptor 1 (CB1) antagonist, such as IM102.

In another embodiment, compounds of formula (I), or pharmaceutically acceptable salts thereof, are administered in combination with a Klothoβ (KLB) and fibroblast growth factor receptor (FGFR) activator, such as MK-3655 (previously known as NGM-313).

In another embodiment, compounds of formula (I), or pharmaceutically acceptable salts thereof, are administered in combination with a chemokine (c-c motif) ligand 24 (CCL24) inhibitor, such as CM 101.

In another embodiment, compounds of formula (I), or pharmaceutically acceptable salts thereof, are administered in combination with an A3 antagonist, such as PBF-1650.

In another embodiment, compounds of formula (I), or pharmaceutically acceptable salts thereof, are administered in combination with a P2x7 receptor antagonist, such as SGM 1019.

In another embodiment, compounds of formula (I), or pharmaceutically acceptable salts thereof, are administered in combination with P2Y13 receptor agonists, such as CER-209.

In another embodiment, compounds of formula (I), or pharmaceutically acceptable salts thereof, are administered in combination with a sulfated oxysterol, such as Dur-928.

In another embodiment, compounds of formula (I), or pharmaceutically acceptable salts thereof, are administered in combination with a leukotriene D4 (LTD4) receptor antagonist, such as MN-001.

In another embodiment, compounds of formula (I), or pharmaceutically acceptable salts thereof, are administered in combination with a type 1 natural killer T cell (NKT1) inhibitor, such as GRI-0621.

In another embodiment, compounds of formula (I), or pharmaceutically acceptable salts thereof, are administered in combination with an anti-lipopolysaccharide (LPS) compound, such as IMM-124E.

In another embodiment, compounds of formula (I), or pharmaceutically acceptable salts thereof, are administered in combination with a VAP1 inhibitor, such as BI1467335.

In another embodiment, compounds of formula (I), or pharmaceutically acceptable salts thereof, are administered in combination with an A3 adenosine receptor agonist, such as CF-102.

In another embodiment, compounds of formula (I), or pharmaceutically acceptable salts thereof, are administered in combination with a SIRT-1 activator, such as NS-20.

In another embodiment, compounds of formula (I), or pharmaceutically acceptable salts thereof, are administered in combination with a nicotinic acid receptor 1 agonist, such as ARI-3037MO.

In another embodiment, compounds of formula (I), or pharmaceutically acceptable salts thereof, are administered in combination with a TLR4 antagonist, such as JKB-121.

In another embodiment, compounds of formula (I), or pharmaceutically acceptable salts thereof, are administered in combination with a ketohexokinase inhibitor, such as PF-06835919.

In another embodiment, compounds of formula (I), or pharmaceutically acceptable salts thereof, are administered in combination with an adiponectin receptor agonist, such as ADP-335.

In another embodiment, compounds of formula (I), or pharmaceutically acceptable salts thereof, are administered in combination with an autotaxin inhibitor, such as PAT-505 and PF8380.

In another embodiment, compounds of formula (I), or pharmaceutically acceptable salts thereof, are administered in combination with a chemokine (c-c motif) receptor 3 (CCR3) antagonist, such as bertilimumab.

In another embodiment, compounds of formula (I), or pharmaceutically acceptable salts thereof, are administered in combination with a chloride channel stimulator, such as cobiprostone and lubiprostone.

In another embodiment, compounds of formula (I), or pharmaceutically acceptable salts thereof, are administered in combination with a heat shock protein 47 (HSP47) inhibitor, such as ND-L02-s0201.

In another embodiment, compounds of formula (I), or pharmaceutically acceptable salts thereof, are administered in combination with a sterol regulatory element-binding protein (SREBP) transcription factor inhibitor, such as CAT-2003 and MDV-4463.

In another embodiment, compounds of formula (I), or pharmaceutically acceptable salts thereof, are administered in combination with a biguanidine, such as metformin.

In another embodiment, compounds of formula (I), or pharmaceutically acceptable salts thereof, are administered in combination with insulin.

In another embodiment, compounds of formula (I), or pharmaceutically acceptable salts thereof, are administered in combination with a glycogen phosphorylase inhibitor and/or a glucose-6-phosphatase inhibitor.

In another embodiment, compounds of formula (I), or pharmaceutically acceptable salts thereof, are administered in combination with a sulfonylurea, such as glipizid, glibenklamid and glimepirid.

In another embodiment, compounds of formula (I), or pharmaceutically acceptable salts thereof, are administered in combination with a meglitinide, such as repaglinide, nateglinide and ormiglitinide.

In another embodiment, compounds of formula (I), or pharmaceutically acceptable salts thereof, are administered in combination with a glucosidase inhibitor, such as acarbose or miglitol.

In another embodiment, compounds of formula (I), or pharmaceutically acceptable salts thereof, are administered in combination with a squalene synthase inhibitor, such as TAK-475.

In another embodiment, compounds of formula (I), or pharmaceutically acceptable salts thereof, are administered in combination with a PTPB1 inhibitor, such as trodusquemine, ertiprotafib, JTT-551 and claramine.

*Preparation of compounds*

The compounds of the invention can be prepared as a free acid or a pharmaceutically acceptable salt thereof by the processes described below. Throughout the following description of such processes it is understood that, where appropriate, suitable protecting groups will be added to, and subsequently removed from the various reactants and intermediates in a manner that will be readily understood by one skilled in the art of organic synthesis. Conventional procedures for using such protecting groups as well as examples of suitable protecting groups are for example described in Greene's Protective Groups in Organic Synthesis by P.G.M Wutz and T.W. Greene, 4th Edition, John Wiley & Sons, Hoboken, 2006.

*General methods*

All solvents used were of analytical grade. Commercially available anhydrous solvents were routinely used for reactions. Starting materials were available from commercial sources or prepared according to literature procedures. 7-Bromo-3,3-dibutyl-8-methoxy-5-phenyl-2,3,4,5-tetrahydro-1,5-benzothiazepine 1,1-dioxide and 3,3-dibutyl-8-hydroxy-7-(methylthio)-5-phenyl-2,3,4,5-tetrahydro-1,5-benzothiazepine 1,1-dioxide may be prepared as described in WO 02/50051 (method 26). 7-Bromo-3-butyl-3-ethyl-8-methoxy-5-phenyl-2,3,4,5-tetrahydro-1,5-benzothiazepine 1,1-dioxide, 3-butyl-3-ethyl-8-hydroxy-7-(methylthio)-5-phenyl-2,3,4,5-tetrahydro-1,5-benzothiazepine 1,1-dioxide, 7-bromo-3-butyl-3-ethyl-8-methoxy-5-phenyl-2,3,4,5-tetrahydro-1,2,5-benzothiadiazepine 1,1-dioxide, 3-butyl-3-ethyl-8-hydroxy-2-(4-methoxybenzyl)-7-(methylthio)-5-phenyl-2,3,4,5-tetrahydro-1,2,5-benzothiadiazepine 1,1-dioxide and 3-butyl-3-ethyl-8-hydroxy-2-methyl-7-(methylthio)-5-phenyl-2,3,4,5-tetrahydro-1,2,5-benzothiadiazepine 1,1-dioxide may be prepared as described in WO 2019/234077 (Intermediates 22, 23, 26, 128 and 152, respectively). 3,3-Dibutyl-8-hydroxy-2-(4-methoxybenzyl)-7-(methylthio)-5-phenyl-2,3,4,5-tetrahydro-1,2,5-benzothiadiazepine 1,1-dioxide may be prepared as described in WO 03/022286 (method 24). 7-Bromo-3,3-dibutyl-5-(4-fluorophenyl)-8-methoxy-2,3,4,5-tetrahydro-1,5-benzothiazepine 1,1-dioxide, 7-bromo-3-butyl-3-ethyl-5-(4-fluorophenyl)-8-methoxy-2,3,4,5-tetrahydro-1,5-benzothiazepine 1,1-dioxide and 3-butyl-3-ethyl-5-(4-fluorophenyl)-8-hydroxy-7-(methylthio)-2,3,4,5-tetrahydro-1,5-benzothiazepine 1,1-dioxide may be prepared as described in WO 2020/161216 (Intermediates 43, 69 and 70, respectively). 7-Bromo-3-butyl-8-methoxy-2-methyl-5-phenyl-2,3,4,5-tetrahydro-1,2,5-benzothiadiazepine 1,1-dioxide, 3-butyl-8-hydroxy-2-methyl-7-(methylthio)-5-phenyl-2,3,4,5-tetrahydro-1,2,5-benzothiadiazepine 1,1-dioxide and 7-bromo-3-butyl-5-(4-fluorophenyl)-8-methoxy-2-methyl-2,3,4,5-tetrahydro-1,2,5-benzothiadiazepine 1,1-dioxide may be prepared as described in WO 2021/110883 (Intermediates 32, 33 and 45, respectively). Enantiopure (R)-7-bromo-3-butyl-8-methoxy-5-phenyl-2,3,4,5-tetrahydro-1,2,5-benzothiadiazepine 1,1-dioxide may be prepared following the procedures described in WO 2021/110883 (see Intermediates 21 to 26), starting from a single enantiomer of 2-aminohexanoic acid (D-Norleucine). 7-Bromo-3-butyl-8-methoxy-3-methyl-5-phenyl-2,3,4,5-tetrahydro-1,5-benzothiazepine 1,1-dioxide and 3-butyl-8-hydroxy-3-methyl-7-(methylthio)-5-phenyl-2,3,4,5-tetrahydro-1,5-benzothiazepine 1,1-dioxide may be prepared as described in WO 2021/110884 (Intermediates 5 and 6, respectively). Room temperature refers to 20 - 25 °C. Solvent mixture compositions are given as volume percentages or volume ratios.

**LCMS:**

**Instrument name:** Agilent 1290 infinity II.

**Method A:** Mobile phase: A: 0.1% HCOOH in water: ACN (95:5), B: ACN; flow rate: 1.5 mL/min; column: ZORBAX XDB C-18 (50 x 4.6 mm) 3.5 μM.

**Method B:** Mobile phase: A: 10 mM $NH_4HCO_3$ in water, B: ACN; flow rate: 1.2 mL/min; column: XBridge C8 (50 x 4.6 mm), 3.5 μM.

**Method C:** Mobile phase: A: 0.1% HCOOH in water: ACN (95:5), B: ACN; flow rate: 1.5 mL/min; column: ATLANTIS dC18 (50 x 4.6 mm), 5 μM.

**Method D:** Mobile phase: A: 10 mM $NH_4OAc$ in water, B: ACN; flow rate: 1.2 mL/min; column: Zorbax Extend C18 (50 x 4.6mm) 5 μM.

**Method E:** Mobile Phase: A: 0.1% TFA in water: ACN (95:5), B: 0.1% TFA in ACN; flow rate: 1.5 mL/min; Column: XBridge C8 (50 x 4.6 mm), 3.5 μM.

**Method F:** Mobile phase: A: 0.1% TFA in water, B: 0.1% TFA in ACN; flow Rate: 0.8 mL/min; column: ZORBAX ECLIPSE PLUS C18 (50 x 2.1 mm), 1.8 μm.

**Method G:** Mobile phase: A: 0.1% TFA in water, B: 0.1% TFA in ACN; flow rate: 0.8 mL/min; column: Acquity UPLC BEH C18 (2.1 x 50 mm), 1.7 μm.

**Method H:** Mobile phase: A: 10 mM $NH_4OAc$, B: 100% ACN; flow rate: 0.8 mL/min; Column: Acquity UPLC BEH C18 (2.1 x 50) mm; 1.7 μm.

**Method I:** Mobile phase: A: 0.1% HCOOH in water: ACN (95:5), B: ACN; flow rate: 0.8 mL/min; Column: ZORBAX ECLIPSE PLUS C18 (2.1 x 50) mm, 1.8 μm.

**Method J:** Mobile phase: A: 0.1% TFA in water, B: 0.1% TFA in ACN; Flow Rate: 1.5 mL/min; Column: XBridge C8 (50 x 4.6 mm), 3.5 μM.

**Instrument Name :** Waters Acquity UPLC I Class / SQ detector 2

**Method K:** Mobile phase: A: 0.1% HCOOH in water: ACN (95:5), B: ACN; Flow Rate: 0.8 mL/min; Column: BEH C18 (50 x 2.1 mm), 1.7 μm.

**UPLC:**

**Instrument name:** Waters Acquity I Class

**Method A:** Mobile phase: A: 0.1% HCOOH in water, B: 0.1% HCOOH in ACN; Flow Rate: 0.8 mL/min; Column: Acquity UPLC HSS T3 (2.1 x 50) mm; 1.8 μm.

**HPLC:**

**Instrument name:** Agilent 1260 Infinity II series instruments as followed using % with UV detection (maxplot).

**Method A:** Mobile phase: A: 10 mM $NH_4HCO_3$ in water, B: ACN; flow rate: 1.0 mL/min; column: XBridge C8 (50 x 4.6 mm, 3.5 μm).

**Method B:** Mobile phase: A: 0.1% TFA in water, B: 0.1% TFA in ACN; flow rate: 2.0 mL/min; column: XBridge C8 (50 x 4.6 mm, 3.5 μm).

**Method C:** Mobile phase: A: 10 mM $NH_4OAc$ in milli-q water, B: ACN; flow rate: 1.0 ml/min; column: Phenomenex Gemini C18 (150 x 4.6 mm, 3.0 μm).

**Method D:** Mobile phase: A: 0.1% TFA in water, B: ACN; flow rate: 1.0 mL/min; column: ATLANTIS dC18 (250 x 4.6 mm, 5.0 μm).

**Method E:** Mobile phase: A: 0.1% TFA in water, B: ACN, flow rate: 2.0 mL/ min; column: X-Bridge C8 (50 X 4.6 mm, 3.5 μm).

**Chiral SFC:**

**Instrument name:** PIC SFC 10 (analytical)

Ratio between $CO_2$ and co-solvent is ranging between 60:40 and 80:20

**Method A:** Mobile phase: 0.5% isopropylamine in IPA; flow rate: 3 mL/min; column: YMC Amylose-SA (250 x 4.6 mm, 5 μm).

**Method B:** Mobile phase: 0.5% isopropylamine in IPA; flow rate: 3 mL/min; column: Chiralpak AD-H (250 x 4.6 mm, 5 μm).

**Method C:** Mobile phase: 20 mM ammonia in methanol; flow rate: 3 mL/min; column: YMC Cellulose-SC (250 x 4.6 mm, 5 μm).

**Method D:** Mobile phase: methanol; flow rate: 3 mL/min; column: Lux A1 (250 x 4.6 mm, 5 μm).

**Method E:** Mobile phase: 0.5% isopropylamine in methanol; flow rate: 5 mL/min; column: Lux C4.

**Method F:** Mobile phase: 0.5% isopropylamine in methanol; flow rate: 3 mL/min; column: YMC Cellulose-SC.

**Method G:** Mobile phase: 0.5% isopropylamine in methanol; flow rate: 3 mL/min; column: Lux A1.

**Method H:** Mobile phase: 0.5% isopropylamine in IPA; flow rate: 3 mL/min; column: Lux A1 (250 x 4.6 mm, 5 μm).

**Method I:** Mobile phase: 0.5% isopropylamine in methanol; flow rate: 3 mL/min; column: Chiral CCS (250 x 4.6 mm, 5 μm).

**Method J:** Mobile phase: 0.5% isopropylamine in IPA; flow rate: 5 mL/min; column: YMC Cellulose-SC AD-H (250 x 4.6 mm, 5 μm).

**Method** K: Mobile phase: 0.5% Isopropylamine in methanol; flow rate: 4 mL/min; column: (R,R)-Whelk-01 (250 x 4.6 mm, 5 μm).

**Method L:** Mobile phase: 0.5% Isopropylamine in IPA; flow rate: 3 mL/min; column: Chiralcel OX-H (250 x 4.6 mm, 5 μm).

**Method M:** Mobile phase: 0.5% Isopropylamine in IPA; flow rate: 5 mL/min; column: YMC Cellulose-SC (250 x 4.6 mm, 5 μm).

**Method N:** Mobile phase: methanol, flow rate: 5 mL/min; column: Chiralcel OX-H (250 x 4.6 mm, 5 μm).

**Method O:** Mobile phase: 0.1% Isopropylamine in IPA:methanol (1:1), flow rate: 3 mL/min; column: Chiralpak AS-H (250 x 4.6 mm, 5 μm).

**Method P:** Mobile phase: 0.5% Isopropylamine in methanol, flow rate: 3 mL/min; column: Chiralpak AS-H (250 x 4.6 mm, 5 μm).

**Method Q:** Mobile phase: IPA, flow rate: 3 mL/min; column: Lux A1 (250 x 4.6 mm, 5 μm).

**Method R:** Mobile phase: 0.1% Isopropylamine in IPA:methanol (1:1), flow rate: 3 mL/min; column: Lux A1 (250 x 4.6 mm, 5 μm).

**Method S:** Mobile phase: 0.5 % Isopropylamine in IPA (1:1), flow rate: 3 mL/min; column: Chiralpak AS-H (250 x 4.6 mm, 5 μm).

**Method T:** Mobile phase: 0.5 % Isopropylamine in IPA (1:1), flow rate: 4 mL/min; column: Chiralpak OX-H (250 x 4.6 mm, 5 μm).

**Method U:** Mobile phase: IPA, flow rate: 3 mL/min; column: Chiralpak AS-H (250 x 4.6 mm, 5 μm). **Method V:** Mobile phase: 0.5% Isopropylamine in methanol, flow rate: 3 mL/min; column: Chiralpak OX-H (250 x 4.6 mm, 5 μm).

**Prep-HPLC:**

**Instrument name:** Agilent 1290 Infinity II
**Method A:** Mobile phase: A: 0.1% TFA in water; Mobile phase; B: 0.1% TFA in ACN; flow rate: 2.0 mL/min; Column: X-Bridge C8 (50 X 4.6 mm, 3.5 μM).
**Method B:** Mobile phase: A: 10 mM $NH_4OAc$ in water; B: ACN; flow rate: 35 mL/min; column: X select C18 (30 x 150 mm, 5 μm).
**Method C:** Mobile phase: A: 10 mM $NH_4HCO_3$ in water; B: ACN; flow rate: 1.0 mL/min; column: XBridge C8 (50 x 4.6 mm, 3.5 μm).

**Method D:** Mobile phase: A: 0.1% HCOOH in water; B: ACN; flow rate: 1.0 mL/min; column: X-select C18 (30 x 150 mm, 5 μm).
**Method E:** Mobile phase: A: 0.1% TFA in water, B: ACN; flow rate: 15 mL/min; column: sunfire C18 (19 x 150 mm, 5μm).

**Chiral Preparative SFC:**

**Instrument name:** PIC SFC 100 and PSC SFC 400
Ratio between $CO_2$ and co-solvent is ranging between 60:40 and 80:20
**Method A:** Mobile phase: 0.5% isopropylamine in IPA; flow rate: 3 mL/min; column: YMC Amylose-SA (250 x 30 mm, 5 μm).
**Method B:** Mobile Phase: 0.5% isopropylamine in IPA; flow rate: 3 mL/min; column: Chiralpak AD-H (250 x 30 mm, 5 μm).
**Method C:** Mobile phase: 20 mM ammonia in methanol; flow rate: 3 mL/min; column: YMC Cellulose-SC (250 x 30 mm, 5 μm).
**Method D:** Mobile phase: methanol; flow rate: 3 mL/min; column: Chiral CCS (250 x 30 mm, 5 μm).
**Method E:** Mobile phase: methanol; flow rate: 3 mL/min; column: Lux A1 (250 x 30 mm, 5 μm).
**Method F:** Mobile Phase: 0.5% isopropylamine in IPA; flow rate: 3 mL/min; column: Lux A1 (250 x 30 mm, 5 μm).
**Method G:** Mobile phase: 0.5% isopropylamine in methanol; flow rate: 3 mL/min; column: Chiral CCS (250 x 30 mm, 5 μm).
**Method H:** Mobile phase: 0.5% Isopropylamine in methanol; flow rate: 4 mL/min; column: (R,R)-Whelk-01 (250 x 30 mm, 5 μm).
**Method I:** Mobile phase: 0.5% Isopropylamine in IPA; flow rate: 5 mL/min; column: YMC Cellulose-SC (250 x 30 mm, 5 μm).
**Method J:** Mobile phase: 0.5% Isopropylamine in IPA; flow rate: 3 mL/min; column: Chiralcel OX-H (250 x 30 mm, 5 μm).
**Method K:** Mobile phase: 0.5% Isopropylamine in methanol; flow rate: 5 mL/min; column: YMC Cellulose-SC (250 x 30 mm, 5 μm).
**Method L:** Mobile phase: Methanol; flow rate: 5 mL/min; column: Chiralcel OX-H (250 x 30 mm, 5 μm).
**Method M:** Mobile phase: 0.5% Isopropylamine in methanol, flow rate: 3 mL/min; column: Chiralpak AS-H (250 x 4.6 mm, 5 μm).
**Method N:** Mobile Phase: 0.1% Isopropylamine in IPA:MeOH (1:1), flow rate: 3 mL/min; column: Chiralpak AS-H (250 x 4.6 mm, 5 μm).

*Abbreviations*

| | |
|---|---|
| ACN | acetonitrile |
| DCM | dichloromethane |
| DMF | dimethylformamide |
| dppf | 1,1'-bis(diphenylphosphino)ferrocene |
| HPLC | high-performance liquid chromatography |
| IPA | isopropyl alcohol |
| LCMS | liquid chromatography - mass spectrometry |
| NMP | *N*-methyl-2-pyrrolidone |
| PE | petroleum ether |
| $Pd_2(dba)_3$ | tris(dibenzylideneacetone)dipalladium(0) |
| PMB | para-methoxybenzyl |
| RT | room temperature |
| RuPhos | 2-Dicyclohexylphosphino-2',6'-diisopropoxybiphenyl |
| SFC | supercritical fluid chromatography |
| TFA | trifluoroacetic acid |
| THF | tetrahydrofuran |
| TLC | thin layer chromatography |
| UPLC | ultra performance liquid chromatography |

The invention will now be described by the following examples which do not limit the invention in any respect.

EXAMPLES

**Intermediate 1**

**3-Butyl-3-ethyl-7-(methylthio)-1,1-dioxido-5-phenyl-2,3,4,5-tetrahydro-1,5-benzothiazepin-8-yl trifluoromethanesulfonate**

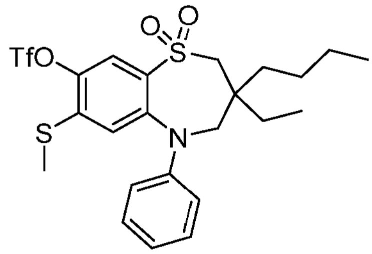

To a stirred solution of 3-butyl-3-ethyl-8-hydroxy-7-(methylthio)-5-phenyl-2,3,4,5-tetrahydro-1,5-benzothiazepine 1,1-dioxide (500 mg, 1.19 mmol) in dry DCM (10 mL) at 0 °C, pyridine (0.2 mL, 2.38 mmol) and then trifluoromethanesulfonic anhydride (0.3 mL, 1.78 mmol) were added dropwise and the reaction mixture was stirred for 1 hour at RT. After completion of the reaction (monitored by TLC), the reaction mixture was diluted with water (10 mL) and the aqueous layer was extracted with DCM (2 x 20 mL). The combined organic layer was washed with water (5 mL) and brine (5 mL) and then dried over anhydrous $Na_2SO_4$. The organic part was filtered, concentrated under vacuum and the resulting crude material was purified by Isolera column chromatography (eluent: 9-10% EtOAc/PE; silica gel: 230-400 mesh) to afford the title compound. **Yield:** 46% (480 mg, white solid). **$^1$H NMR** (400 MHz, DMSO-$d_6$): δ 7.70 (s, 1H), 7.40 (t, *J* = 9.6 Hz, 4H), 7.17 (t, *J* = 9.2 Hz, 1H), 6.53 (s, 1H), 3.88-3.80 (m, 2H), 3.53-3.48 (m, 2H), 2.18 (s, 3H), 1.58-1.51 (m, 1H), 1.45-1.35 (m, 3H), 0.89-0.83 (m, 4H), 0.69-0.79 (m, 6H). **LCMS:** (Method B) 552.2 ($M^+$+H), Rt. 3.21 min, 98.21%(Max).

**Intermediate 2**

**Methyl 3-butyl-3-ethyl-7-(methylthio)-5-phenyl-2,3,4,5-tetrahydro-1,5-benzothiazepine-8-carboxylate 1,1-dioxide**

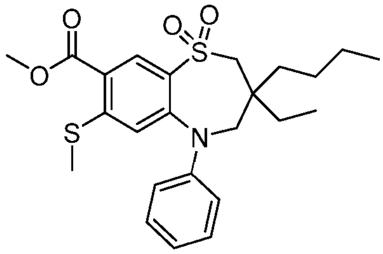

To a stirred solution of 3-butyl-3-ethyl-7-(methylthio)-1,1-dioxido-5-phenyl-2,3,4,5-tetrahydro-1,5-benzothiazepin-8-yl trifluoromethanesulfonate (Intermediate 1; 500 mg, 0.90 mmol) in degassed methanol (10 mL) and DMF (5 mL), triethylamine (0.18 mL, 1.35 mmol), dppf (126 mg, 0.20 mmol) and $Pd_2(dba)_3$ (104 mg, 0.10 mmol) were added at RT. The resulting reaction mixture was stirred for 16 hours at 70 °C in an autoclave, under 5 kg carbon monoxide gas pressure. After completion of the reaction (monitored by TLC), the reaction mixture was diluted with water (20 mL), and the aqueous layer was extracted with EtOAc (2 x 50 mL). The combined organic layer was washed with water (15 mL) and brine (15 mL) and then dried over anhydrous $Na_2SO_4$. The organic part was filtered, concentrated under vacuum and the resulting crude material was purified by Isolera column chromatography (eluent: 10-15% EtOAc/PE; silica gel: 230-400 mesh) to afford the title compound. **Yield:** 52% (215 mg, off-white solid).

**$^1$H NMR** (400 MHz, DMSO-$d_6$): δ 8.41 (s, 1H), 7.51-7.45 (m, 4H), 7.30-7.26 (m, 1H), 6.28 (s, 1H), 3.98-3.95 (m, 2H), 3.82 (s, 3H), 3.56-3.53 (m, 2H), 1.89 (s, 3H), 1.59-1.56 (m, 1H), 1.43-1.31 (m, 3H), 1.24-1.04 (m, 2H), 0.94-0.87 (m,

2H), 0.72-0.65 (m, 6H). **LCMS:** (Method A) 462.9 ($M^{+}$+H), Rt. 3.23 min, 77.43% (Max).

**Intermediate 3**

**3-Butyl-3-ethyl-8-(hydroxymethyl)-7-(methylthio)-5-phenyl-2,3,4,5-tetrahydro-1,5-benzothiazepine 1,1-dioxide**

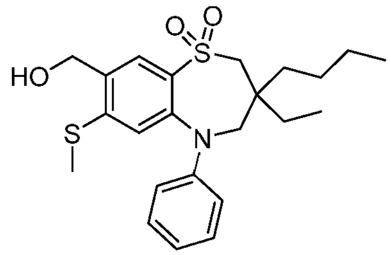

To a stirred solution of methyl 3-butyl-3-ethyl-7-(methylthio)-5-phenyl-2,3,4,5-tetrahydro-1,5-benzothiazepine-8-carboxylate 1,1-dioxide (Intermediate 2; 200 mg, 0.43 mmol) in dry THF (10 mL) at 0 °C, lithium aluminium hydride (2M in THF, 0.4 mL, 0.86 mmol) was added dropwise and the reaction mixture was stirred for 1 hour at RT. After completion of the reaction (monitored by TLC), the reaction mixture was quenched with saturated aqueous ammonium chloride solution (5 mL) and the aqueous layer was extracted with EtOAc (2 x 15 mL). The combined organic layer was washed with water (10 mL), brine (10 mL) and dried over anhydrous $Na_2SO_4$. The organic part was filtered, concentrated under vacuum and the resulting crude was forwarded to the next step without any further purification. **Yield:** 83% (210 mg, white solid).

**$^1$H NMR** (400 MHz, DMSO-*d*$_6$): δ 7.92 (s, 1H), 7.32-7.30 (m, 3H), 7.19-7.16 (m, 2H), 7.20-6.99 (m, 1H), 6.61 (s, 1H), 5.47 (s, 2H), 4.45 (s, 2H), 3.76 (s, 2H), 2.16 (s, 3H), 1.54-1.36 (m, 4H), 1.17-1.06 (m, 4H), 0.73-0.65 (m, 6H).

**Intermediate 4**

**Ethyl-2-(((3-butyl-3-ethyl-7-(methylthio)-1,1-dioxido-5-phenyl-2,3,4,5-tetrahydro-1,5-benzothiazepin-8-yl) methyl)thio)acetate**

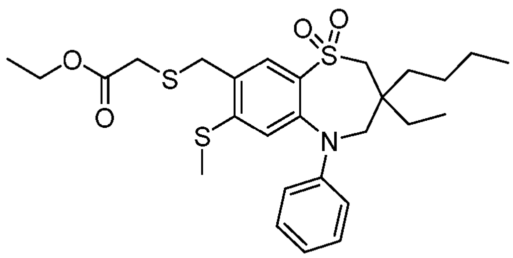

To a stirred solution of 3-butyl-3-ethyl-8-(hydroxymethyl)-7-(methylthio)-5-phenyl-2,3,4,5-tetrahydro-1,5-benzothiazepine 1,1-dioxide (Intermediate 3; 50 mg, 0.11 mmol) in DCM (10 mL) at 0 °C, zinc iodide (17.3 mg, 0.05 mmol) and then ethyl 2-mercaptoacetate (34 mg, 0.28 mmol) were added and the reaction mixture was stirred for 1 hour at RT. After completion of the reaction (monitored by TLC), the reaction mixture was quenched with water (10 mL) and the aqueous layer was extracted with DCM (2 x 10 mL). The combined organic layer was washed with water (10 mL), brine (10 mL) and dried over anhydrous $Na_2SO_4$. The organic part was filtered, concentrated under vacuum and the resulting crude material was forwarded to the next step without any further purification. **Yield:** 70 mg (crude, light yellow solid).

**$^1$H NMR** (400 MHz, DMSO-*d*$_6$): δ 7.74 (s, 1H), 7.33 (t, *J* = 8.4 Hz, 2H), 7.22 (d, *J* = 7.6 Hz, 2H), 7.04 (t, *J* = 7.6 Hz, 1H), 6.58 (s, 1H), 4.14-4.07 (m, 2H), 3.88 (s, 2H), 3.77-3.73 (m, 2H), 3.35-3.34 (m, 2H), 3.31-3.30 (m, 2H), 2.15 (s, 3H), 1.55-1.50 (m, 1H), 1.49-.39 (m, 3H), 1.38-1.34 (m, 3H), 1.27-1.18 (m, 4H), 0.75-0.70 (m, 6H).

**Intermediate 5**

**3,3-dibutyl-7-(methylthio)-1,1-dioxido-5-phenyl-2,3,4,5-tetrahydro-1,5-benzothiazepin-8-yl trifluoromethanesulfonate**

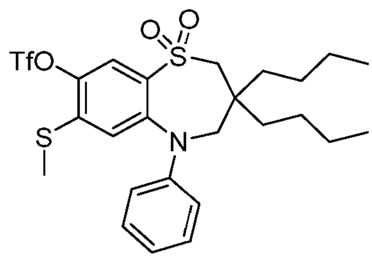

To a stirred solution of 3,3-dibutyl-8-hydroxy-7-(methylthio)-5-phenyl-2,3,4,5-tetrahydro-1,5-benzothiazepine 1,1-dioxide (1.0 g, 2.23 mmol) in dry DCM (10 mL) at 0 °C, pyridine (0.35 mL, 4.46 mmol) and then trifluoromethanesulfonic anhydride (0.56 mL, 3.35 mmol) were added dropwise and the reaction mixture was stirred 1 hour at RT. After completion of the reaction (monitored by TLC), the reaction mixture was quenched with ice-cold water (20 mL) and the aqueous layer was extracted with DCM (2 x 50 mL). The combined organic layer was washed with water (10 mL), brine (10 mL) and then dried over anhydrous $Na_2SO_4$. The organic part was filtered, concentrated under vacuum and the resulting crude material was purified by Isolera column chromatography (eluent: 20% EtOAc/PE; silica gel: 230-400 mesh). The obtained solid was triturated with petroleum ether (2 x 10 mL) and dried under vacuum to afford the title compound. **Yield:** 85% (1.1 g, off-white solid).

**$^1$H NMR** (400 MHz, DMSO-$d_6$): δ 7.70 (s, 1H), 7.43-7.30 (m, 4H), 7.18 (t, $J$ = 6.8 Hz, 1H), 6.53 (s, 1H), 3.90-3.88 (m, 2H), 3.54 (s, 2H), 2.18 (s, 3H), 1.65-1.20 (m, 4H), 1.20-0.90 (m, 8H), 0.80-0.62 (m, 6H). **LCMS:** (Method E) 579.9 ($M^+$ +H), Rt. 3.52 min, 96.63% (Max).

**Intermediate 6**

**Methyl 3,3-dibutyl-7-(methylthio)-5-phenyl-2,3,4,5-tetrahydro-1,5-benzothiazepine-8-carboxylate 1,1-dioxide**

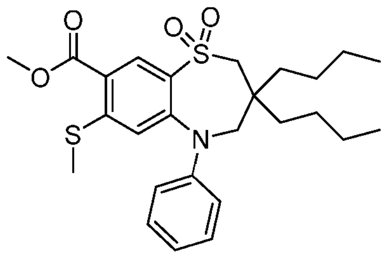

To a solution of 3,3-dibutyl-7-(methylthio)-1,1-dioxido-5-phenyl-2,3,4,5-tetrahydro-1,5-benzothiazepin-8-yl trifluoromethanesulfonate (Intermediate 5; 1.10 g, 1.80 mmol) in degassed methanol (10 mL) and DMF (5 mL), triethylamine (0.4 mL, 2.80 mmol), dppf (126 mg, 0.20 mmol) and $Pd_2(dba)_3$ (104 mg, 0.10 mmol) were added at RT. The resulting reaction mixture was stirred for 16 hours at 70 °C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL) and then dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 67% (0.62 g, off-white solid).

**$^1$H NMR** (400 MHz, DMSO-$d_6$): δ 8.41 (s, 1H), 7.51-7.45 (m, 4H), 7.27 (s, 1H), 6.29 (s, 1H), 4.05-3.90 (m, 2H), 3.82 (s, 3H), 3.62-3.48 (m, 2H), 1.89 (s, 3H), 1.60-1.20 (m, 4H), 1.20-0.80 (m, 8H), 0.78-0.55 (m, 6H). **LCMS:** (Method E) 489.9 ($M^+$+H), Rt. 3.37 min, 94.23% (Max).

**Intermediate 7**

**3,3-dibutyl-8-(hydroxymethyl)-7-(methylthio)-5-phenyl-2,3,4,5-tetrahydro-1,5-benzothiazepine 1,1-dioxide**

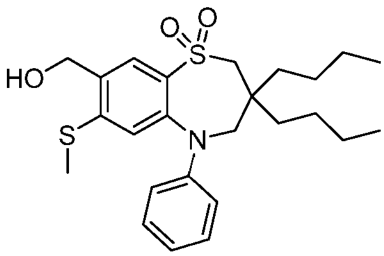

To a stirred solution of methyl 3,3-dibutyl-7-(methylthio)-5-phenyl-2,3,4,5-tetrahydro-1,5-benzothiazepine-8-carboxylate 1,1-dioxide (Intermediate 6; 0.3 g, 0.6 mmol) in dry THF (6 mL) at 0 °C, lithium aluminium hydride (1M in THF, 0.9 mL, 0.9 mmol) was added dropwise and the reaction mixture was stirred for 1 hour at RT. After completion of the reaction (monitored by TLC), the reaction mixture was quenched with saturated aqueous ammonium chloride solution (5 mL) and the aqueous layer was extracted with EtOAc (2 x 15 mL). The combined organic layer was washed with water (10 mL), brine (10 mL) and then dried over anhydrous $Na_2SO_4$. The organic part was filtered, concentrated under vacuum and the resulting crude was forwarded to the next step without any purification. **Yield:** 300 mg (crude, off-white solid).

**$^1$H NMR** (400 MHz, DMSO-$d_6$): δ 7.91 (s, 1H), 7.32-7.28 (m, 2H), 7.19-7.17 (m, 2H), 7.02-6.99 (m, 1H), 6.59 (s, 1H), 5.47 (t, *J* = 5.6 Hz, 1H), 4.43 (d, *J* = 5.2 Hz, 2H), 3.90-3.65 (m, 2H), 3.40-3.30 (m, 2H), 2.15 (s, 3H), 1.50-1.40 (m, 2H), 1.40-1.30 (m, 2H), 1.20-0.98 (m, 8H), 0.80-0.70 (m, 6H). **LCMS:** (Method A) 462.1 ($M^+$+H), Rt. 2.94 min, 97.01% (Max).

**Intermediate 8**

**Ethyl 2-(((3,3-dibutyl-7-(methylthio)-1,1-dioxido-5-phenyl-2,3,4,5-tetrahydro-1,5-benzothiazepin-8-yl)methyl) thio)acetate**

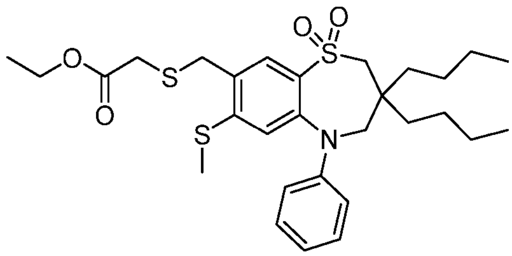

To a stirred solution of 3,3-dibutyl-8-(hydroxymethyl)-7-(methylthio)-5-phenyl-2,3,4,5-tetrahydro-1,5-benzothiazepine 1,1-dioxide (Intermediate 7; 0.08 g, 0.17 mmol) in DCM (3 mL) at 0 °C, zinc iodide (0.03 g, 0.09 mmol) and then ethyl 2-mercaptoacetate (0.04 g, 0.35 mmol) were added and the reaction mixture was stirred for 1 hour at RT. After completion of the reaction (monitored by TLC), the reaction mixture was diluted with water (10 mL) and the aqueous layer was extracted with DCM (2 x 20 mL). The combined organic layer was washed with water (5 mL) and with brine (5 mL) and then dried over anhydrous $Na_2SO_4$. The organic part was filtered, concentrated under vacuum and the resulting material was forwarded to the next step without any further purification. **Yield:** 80% (90 mg, brown solid).

**LCMS:** (Method E) 581.0 ($M^+$+18), Rt. 3.44 min, 80.16% (Max).

**Intermediate 9**

**3-Butyl-3-methyl-7-(methylthio)-1,1-dioxido-5-phenyl-2,3,4,5-tetrahydro-1,5-benzothiazepin-8-yl trifluoro-methanesulfonate**

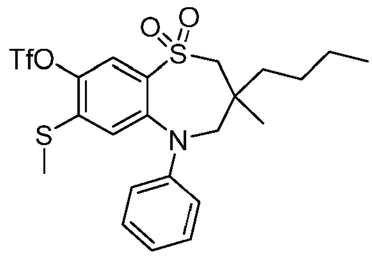

To a stirred solution of 3-butyl-8-hydroxy-3-methyl-7-(methylthio)-5-phenyl-2,3,4,5-tetrahydro-1,5-benzothiazepine 1,1-dioxide (1.0 g, 2.46 mmol) in dry DCM (10 mL) at 0 °C, pyridine (0.24 g, 4.93 mmol) was added dropwise. Then trifluoromethanesulfonic anhydride (0.65 g, 3.69 mmol) was added dropwise at 0 °C and the reaction mixture was stirred for 1 hour at RT. After completion of the reaction (monitored by TLC), the reaction mixture was quenched with ice-cold water (20 mL). The aqueous layer was extracted with DCM (2 x 50 mL) and the combined organic layer was washed with water (10 mL) and brine (10 mL). The organic layer was dried over anhydrous $Na_2SO_4$, filtered and concentrated under vacuum. The resulting crude material was purified by Isolera column chromatography (eluent: 20% EtOAc/PE; silica gel: 230-400 mesh) to afford a sticky solid. This sticky solid was further purified by trituration with petroleum ether (2 x 10 mL) and dried to afford the title compound. **Yield:** 68% (0.9 g, off-white solid).

**LCMS:** (Method E) 538.0 ($M^+$+H), Rt. 3.30 min, 97.12% (Max).

**Intermediate 10**

**Methyl 3-butyl-3-methyl-7-(methylthio)-5-phenyl-2,3,4,5-tetrahydro-1,5-benzothiazepine-8-carboxylate 1,1-dioxide**

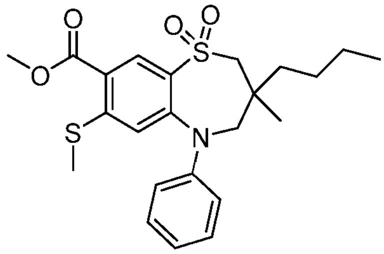

To a solution of 3-butyl-3-methyl-7-(methylthio)-1,1-dioxido-5-phenyl-2,3,4,5-tetrahydro-1,5-benzothiazepin-8-yl trifluoromethanesulfonate (Intermediate 9; 0.9 g, 1.67 mmol) in degassed methanol (15 mL) and DMF (10 mL), triethylamine (0.32 mL, 2.51 mmol), dppf (0.10 g, 2.00 mmol) and $Pd_2(dba)_3$ (0.09 g, 1.01 mmol) were added at RT. The resulting reaction mixture was stirred for 16 hours at 75 °C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL) and brine (15 mL) and then dried over anhydrous $Na_2SO_4$. The organic layer was filtered, concentrated under vacuum and the resulting crude material was purified by Isolera column chromatography (eluent: 15% EtOAc/PE; silica gel: 230-400 mesh) to afford the title compound. **Yield:** 67.5% (0.5 g, off-white solid).

**LCMS:** (Method E) 448.3 ($M^+$+H), Rt. 2.93 min, 96.13% (Max).

**Intermediate 11**

**3-Butyl-8-(hydroxymethyl)-3-methyl-7-(methylthio)-5-phenyl-2,3,4,5-tetrahydro-1,5-benzothiazepine 1,1-dioxide**

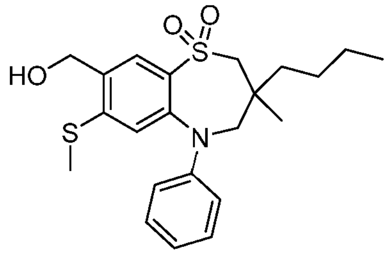

To a stirred solution of methyl 3-butyl-3-methyl-7-(methylthio)-5-phenyl-2,3,4,5-tetrahydro-1,5-benzothiazepine-8-carboxylate 1,1-dioxide (Intermediate 10; 0.25 g, 0.56 mmol) in dry THF (10 mL) at 0 °C, lithium aluminium hydride (2M solution in THF, 0.27 mL, 0.56 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 15 mL). The combined organic layer was washed with water (10 mL), brine (10 mL) and dried over anhydrous $Na_2SO_4$. The organic part was filtered, concentrated under vacuum to afford the title compound which was forwarded to the next step without any further purification. **Yield:** 92% (0.22 g, off-white solid).

**LCMS:** (Method A) 419.9 ($M^+$+H), Rt. 2.72 min, 98.23% (Max).

**Intermediate 12**

**Ethyl 2-(((3-butyl-3-methyl-7-(methylthio)-1,1-dioxido-5-phenyl-2,3,4,5-tetrahydro-1,5-benzothiazepin-8-yl) methyl)thio)acetate**

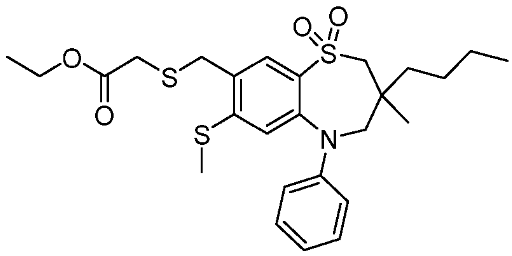

To a stirred solution of 3-butyl-8-(hydroxymethyl)-3-methyl-7-(methylthio)-5-phenyl-2,3,4,5-tetrahydro-1,5-benzothiazepine 1,1-dioxide (Intermediate 11; 0.15 g, 0.36 mmol) in DCM (5 mL) at 0 °C, zinc iodide (0.06 g, 0.18 mmol) and ethyl 2-mercaptoacetate (0.08 g, 0.72 mmol) were added and the reaction mixture was stirred for 2 hours at RT. After completion of the reaction (monitored by TLC), the reaction mixture was diluted with water (10 mL) and the aqueous layer was extracted with DCM (2 x 20 mL). The combined organic layer was washed with water (5 mL) and with brine (5 mL) and then dried over anhydrous $Na_2SO_4$. The organic part was concentrated under vacuum and the resulting crude material was purified by Isolera column chromatography (eluent: 20% EtOAc/PE; silica gel: 230-400 mesh) to afford the title compound. **Yield:** 75.26%, (0.14 g, white solid).

**LCMS:** (Method B) 539.0 ($M^+$+18), Rt. 2.86 min, 97.00% (Max).

**Intermediate 13**

**3-Butyl-3-ethyl-5-(4-fluorophenyl)-7-(methylthio)-1,1-dioxido-2,3,4,5-tetrahydro-1,5-benzothiazepin-8-yl trifluoromethanesulfonate**

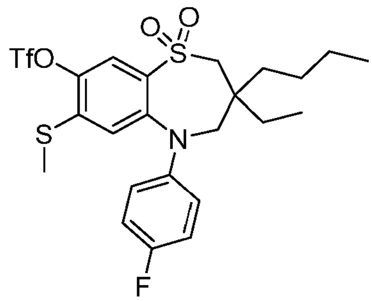

To a stirred solution of 3-butyl-3-ethyl-5-(4-fluorophenyl)-8-hydroxy-7-(methylthio)-2,3,4,5-tetrahydro-1,5-benzothiazepine 1,1-dioxide (3 g, 6.85 mmol) in DCM (30 mL) at 0 °C, pyridine (1.08 mL, 13.71 mmol) and then trifluoromethanesulfonic anhydride (1.36 mL, 8.22 mmol) were added dropwise and the reaction mixture was stirred for 1 hour at RT. After completion of the reaction (monitored by TLC), the reaction mixture was diluted with water (20 mL) and the aqueous layer was extracted with DCM (2 x 50 mL). The combined organic layer was washed with water (20 mL), brine (20 mL) and then dried over anhydrous $Na_2SO_4$. The organic part was filtered, concentrated under vacuum and the resulting crude material was purified by Isolera column chromatography (eluent: 10-20% EtOAc/PE; silica gel: 230-400 mesh) to afford the title compound. **Yield:** 89.7% (3.5 g, white solid).

**Intermediate 14**

**Methyl 3-butyl-3-ethyl-5-(4-fluorophenyl)-7-(methylthio)-2,3,4,5-tetrahydro-1,5-benzothiazepine-8-carboxylate 1,1-dioxide**

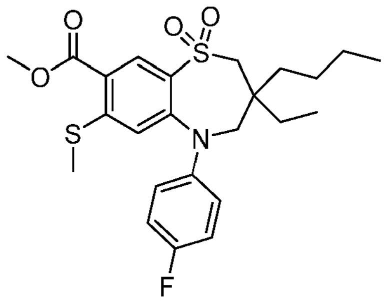

To a solution of 3-butyl-3-ethyl-5-(4-fluorophenyl)-7-(methylthio)-1,1-dioxido-2,3,4,5-tetrahydro-1,5-benzothiazepin-8-yl trifluoromethanesulfonate (Intermediate 13; 3.5 g, 6.15 mmol) in degassed methanol (35 mL) and DMF (20 mL), triethylamine (1.28 mL, 9.22 mmol), dppf (0.41 g, 0.73 mmol) and $Pd_2(dba)_3$ (0.34 g, 0.37 mmol) were added at RT. The resulting reaction mixture was stirred for 16 hours at 75 °C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL) and then dried over anhydrous $Na_2SO_4$. The organic layer was filtered, concentrated under vacuum and the resulting crude material was purified by Isolera column chromatography (eluent: 20 % EtOAc/PE; silica gel: 230-400 mesh) to afford the title compound. **Yield:** 16% (0.45 g, off-white solid).

**$^1$H NMR** (400 MHz, DMSO-*d*$_6$): δ 8.40 (s, 1H), 7.60-7.57 (m, 2H), 7.32 (t, J = 8.80 Hz, 2H), 6.21 (s, 1H), 3.95-3.92 (m, 2H), 3.81 (s, 3H), 3.58 (s, 2H), 1.93 (s, 3H), 1.58-1.50 (m, 1H), 1.40-1.32 (m, 3H), 1.19-1.16 (m, 2H), 1.09-1.08 (m, 1H), 0.86-0.84 (m, 1H), 0.73-0.64 (m, 6H). **LCMS:** (Method E) 480.0 ($M^+$+H), Rt. 3.17 min, 91.42% (Max).

**Intermediate 15**

**3-Butyl-3-ethyl-5-(4-fluorophenyl)-8-(hydroxymethyl)-7-(methylthio)-2,3,4,5-tetrahydro-1,5-benzothiazepine 1,1-dioxide**

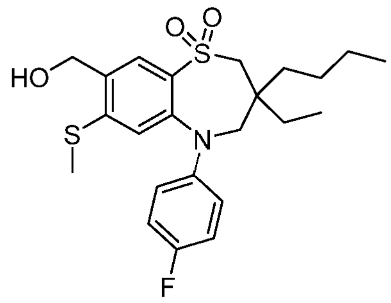

To a stirred solution of methyl 3-butyl-3-ethyl-5-(4-fluorophenyl)-7-(methylthio)-2,3,4,5-tetrahydro-1,5-benzothiazepine-8-carboxylate 1,1-dioxide (Intermediate 14; 1.7 g, 3.54 mmol) in THF (20 mL) at 0 °C, lithium aluminium hydride (2M solution in THF, 2.6 mL, 5.32 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 25 mL). The combined organic layer was washed with water (15 mL), brine (15 mL) and dried over anhydrous $Na_2SO_4$. The organic part was filtered, concentrated under vacuum and the resulting crude was forwarded to the next step without any further purification. **Yield:** 1.7 g (crude, off-white solid).

**$^1$H NMR** (400 MHz, DMSO-$d_6$): δ 7.89 (s, 1H), 7.14-7.24 (m, 5H), 6.51 (s, 1H), 5.45-5.48 (m, 2H), 4.43-4.42 (m, 2H), 3.78-3.71 (m, 2H), 1.53 (s, 3H), 1.51-1.50 (m, 4H), 1.38-1.30 (m, 4H), 1.24-1.00 (m, 6H). **LCMS:** (Method G) 451.0 ($M^+$+H), Rt. 2.57 min, 94.36% (Max).

**Intermediate 16**

**Ethyl 2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetate**

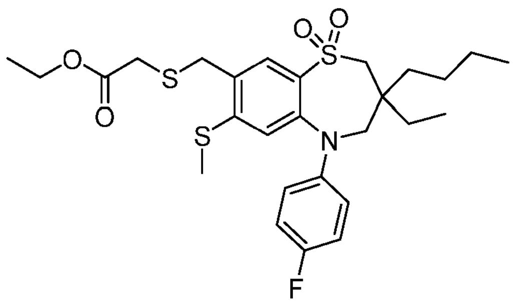

To a stirred solution of 3-butyl-3-ethyl-5-(4-fluorophenyl)-8-(hydroxymethyl)-7-(methylthio)-2,3,4,5-tetrahydro-1,5-benzothiazepine 1,1-dioxide (Intermediate 15; 0.3 g, 0.66 mmol) in DCM (10 mL) at 0 °C, zinc iodide (0.11 g, 0.33 mmol) and ethyl 2-mercaptoacetate (0.16 g, 1.33 mmol) were added and the reaction mixture was stirred for 2 hours at RT. After completion of the reaction (monitored by TLC), the reaction mixture was diluted with water (50 mL) and the aqueous layer was extracted with DCM (2 x 25 mL). The combined organic layer was washed with water (5 mL) and with brine (5 mL) and then dried over anhydrous $Na_2SO_4$. The organic part was concentrated under vacuum and the resulting crude material was forwarded to the next step without any purification. **Yield:** 368 mg (crude, light yellow solid).

**LCMS:** (Method B) 571.1 ($M^+$+18), Rt. 2.91 min, 64.26% (Max).

**Intermediate 17**

**3,3-dibutyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate**

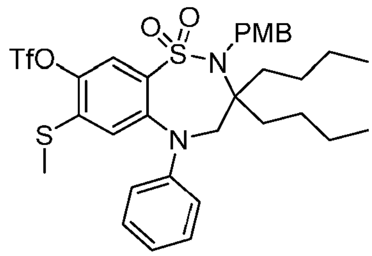

To a stirred solution of 3,3-dibutyl-8-hydroxy-2-(4-methoxybenzyl)-7-(methylthio)-5-phenyl-2,3,4,5-tetrahydro-1,2,5-benzothiadiazepine 1,1-dioxide (0.5 g, 0.88 mmol) in dry DCM (5 mL) at 0 °C, pyridine (0.14 mL, 1.76 mmol) was added. Then trifluoromethanesulfonic anhydride (0.22 mL, 1.32 mmol) was added dropwise and the reaction mixture was stirred for 1 hour at RT. After completion of the reaction (monitored by TLC), the reaction mixture was quenched with ice-cold water (20 mL) and the aqueous layer was extracted with DCM (2 x 20 mL). The combined organic layer was washed with water (10 mL) and with brine (10 mL) and then dried over anhydrous $Na_2SO_4$. The organic part was filtered and concentrated under vacuum and the resulting crude material was purified by Isolera column chromatography (eluent: 20% EtOAc/PE; silica gel: 230-400 mesh) to afford the title compound. **Yield:** 91% (0.6 g, white solid).

**LCMS:** (Method J) 701.3 ($M^+$+H), Rt. 3.49 min, 93.52% (Max).

**Intermediate 18**

**Methyl 3,3-dibutyl-2-(4-methoxybenzyl)-7-(methylthio)-5-phenyl-2,3,4,5-tetrahydro-1,2,5-benzothiadiazepine-8-carboxylate 1,1-dioxide**

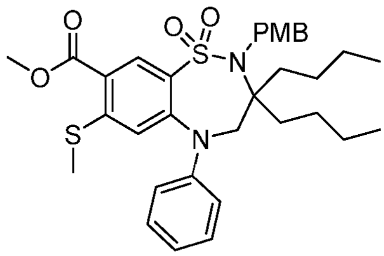

To a solution of 3,3-dibutyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate (Intermediate 17; 0.6 g, 0.86 mmol) in degassed methanol (10 mL) and DMF (5 mL), triethylamine (0.18 mL, 1.28 mmol), dppf (57 mg, 0.10 mmol) and $Pd_2(dba)_3$ (47 mg, 0.05 mmol) were added at RT. The resulting reaction mixture was stirred for 16 hours at 70 °C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL) and with brine (15 mL) and then dried over anhydrous $Na_2SO_4$. The organic part was filtered, concentrated under vacuum and the resulting crude material was purified by Isolera column chromatography (eluent: 12% EtOAc/PE; silica gel: 230-400 mesh) to afford the title compound. **Yield:** 28% (0.17 g, off-white solid).

**LCMS:** (Method J) 610.9 ($M^+$+H), Rt. 3.51 min, 87.25% (Max).

**Intermediate 19**

**3,3-dibutyl-8-(hydroxymethyl)-2-(4-methoxybenzyl)-7-(methylthio)-5-phenyl-2,3,4,5-tetrahydro-1,2,5-benzothiadiazepine 1,1-dioxide**

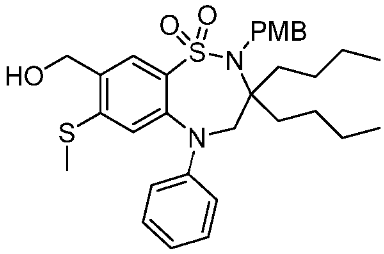

To a stirred solution of methyl 3,3-dibutyl-2-(4-methoxybenzyl)-7-(methylthio)-5-phenyl-2,3,4,5-tetrahydro-1,2,5-benzothiadiazepine-8-carboxylate 1,1-dioxide (Intermediate 18; 0.17 g, 0.28 mmol) in dry THF (4 mL) at 0 °C, lithium aluminium hydride (1M solution in THF, 0.41 mL, 0.42 mmol) was added dropwise and the reaction mixture was stirred for 20 minutes at RT. After completion of the reaction (monitored by TLC), the reaction mixture was quenched with saturated aqueous ammonium chloride solution (10 mL) and the aqueous layer was extracted with EtOAc (2 x 20 mL). The combined organic layer was washed with water (10 mL) and with brine (10 mL) and then dried over anhydrous $Na_2SO_4$. The organic part was filtered, concentrated under vacuum and the resulting crude was forwarded to the next step without any further purification. **Yield:** 87% (0.15 g, off-white solid).

**Intermediate 20**

**Ethyl 2-(((3,3-dibutyl-2-(4-methoxybenzyl)-7-(methylthio)-1,1-dioxido-5-phenyl-2,3,4,5-tetrahydro-1,2,5-benzothiadiazepin-8-yl)methyl)thio)acetate**

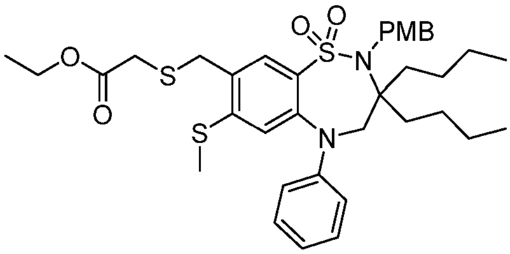

To a stirred solution of 3,3-dibutyl-8-(hydroxymethyl)-2-(4-methoxybenzyl)-7-(methylthio)-5-phenyl-2,3,4,5-tetrahydro-1,2,5-benzothiadiazepine 1,1-dioxide (Intermediate 19; 0.15 g, 0.26 mmol) in DCM (5 mL) at 0 °C, zinc iodide (0.04 g, 0.13 mmol) and ethyl 2-mercaptoacetate (0.06 g, 0.52 mmol) were added and the reaction mixture was stirred for 1 hour at RT. After completion of the reaction

(monitored by TLC), the reaction mixture was diluted with water (10 mL) and the aqueous layer was extracted with DCM (2 x 20 mL). The combined organic layer was washed with water (10 mL) and with brine (10 mL) and then dried over anhydrous $Na_2SO_4$. The organic part was concentrated under vacuum and the resulting crude material was purified by Isolera column chromatography (eluent: 12% EtOAc/PE; silica gel: 230-400 mesh) to afford the title compound. **Yield:** 59.2%, (0.16 g, white sticky solid).

**LCMS:** (Method B) 702.0 ($M^+$+18), Rt. 3.24 min, 54.22% (Max).

**Intermediate 21**

**Ethyl 2-(((3,3-dibutyl-7-(methylthio)-1,1-dioxido-5-phenyl-2,3,4,5-tetrahydro-1,2,5-benzothiadiazepin-8-yl) methyl)thio)acetate**

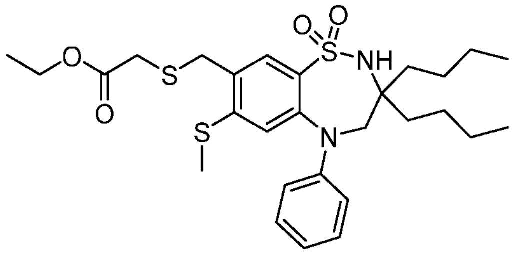

To a stirred solution of ethyl 2-(((3,3-dibutyl-2-(4-methoxybenzyl)-7-(methylthio)-1,1-dioxido-5-phenyl-2,3,4,5-tetrahydro-1,2,5-benzothiadiazepin-8-yl)methyl)thio)acetate (Intermediate 20; 0.16 g, 0.23 mmol) in toluene (3 mL) at 0 °C, triphenylamine (0.12 g, 0.47 mmol) and 2,2,2-trifluoroacetic acid (0.18 mL, 2.34 mmol) were added dropwise and the resulting reaction mixture was stirred for 16 hours at RT. After completion of the reaction (monitored by TLC), the reaction mixture was diluted with ice-cold water (10 mL) and the aqueous layer was extracted with EtOAc (2 x 10 mL). The combined organic layer was washed with water (5 mL) and with brine (5 mL) and then dried over anhydrous $Na_2SO_4$. The organic part was concentrated under vacuum and the resulting crude material was purified by Isolera column chromatography (eluent: 20% EtOAc/PE; silica gel: 230-400 mesh) to afford the title compound. **Yield:** 58% (85 mg, light brown solid).

**$^1$H NMR** (400 MHz, DMSO-$d_6$): δ 7.51 (s, 1H), 7.55-7.20 (m, 5H), 7.20-7.05 (m, 1H), 6.44 (s, 1H), 4.12 (q, J = 6.8 Hz, 2H), 4.10-3.90 (m, 2H), 3.83 (s, 2H), 3.27 (s, 2H), 2.08 (s, 3H), 1.62-1.45 (m, 2H), 1.45-1.28 (m, 2H), 1.28-1.15 (m, 6H), 1.15-1.00 (m, 2H), 1.00-0.82 (m, 3H), 0.82-0.68 (m, 6H) **LCMS:** (Method A) 563.2 ($M^+$-H), Rt. 3.23 min, 89.96% (Max).

**Intermediate 22**

**3-butyl-3-ethyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate**

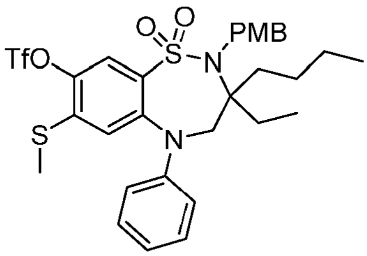

To a stirred solution of 3-butyl-3-ethyl-8-hydroxy-2-(4-methoxybenzyl)-7-(methylthio)-5-phenyl-2,3,4,5-tetrahydro-1,2,5-benzothiadiazepine 1,1-dioxide (1.60 g, 2.96 mmol) in dry DCM (10 mL) at 0 °C, pyridine (0.72 mL, 8.88 mmol) and trifluoromethanesulfonic anhydride (0.75 mL, 4.44 mmol) were added dropwise and the reaction mixture was stirred for 1 hour at RT. After completion of the reaction (monitored by TLC), the reaction mixture was diluted with water (10 mL) and the aqueous layer was extracted with DCM (2 x 20 mL). The combined organic layer was washed with brine (5 mL), dried over anhydrous $Na_2SO_4$, filtered and concentrated under vacuum. The resulting crude material was purified by Isolera column chromatography (eluent: 20-22% EtOAc/PE; silica gel: 230-400 mesh) to afford the title compound. **Yield:** 62.8% (1.4 g, white solid).

**LCMS:** (Method A) 673.0 ($M^+$+H), Rt. 3.53 min, 89.35%(Max).

**Intermediate 23**

**Methyl 3-butyl-3-ethyl-2-(4-methoxybenzyl)-7-(methylthio)-5-phenyl-2,3,4,5-tetrahydro-1,2,5-benzothiadiazepine-8-carboxylate 1,1-dioxide**

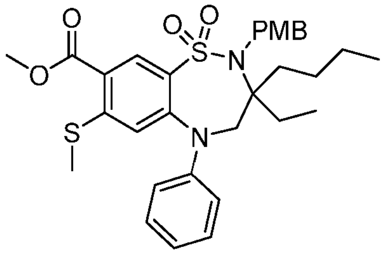

To a stirred solution of 3-butyl-3-ethyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate (Intermediate 22; 1.4 g, 2.08 mmol) in degassed methanol (20 mL) and DMF (5 mL), triethylamine (0.29 mL, 2.08 mmol), dppf (138 mg, 0.25 mmol) and $Pd_2(dba)_3$ (110 mg, 0.13 mmol) were added at RT. The resulting reaction mixture was stirred for 16 hours at 70 °C in an autoclave, under 10 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL) and with brine (15 mL) and then dried over anhydrous $Na_2SO_4$. The organic part was filtered, concentrated under vacuum and the resulting crude material was purified by Isolera column chromatography (eluent: 10-12% EtOAc/PE; silica gel: 230-400 mesh) to afford the title compound. **Yield:** 67% (1 g, off-white solid).

**LCMS:** (Method A) 583.2 ($M^+$+H), Rt. 3.26 min, 81.20% (Max).

**Intermediate 24**

**3-Butyl-3-ethyl-8-(hydroxymethyl)-2-(4-methoxybenzyl)-7-(methylthio)-5-phenyl-2,3,4,5-tetrahydro-1,2,5-benzothiadiazepine 1,1-dioxide**

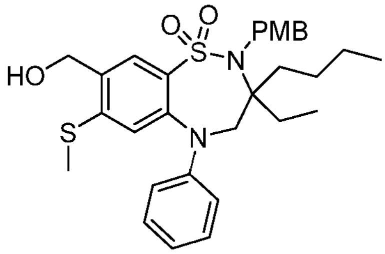

To a stirred solution of methyl 3-butyl-3-ethyl-2-(4-methoxybenzyl)-7-(methylthio)-5-phenyl-2,3,4,5-tetrahydro-1,2,5-benzothiadiazepine-8-carboxylate 1,1-dioxide (Intermediate 23; 1 g, 1.72 mmol) in dry THF (10 mL) at 0 °C, lithium aluminium hydride (2M in THF, 1.27 mL, 2.54 mmol) was added dropwise and the reaction mixture was stirred for 1 hour at RT. After completion of the reaction (monitored by TLC), the reaction mixture was cooled to 0 °C and the reaction mixture was quenched with saturated aqueous ammonium chloride solution (10 mL). The aqueous layer was extracted with EtOAc (2 x 20 mL) and the combined organic layer was washed with water (10 mL), with brine (10 mL) and then dried over anhydrous $Na_2SO_4$. The organic part was filtered, concentrated under vacuum and the resulting crude material was purified by Isolera column chromatography (eluent: 20-25% EtOAc/PE; silica gel: 230-400 mesh) to afford the title compound. **Yield:** 51% (550 mg, off-white solid).

**Intermediate 25**

**Ethyl 2-(((3-butyl-3-ethyl-2-(4-methoxybenzyl)-7-(methylthio)-1,1-dioxido-5-phenyl-2,3,4,5-tetrahydro-1,2,5-benzothiadiazepin-8-yl)methyl)thio)acetate**

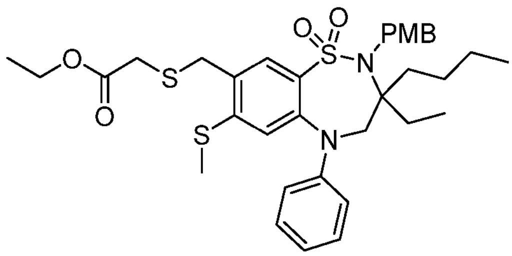

To a stirred solution of 3-butyl-3-ethyl-8-(hydroxymethyl)-2-(4-methoxybenzyl)-7-(methylthio)-5-phenyl-2,3,4,5-tetrahydro-1,2,5-benzothiadiazepine 1,1-dioxide (Intermediate 24; 200 mg, 0.36 mmol) in DCM (5 mL) at 0 °C, zinc iodide (60 mg, 0.18 mmol) and ethyl 2-mercaptoacetate (43.3 mg, 0.36 mmol) were added and the reaction mixture was stirred for 2 hours at RT. After completion of the reaction (monitored by TLC), the reaction mixture was diluted with water (10 mL) and the aqueous layer was extracted with EtOAc (2 x 15 mL). The combined organic layer was washed with water (5 mL) and with brine (5 mL) and dried over anhydrous $Na_2SO_4$. The organic part was filtered, concentrated under vacuum and the resulting crude material was forwarded to the next step without any further purification. **Yield:** 32.8% ( 160 mg, light yellow sticky solid).

**Intermediate 26**

**Ethyl 2-(((3-butyl-3-ethyl-7-(methylthio)-1,1-dioxido-5-phenyl-2,3,4,5-tetrahydro-1,2,5-benzothiadiazepin-8-yl) methyl)thio)acetate**

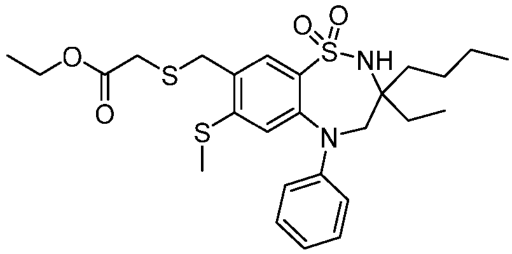

To a stirred solution of ethyl 2-(((3-butyl-3-ethyl-2-(4-methoxybenzyl)-7-(methylthio)-1,1-dioxido-5-phenyl-2,3,4,5-tetrahydro-1,2,5-benzothiadiazepin-8-yl)methyl)thio)acetate (Intermediate 25; 160 mg, 0.24 mmol) in toluene (10 mL) at 0 °C, triphenylamine (120 mg, 0.48 mmol) and trifluoroacetic acid (0.19 mL, 2.44 mmol) were added dropwise and the reaction mixture was stirred for 16 hours at RT. After completion of the reaction (monitored by TLC), the reaction mixture was quenched with ice-cold water (10 mL) and the aqueous layer was extracted with EtOAc (2 x 10 mL). The combined organic layer was washed with water (10 mL) and with brine (10 mL) and then dried over anhydrous $Na_2SO_4$. The organic part was filtered, concentrated under vacuum and the resulting crude material was purified by Isolera column chromatography (eluent: 20-25% EtOAc/PE; silica gel: 230-400 mesh) to afford the title compound. **Yield:** 73.2% (100 mg, light brown solid).

**LCMS:** (Method A) 535.21 ($M^+$-H), Rt. 2.96 min, 95.72% (Max).

**Intermediate 27**

**3-Butyl-2-methyl-7-(methylthio)-1,1-dioxido-5-phenyl-2,3,4,5-tetrahydro-1,2,5-benzothiadiazepin-8-yl trifluoromethanesulfonate**

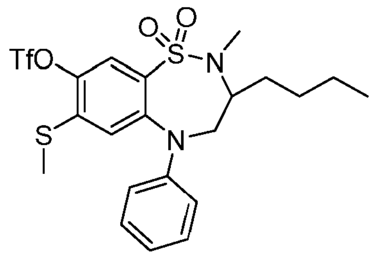

To a stirred solution of 3-butyl-8-hydroxy-2-methyl-7-(methylthio)-5-phenyl-2,3,4,5-tetrahydro-1,2,5-benzothiadiazepine 1,1-dioxide (1.0 g, 2.460 mmol) in dry DCM (10mL) at 0 °C, pyridine (0.38 mL, 4.92 mmol) was added dropwise. Trifluoromethanesulfonic anhydride (0.83 mL, 2.95 mmol) was then added and the reaction mixture was stirred for 1 hour at RT. After completion of the reaction (monitored by TLC), the reaction mixture was diluted with water (10 mL) and the aqueous layer was extracted with DCM (2 x 20 mL). The combined organic layer was washed with brine (5 mL), dried over anhydrous $Na_2SO_4$, filtered and concentrated under vacuum. The resulting crude material was purified by Isolera column chromatography (eluent: 20-22% EtOAc/PE; silica gel: 230-400 mesh) to afford the title compound. **Yield:** 63% (1.0 g, white solid).

**UPLC-MS:** (Method A) 539.1 ($M^+$+H), Rt. 2.64 min, 92.69%(Max).

**Intermediate 28**

**Methyl 3-butyl-7-(methylthio)-5-phenyl-2,3,4,5-tetrahydro-1,2,5-benzothiadiazepine-8-carboxylate 1,1-dioxide**

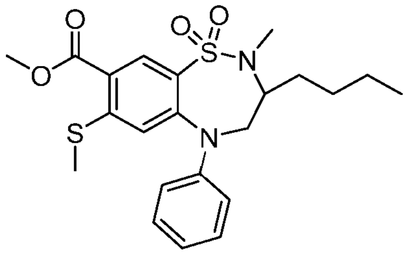

To a stirred solution of 3-butyl-2-methyl-7-(methylthio)-1,1-dioxido-5-phenyl-2,3,4,5-tetrahydro-1,2,5-benzothiadiazepin-8-yl trifluoromethanesulfonate (Intermediate 27; 1.0 g, 1.85 mmol) in degassed methanol (15 mL) and DMF (10 mL), triethylamine (0.38 mL, 2.78 mmol), dppf (0.13 g, 0.22 mmol) and $Pd_2(dba)_3$ (0.102 g, 0.11 mmol) were added at RT. The resulting reaction mixture was then stirred for 16 hours at 70 °C in an autoclave, under 10 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), with brine (15 mL) and then dried over anhydrous $Na_2SO_4$. The organic part was filtered, concentrated under vacuum and the resulting crude material was purified by Isolera column chromatography (eluent: 10-12% EtOAc/PE; silica gel: 230-400 mesh) to afford the title compound. **Yield:** 50.4% (600 mg, off-white solid).

**UPLC-MS:** (Method A) 449.1($M^+$+H), Rt. 2.46 min, 69.04% (Max).

**Intermediate 29**

**3-Butyl-8-(hydroxymethyl)-2-methyl-7-(methylthio)-5-phenyl-2,3,4,5-tetrahydro-1,2,5-benzothiadiazepine 1,1-dioxide**

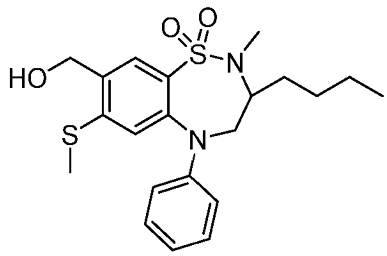

To a stirred solution of methyl 3-butyl-7-(methylthio)-5-phenyl-2,3,4,5-tetrahydro-1,2,5-benzothiadiazepine-8-carboxylate 1,1-dioxide (Intermediate 28; 0.6 g, 1.34 mmol) in dry THF (10 mL) at 0 °C, lithium aluminium hydride (2M in THF, 1.05 mL, 2.10 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 20 mL). The combined organic layer was washed with water (10 mL) and with brine (10 mL) and then dried over anhydrous $Na_2SO_4$. The organic part was filtered, concentrated under vacuum and the resulting crude was purified by Isolera column chromatography (eluent: 20-25% EtOAc/PE; silica gel: 230-400 mesh) to afford the title compound. **Yield:** 86% (500 mg, off-white solid).

**Intermediate 30**

**Ethyl 2-(((3-butyl-2-methyl-7-(methylthio)-1,1-dioxido-5-phenyl-2,3,4,5-tetrahydro-1,2,5-benzothiadiazepin-8-yl)methyl)thio)acetate**

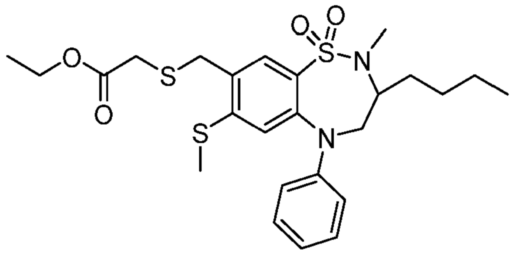

To a stirred solution of 3-butyl-8-(hydroxymethyl)-2-methyl-7-(methylthio)-5-phenyl-2,3,4,5-tetrahydro-1,2,5-benzothiadiazepine 1,1-dioxide (Intermediate 29; 0.25 g, 0.59 mmol) in DCM (5 mL) at 0 °C, zinc (II) iodide (0.09 g, 0.29 mmol) and ethyl 2-mercaptoacetate (0.14 g, 1.19 mmol) were added and the reaction mixture was stirred for 1 hour at RT. After completion of the reaction (monitored by TLC), the reaction mixture was diluted with water (10 mL) and the aqueous layer was extracted with EtOAc (2 x 10 mL). The combined organic layer was washed with water (10 mL) and with brine (10 mL) and then dried over anhydrous $Na_2SO_4$. The organic part was filtered, concentrated under vacuum and the resulting crude material was forwarded as such to the next step without any purification. **Yield:** 48% (0.2 g, light yellow solid).

**Intermediate 31**

**7-Bromo-3-butyl-3-ethyl-8-hydroxy-5-phenyl-2,3,4,5-tetrahydro-1,5-benzothiazepine 1,1-dioxide**

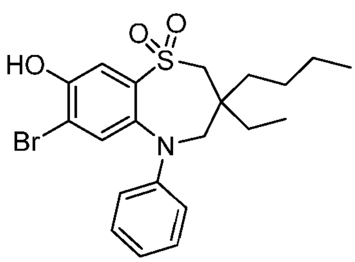

To a stirred solution of 7-bromo-3-butyl-3-ethyl-8-methoxy-5-phenyl-2,3,4,5-tetrahydro-1,5-benzothiazepine 1,1-dioxide (3 g, 6.43 mmol) in DCM (10 mL) at -15 °C, $BBr_3$ (1M in DCM, 12.86 mL, 12.86 mmol) was added dropwise and the reactiom mixture was stirred for 10 minutes. After completion of the reaction (monitored by TLC), the reaction mixture was quenched with methanol (10 mL) at 0 °C and was concentrated under vacuum. The resulting crude material was purified by Isolera column chromatography (eluent: 0-30 % EtOAc/PE; silica gel: 230-400 mesh) to afford the title compound. **Yield:** 48% (2.9 g, brown solid).

**Intermediate 32**

**3-Butyl-3-ethyl-8-hydroxy-7-methoxy-5-phenyl-2,3,4,5-tetrahydro-1,5-benzothiazepine 1,1-dioxide**

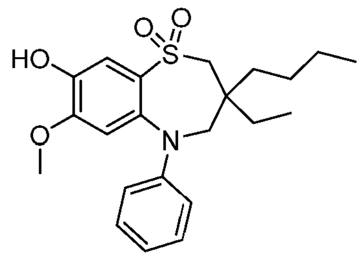

To a stirred solution of 7-bromo-3-butyl-3-ethyl-8-hydroxy-5-phenyl-2,3,4,5-tetrahydro-1,5-benzothiazepine 1,1-dioxide (Intermediate 31; 2.0 g, 4.42 mmol) in sodium methoxide (28% in methanol, 15 mL), copper(I) bromide (0.63 g, 4.42 mmol) was added at RT and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL) and with brine (15 mL) and dried over anhydrous $Na_2SO_4$. The organic layer was concentrated under vacuum and the resulting crude material was purified by Isolera column chromatography (eluent: 0-30% EtOAc/PE; silica gel: 230-400 mesh) to afford the title compound. **Yield:** 48% (2 g, brown solid).

**LCMS:** (Method A) 404.1 ($M^+$+H), Rt. 2.94 min, 98.03% (Max).

**Intermediate** 33

**3-Butyl-3-ethyl-7-methoxy-1,1-dioxido-5-phenyl-2,3,4,5-tetrahydro-1,5-benzothiazepin-8-yl trifluoromethane-sulfonate**

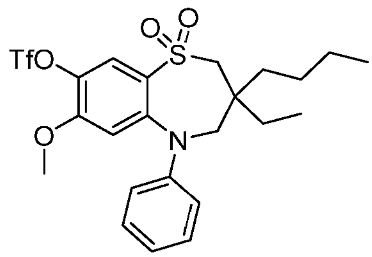

To a stirred solution of 3-butyl-3-ethyl-8-hydroxy-7-methoxy-5-phenyl-2,3,4,5-tetrahydro-1,5-benzothiazepine 1,1-dioxide (Intermediate 32; 1.0 g, 2.48 mmol) in dry DCM (10 mL) at 0 °C, pyridine (0.2 mL, 2.48 mmol) and then trifluoromethanesulfonic anhydride (0.41 mL, 2.48 mmol) were added and the reaction mixture was stirred for 1 hour at RT. After completion of the reaction (monitored by TLC), the reaction mixture was diluted with water (10 mL) and the aqueous layer was extracted with DCM (2 x 20 mL). The combined organic layer was washed with brine (5 mL) and with brine (10 mL) and then dried over anhydrous $Na_2SO_4$. The organic part was concentrated under vacuum and the resulting crude material was purified by Isolera column chromatography (eluent: 7-8% EtOAc/PE; silica gel: 230-400 mesh) to afford the title compound. **Yield:** 68% (900 mg, white solid).

**LCMS:** (Method A) 536.1 ($M^+$+H), Rt. 3.10 min, 97.27%(Max).

**Intermediate 34**

**Methyl 3-butyl-3-ethyl-7-methoxy-5-phenyl-2,3,4,5-tetrahydro-1,5-benzothiazepine-8-carboxylate 1,1-dioxide**

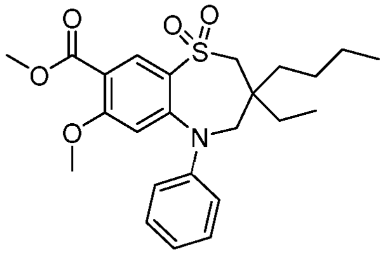

To a stirred solution of 3-butyl-3-ethyl-7-methoxy-1,1-dioxido-5-phenyl-2,3,4,5-tetrahydro-1,5-benzothiazepin-8-yl trifluoromethanesulfonate (Intermediate 33; 0.9 g, 1.68 mmol) in degassed methanol (15 mL) and DMF (5 mL), triethylamine (0.35 mL, 2.52 mmol), $Pd_2(dba)_3$ (0.08 g, 0.08 mmol) and dppf (0.11 g, 0.20 mmol) were added at RT. The resulting reaction mixture was stirred for 16 hours at 70 °C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL) and with brine (15 mL) and then dried over anhydrous $Na_2SO_4$. The organic part was filtered, concentrated under vacuum and the resulting crude material was purified by Isolera column chromatography (eluent: 0-30% EtOAc/PE; silica gel: 230-400 mesh) to afford the title compound. **Yield:** 16% (0.6 g, off-white solid).

**Intermediate 35**

**3-Butyl-3-ethyl-8-(hydroxymethyl)-7-methoxy-5-phenyl-2,3,4,5-tetrahydro-1,5-benzothiazepine 1,1-dioxide**

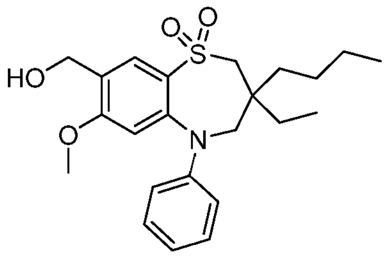

To a stirred solution of methyl 3-butyl-3-ethyl-7-methoxy-5-phenyl-2,3,4,5-tetrahydro-1,5-benzothiazepine-8-carboxylate 1,1-dioxide (Intermediate 34; 0.2 g, 0.45 mmol) in THF (5 mL) at 0 °C, lithium aluminium hydride (2M in THF, 0.22 mL, 0.44 mmol) was added dropwise and the reaction mixture was stirred for 1 hour at RT. After completion of the reaction (monitored by TLC), the reaction mixture was cooled to 0 °C and quenched with saturated aqueous ammonium chloride solution (10 mL). The aqueous layer was extracted with EtOAc (2 x 20 mL) and the combined organic layer was washed with water (10 mL) and with brine (10 mL) and then dried over anhydrous $Na_2SO_4$. The organic part was filtered, concentrated under vacuum and the resulting crude was purified by Isolera column chromatography (eluent: 20% EtOAc/PE; silica gel: 230-400 mesh) to afford the title compound. **Yield:** 95% (190 mg, white solid).

**Intermediate 36**

**Ethyl 2-(((3-butyl-3-ethyl-7-methoxy-1,1-dioxido-5-phenyl-2,3,4,5-tetrahydro-1,5-benzothiazepin-8-yl)methyl) thio)acetate**

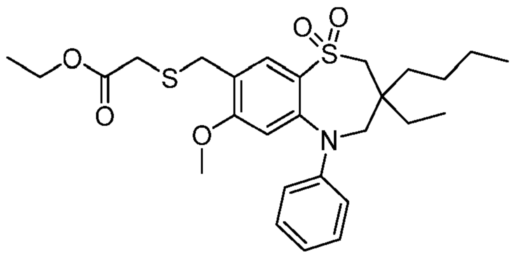

To a stirred solution of 3-butyl-3-ethyl-8-(hydroxymethyl)-7-methoxy-5-phenyl-2,3,4,5-tetrahydro-1,5-benzothiazepine 1,1-dioxide (Intermediate 35; 0.19 g, 0.46 mmol) in DCM at 0 °C, ethyl 2-mercaptoacetate (0.11 g, 0.91 mmol) and then zinc (II) iodide (0.07 g, 0.23 mmol) were added, and the reaction mixture was stirred for 3 hours at RT. After completion of the reaction (monitored by TLC), the reaction mixture was quenched with water (5 mL) and the aqueous layer was extracted with DCM (2 x 10 mL). The combined organic layer was washed with water (5 mL) and with brine (5 mL) and then dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 60% (0.15 g, white solid).

**Intermediate 37**

**Methyl 2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoate**

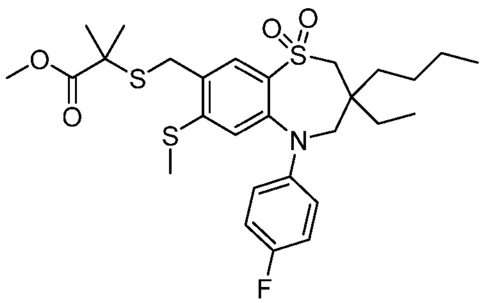

To a stirred solution of 3-butyl-3-ethyl-5-(4-fluorophenyl)-8-(hydroxymethyl)-7-(methylthio)-2,3,4,5-tetrahydro-1,5-benzothiazepine 1,1-dioxide (Intermediate 15; 250 mg, 0.58 mmol) in DCM (5 mL) at 0 °C, methyl 2-mercapto-2-methylpropanoate (81 mg, 0.61 mmol) and zinc iodide (70 mg, 0.22 mmol) were added and the reaction mixture was stirred for 3 hours at RT. After completion of the reaction (monitored by TLC), the reaction mixture was quenched with water (5 mL) and the aqueous layer was extracted with DCM (2 x 10 mL). The combined organic layer was washed with brine (10 mL), dried over anhydrous $Na_2SO_4$, filtered and concentrated under vacuum to afford the title compound. **Yield:** 76.2% (230 mg, brown gum).

**Intermediate 38**

Methyl 2-(((3,3-dibutyl-7-(methylthio)-1,1-dioxido-5-phenyl-2,3,4,5-tetrahydro-1,5-benzothiazepin-**8-yl)methyl)thio)-2-methylpropanoate**

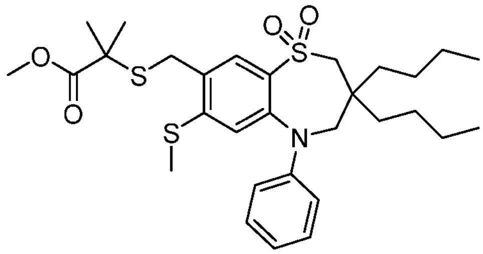

To a stirred solution of 3,3-dibutyl-8-(hydroxymethyl)-7-(methylthio)-5-phenyl-2,3,4,5-tetrahydro-1,5-benzothiazepine 1,1-dioxide (Intermediate 7, 0.15 g, 0.33 mmol) in DCM (5 mL) at 0 °C, methyl 2-mercapto-2-methylpropanoate (0.05 g, 0.36 mmol) and zinc (II) iodide (0.05 g, 0.16 mmol) were added and the reaction mixture was stirred for 1 hour at RT. After completion of the reaction (monitored by TLC), the reaction mixture was quenched with water (5 mL) and the aqueous layer was extracted with DCM (2 x 10 mL). The combined organic layer was washed with brine (10 mL), dried over anhydrous $Na_2SO_4$, filtered and concentrated under vacuum to afford the title compound. **Yield:** 48% (0.09 g, brown gum).

**Intermediate 39**

**Methyl 2-(((3-butyl-3-methyl-7-(methylthio)-1,1-dioxido-5-phenyl-2,3,4,5-tetrahydro-1,5-benzothiazepin-8-yl) methyl)thio)-2-methylpropanoate**

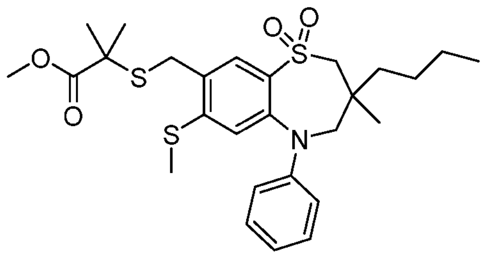

To a stirred solution of 3-butyl-8-(hydroxymethyl)-3-methyl-7-(methylthio)-5-phenyl-2,3,4,5-tetrahydro-1,5-benzothiazepine 1,1-dioxide (Intermediate 11; 0.07 g, 0.17 mmol) in DCM (5 mL) at 0 °C, methyl 2-mercapto-2-methylpropanoate (0.05 g,0.33 mmol) and zinc (II) iodide (16 mg, 0.09 mmol) were added and the reaction mixture was stirred for 1 hour at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 20% EtOAc/PE; silica gel: 230-400 mesh) to afford the title compound. **Yield:** 75% (0.05 g, white solid).

Intermediate 40

3-Butyl-3-ethyl-2-methyl-7-(methylthio)-1,1-dioxido-5-phenyl-2,3,4,5-tetrahydro-1,2,5-benzothiadiazepin-8-yl trifluoromethanesulfonate

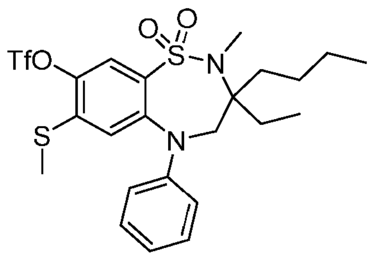

To a stirred solution of 3-butyl-3-ethyl-8-hydroxy-2-methyl-7-(methylthio)-5-phenyl-2,3,4,5-tetrahydro-1,2,5-benzothiadiazepine 1,1-dioxide (1.5 g, 2.77 mmol) in DCM (10 mL) at 0 °C, pyridine (0.67 mL, 8.32 mmol) and then trifluoromethanesulfonic anhydride (0.14 mL, 1.76 mmol) were added dropwise and the reaction mixture was stirred for 1 hour at RT. After completion of the reaction (monitored by TLC), the reaction mixture was diluted with water (20 mL) and the aqueous layer was extracted with DCM (2 x 20 mL). The combined organic layer was washed with water (20 mL), brine (20 mL) and then dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford the title compound. **Yield:** 97% (1.85 g, white solid).
**LCMS:** (Method A) 567.1 ($M^+$+H), Rt. 3.32 min, 97.97% (Max).

**Intermediate 41**

**Methyl 3-butyl-3-ethyl-2-methyl-7-(methylthio)-5-phenyl-2,3,4,5-tetrahydro-1,2,5-benzothiadiazepine-8-carboxylate 1,1-dioxide**

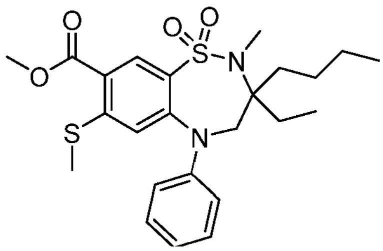

To a solution of 3-butyl-3-ethyl-2-methyl-7-(methylthio)-1,1-dioxido-5-phenyl-2,3,4,5-tetrahydro-1,2,5-benzothiadiazepin-8-yl trifluoromethanesulfonate (Intermediate 40; 1.85 g, 3.26 mmol) in degassed methanol (10 mL) and DMF (5 mL) at RT, triethylamine (0.45 mL, 3.26 mmol), dppf (0.22 g, 0.39 mmol) and $Pd_2(dba)_3$ (0.18 g, 0.19 mmol) were added. The resulting reaction mixture was stirred for 16 hours at 75 °C in an autoclave, under 5 kg carbon monoxide gas pressure. After completion of the reaction (monitored by TLC), the reaction mixture was diluted with water (20 mL) and the aqueous layer was extracted with EtOAc (2 x 20 mL). The combined organic layer was washed with water (10 mL), brine (10 mL) and then dried over anhydrous $Na_2SO_4$. The organic layer was filtered, concentrated under vacuum and the resulting crude material was purified by Isolera column chromatography (eluent: 12 % EtOAc/PE; silica gel: 230-400 mesh) to afford the title compound. **Yield:** 48.5% (1 g, off-white solid).
**LCMS:** (Method A) 477.1 ($M^+$+H), Rt. 3.16 min, 75.49% (Max).

**Intermediate 42**

**3-Butyl-3-ethyl-8-(hydroxymethyl)-2-methyl-7-(methylthio)-5-phenyl-2,3,4,5-tetrahydro-1,2,5-benzothiadiazepine 1,1-dioxide**

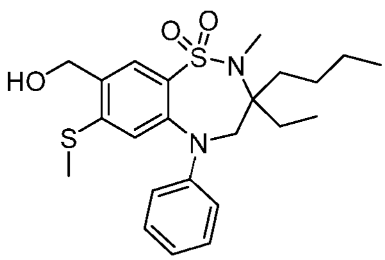

To a stirred solution of methyl 3-butyl-3-ethyl-2-methyl-7-(methylthio)-5-phenyl-2,3,4,5-tetrahydro-1,2,5-benzothiadiazepine-8-carboxylate 1,1-dioxide (Intermediate 41; 1 g, 2.09 mmol) in THF (10 mL) at 0 °C, lithium aluminium hydride (2M solution in THF, 1.57 mL, 3.15 mmol) was added dropwise and the reaction mixture was stirred for 1 hour at RT. After completion of the reaction (monitored by TLC), the reaction mixture was quenched with saturated aqueous ammonium chloride solution (5 mL) and the aqueous layer was extracted with EtOAc (2 x 20 mL). The combined organic

layer was washed with water (15 mL), brine (15 mL) and then dried over anhydrous $Na_2SO_4$. The organic part was filtered, concentrated under vacuum and the resulting crude was purified by Isolera column chromatography (eluent: 20% EtOAc/PE; silica gel: 230-400 mesh) to afford the title compound. **Yield:** 53% (0.5 g, off-white solid).

**$^1$H NMR** (400 MHz, $CDCl_3$): δ 7.83 (s, 1H), 7.42-7.38 (m, 2H), 724-7.19 (m, 3H), 6.40 (s, 1H), 4.67-4.65 (d, J = 6.4 Hz, 2H), 4.40-4.28 (m, 2H), 3.04 (s, 3H), 2.11-2.07 (m, 4H), 1.90-1.87 (m, 2H), 1.56-1.55 (m, 1H), 1.28-1.11 (m, 2H), 1.02-0.95 (m, 2H), 0.82-0.75 (m, 6H).

***Separation of enantiomers:***

(S)-3-butyl-3-ethyl-8-(hydroxymethyl)-2-methyl-7-(methylthio)-5-phenyl-2,3,4,5-tetrahydro-1,2,5-benzothiadiazepine 1,1-dioxide and (R)-3-butyl-3-ethyl-8-(hydroxymethyl)-2-methyl-7-(methylthio)-5-phenyl-2,3,4,5-tetrahydro-1,2,5-benzothiadiazepine 1,1-dioxide

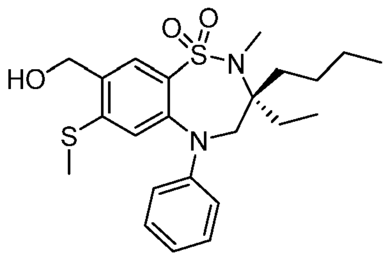 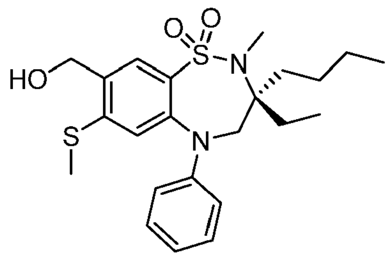

The two enantiomers of racemic 3-butyl-3-ethyl-8-(hydroxymethyl)-2-methyl-7-(methylthio)-5-phenyl-2,3,4,5-tetrahydro-1,2,5-benzothiadiazepine 1,1-dioxide (0.3 g, 0.67 mmol) were separated by chiral SFC (Method H). The material was concentrated under vacuum at 45 °C. The first eluting fraction corresponded to enantiomer 1 and the second eluting fraction corresponded to enantiomer 2. The absolute configuration of the two enantiomers is not known.

Enantiomer 1: **Yield:** 39% (0.12 g, white solid). **Chiral HPLC** (Method H) Rt. 3.16 min, 99.85% (Max). Enantiomer 2: **Yield:** 32.9% (0.1 g, white solid). **Chiral HPLC** (Method H) Rt. 3.84 min, 98.34% (Max).

**Intermediate 43**

**Ethyl 2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetate**

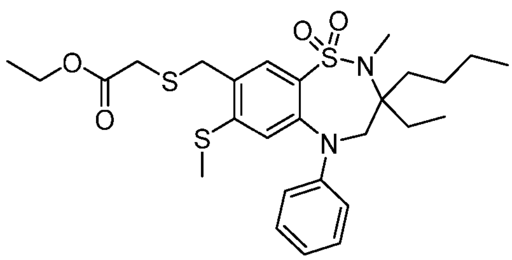

To a stirred solution of 3-butyl-3-ethyl-8-(hydroxymethyl)-2-methyl-7-(methylthio)-5-phenyl-2,3,4,5-tetrahydro-1,2,5-benzothiadiazepine 1,1-dioxide (Intermediate 42; 500 mg, 1.11 mmol) in DCM (5 mL) at 0 °C, zinc iodide (356 mg, 1.11 mmol) and ethyl 2-mercaptoacetate (134 mg, 1.11 mmol) were added and the reaction mixture was stirred for 2 hours at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 12% EtOAc/PE; silica gel: 230-400 mesh) to afford the title compound. **Yield:** 25.7% (0.15 g, colourless gum).

**$^1$H NMR** (400 MHz, DMSO-ds): δ 7.59 (s, 1H), 7.42-7.38 (m, 2H), 7.34-7.32 (m, 2H), 7.18-7.14 (m, 1H), 6.41 (s, 1H), 4.38-4.2(m, 1H), 4.17-4.13 (m, 3H), 3.82 (s, 2H), 3.28 (s, 3H), 3.17 (s, 2H), 2.89 (s, 3H), 2.07 (s, 3H), 1.92-1.80 (m, 2H), 1.46-1.49 (m, 2H), 1.21-1.18 (m, 2H), 0.96-0.90 (m, 2H), 0.75-0.69 (m, 6H). LCMS: (Method A) 549.2 ($M^+$-H), Rt. 3.23 min, 99.79% (Max). HPLC: (Method B) Rt. 6.67 min, 99.83% (Max).

Intermediate 44

Ethyl (S)-2-(((3-butyl-3-ethyl-2-methyl-7-(methylthio)-1,1-dioxido-5-phenyl-2,3,4,5-tetrahydro-**1,2,5-benzothiadiazepin-8-yl)methyl)thio)acetate** and ethyl (R)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetate

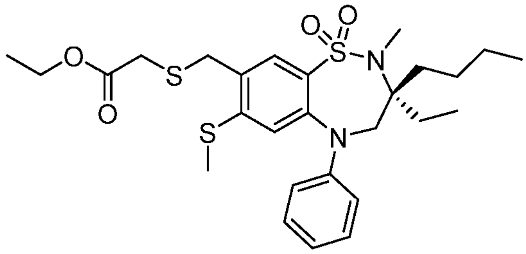

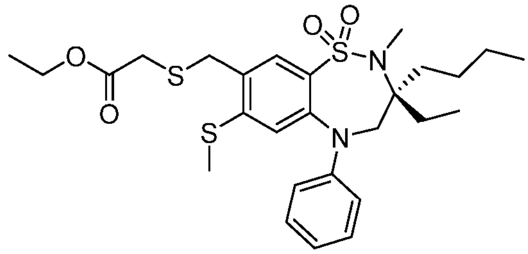

Enantiomers 1 and 2 of the title compound were obtained following the same procedure as described for Intermediate 43, butstarting from 50 mg of enantiomers 1 and 2 of Intermediate 42, respectively. The absolute configuration of the two enantiomers is not known.

Enantiomer 1: **Yield:** 73% (45 mg, colourless gum). **$^1$H NMR** (400 MHz, DMSO-ds): δ 7.59 (s, 1H), 7.42-7.38 (m, 2H), 7.35-7.33 (m, 2H), 7.18-7.16 (m, 1H), 6.41 (s, 1H), 4.20-4.13 (m, 2H), 4.12-4.08 (m, 2H), 3.82 (s, 2H), 3.28 (s, 2H), 2.90 (s, 3H), 2.07 (s, 3H), 1.99-1.82 (m, 1H), 1.80-1.78 (m, 1H), 1.55-1.48 (m, 2H), 1.23-1.16 (m, 5H), 1.02-0.99 (m, 2H), 0.74-0.69 (m, 6H).

Enantiomer 2: **Yield:** 74% (45 mg, colourless gum). **$^1$H NMR** (400 MHz, DMSO-ds): δ 7.60 (s, 1H), 7.42-7.38 (m, 2H), 7.33-7.31 (m, 2H), 7.18-7.14 (m, 1H), 6.41 (s, 1H), 4.20-4.13 (m, 2H), 4.12-4.08 (m, 2H), 3.82 (s, 2H), 3.28 (s, 2H), 2.90 (s, 3H), 2.07 (s, 3H), 1.99-1.82 (m, 1H), 1.80-1.78 (m, 1H), 1.55-1.48 (m, 2H), 1.24-1.16 (m, 5H), 1.02-0.99 (m, 2H), 0.74-0.69 (m, 6H).

**Intermediate 45**

**3,3-dibutyl-8-methoxy-2-methyl-7-(methylthio)-5-phenyl-2,3,4,5-tetrahydro-1,2,5-benzothiadiazepine 1,1-dioxide**

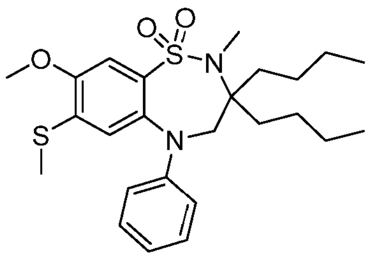

To a stirred solution of 3,3-dibutyl-8-hydroxy-7-(methylthio)-5-phenyl-2,3,4,5-tetrahydro-1,2,5-benzothiadiazepine 1,1-dioxide (0.38 g, 0.85 mmol) in NMP (5 mL) at 0 °C, $Cs_2CO_3$ (0.55 g, 1.69 mmol) was added. Methyl iodide (0.11 mL,1.69 mmol) was added dropwise and the reaction mixture was stirred for 16 hours at room temperature. After completion of the reaction (monitored by TLC), the reaction mixture was quenched with water (20 mL) and the aqueous layer was extracted with EtOAc (2 x 20 mL). The combined organic layer was washed with water (15 mL) and with brine (15 mL) and then dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 84% (0.34 g, off-white solid).

**LCMS:** (Method E) 477.3 ($M^+$+H), Rt. 3.30 min, 99.44% (Max).

**Intermediate 46**

**3,3-Dibutyl-8-hydroxy-2-methyl-7-(methylthio)-5-phenyl-2,3,4,5-tetrahydro-1,2,5-benzothiadiazepine 1,1-dioxide**

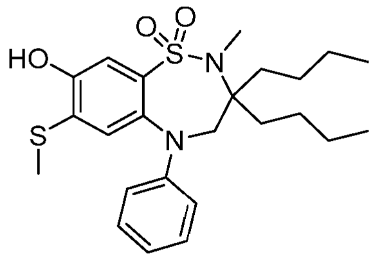

To a stirred solution of 3,3-dibutyl-8-methoxy-2-methyl-7-(methylthio)-5-phenyl-2,3,4,5-tetrahydro-1,2,5-benzothiadiazepine 1,1-dioxide (Intermediate 45; 0.34 g, 0.71 mmol) in DMF (5 mL), sodium thiomethoxide (0.25 g, 3.57 mmol) was added at room temperature and the reaction mixture was then heated for 16 hours at 100 °C. After completion of the reaction (monitored by TLC), the reaction mixture was quenched with water (20 mL) and the aqueous layer was extracted with EtOAc (2 x 25 mL). The combined organic layer was washed with water (15 mL) and with brine (15 mL) and dried over anhydrous $Na_2SO_4$. The organic layer was filtered and concentrated under vacuum. The resulting crude material was purified by Isolera column chromatography (eluent: 0-30% EtOAc/PE; silica gel: 230-400 mesh) to afford the title compound. **Yield:** 62.4% (0.3 g, white solid).

**LCMS:** (Method A) 463.1 ($M^+$+H), Rt. 4.18 min, 68.61% (Max).

**Intermediate 47**

**3,3-Dibutyl-2-methyl-7-(methylthio)-1,1-dioxido-5-phenyl-2,3,4,5-tetrahydro-1,2,5-benzothiadiazepin-8-yl trifluoromethanesulfonate**

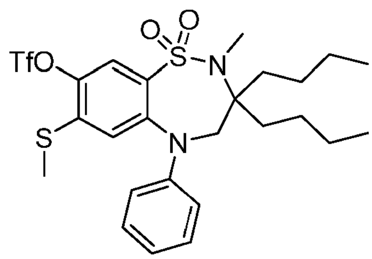

To a stirred solution of 3,3-dibutyl-8-hydroxy-2-methyl-7-(methylthio)-5-phenyl-2,3,4,5-tetrahydro-1,2,5-benzothiadiazepine 1,1-dioxide (Intermediate 46; 0.3 g, 0.65 mmol) in DCM (5 mL) at 0 °C, pyridine (0.10 mL, 1.29 mmol) and then trifluoromethanesulfonic anhydride (0.16 mL, 0.97 mmol) were added dropwise and the reaction mixture was stirred for 30 minutes at RT. After completion of the reaction (monitored by TLC), the reaction mixture was diluted with water (10 mL) and the aqueous layer was extracted with DCM (2 x 20 mL). The combined organic layer was washed with water (20 mL), brine (20 mL), and then dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford the title compound. **Yield:** 89% (0.35 g, off-white solid).

**LCMS:** (Method A) 595.1 ($M^+$+H), Rt. 3.65 min, 98.61% (Max).

**Intermediate 48**

**Methyl 3,3-dibutyl-2-methyl-7-(methylthio)-5-phenyl-2,3,4,5-tetrahydro-1,2,5-benzothiadiazepine-8-carboxylate 1,1-dioxide**

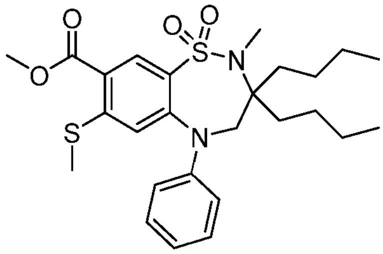

To a solution of 3,3-dibutyl-2-methyl-7-(methylthio)-1,1-dioxido-5-phenyl-2,3,4,5-tetrahydro-1,2,5-benzothiadiazepin-8-yl trifluoromethanesulfonate (Intermediate 47; 0.35 g, 0.59 mmol) in degassed methanol (10 mL) and DMF (5 mL) at RT, triethylamine (0.12 mL, 0.88 mmol), dppf (0.03 g, 0.04 mmol) and $Pd_2(dba)_3$ (0.04 g, 0.07 mmol) were added. The resulting reaction mixture was stirred for 16 hours at 75 °C in an autoclave. under 5 kg carbon monoxide gas pressure. After completion of the reaction (monitored by TLC), the reaction mixture was diluted with water (20 mL) and the aqueous layer was extracted with EtOAc (2 x 20 mL). The combined organic layer was washed with water (10 mL), brine (10 mL), and then dried over anhydrous $Na_2SO_4$. The organic layer was filtered, concentrated under vacuum and the resulting crude material was purified by Isolera column chromatography (eluent: 12% EtOAc/PE; silica gel: 230-400 mesh) to afford the title compound. **Yield:** 39.3% (0.18 g, white solid).

**LCMS:** (Method A) 505.2 ($M^{+}$+H), Rt. 3.41 min, 64.78% (Max).

Intermediate 49

**3,3-Dibutyl-8-(hydroxymethyl)-2-methyl-7-(methylthio)-5-phenyl-2,3,4,5-tetrahydro-1,2,5-**benzothiadiazepine 1,1-dioxide

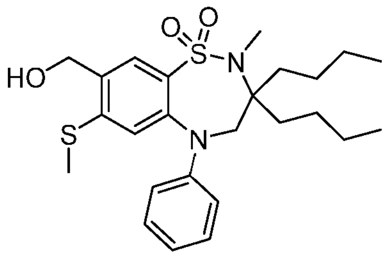

To a stirred solution of methyl 3,3-dibutyl-2-methyl-7-(methylthio)-5-phenyl-2,3,4,5-tetrahydro-1,2,5-benzothiadiazepine-8-carboxylate 1,1-dioxide (Intermediate 48; 0.18 g, 0.36 mmol) in THF (5 mL) at 0 °C, lithium aluminium hydride (2.4M in THF, 0.22 mL, 0.54 mmol) was added dropwise and the reaction mixture was stirred for 1 hour at RT. After completion of the reaction (monitored by TLC), the reaction mixture was quenched with saturated aqueous ammonium chloride solution (5 mL) and the aqueous layer was extracted with EtOAc (3 x 20 mL). The combined organic layer was washed with water (15 mL), brine (15 mL), and dried over anhydrous $Na_2SO_4$. The organic part was filtered, concentrated under vacuum and the resulting crude was purified by Isolera column chromatography (eluent: 25% EtOAc/PE; silica gel: 230-400 mesh) to afford the title compound. **Yield:** 64.3% (0.11 g, off-white solid).

**$^1$H NMR** (400 MHz, DMSO-ds): δ 7.75 (s, 1H), 7.4-7.36 (m, 2H), 730-7.28 (m, 2H), 7.14-7.11 (m, 1H), 6.43 (s, 1H), 5.36-5.33 (m, 1H), 4.04-4.39 (m, 2H), 4.16-4.04 (m, 2H), 2.87 (s, 3H), 2.07 (s, 3H), 1.99-1.79 (m, 2H), 1.50-1.45 (m, 2H), 1.24-1.09 (m, 8H), 0.98-0.97 (m, 6H). **LCMS:** (Method A) 477.0 ($M^{+}$+H), Rt. 3.29 min, 99.42% (Max).

**Intermediate 50**

**Ethyl 2-(((3,3-dibutyl-2-methyl-7-(methylthio)-1,1-dioxido-5-phenyl-2,3,4,5-tetrahydro-1,2,5-benzothiadiazepin-8-yl)methyl)thio)acetate**

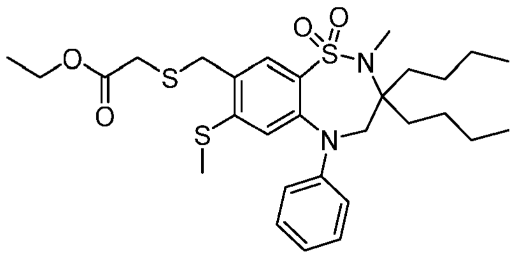

To a stirred solution of 3,3-dibutyl-8-(hydroxymethyl)-2-methyl-7-(methylthio)-5-phenyl-2,3,4,5-tetrahydro-1,2,5-benzothiadiazepine 1,1-dioxide (Intermediate 49; 0.11 g, 0.23 mmol) in DCM (5 mL) at 0 °C, zinc (II) iodide (0.037 g, 0.12 mmol) and ethyl 2-mercaptoacetate (0.05 g, 0.46 mmol) were added and the reaction mixture was stirred for 1 hour at RT. After completion of the reaction (monitored by TLC), the reaction mixture was quenched with water (5 mL) and the aqueous layer was extracted with DCM (2 x 10 mL). The combined organic layer was washed with brine (20 mL), dried over anhydrous $Na_2SO_4$, filtered and concentrated under vacuum. The resulting crude material was purified by Isolera column chromatography (eluent: 12% EtOAc/PE; silica gel: 230-400 mesh) to afford the title compound. **Yield:** 82% (0.11 g, off-white solid).

**$^1$H NMR** (400 MHz, DMSO-ds): δ 7.59 (s, 1H), 7.42-7.38 (m, 2H), 7.35-7.33 (m, 2H), 7.18-7.16 (m, 1H), 6.41 (s, 1H), 4.20-4.13 (m, 2H), 4.12-4.10 (m, 2H), 3.82 (s, 2H), 3.28 (s, 2H), 2.90 (s, 3H), 2.07 (s, 3H), 1.99-1.82 (m, 2H), 1.54-1.47 (m, 2H), 1.24-1.00 (m, 11H), 0.75-0.72 (m, 6H). **HPLC:** (Method B) Rt. 6.67 min, 99.83% (Max).

**Intermediate 51**

**Methyl 2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoate**

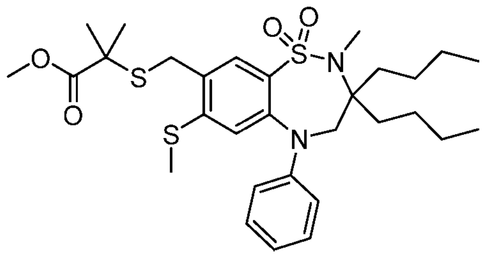

To a stirred solution of 3,3-dibutyl-8-(hydroxymethyl)-2-methyl-7-(methylthio)-5-phenyl-2,3,4,5-tetrahydro-1,2,5-benzothiadiazepine 1,1-dioxide (Intermediate 49; 0.38 g, 0.79 mmol) in DCM (10 mL) at 0 °C, methyl 2-mercapto-2-methylpropanoate (0.21 g, 1.59 mmol) and zinc iodide (0.13 g, 0.39 mmol) were added and the reaction mixture was stirred for 2 hours at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 17% EtOAc/PE; silica gel: 230-400 mesh) to afford the title compound. **Yield:** 87% (0.4 g, white solid).

**$^1$H NMR** (400 MHz, DMSO-ds): δ 7.57 (s, 1H), 7.32-7.42 (m, 4H), 7.16-7.18 (m, 1H), 6.37 (s, 1H), 4.18-4.26 (m, 2H), 3.82 (s, 2H), 3.64 (s, 3H), 2.89 (s, 3H), 2.06 (s, 3H), 1.82-1.89 (m, 2H), 1.48 (s, 7H), 1.42-1.43 (m, 1H), 1.10-1.23 (m, 8H), 0.67-0.75 (m, 6H).

Intermediate 52

Methyl 2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)-2-methylpropanoate

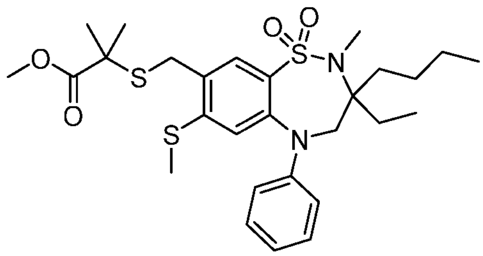

To a stirred solution of 3-butyl-3-ethyl-8-(hydroxymethyl)-2-methyl-7-(methylthio)-5-phenyl-2,3,4,5-tetrahydro-1,2,5-benzothiadiazepine 1,1-dioxide (Intermediate 42; 0.4 g, 0.89 mmol) in DCM (10 mL) at 0 °C, methyl 2-mercapto-2-methylpropanoate (0.24 g, 1.78 mmol) and zinc iodide (0.14 g, 0.45 mmol) were added and the reaction mixture was for 2 hours at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 17% EtOAc/PE; silica gel: 230-400 mesh) to afford the title compound. **Yield:** 83.3% (0.42 g, white solid).

**$^1$H NMR** (400 MHz, DMSO-ds): δ 7.58 (s, 1H), 7.43-7.30 (m, 4H), 7.18-7.16 (m, 1H), 6.37 (s, 1H), 4.16 (s, 2H), 3.82 (s, 2H), 3.65 (s, 3H), 2.89 (s, 3H), 2.07 (s, 3H), 1.87-1.84 (m, 2H), 1.48 (s, 8H), 1.18-1.11 (m, 2H), 0.91-0.72 (m, 2H), 0.69-0.51 (m, 6H).

**Intermediate 53**

**Methyl 2-(((3-butyl-3-ethyl-7-methoxy-1,1-dioxido-5-phenyl-2,3,4,5-tetrahydro-1,5-benzothiazepin-8-yl)methyl) thio)-2-methylpropanoate**

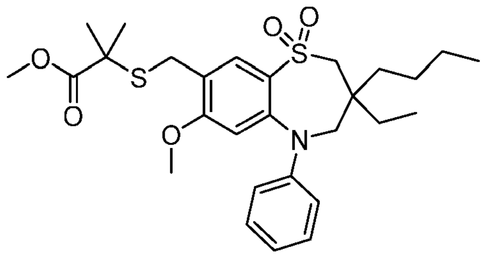

To a stirred solution of 3-butyl-3-ethyl-8-(hydroxymethyl)-7-methoxy-5-phenyl-2,3,4,5-tetrahydro-1,5-benzothiazepine 1,1-dioxide (Intermediate 35; 0.35 g, 0.83 mmol) in DCM (5 mL) at 0 °C, methyl 2-mercapto-2-methylpropanoate (0.22 g, 1.68 mmol) and zinc (II) iodide (0.13 g, 0.42 mmol) were added and the reaction mixture was stirred for 1 hour at RT. After completion of the reaction (monitored by TLC), the reaction mixture was quenched with water (15 mL) and the aqueous layer extracted with DCM (2 X 15 mL). The combined organic layer was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 67.3% (0.35 g, white solid).

**Intermediate 54**

**7-Bromo-3-butyl-8-hydroxy-3-methyl-5-phenyl-2,3,4,5-tetrahydro-1,5-benzothiazepine 1,1-dioxide**

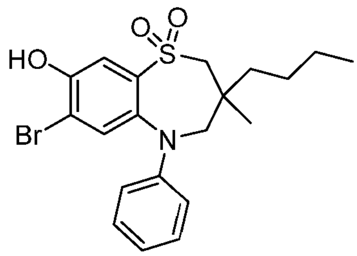

To a stirred solution of 7-bromo-3-butyl-8-methoxy-3-methyl-5-phenyl-2,3,4,5-tetrahydro-1,5-benzothiazepine 1,1-dioxide (2.5 g, 5.53 mmol) in DCM (10 mL) at -15 °C, $BBr_3$ (1M in DCM, 11.05 mL, 11.05 mmol) was added dropwise and the reaction mixture was stirred for 10 minutes. After completion of the reaction (monitored by TLC), the reaction mixture was quenched with methanol (10 mL) and concentrated under vacuum. The resulting crude material was purified by Isolera column chromatography (eluent: 0-30% EtOAc/PE; silica gel: 230-400 mesh) to afford the title compound. **Yield:** 89% (2.1 g, brown solid).

**LCMS:** (Method E) 440.1 ($M^+$+2), Rt. 2.57 min, 96.55% (Max).

**Intermediate 55**

**3-Butyl-8-hydroxy-7-methoxy-3-methyl-5-phenyl-2,3,4,5-tetrahydro-1,5-benzothiazepine 1,1-dioxide**

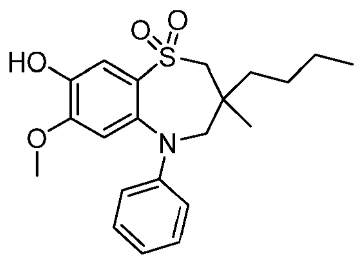

To a stirred solution of 7-bromo-3-butyl-8-hydroxy-3-methyl-5-phenyl-2,3,4,5-tetrahydro-1,5-benzothiazepine 1,1-dioxide (Intermediate 54; 2.1 g, 4.79 mmol) in sodium methoxide (30% in methanol, 20 mL), copper(I) bromide (0.69 g, 4.79 mmol) was added at RT and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL), brine (15 mL) and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 85% (1.6 g, brown solid).

**LCMS:** (Method A) 390.1 ($M^+$+H), Rt. 2.94 min, 99.05% (Max).

**Intermediate 56**

**3-Butyl-7-methoxy-3-methyl-1,1-dioxido-5-phenyl-2,3,4,5-tetrahydro-1,5-benzothiazepin-8-yl trifluoromethanesulfonate**

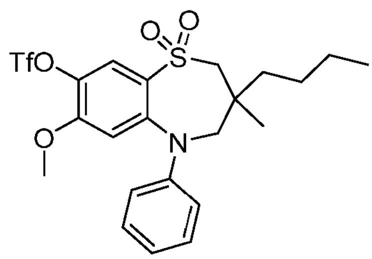

To a stirred solution of 3-butyl-8-hydroxy-7-methoxy-3-methyl-5-phenyl-2,3,4,5-tetrahydro-1,5-benzothiazepine 1,1-dioxide (Intermediate 55; 1.3 g, 3.34 mmol) in DCM (10 mL) at 0 °C, pyridine (0.26 mL, 3.34 mmol) and then trifluoromethanesulfonic anhydride (0.55 mL, 3.34 mmol) were added dropwise and the reaction mixture was stirred for 1 hour at RT. After completion of the reaction (monitored by TLC), the reaction mixture was diluted with water (20 mL) and the aqueous layer was extracted with DCM (2 x 50 mL). The combined organic layer was washed with water (10 mL), brine (10 mL) and dried over anhydrous $Na_2SO_4$. The organic part was filtered, concentrated under vacuum and the resulting crude material was purified by Isolera column chromatography (eluent: 20% EtOAc/PE; silica gel: 230-400 mesh). The obtained sticky solid was triturated with petroleum ether (2 x 10 mL) and dried under vacuum to afford the title compound. **Yield:** 73.9% (1.3 g, off-white solid).

**$^1$H NMR** (400 MHz, $CDCl_3$): δ 7.87 (s, 1H), 7.41-7.37 (m, 2H), 7.20-7.14 (m, 3H), 6.33 (s, 1H), 3.97-3.96 (m,1H), 3.79-3.76 (m, 1H), 3.66 (s, 3H), 3.28-3.18 (m, 2H), 1.56-1.52 (m, 1H),1.49-1.39 (m, 1H), 1.38-1.32 (m, 2H),1.27-1.22 (m, 5H), 0.87-0.79 (m, 3H). **LCMS:** (Method E) 522.2 ($M^+$+H), Rt. 2.77 min, 99.09% (Max).

**Intermediate 57**

**Methyl 3-butyl-7-methoxy-3-methyl-5-phenyl-2,3,4,5-tetrahydro-1,5-benzothiazepine-8-carboxylate 1,1-dioxide**

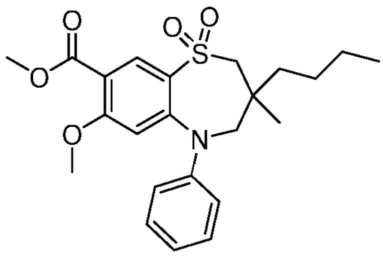

To a solution of 3-butyl-7-methoxy-3-methyl-1,1-dioxido-5-phenyl-2,3,4,5-tetrahydro-1,5-benzothiazepin-8-yl trifluoromethanesulfonate (Intermediate 56; 1.30 g, 2.49 mmol) in degassed methanol (15 mL) and DMF (10 mL) at RT, triethylamine (0.51 mL, 3.74 mmol), dppf (0.17 g, 0.29 mmol) and $Pd_2(dba)_3$ (0.11 g, 0.13 mmol) were added. The resulting reaction mixture was stirred for 16 hours at 75°C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL) and dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 64.24% (0.7 g, off-white solid).

**$^1$H NMR** (400 MHz, $CDCl_3$): δ 8.58 (s, 1H), 7.44-7.41 (m, 2H), 7.25-7.20 (m, 3H), 6.12 (s, 1H), 4.07-4.04 (m,1H), 3.91 (s, 1H), 3.86 (s, 3H), 3.59 (s, 3H), 3.37-3.34 (m, 1H), 3.26-3.22 (m, 1H), 1.62-1.52 (m, 1H),1.38-1.36 (m, 1H), 1.22-1.04 (m, 7H), 0.80-0.77 (m, 3H). **LCMS:** (Method A) 432.1 ($M^+$+H), Rt. 2.72 min, 99.28 % (Max).

**Intermediate 58**

**3-Butyl-8-(hydroxymethyl)-7-methoxy-3-methyl-5-phenyl-2,3,4,5-tetrahydro-1,5-benzothiazepine 1,1-dioxide**

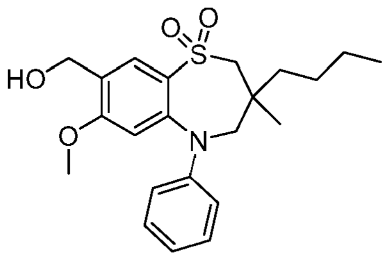

To a stirred solution of methyl 3-butyl-7-methoxy-3-methyl-5-phenyl-2,3,4,5-tetrahydro-1,5-benzothiazepine-8-carboxylate 1,1-dioxide (Intermediate 57; 0.7 g, 1.62 mmol) in THF (10 mL) at 0 °C, lithium aluminium hydride (2M in THF,

0.81 mL, 1.62 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 25 mL). The combined organic layer was washed with water (15 mL), brine (15 mL) and dried over anhydrous $Na_2SO_4$. The organic part was filtered, concentrated under vacuum and the resulting crude was forwarded to the next step without any further purification. **Yield:** 600 mg (crude, off-white solid).

**$^1$H NMR** (400 MHz, $CDCl_3$): δ 7.94 (s, 1H), 7.34-7.30 (m, 2H), 7.29-7.28 (m, 2H), 7.14-7.03 (m, 1H), 6.36 (s, 1H), 4.66 (s, 2H), 3.87-3.78 (m, 1H), 3.67 (s, 4H), 3.26-3.22 (m, 1H), 3.26-3.22 (m, 1H), 1.45-1.30 (m, 6H), 1.28-1.12 (m, 3H), 0.84-0.81 (m, 3H). **LCMS:** (Method A) 404.1($M^+$+H), Rt. 2.44 min, 99.91% (Max).

**Intermediate 59**

**Ethyl 2-(((3-butyl-7-methoxy-3-methyl-1,1-dioxido-5-phenyl-2,3,4,5-tetrahydro-1,5-benzothiazepin-8-yl)methyl)thio)acetate**

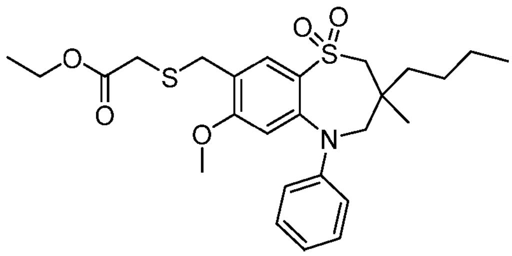

To a stirred solution of 3-butyl-8-(hydroxymethyl)-7-methoxy-3-methyl-5-phenyl-2,3,4,5-tetrahydro-1,5-benzothiazepine 1,1-dioxide (Intermediate 58; 0.3 g, 0.74 mmol) in DCM (10 mL) at 0 °C, ethyl 2-mercaptoacetate (0.18 g, 1.49 mmol) and zinc (II) iodide (0.12 g, 0.37 mmol) were added and the reaction mixture was stirred for 1 hour at RT. After completion of the reaction (monitored by TLC), the reaction mixture was quenched with water (20 mL) and the aqueous layer was extracted with DCM (2 x 20 mL). The combined organic part was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 66.5% (0.25 g, white solid).

**Intermediate** 60

7-Bromo-3-butyl-5-(4-fluorophenyl)-8-hydroxy-2-methyl-2,3,4,5-tetrahydro-1,2,5-benzothiadiazepine 1,1-dioxide

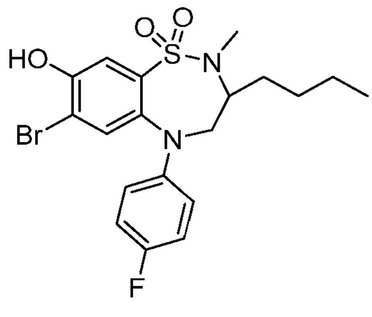

To a stirred solution of 7-bromo-3-butyl-5-(4-fluorophenyl)-8-methoxy-2-methyl-2,3,4,5-tetrahydro-1,2,5-benzothiadiazepine 1,1-dioxide (2.0 g, 4.24 mmol) in DCM (20 mL) at -15 °C, $BBr_3$ (1M in DCM, 4.24 mL, 4.24 mmol) was added dropwise and the reaction mixture was stirred for 10 minutes at that temperature. After completion of the reaction (monitored by TLC), the reaction mixture was quenched with methanol (15 mL) and the reaction mixture was concentrated under vacuum. The resulting crude material was purified by Isolera column chromatography (eluent: 0-30% EtOAc/PE; silica gel: 230-400 mesh) to afford the title compound. **Yield:** 80% (1.6 g, brown solid).

**$^1$H NMR** (400 MHz, DMSO-ds): δ 11.09 (s,1H), 7.50 (s, 1H), 7.45 (s, 1H), 7.01 (t, J = 8.8 Hz, 2H), 6.59-6.56 (m,

2H), 4.04-3.97 (m, 1H), 3.86-3.82 (m, 1H), 3.25-3.22 (m, 1H), 2.46 (s, 3H), 1.49-1.46 (m, 1H), 1.30-1.32 (m, 5H), 0.92-0.90 (m, 3H). **LCMS:** (Method A) 455 ($M^+$-2), Rt. 2.67 min, 97.17%(Max).

**Intermediate 61**

**3-Butyl-5-(4-fluorophenyl)-8-hydroxy-7-methoxy-2-methyl-2,3,4,5-tetrahydro-1,2,5-benzothiadiazepine 1,1-dioxide**

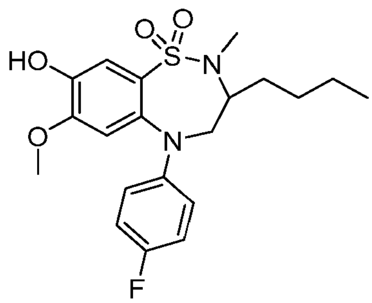

To a stirred solution of 7-bromo-3-butyl-5-(4-fluorophenyl)-8-hydroxy-2-methyl-2,3,4,5-tetrahydro-1,2,5-benzothiadiazepine 1,1-dioxide (Intermediate 60; 1.6 g, 3.50 mmol) in sodium methoxide (30% solution in methanol, 10 mL), copper(I) bromide (0.50 g, 3.50 mmol) was added at RT and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL), brine (15 mL), and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 85% (1.4 g, brown solid).

**$^1$H NMR** (400 MHz, DMSO-ds): δ 9.87 (s,1H), 7.26 (s, 1H), 6.99-6.95 (m, 2H), 6.88 (s, 1H), 6.56-6.53 (m, 2H), 4.06-3.97 (m, 1H), 3.92-3.86 (m, 1H), 3.33 (s, 3H), 3.21-3.16 (m, 1H), 2.40 (s, 3H), 1.6-1.56 (m, 1H), 1.48-1.45 (m,1H), 1.34-1.29 (m, 4H), 0.92-0.89 (m, 3H). **LCMS:** (Method K) 409.2 ($M^+$+H), Rt. 2.27 min, 86.65% (Max).

**Intermediate 62**

**3-Butyl-5-(4-fluorophenyl)-7-methoxy-2-methyl-1,1-dioxido-2,3,4,5-tetrahydro-1,2,5-benzothiadiazepin-8-yl trifluoromethanesulfonate**

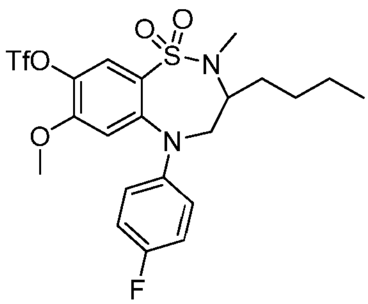

To a stirred solution of 3-butyl-5-(4-fluorophenyl)-8-hydroxy-7-methoxy-2-methyl-2,3,4,5-tetrahydro-1,2,5-benzothiadiazepine 1,1-dioxide (Intermediate 61; 1.4 g, 3.43 mmol) in DCM (15 mL) at 0 °C, pyridine (0.55 mL, 6.85 mmol) and trifluoromethanesulfonic anhydride (0.86 mL, 5.14 mmol) were added dropwise and the reaction mixture was stirred for 1 hour at RT. After completion of the reaction (monitored by TLC), the reaction mixture was quenched with ice-cold water (20 mL) and the aqueous layer was extracted with DCM (2 x 50 mL). The combined organic layer was washed with water (10 mL), brine (10 mL), and dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford sticky solid. This sticky solid was triturated with petroleum ether (2 x 10 mL) and dried under vacuum to afford the

title compound. **Yield:** 76% (1.5 g, off-white solid).

**$^1$H NMR** (400 MHz, DMSO-ds): δ 7.78 (s,1H), 7.13-7.17 (m, 3H), 6.92-6.89 (m, 2H), 4.08-4.02 (m, 1H), 3.83 (s, 3H), 3.73-3.71 (m, 1H), 3.59-3.51 (m, 1H), 2.68 (s, 3H), 1.56-1.55 (m, 2H), 1.37-1.33 (m, 4H), 0.91-0.88 (m, 3H). **LCMS:** (Method E) 541.2 ($M^+$+H), Rt. 2.74 min, 94.32% (Max).

Intermediate 63

Methyl 3-butyl-5-(4-fluorophenyl)-7-methoxy-2-methyl-2,3,4,5-tetrahydro-1,2,5-benzothiadiazepine-8-carboxylate 1,1-dioxide

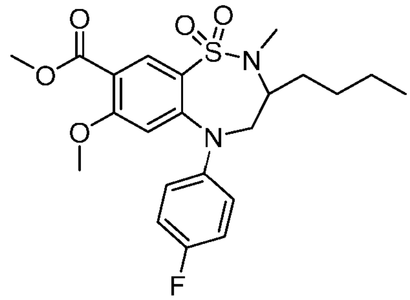

To a solution of 3-butyl-5-(4-fluorophenyl)-7-methoxy-2-methyl-1,1-dioxido-2,3,4,5-tetrahydro-1,2,5-benzothiadiazepin-8-yl trifluoromethanesulfonate (Intermediate 62; 1.5 g, 2.77 mmol) in degassed methanol (15 mL) and DMF (10 mL) at RT, triethylamine (0.58 mL, 4.16 mmol), dppf (0.18 g, 0.33 mmol) and $Pd_2(dba)_3$ (0.15 g, 0.16 mmol) were added. The resulting reaction mixture was stirred for 16 hours at 75°C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL), and dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 86% (1.1 g, off-white solid).

**$^1$H NMR** (400 MHz, DMSO-ds): δ 8.15 (s,1H), 7.18-7.06 (m, 4H), 6.60 (s, 1H), 4.05-4.00 (m, 1H), 3.79 (s, 4H), 3.66 (s, 4H), 2.68 (s, 3H), 1.57-1.53 (m, 2H), 1.38-1.26 (m, 4H), 0.91-0.87(m, 3H). **LCMS:** (Method A) 451.1 ($M^+$+H), Rt. 3.08 min, 97.59% (Max).

**Intermediate 64**

**3-Butyl-5-(4-fluorophenyl)-8-(hydroxymethyl)-7-methoxy-2-methyl-2,3,4,5-tetrahydro-1,2,5-benzothiadiazepine 1,1-dioxide**

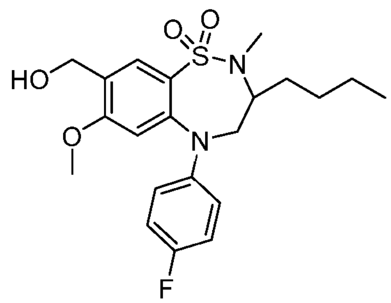

To a stirred solution of methyl 3-butyl-5-(4-fluorophenyl)-7-methoxy-2-methyl-2,3,4,5-tetrahydro-1,2,5-benzothiadiazepine-8-carboxylate 1,1-dioxide (Intermediate 63; 1.1 g, 2.44 mmol) in THF (10 mL) at 0 °C, lithium aluminium hydride (1M in THF, 2.44 mL, 2.44 mmol) was added and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 15 mL). The combined organic layer was washed

with water (10 mL), brine (10 mL), and dried over anhydrous $Na_2SO_4$. The organic part was filtered, concentrated under vacuum and the resulting crude was forwarded to the next step without any further purification. **Yield:** 87% (0.9 g, off-white solid).

**$^1$H NMR** (400 MHz, DMSO-ds): δ 7.89 (s, 1H), 7.02 (t, J = 8.8 Hz, 2H), 6.85 (s, 1H), 6.70-6.67 (m, 2H), 5.33-5.30 (m, 1H), 4.53-4.50 (m, 2H), 4.04-3.99 (m, 1H), 3.77(s, 4H), 3.34-3.32 (m, 1H), 2.45 (s, 3H), 1.57-1.56 (m, 2H), 1.35-1.31 (m, 4H), 0.92-0.89 (m, 3H).

**Intermediate 65**

**Ethyl 2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetate**

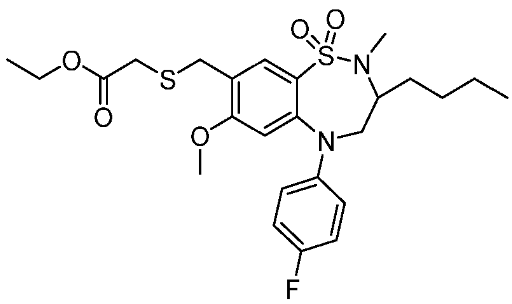

To a stirred solution of 3-butyl-5-(4-fluorophenyl)-8-(hydroxymethyl)-7-methoxy-2-methyl-2,3,4,5-tetrahydro-1,2,5-benzothiadiazepine 1,1-dioxide (Intermediate 64; 0.35 g, 0.83 mmol) in DCM (10 mL) at 0 °C, ethyl 2-mercaptoacetate (0.199 g, 1.66 mmol) and zinc iodide (0.13 g, 0.41 mmol) were added and the reaction mixture was stirred for 2 hours at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 15% EtOAc/PE; silica gel: 230-400 mesh) to afford the title compound. **Yield:** 79% (0.37 g, white gum).

**$^1$H NMR** (400 MHz, DMSO-ds): δ 7.74 (s, 1H), 7.03 (s, 2H), 6.88 (s, 1H), 6.73 (s, 2H), 4.12-4.00 (m, 3H), 3.83 (s, 2H), 3.77 (s, 3H), 3.38 (s, 2H), 2.51-2.47 (m, 3H), 1.54 (s, 2H), 1.34 (s, 3H), 1.23-1.21 (m, 4H), 0.91-0.93 (m, 3H).

**Intermediate 66**

**7-Bromo-3,3-dibutyl-8-hydroxy-5-phenyl-2,3,4,5-tetrahydro-1,5-benzothiazepine 1,1-dioxide**

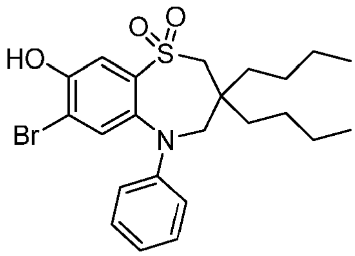

To a stirred solution of 7-bromo-3,3-dibutyl-8-methoxy-5-phenyl-2,3,4,5-tetrahydro-1,5-benzothiazepine 1,1-dioxide (3.3 g, 6.67 mmol) in DCM (5 mL) at -15 °C, $BBr_3$ (1M in DCM, 12.86 mL, 12.86 mmol) was added dropwise and the reaction mixture was stirred for 10 minutes. After completion of the reaction (monitored by TLC), the reaction mixture was quenched with methanol (10 mL) at 0 °C and concentrated under vacuum. The resulting crude material was purified by Isolera column chromatography (eluent: 0-30% EtOAc/PE; silica gel: 230-400 mesh) to afford the title compound. **Yield:** 86% (2.5 g, brown solid).

**LCMS:** (Method A) 480.0 ($M^+$+H), Rt. 3.16 min, 70.09% (Max).

**Intermediate 67**

**3,3-Dibutyl-8-hydroxy-7-methoxy-5-phenyl-2,3,4,5-tetrahydro-1,5-benzothiazepine 1,1-dioxide**

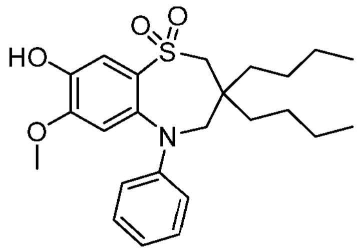

To a stirred solution of 7-bromo-3,3-dibutyl-8-hydroxy-5-phenyl-2,3,4,5-tetrahydro-1,5-benzothiazepine 1,1-dioxide (Intermediate 66; 2 g, 4.16 mmol) in sodium methoxide (28% in methanol, 20 mL) at RT, copper(I) bromide (0.59 g, 4.16 mmol) was added and the reaction mixture was heated for 6 hours at 85 °C. After completion of the reaction (monitored by TLC), the reaction mixture was quenched with water (10 mL) and the aqueous layer extracted with EtOAc (2 x 50 mL). The combined organic layer was washed with water (25 mL), brine (25 mL), and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 59.4% (1.2 g, brown solid).

**LCMS:** (Method A) 430.1 ($M^{+}$+H), Rt. 2.70 min, 89.19% (Max).

**Intermediate 68**

3,3-Dibutyl-7-methoxy-1,1-dioxido-5-phenyl-2,3,4,5-tetrahydro-1,5-benzothiazepin-8-yl **trifluoromethanesulfonate**

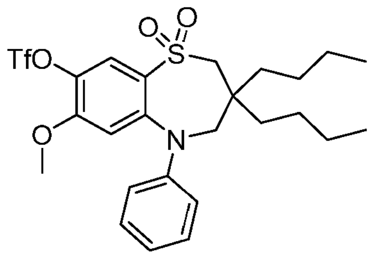

To a stirred solution of 3,3-dibutyl-8-hydroxy-7-methoxy-5-phenyl-2,3,4,5-tetrahydro-1,5-benzothiazepine 1,1-dioxide (Intermediate 67; 1.2 g, 2.78 mmol) in dry DCM (10 mL) at 0 °C, pyridine (0.11 mL, 1.39 mmol) and then trifluoromethanesulfonic anhydride (0.46 mL, 2.78 mmol) were added dropwise and the reaction mixture was stirred for 1 hour at RT. After completion of the reaction (monitored by TLC), the reaction mixture was diluted with water (10 mL) and the aqueous layer was extracted with DCM (2 x 50 mL). The combined organic layer was washed with water (5 mL), brine (5 mL), and dried over anhydrous $Na_2SO_4$. The organic part was filtered, concentrated under vacuum and the resulting crude material was purified by Isolera column chromatography (eluent: 20% EtOAc/PE; silica gel: 230-400 mesh). The obtained sticky solid of compound was triturated with petroleum ether (2 x 10 mL) and dried under vacuum to afford the title compound. **Yield:** 61% (1.1 g, off-white solid).

**LCMS:** (Method A) 564.1 ($M^{+}$+H), Rt. 3.31 min, 89.09% (Max).

**Intermediate 69**

**Methyl 3,3-dibutyl-7-methoxy-5-phenyl-2,3,4,5-tetrahydro-1,5-benzothiazepine-8-carboxylate 1,1-dioxide**

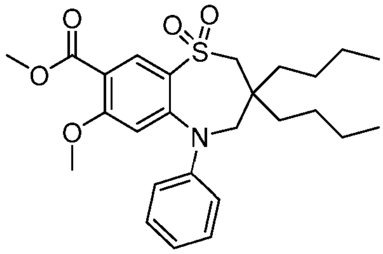

To a solution of 3,3-dibutyl-7-methoxy-1,1-dioxido-5-phenyl-2,3,4,5-tetrahydro-1,5-benzothiazepin-8-yl trifluoromethanesulfonate (Intermediate 68; 1.10 g, 1.95 mmol) in degassed methanol (10 mL) and DMF (15 mL) at RT, triethylamine (0.29 g, 2.93 mmol), dppf (0.13 g, 0.23 mmol) and $Pd_2(dba)_3$ (0.09 g, 0.09 mmol) were added. The resulting reaction mixture was stirred for 16 hours at 70°C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL), and dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 74.4% (0.8 g, off-white solid). **LCMS:** (Method A) 474.1 ($M^+$+H), Rt. 3.08 min, 86.01% (Max).

**Intermediate 70**

**3,3-Dibutyl-8-(hydroxymethyl)-7-methoxy-5-phenyl-2,3,4,5-tetrahydro-1,5-benzothiazepine 1,1-dioxide**

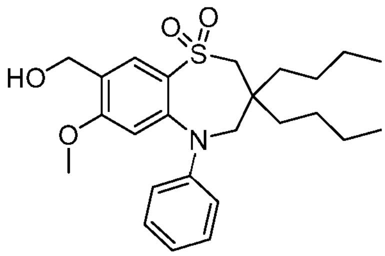

To a stirred solution of methyl 3,3-dibutyl-7-methoxy-5-phenyl-2,3,4,5-tetrahydro-1,5-benzothiazepine-8-carboxylate 1,1-dioxide (Intermediate 69; 0.8 g, 1.69 mmol) in THF (10 mL) at 0 °C, lithium aluminium hydride (2M in THF, 1.69 mL, 1.69 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 50 mL). The combined organic layer was washed with water (20 mL), brine (20 mL), and dried over anhydrous $Na_2SO_4$. The organic part was filtered, concentrated under vacuum and the resulting crude was forwarded to the next step without any further purification. **Yield:** 95% (0.75 g, off-white solid).

**LCMS:** (Method E) 446.3 ($M^+$+H), Rt. 2.66 min, 95.15% (Max).

**Intermediate 71**

**Ethyl 2-(((3,3-dibutyl-7-methoxy-1,1-dioxido-5-phenyl-2,3,4,5-tetrahydro-1,5-benzothiazepin-8-yl)methyl)thio) acetate**

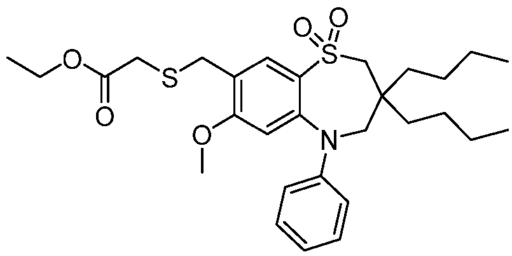

To a stirred solution of 3,3-dibutyl-8-(hydroxymethyl)-7-methoxy-5-phenyl-2,3,4,5-tetrahydro-1,5-benzothiazepine 1,1-dioxide (Intermediate 70; 0.2 g, 0.45 mmol) in DCM (5 mL) at 0 °C, ethyl 2-mercaptoacetate (0.108 g, 0.89 mmol) and zinc (II) iodide (0.07 g, 0.22 mmol) were added and the reaction mixture was stirred for 1 hour at RT. After completion of the reaction (monitored by TLC), the reaction mixture was quenched with water (15 mL) and the aqueous layer was extracted with DCM (2 x 20 mL). The combined organic layer was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 61% (0.15 g, white solid).

**Intermediate 72**

**7-Bromo-3,3-dibutyl-5-(4-fluorophenyl)-8-hydroxy-2,3,4,5-tetrahydro-1,5-benzothiazepine 1,1-dioxide**

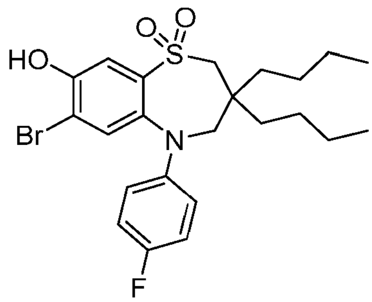

To a stirred solution of 7-bromo-3,3-dibutyl-5-(4-fluorophenyl)-8-methoxy-2,3,4,5-tetrahydro-1,5-benzothiazepine 1,1-dioxide (2 g, 3.90 mmol) in DCM (20 mL) at 0 °C, $BBr_3$ (1M solution in DCM, 3.90 mL, 3.90 mmol) was added dropwise at -15 °C and the reaction mixture was stirred for 10 minutes. After completion of the reaction (monitored by TLC), the reaction mixture was quenched with methanol (20 mL) and the organic part was concentrated under vacuum. The resulting solid was partitioned between water (5 mL) and EtOAc (5 mL) and the aqueous layer was extracted with EtOAc (2 x 15 mL). The combined organic layer was washed with water (10 mL) and with brine (15 mL) and then dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford the title compound. **Yield:** 87% (1.69 g, white solid).

**LCMS:** (Method A) 496.1 ($M^+$-H), Rt. 3.07 min, 94.28% (Max).

**Intermediate 73**

**3,3-Dibutyl-5-(4-fluorophenyl)-8-hydroxy-7-methoxy-2,3,4,5-tetrahydro-1,5-benzothiazepine 1,1-dioxide**

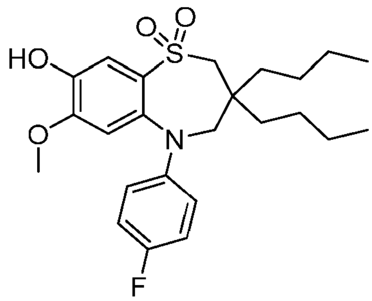

To a stirred solution of 7-bromo-3,3-dibutyl-5-(4-fluorophenyl)-8-hydroxy-2,3,4,5-tetrahydro-1,5-benzothiazepine 1,1-dioxide (Intermediate 72; 1.69 g, 3.39 mmol) in sodium methoxide (28% in methanol, 20 mL), copper(I) bromide (0.59 g, 4.16 mmol) was added at room temperature and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL) and with brine (15 mL) and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound.

**Yield:** 97% (1.5 g, brown solid). **LCMS:** (Method A) 450.2 ($M^+$+H), Rt. 3.26 min, 98.43% (Max).

**Intermediate 74**

**3,3-Dibutyl-5-(4-fluorophenyl)-7-methoxy-1,1-dioxido-2,3,4,5-tetrahydro-1,5-benzothiazepin-8-yl trifluoromethanesulfonate**

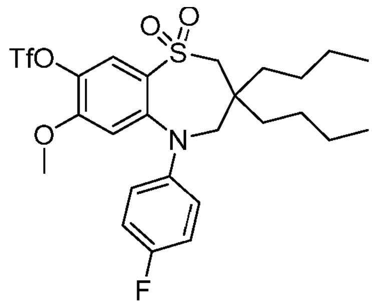

To a stirred solution of of 3,3-dibutyl-5-(4-fluorophenyl)-8-hydroxy-7-methoxy-2,3,4,5-tetrahydro-1,5-benzothiazepine 1,1-dioxide (Intermediate 73; 1.5 g, 3.34 mmol) in DCM (10 mL) at 0 °C, pyridine (0.27 mL, 3.34 mmol) and then trifluoromethanesulfonic anhydride (0.56 mL, 0.33 mmol) were added dropwise and the reaction mixture was stirred for 30 minutes at RT. After completion of the reaction (monitored by TLC), the reaction mixture was diluted with water (5 mL) and the aqueous layer was extracted with DCM (2 x 20 mL). The combined organic layer was washed with water (20 mL), brine (20 mL), and dried over anhydrous $Na_2SO_4$. The organic part was filtered, concentrated under vacuum and the resulting crude material was purified by Isolera column chromatography (eluent: 8-10% EtOAc/PE; silica gel: 230-400 mesh) to afford the title compound. **Yield:** 77% (1.5 g, off-white solid).

**LCMS:** (Method G) 582.0 ($M^+$+H), Rt. 2.76 min, 99.01% (Max).

Intermediate 75

Methyl 3,3-dibutyl-5-(4-fluorophenyl)-7-methoxy-2,3,4,5-tetrahydro-1,5-benzothiazepine-8-carboxylate 1,1-dioxide

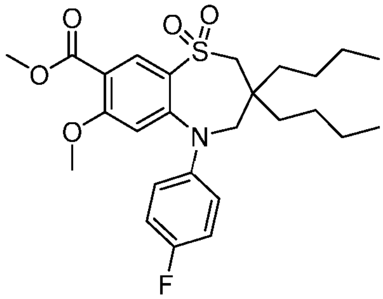

To a solution of 3,3-dibutyl-5-(4-fluorophenyl)-7-methoxy-1,1-dioxido-2,3,4,5-tetrahydro-1,5-benzothiazepin-8-yl trifluoromethanesulfonate (Intermediate 74; 1.50 g, 2.58 mmol) in degassed methanol (15 mL) and DMF (10 mL) at RT, triethylamine (0.75 mL, 5.42 mmol), dppf (0.24 g, 0.43 mmol) and $Pd_2(dba)_3$ (0.16 g, 0.18 mmol) were added. The resulting reaction mixture was stirred for 16 hours at 75 °C in an autoclave, under 5 kg carbon monoxide gas pressure. After completion of the reaction (monitored by TLC), the reaction mixture was diluted with water (20 mL) and the aqueous layer was extracted with EtOAc (2 x 20 mL). The combined organic layer was washed with water (10 mL), brine (10 mL) and dried over anhydrous $Na_2SO_4$. The organic layer was filtered, concentrated under vacuum and the resulting crude material was purified by Isolera column chromatography (eluent: 10-15% EtOAc/PE; silica gel: 230-400 mesh) to afford the title compound. **Yield:** 77% (1 g, white solid).

**$^1$H NMR** (400 MHz, DMSO-$d_6$): δ 8.25 (s, 1H), 7.54-7.51 (m, 2H), 730-7.26 (m, 2H), 6.03 (s, 1H), 3.95-3.90 (m, 2H), 3.76 (s, 3H), 3.50 (s, 5H), 1.45-1.42 (m, 2H), 1.33-1.26 (m, 2H), 1.13-1.08 (m, 4H), 0.99-0.92 (m, 4H), 0.75-0.72 (m, 6H). **LCMS:** (Method A) 492.2 ($M^+$+H), Rt. 3.05 min, 97.78% (Max).

**Intermediate 76**

**3,3-Dibutyl-5-(4-fluorophenyl)-8-(hydroxymethyl)-7-methoxy-2,3,4,5-tetrahydro-1,5-benzothiazepine 1,1-dioxide**

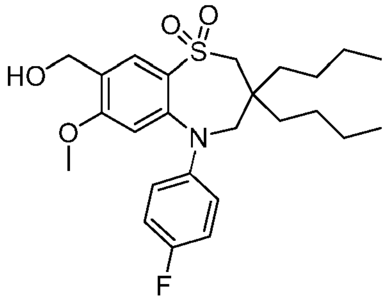

To a stirred solution of methyl 3,3-dibutyl-5-(4-fluorophenyl)-7-methoxy-2,3,4,5-tetrahydro-1,5-benzothiazepine-8-carboxylate 1,1-dioxide (Intermediate 75; 1.5 g, 3.05 mmol) in THF (10 mL) at 0 °C, lithium aluminium hydride (2M in THF, 1.53 mL, 3.05 mmol) was added dropwise and the reaction mixture was stirred for 30 minutes at RT. After completion of the reaction (monitored by TLC), the reaction mixture was quenched with saturated aqueous ammonium chloride solution (5 mL) and the aqueous layer was extracted with EtOAc (3 x 20 mL). The combined organic layer was washed with water (15 mL), brine (15 mL) and dried over anhydrous $Na_2SO_4$. The organic part was filtered, concentrated under vacuum and the resulting crude was purified by Isolera column chromatography (eluent: 25% EtOAc/PE; silica gel: 230-400 mesh) to afford the title compound. **Yield:** 71% (1 g, off-white solid).

**Intermediate 77**

**Ethyl 2-(((3,3-dibutyl-5-(4-fluorophenyl)-7-methoxy-1,1-dioxido-2,3,4,5-tetrahydro-1,5-benzothiazepin-8-yl) methyl)thio)acetate**

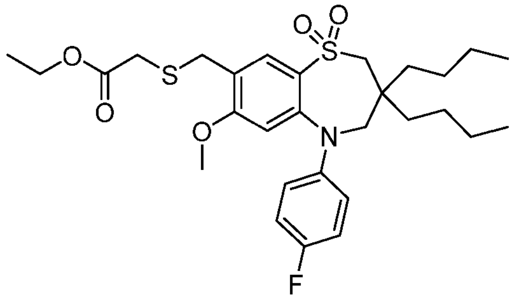

To a stirred solution of 3,3-dibutyl-5-(4-fluorophenyl)-8-(hydroxymethyl)-7-methoxy-2,3,4,5-tetrahydro-1,5-benzothiazepine 1,1-dioxide (Intermediate 76; 100 mg, 0.22 mmol) in DCM (10 mL) at 0 °C, ethyl 2-mercaptoacetate (25.9 mg, 0.22 mmol) and zinc iodide (68.8 mg, 0.22 mmol) were added and the reaction mixture was stirred for 1 hour at RT. After completion of the reaction (monitored by TLC), the reaction mixture was quenched with water (5 mL) and the aqueous layer was extracted with DCM (2 x 10 mL). The combined organic layer was washed with brine (10 mL), dried over anhydrous $Na_2SO_4$, filtered and concentrated under vacuum. The resulting crude material was purified by Isolera column chromatography (eluent: 0-30% EtOAc/PE; silica gel: 230-400 mesh) to afford the title compound. **Yield:** 82% (0.1 g, brown gum).

Intermediate 78

7-Bromo-3-butyl-3-ethyl-5-(4-fluorophenyl)-8-hydroxy-2,3,4,5-tetrahydro-1,5-benzothiazepine 1,1-dioxide

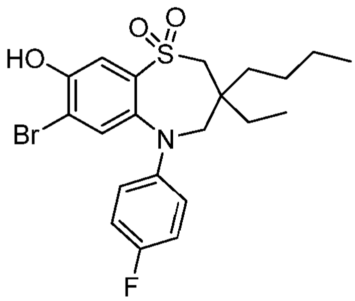

To a stirred solution of 7-bromo-3-butyl-3-ethyl-5-(4-fluorophenyl)-8-methoxy-2,3,4,5-tetrahydro-1,5-benzothiazepine 1,1-dioxide (3 g, 6.19 mmol) in DCM (20 mL) at -15 °C, $BBr_3$ (1M in DCM, 12.39 mL, 12.39 mmol) was added dropwise and the reaction mixture was stirred for 10 minutes. After completion of the reaction (monitored by TLC), the reaction mixture was quenched with methanol (15 mL) and the reaction mixture was concentrated under vacuum. The resulting crude material was purified by Isolera column chromatography (eluent: 0-30 % EtOAc/PE; silica gel: 230-400 mesh) to afford the title compound. **Yield:** 82% (2.38 g, brown solid).

$^1$**H NMR** (400 MHz, DMSO-ds): $\delta$ 10.83 (s, 1H), 7.59 (s, 1H), 7.12-7.05 (m, 5H), 3.65-3.35 (m, 2H), 3.25 (s, 2H), 1.47-1.45 (m, 1H), 1.37-1.33 (m, 3H), 1.18-1.01 (m, 4H), 0.77-0.71 (m, 6H). **LCMS:** (Method K) 471.1 ($M^+$+H), Rt. 2.46 min, 97.17% (Max).

**Intermediate 79**

**3-Butyl-3-ethyl-5-(4-fluorophenyl)-8-hydroxy-7-methoxy-2,3,4,5-tetrahydro-1,5-benzothiazepine 1,1-dioxide**

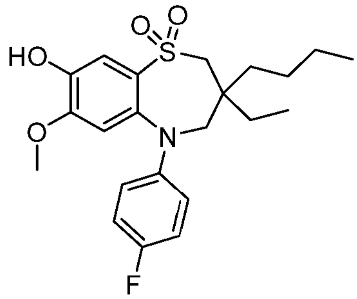

To a stirred solution of 7-bromo-3-butyl-3-ethyl-5-(4-fluorophenyl)-8-hydroxy-2,3,4,5-tetrahydro-1,5-benzothiazepine 1,1-dioxide (Intermediate 78; 2.3 g, 4.89 mmol) in sodium methoxide (30% solution in methanol, 20 mL) at RT, copper(I) bromide (0.70 g, 4.89 mmol) was added and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL), brine (15 mL), and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 95% (2.0 g, brown solid).

**LCMS:** (Method E) 422.3 ($M^+$+H), Rt. 2.45 min, 98.53% (Max).

**Intermediate 80**

**3-Butyl-3-ethyl-5-(4-fluorophenyl)-7-methoxy-1,1-dioxido-2,3,4,5-tetrahydro-1,5-benzothiazepin-8-yl trifluoromethanesulfonate**

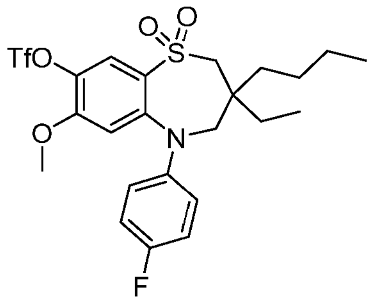

To a stirred solution of 3-butyl-3-ethyl-5-(4-fluorophenyl)-8-hydroxy-7-methoxy-2,3,4,5-tetrahydro-1,5-benzothiazepine 1,1-dioxide (Intermediate 79; 2 g, 4.74 mmol) in dry DCM (15 mL) at 0 °C, pyridine (0.37 mL, 4.74 mmol) and then trifluoromethanesulfonic anhydride (0.8 mL, 4.74 mmol) were added dropwise and the reaction mixture was stirred for 1 hour at RT. After completion of the reaction (monitored by TLC), the reaction mixture was diluted with water (20 mL) and the aqueous layer was extracted with DCM (2 x 50 mL). The combined organic layer was washed with water (10 mL), brine (10 mL) and dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford sticky solid. The obtained sticky solid was triturated with petroleum ether (2 x 10 mL) and dried under vacuum to afford the title compound. **Yield:** 74.6% (2 g, off-white solid).

**LCMS:** (Method E) 554.2 ($M^+$+H), Rt. 2.82 min, 98.24% (Max).

**Intermediate 81**

**Methyl 3-butyl-3-ethyl-5-(4-fluorophenyl)-7-methoxy-2,3,4,5-tetrahydro-1,5-benzothiazepine-8-carboxylate 1,1-dioxide**

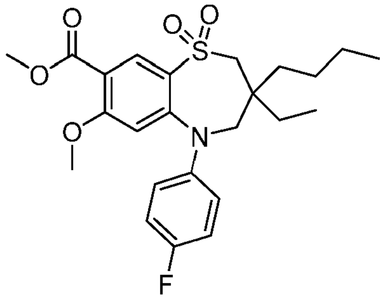

To a solution of 3-butyl-3-ethyl-5-(4-fluorophenyl)-7-methoxy-1,1-dioxido-2,3,4,5-tetrahydro-1,5-benzothiazepin-8-yl trifluoromethanesulfonate (Intermediate 80; 2 g, 3.61 mmol) in degassed methanol (15 mL) and DMF (10 mL) at RT, triethylamine (0.75 mL, 5.42 mmol), dppf (0.24 g, 0.43 mmol) and $Pd_2(dba)_3$ (0.17 g, 0.18 mmol) were added.The resulting reaction mixture was stirred for 16 hours at 75°C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), brine (15 mL), and dried over anhydrous $Na_2SO_4$. The organic part was filtered, concentrated under vacuum and the resulting crude material was purified by Isolera column chromatography (eluent: 15% EtOAc/PE; silica gel: 230-400 mesh) to afford the title compound. **Yield:** 90% (1.5 g, off-white solid).

**$^1$H NMR** (400 MHz, $CDCl_3$): δ 8.58 (s, 1H), 7.28-7.27 (m, 2H), 7.24-7.13 (m, 2H), 5.99 (s, 1H), 3.94 (s, 2H), 3.87 (s, 3H), 3.59 (s, 3H), 3.29 (s, 2H), 1.68-1.65 (m, 1H), 1.59-1.50 (m, 3H), 1.27-1.11 (m, 4H), 0.84-0.73 (m, 6H). **LCMS:** (Method A) 464.2 ($M^+$+H), Rt. 2.78 min, 98.72% (Max).

**Intermediate 82**

**3-Butyl-3-ethyl-5-(4-fluorophenyl)-8-(hydroxymethyl)-7-methoxy-2,3,4,5-tetrahydro-1,5-benzothiazepine 1,1-dioxide**

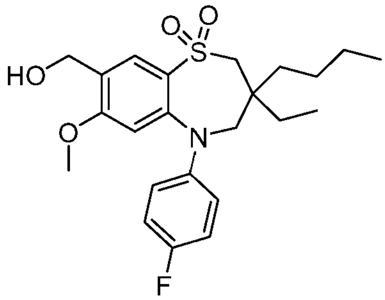

To a stirred solution of methyl 3-butyl-3-ethyl-5-(4-fluorophenyl)-7-methoxy-2,3,4,5-tetrahydro-1,5-benzothiazepine-8-carboxylate 1,1-dioxide (Intermediate 81; 1.5 g, 3.24 mmol) in THF (10 mL) at 0 °C, lithium aluminium hydride (2M in THF, 1.62 mL, 3.24 mmol) was added dropwise and the reaction mixture was stirred for 1 hour at RT. After completion of the reaction (monitored by TLC), the reaction mixture was quenched with saturated aqueous ammonium chloride solution (10 mL) and the aqueous layer was extracted with EtOAc (2 x 15 mL). The combined organic layer was washed with water (10 mL), brine (10 mL), and dried over anhydrous $Na_2SO_4$. The organic part was filtered, concentrated under vacuum and the resulting crude material was forwarded to the next step without any further purification. **Yield:** 98% (1.4 g, off-white solid).

**$^1$H NMR** (400 MHz, $CDCl_3$): δ 7.94 (s, 1H), 7.29-7.27 (m, 2H), 7.19-7.15 (m, 2H), 6.17 (s, 1H), 4.64 (s, 2H), 3.78-3.74 (m, 2H), 3.63 (s, 3H), 3.21-3.20 (s, 2H), 1.66-1.61 (m, 2H), 1.50-1.41 (m, 2H), 1.30-1.09 (m, 4H), 0.83-0.76 (m, 6H).

**Intermediate 83**

**Methyl 2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoate**

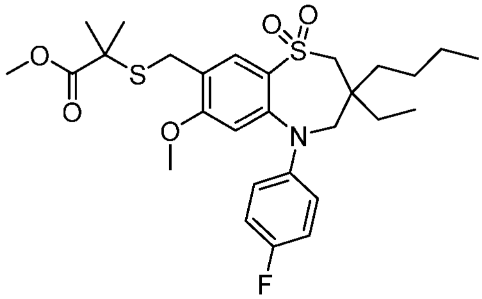

To a stirred solution of 3-butyl-3-ethyl-5-(4-fluorophenyl)-8-(hydroxymethyl)-7-methoxy-2,3,4,5-tetrahydro-1,5-benzothiazepine 1,1-dioxide (Intermediate 82; 500 mg, 1.15 mmol) in DCM (5 mL) at 0 °C, methyl 2-mercapto-2-methylpropanoate (308 mg, 2.29 mmol) and zinc (II) iodide (183 mg, 0.57 mmol) were added and the reaction mixture was stirred for 1 hour at RT. After completion of the reaction (monitored by TLC), the reaction mixture was quenched with water (15 mL) and the aqueous layer was extracted with DCM (2 x 20 mL). The combined organic layer was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 79% (0.5 g, white solid).

**Intermediate 84**

**7-Bromo-3-butyl-8-hydroxy-2-methyl-5-phenyl-2,3,4,5-tetrahydro-1,2,5-benzothiadiazepine 1,1-dioxide**

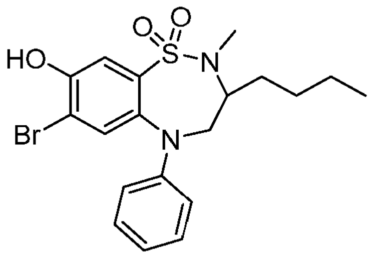

To a stirred solution of 7-bromo-3-butyl-8-methoxy-2-methyl-5-phenyl-2,3,4,5-tetrahydro-1,2,5-benzothiadiazepine 1,1-dioxide (2.8 g, 6.18 mmol) in DCM (20 mL) at -15 °C, $BBr_3$ (1M in DCM, 1.17 mL, 12.35 mmol) was added dropwise and the reaction mixture was stirred for 10 minutes. After completion of the reaction (monitored by TLC), the reaction mixture was quenched with methanol (10 mL) at 0 °C and concentrated under vacuum. The resulting crude material was purified by Isolera column chromatography (eluent: 0-30% EtOAc/PE; silica gel: 230-400 mesh) to afford the title compound. **Yield:** 75% (2.1 g, brown solid.

**LCMS:** (Method A) 439.0 ($M^+$), Rt. 2.50 min, 97.55% (Max).

**Intermediate 85**

**3-Butyl-8-hydroxy-7-methoxy-2-methyl-5-phenyl-2,3,4,5-tetrahydro-1,2,5-benzothiadiazepine 1,1-dioxide**

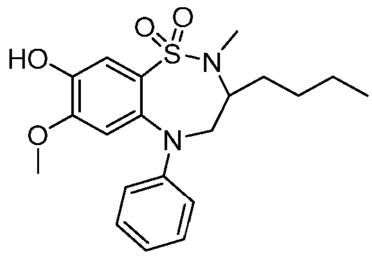

To a stirred solution of 7-bromo-3-butyl-8-hydroxy-2-methyl-5-phenyl-2,3,4,5-tetrahydro-1,2,5-benzothiadiazepine 1,1-dioxide (Intermediate 84; 2.1 g, 4.78 mmol) in sodium methoxide (30% in methanol, 20 mL) at RT, copper(I) bromide (0.69 g, 4.78 mmol) was added and the reaction mixture was heated for 6 hours at 85 °C. After completion of the reaction (monitored by TLC), the reaction mixture was quenched with water (10 mL) and the aqueous layer was extracted with EtOAc (2 x 25 mL). The combined organic layer was washed with water (15 mL) and brine (15 mL) and dried over anhydrous $Na_2SO_4$. The organic layer was filtered, concentrated under vacuum and the resulting crude material was purified by Isolera column chromatography (eluent: 0-30% EtOAc/PE; silica gel: 230-400 mesh) to afford the title compound. **Yield:** 79% (1.45 g, brown solid).

**LCMS:** (Method A) 391.2 ($M^+$+H), Rt. 2.46 min, 98.62% (Max).

**Intermediate 86**

**3-Butyl-7-methoxy-2-methyl-1,1-dioxido-5-phenyl-2,3,4,5-tetrahydro-1,2,5-benzothiadiazepin-8-yl trifluoromethanesulfonate**

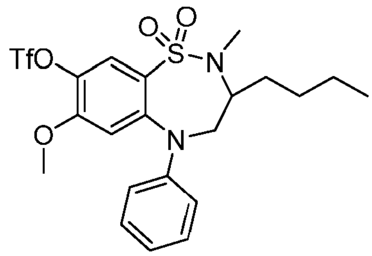

To a stirred solution of 3-butyl-8-hydroxy-7-methoxy-2-methyl-5-phenyl-2,3,4,5-tetrahydro-1,2,5-benzothiadiazepine 1,1-dioxide (Intermediate 85; 1.5 g, 3.84 mmol) in DCM (10 mL) at 0 °C, pyridine (0.30 mL, 3.84 mmol) and then trifluoromethanesulfonic anhydride (0.64 mL, 3.84 mmol) were added dropwise and the reaction mixture was stirred for 1 hour at RT. After completion of the reaction (monitored by TLC), the reaction mixture was diluted with water (20 mL) and the aqueous layer was extracted with DCM (2 x 50 mL). The combined organic layer was washed with water (10 mL), brine (10 mL) and dried over anhydrous $Na_2SO_4$. The organic part was filtered, concentrated under vacuum and the resulting crude material was purified by Isolera column chromatography (eluent: 20% EtOAc/PE; silica gel: 230-400 mesh) to afford a sticky solid. The obtained solid was triturated with petroleum ether (2 x 10 mL) and dried under vacuum to afford the title compound. **Yield:** 74.6% (1.5 g, off-white solid).

**$^1$H NMR** (400 MHz, $CDCl_3$): δ 7.80 (s, 1H), 7.36-7.34 (m, 2H), 7.06-7.04 (m, 1H), 6.98-6.97 (m, 2H), 6.65 (s, 1H), 4.05-4.02 (m, 1H), 3.77 (s, 5H), 2.75 (s, 3H),1.78-1.67 (m, 1H), 1.57-1.50 (m, 3H),1.48-1.30 (m, 2H), 0.95-0.85 (m, 3H).

**Intermediate 87**

**Methyl 3-butyl-7-methoxy-2-methyl-5-phenyl-2,3,4,5-tetrahydro-1,2,5-benzothiadiazepine-8-carboxylate 1,1-dioxide**

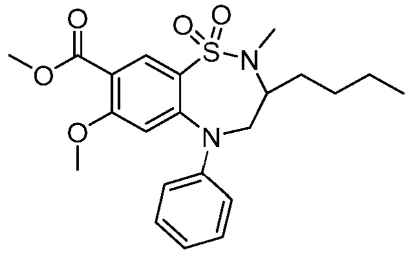

To a solution of 3-butyl-7-methoxy-2-methyl-1,1-dioxido-5-phenyl-2,3,4,5-tetrahydro-1,2,5-benzothiadiazepin-8-yl trifluoromethanesulfonate (Intermediate 86; 1.5 g, 2.87 mmol) in degassed methanol (15 mL) and dry DMF (10 mL) at RT, triethylamine (0.39 mL, 2.87 mmol), dppf (0.19 g, 0.34 mmol) and $Pd_2(dba)_3$ (0.13 g, 0.14 mmol) were added. The resulting reaction mixture was stirred for 16 h at 75°C in an autoclave, under 5 kg carbon monoxide gas pressure. After completion of the reaction (monitored by TLC), the reaction mixture was diluted with water (20 mL) and the aqueous layer was extracted with EtOAc (2 x 50 mL). The combined organic layer was washed with water (15 mL), with brine (15 mL) and dried over anhydrous $Na_2SO_4$. The organic layer was filtered, concentrated under vacuum and the resulting crude material was purified by Isolera column chromatography (eluent: 15% EtOAc/PE; silica gel: 230-400 mesh) to afford the title compound. **Yield:** 70.6% (0.9 g, off-white solid).

**LCMS:** (Method B) 433.1 ($M^+$+H), Rt. 2.45 min, 97.35% (Max).

**Intermediate 88**

**3-Butyl-8-(hydroxymethyl)-7-methoxy-2-methyl-5-phenyl-2,3,4,5-tetrahydro-1,2,5-benzothiadiazepine 1,1-dioxide**

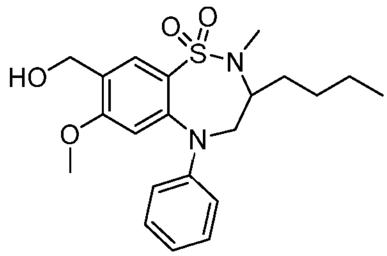

To a stirred solution of methyl 3-butyl-7-methoxy-2-methyl-5-phenyl-2,3,4,5-tetrahydro-1,2,5-benzothiadiazepine-8-carboxylate 1,1-dioxide (Intermediate 87; 0.9 g, 2.08 mmol) in THF (10 mL) at 0 °C, lithium aluminium hydride (2M in THF, 2.08 mL, 4.16 mmol) was added dropwise and the reaction mixture was stirred for 1 hour at RT. After completion of the reaction (monitored by TLC), the reaction mixture was quenched with saturated $NH_4Cl$ solution (10 mL) and the aqueous layer was extracted with EtOAc (2 x 25 mL). The combined organic layer was washed with water (10 mL), with brine (10 mL) and dried over anhydrous $Na_2SO_4$. The organic part was filtered, concentrated under vacuum and the resulting crude was forwarded to the next step without any further purification. **Yield:** 87% (0.8 g, off-white solid).

**$^1$H NMR** (400 MHz, $CDCl_3$): δ 7.92 (s, 1H), 7.28-7.25 (m, 2H), 6.93-6.90 (m, 1H), 6.86-6.84 (m, 2H), 6.68 (s, 1H), 4.75-4.72 (m, 2H), 4.15-4.13 (m, 1H), 4.06-3.99 (m, 1H), 3.51 (s, 3H), 3.47-3.46 (m, 1H), 2.65 (s, 3H), 1.6-1.36 (m, 3H),1.30-1.26 (m, 3H), 0.96-0.85 (m, 3H).

**Intermediate 89**

**Ethyl 2-(((3-butyl-7-methoxy-2-methyl-1,1-dioxido-5-phenyl-2,3,4,5-tetrahydro-1,2,5-benzothiadiazepin-8-yl) methyl)thio)acetate**

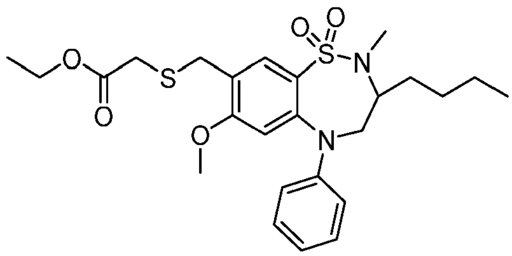

To a stirred solution of 3-butyl-8-(hydroxymethyl)-7-methoxy-2-methyl-5-phenyl-2,3,4,5-tetrahydro-1,2,5-benzothiadiazepine 1,1-dioxide (Intermediate 88; 0.4 g, 0.98 mmol) in DCM (5 mL) at 0 °C, ethyl 2-mercaptoacetate (0.12 g, 0.99 mmol) and zinc (II) iodide (0.32 g, 0.99 mmol) were added and the reaction mixture was stirred for 1 hour at RT. After completion of the reaction (monitored by TLC), the reaction mixture was quenched with water (15 mL) and the aqueous layer was extracted with DCM (2 x 20 mL). The combined organic layer was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 69.9% (0.35 g, white solid).

**Intermediate 90**

**Methyl 2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoate**

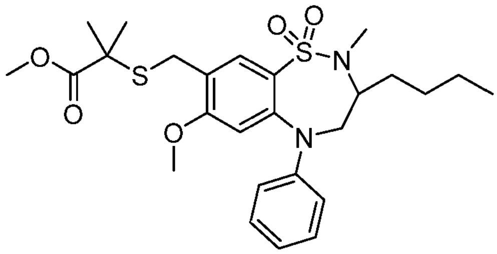

To a stirred solution of 3-butyl-8-(hydroxymethyl)-7-methoxy-2-methyl-5-phenyl-2,3,4,5-tetrahydro-1,2,5-benzothiadiazepine 1,1-dioxide (Intermediate 88; 0.4 g, 0.99 mmol) in DCM (5 mL) at 0 °C, ethyl 2-mercaptoacetate (0.12 g, 0.99 mmol) and zinc (II) iodide (0.32 g, 0.99) were added and the reaction mixture was stirred for 1 hour at RT. After completion of the reaction (monitored by TLC), the reaction mixture was quenched with water (5 mL) and the aqueous layer was extracted with DCM (2 x 20 mL). The combined organic part was washed with brine (10 mL), dried over anhydrous $Na_2SO_4$, filtered and concentrated under vacuum. The resulting crude material was purified by Isolera column chromatography (eluent: 20% EtOAc/PE; silica gel: 230-400 mesh) to afford the title compound. **Yield:** 58.3% (0.3 g, white solid).

**Intermediate 91**

**Ethyl 2-(((3-butyl-3-ethyl-5-(4-fluorophenyl)-7-methoxy-1,1-dioxido-2,3,4,5-tetrahydro-1,5-benzothiazepin-8-yl)methyl)thio)acetate**

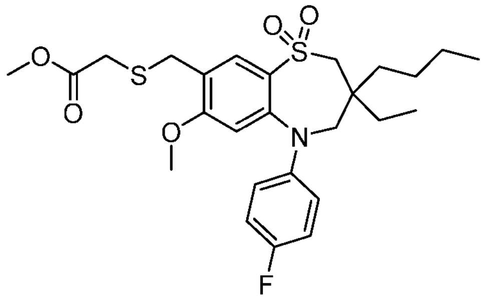

To a stirred solution of 3-butyl-3-ethyl-5-(4-fluorophenyl)-8-(hydroxymethyl)-7-methoxy-2,3,4,5-tetrahydro-1,5-benzothiazepine 1,1-dioxide (Intermediate 82; 0.5 g, 1.15 mmol) in DCM (10 mL) at 0 °C, ethyl 2-mercaptoacetate (0.28 g, 2.29 mmol) and zinc (II) iodide (0.18 g, 0.57 mmol) were added and the reaction mixture was stirred for 1 hour at RT. After completion of the reaction (monitored by TLC), the reaction mixture was quenched with water (5 mL) and the aqueous layer was extracted with DCM (2 x 15 mL). The combined organic layer was washed with brine (20 mL) and dried over anhydrous $Na_2SO_4$. The organic part was filtered and concentrated under vacuum. The resulting crude material was purified by Isolera column chromatography (eluent: 20% EtOAc/PE; silica gel: 230-400 mesh) to afford the title compound. **Yield:** 81% (500 mg, white solid).

**Intermediate 92**

**Methyl 2-(((3-butyl-7-methoxy-3-methyl-1,1-dioxido-5-phenyl-2,3,4,5-tetrahydro-1,5-benzothiazepin-8-yl) methyl)thio)-2-methylpropanoate**

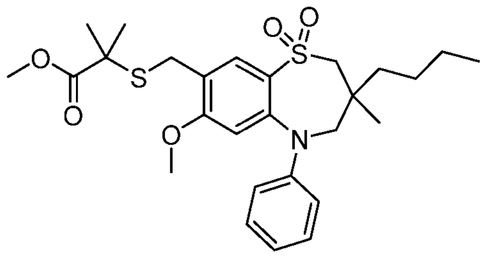

To a stirred solution of 3-butyl-8-(hydroxymethyl)-7-methoxy-3-methyl-5-phenyl-2,3,4,5-tetrahydro-1,5-benzothiazepine 1,1-dioxide (Intermediate 58; 0.25 g, 0.62 mmol) in DCM (10 mL) at 0 °C, methyl 2-mercapto-2-methylpropanoate (0.083 g, 0.62 mmol) and zinc (II) iodide (0.39 g, 1.24 mmol) were added and the reaction mixture was stirred for 1 hour at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 20% EtOAc/PE; silica gel: 230-400 mesh) to afford the title compound. **Yield:** 78% (0.25 g, white solid).

**Intermediate 93**

**Methyl 2-(((3,3-dibutyl-7-methoxy-1,1-dioxido-5-phenyl-2,3,4,5-tetrahydro-1,5-benzothiazepin-8-yl)methyl) thio)-2-methylpropanoate**

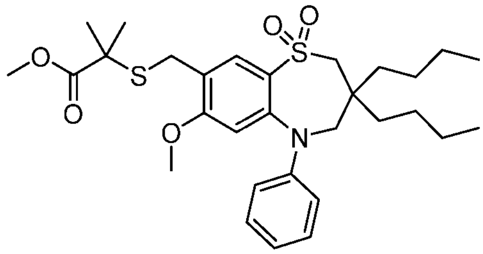

To a stirred solution of 3,3-dibutyl-8-(hydroxymethyl)-7-methoxy-5-phenyl-2,3,4,5-tetrahydro-1,5-benzothiazepine 1,1-dioxide (Intermediate 70; 200 mg, 0.45 mmol) in DCM (5 mL) at 0 °C, methyl 2-mercapto-2-methylpropanoate (60.2 mg, 0.45 mmol) and zinc (II) iodide (143 mg, 0.45 mmol) were added and the reaction mixture was stirred for 1 hour at RT. After completion of the reaction (monitored by TLC), the reaction mixture was quenched with water (5 mL) and the aqueous layer was extracted with DCM (2 x 15 mL). The combined organic layer was washed with brine (20 mL), dried over anhydrous $Na_2SO_4$, filtered and concentrated under vacuum. The resulting crude material was purified by Isolera column chromatography (eluent: 20% EtOAc/PE; silica gel: 230-400 mesh) to afford the title compound. **Yield:** 91% (230 mg, white solid).

**Intermediate 94**

**7-Bromo-3-butyl-3-ethyl-8-methoxy-2-(4-methoxybenzyl)-5-phenyl-2,3,4,5-tetrahydro-1,2,5-benzothiadiazepine 1,1-dioxide**

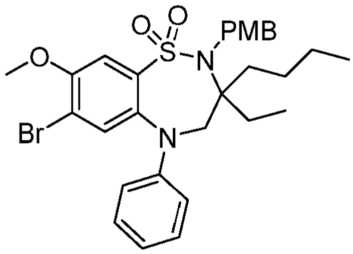

To a stirred solution of 7-bromo-3-butyl-3-ethyl-8-methoxy-5-phenyl-2,3,4,5-tetrahydro-1,2,5-benzothiadiazepine 1,1-dioxide (4 g, 8.56 mmol) in NMP (40 mL), $Cs_2CO_3$ (5.56 g, 17.13 mmol) at 0 °C, then 1-(chloromethyl)-4-methoxybenzene (2.01 g, 12.84 mmol) was added dropwise and the reaction mixture was stirred for 16 hours at RT. After completion of the reaction (monitored by TLC), the reaction mixture was diluted with water (50 mL) and the aqueous layer was extracted with ethyl acetate (2 x 50 mL). The combined organic layer was washed with brine (30 mL), dried over anhydrous $Na_2SO_4$, filtered and concentrated under reduced pressure. The resulting crude was purified by Isolera column chromatography (eluent: 0-50% EtOAc/PE; silica gel: 230-400 mesh) to afford the title compound. **Yield:** 63% (5 g, yellow gum).

**LCMS:** (Method G) 587.1 ($M^{+}$+H), Rt. 2.92 min, 63.50% (Max).

**Intermediate 95**

**7-Bromo-3-butyl-3-ethyl-8-hydroxy-2-(4-methoxybenzyl)-5-phenyl-2,3,4,5-tetrahydro-1,2,5-benzothiadiazepine 1,1-dioxide**

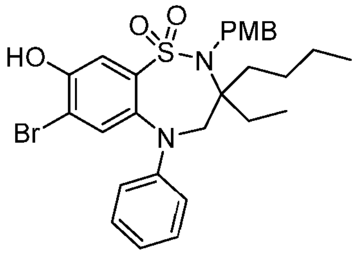

To a stirred solution of 7-bromo-3-butyl-3-ethyl-8-methoxy-2-(4-methoxybenzyl)-5-phenyl-2,3,4,5-tetrahydro-1,2,5-benzothiadiazepine 1,1-dioxide (Intermediate 94; 5 g, 8.51 mmol) in DMSO (50 mL) at 0 °C, lithium chloride (1.80 g, 42.5 mmol) was added and the reaction mixture was stirred for 24 hours at 140 °C. After completion of the reaction (monitored by UPLC), the reaction mixture was quenched with ice-cold water (25 mL) and the obtained solid was filtered off. The resulting crude was purified by Isolera column chromatography (eluent: 0-15% EtOAc/PE; silica gel: 230-400 mesh) to afford the title compound. **Yield:** 28% (1.4 g, off-white solid).

**LCMS:** (Method E) 575.1 ($M^{+}$+2), Rt. 2.71 min, 97.53% (Max).

**Intermediate 96**

**3-Butyl-3-ethyl-8-hydroxy-7-methoxy-2-(4-methoxybenzyl)-5-phenyl-2,3,4,5-tetrahydro-1,2,5-benzothiadiazepine 1,1-dioxide**

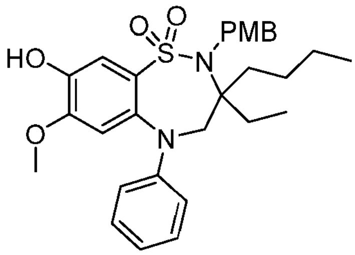

To a stirred solution of 7-bromo-3-butyl-3-ethyl-8-hydroxy-2-(4-methoxybenzyl)-5-phenyl-2,3,4,5-tetrahydro-1,2,5-benzothiadiazepine 1,1-dioxide (Intermediate 95; 1 g, 1.74 mmol) in methanol (15 mL) at 0 °C, sodium methoxide (25% in methanol, 2 mL, 8.72 mmol) and copper(I) bromide (0.25 g, 1.74 mmol) were added and the reaction mixture was stirred for 16 hours at 75 °C. After completion of the reaction (monitored by UPLC), the reaction mixture was diluted with water (20 mL) and the aqueous layer was extracted with EtOAc (50 mL). The combined organic layer was washed with brine (30 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 62% (0.66 g, off-white solid).

**LCMS:** (Method B) 523.1 ($M^{+}$-H), Rt. 2.71 min, 85.40% (Max).

**Intermediate 97**

**3-Butyl-3-ethyl-7-methoxy-2-(4-methoxybenzyl)-1,1-dioxido-5-phenyl-2,3,4,5-tetrahydro-1,2,5-benzothiadiazepin-8-yl trifluoromethanesulfonate**

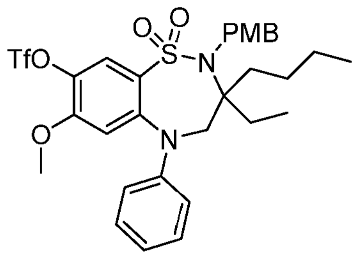

To a stirred solution of *3-butyl-3-ethyl-8-hydroxy-7-methoxy-2-(4-methoxybenzyl)-5-phenyl-2,3,4,5-tetrahydro-1,2,5-benzothiadiazepine 1,1-dioxide* (Intermediate 96; 660 mg, 1.26 mmol) in DCM (10 mL) at 0 °C were added pyridine (0.21 mL, 2.52 mmol) followed by triflic anhydride (0.32 mL, 1.89 mmol), and the reaction mixture was stirred for 1 hour at RT. After completion of the reaction (monitored by TLC), the reaction mixture was diluted with water (25 mL) and the aqueous layer was extracted with DCM (2 x 25 mL). The combined organic layer was washed with brine (30 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 76% (0.65 g, off-white solid).

**Intermediate 98**

**Methyl 3-butyl-3-ethyl-7-methoxy-2-(4-methoxybenzyl)-5-phenyl-2,3,4,5-tetrahydro-1,2,5-benzothiadiazepine-8-carboxylate 1,1-dioxide**

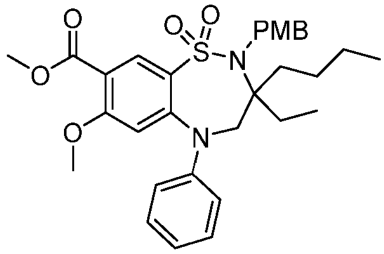

A stirred solution of 3-butyl-3-ethyl-7-methoxy-2-(4-methoxybenzyl)-1,1-dioxido-5-phenyl-2,3,4,5-tetrahydro-1,2,5-benzothiadiazepin-8-yl trifluoromethanesulfonate (Intermediate 97; 650 mg, 0.99 mmol) in methanol (10 mL) and DMF (5 mL) was purged with nitrogen gas for 15 minutes. $Pd_2(dba)_3$ (91 mg, 0.10 mmol), triethylamine (0.27 mL, 1.98 mmol) and dppf (54.9 mg, 0.10 mmol) were then added and the reaction mixture was stirred for 16 hours at 70 °C in a tinyclave, under 5 kg carbon monoxide gas pressure. After completion of the reaction (monitored by UPLC), the reaction mixture was filtered through a celite bed and the filtrate was concentrated to afford the crude compound. The resulting crude was purified by Isolera column chromatography (eluent: 0-30% EtOAc/PE; silica gel: 230-400 mesh) to afford the title compound. Yield: 34% (0.21 g, white solid).

**LCMS:** (Method B) 567.3 ($M^{+}$+H) Rt. 2.74 Min, 91.55% (Max).

**Intermediate 99**

**3-Butyl-3-ethyl-8-(hydroxymethyl)-7-methoxy-2-(4-methoxybenzyl)-5-phenyl-2,3,4,5-tetrahydro-1,2,5-benzothiadiazepine 1,1-dioxide**

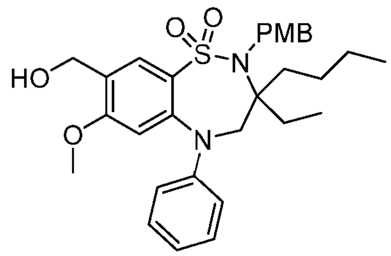

To a stirred solution of methyl 3-butyl-3-ethyl-7-methoxy-2-(4-methoxybenzyl)-5-phenyl-2,3,4,5-tetrahydro-1,2,5-benzothiadiazepine-8-carboxylate 1,1-dioxide (Intermediate 98; 20 mg, 0.04 mmol) in THF (10 mL), $LiAlH_4$ (2M solution in THF, 0.035 mL, 0.04 mmol) was added dropwise at 0 °C and the reaction mixture was stirred for 30 minutes at RT. After completion of the reaction (monitored by TLC), the reaction mixture was quenched with saturated aqueous ammonium chloride solution (25 mL) at 0 °C, and the aqueous layer was extracted with EtOAc (15 mL). The organic layer was dried over anhydrous $Na_2SO_4$ and concentrated under vacuum. The crude was forwarded to the next step without any further purification. **Yield:** 89% (19 mg, crude).

**LCMS:** (Method B) 539.1 ($M^+$+H), Rt. 2.60 Min, 92.50% (Max)

**Intermediate 100**

**Ethyl 2-(((3-butyl-3-ethyl-7-methoxy-2-(4-methoxybenzyl)-1,1-dioxido-5-phenyl-2,3,4,5-tetrahydro-1,2,5-benzothiadiazepin-8-yl)methyl)thio)acetate**

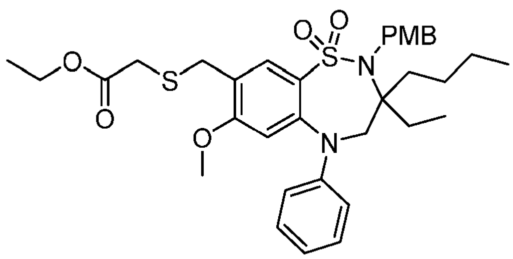

To a stirred solution of 3-butyl-3-ethyl-8-(hydroxymethyl)-7-methoxy-2-(4-methoxybenzyl)-5-phenyl-2,3,4,5-tetrahydro-1,2,5-benzothiadiazepine 1,1-dioxide (Intermediate 99; 180 mg, 0.32 mmol) in DCM (5 mL) at 0 °C, ethyl 2-mercaptoacetate (39.0 mg, 0.32 mmol) followed by zinc iodide (104 mg, 0.32 mmol) were added. The reaction mixture was then stirred for 3 hours at RT. After completion of the reaction (monitored by TLC), the reaction mixture was concentrated and quenched with water (30 mL). The aqueous layer was extracted with EtOAc (2 x 15 mL). The combined organic layer was separated, dried over anhydrous $Na_2SO_4$ and concentrated under high vacuum. The resulting crude was forwarded to the next step as such without any further purification. **Yield:** 250 mg (crude, off-white solid).

**Intermediate 101**

**2-(((3-Butyl-3-ethyl-7-methoxy-2-(4-methoxybenzyl)-1,1-dioxido-5-phenyl-2,3,4,5-tetrahydro-1,2,5-benzothiadiazepin-8-yl)methyl)thio)acetic acid**

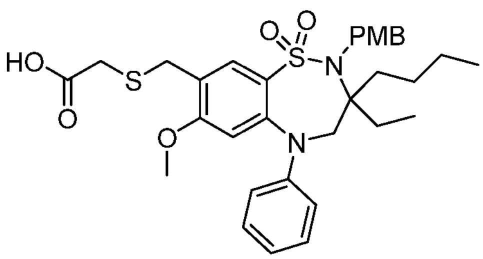

To a stirred solution of ethyl 2-(((3-butyl-3-ethyl-7-methoxy-2-(4-methoxybenzyl)-1,1-dioxido-5-phenyl-2,3,4,5-tetrahydro-1,2,5-benzothiadiazepin-8-yl)methyl)thio)acetate (Intermediate 100; 200 mg, 0.31 mmol) in a mixture of 1,4-dioxane and water (1:1; 4 mL), LiOH (74.7 mg, 3.12 mmol) was added and the reaction mixture was stirred for 1 hour at RT. After completion of the reaction (monitored by TLC), the reaction mixture was quenched with dilute HCl (1.5 N, 10 mL) and the aqueous layer was extracted with EtOAc (2 x 15 mL). The combined organic layer was separated, dried over anhydrous $Na_2SO_4$ and concentrated under high vacuum. The resulting crude was purified by Isolera column chromatography (eluent: 0-30% EtOAc/PE; silica gel: 230-400 mesh) to afford the title compound. **Yield:** 42% (0.2 g, off-white solid).
**UPLC:** (Method B) 611.0 ($M^+$-H), Rt. 1.51 Min, 50.26% (Max).

**Intermediate 102**

**3-Butyl-7-(dimethylamino)-3-ethyl-8-methoxy-2-(4-methoxybenzyl)-5-phenyl-2,3,4,5-tetrahydro-1,2,5-benzothiadiazepine 1,1-dioxide**

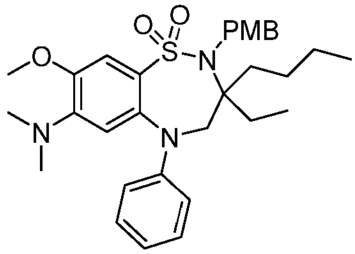

To a stirred solution of 7-bromo-3-butyl-3-ethyl-8-methoxy-2-(4-methoxybenzyl)-5-phenyl-2,3,4,5-tetrahydro-1,2,5-benzothiadiazepine 1,1-dioxide (Intermediate 94; 0.5 g, 0.85 mmol) in 1,4-dioxane (5 mL), dimethylamine (40% in aqueous solution, 0.2 mL, 1.70 mmol), sodium *tert*-butoxide (0.49 g, 5.11 mmol), $Pd_2(dba)_3$ (0.08 g, 0.09 mmol) and RuPhos (0.08 g, 0.17 mmol) were added and the reaction mixture was stirred for 2 hours at 70 °C. After completion of the reaction (monitored by TLC), the reaction mixture was diluted with water (25 mL) and the aqueous layer was extracted with EtOAc (2 x 25 mL). The organic layer was washed with brine (25 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 98% (0.5 g, off-white solid).
**LCMS:** (Method A) 552.3 ($M^+$+H), Rt. 2.29 min, 92.37% (Max).

**Intermediate 103**

**3-Butyl-7-(dimethylamino)-3-ethyl-8-hydroxy-2-(4-methoxybenzyl)-5-phenyl-2,3,4,5-tetrahydro-1,2,5-benzothiadiazepine 1,1-dioxide**

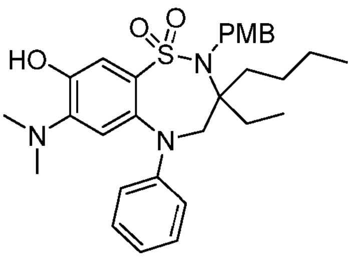

To a stirred solution of 3-butyl-7-(dimethylamino)-3-ethyl-8-methoxy-2-(4-methoxybenzyl)-5-phenyl-2,3,4,5-tetrahydro-1,2,5-benzothiadiazepine 1,1-dioxide (Intermediate 102; 1.0 g, 1.81 mmol) in DMF (10 mL), sodium thiomethoxide (0.64 g, 9.06 mmol) was added and the reaction mixture was stirred for 6 hours at 70 °C. After completion of the reaction (monitored by TLC), the reaction mixture was quenched with water (25 mL) and the aqueous layer was extracted with EtOAc (2 x 25 mL). The organic layer was separated, dried over anhydrous $Na_2SO_4$, filtered and concentrated under vacuum.
The resulting crude was purified by Isolera column chromatography (eluent: 0-30% EtOAc/PE; silica gel:

230-400 mesh) to afford the title compound. **Yield:** 75% (0.8 g, off-white solid).

**LCMS:** (Method B) 538.3 ($M^{+}$+H), Rt. 2.49 min, 91.11% (Max).

**Intermediate 104**

**3-Butyl-7-(dimethylamino)-3-ethyl-2-(4-methoxybenzyl)-1,1-dioxido-5-phenyl-2,3,4,5-tetrahydro-1,2,5-benzothiadiazepin-8-yl trifluoromethanesulfonate**

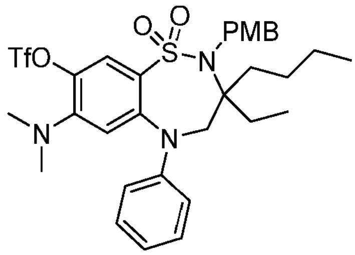

To a stirred solution of 3-butyl-7-(dimethylamino)-3-ethyl-8-hydroxy-2-(4-methoxybenzyl)-5-phenyl-2,3,4,5-tetrahydro-1,2,5-benzothiadiazepine 1,1-dioxide (Intermediate 103; 1.3 g, 2.42 mmol) in DCM (10 mL) at 0 °C, pyridine (0.39 mL, 4.84 mmol) and trifluoromethanesulfonic anhydride (0.4 mL, 2.418 mmol) were added dropwise and the reaction mixture was stirred for 30 minutes at RT. After completion of the reaction (monitored by TLC), the reaction mixture was quenched with water (25 mL) and the aqueous layer was extracted with DCM (2 x 25 mL). The combined organic layer was separated, dried over anhydrous $Na_2SO_4$, filtered and concentrated under high vacuum. The resulting crude was purified by Isolera column chromatography (eluent: 0-30% EtOAc/PE; silica gel: 230-400 mesh) to afford the title compound. **Yield:** 45% (1.0 g, brown solid).

**LCMS:** (Method B), 670.3 ($M^{+}$+H), Rt. 2.82 min,72.59% (Max).

**Intermediate 105**

**Methyl 3-butyl-7-(dimethylamino)-3-ethyl-2-(4-methoxybenzyl)-5-phenyl-2,3,4,5-tetrahydro-1,2,5-benzothiadiazepine-8-carboxylate 1,1-dioxide**

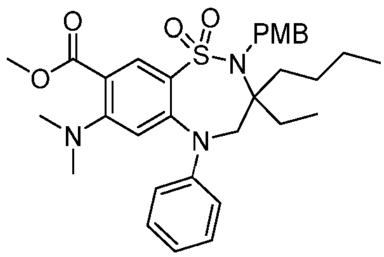

To a stirred solution of 3-butyl-7-(dimethylamino)-3-ethyl-2-(4-methoxybenzyl)-1,1-dioxido-5-phenyl-2,3,4,5-tetrahydro-1,2,5-benzothiadiazepin-8-yl trifluoromethanesulfonate (Intermediate 104; 0.4 g, 0.59 mmol) in methanol (15 mL) and DMF (10 mL) were added triethylamine (0.08 mL, 0.59 mmol), $Pd_2(dba)_3$ (0.03 g, 0.03 mmol) and dppf (0.04 g, 0.07 mmol) under nitrogen and reaction mixture was stirred for 16 hours at 80 °C in a tinyclave, under 5 kg carbon monoxide gas pressure. After completion of the reaction (monitored by TLC), the reaction mixture was concentrated under vacuum and quenched with water (25 mL). The aqueous layer was extracted with EtOAc (2 x 25 mL) and the combined organic layer was dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 11% (0.06 g, brown solid). **LCMS:** (Method B) 580.3 ($M^{+}$+H), Rt. 2.62 min, 44.08% (Max).

**Intermediate 106**

**3-Butyl-7-(dimethylamino)-3-ethyl-8-(hydroxymethyl)-2-(4-methoxybenzyl)-5-phenyl-2,3,4,5-tetrahydro-1,2,5-benzothiadiazepine 1,1-dioxide**

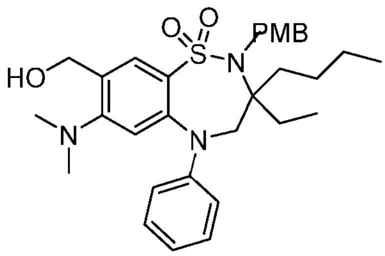

To a stirred solution of methyl 3-butyl-7-(dimethylamino)-3-ethyl-2-(4-methoxybenzyl)-5-phenyl-2,3,4,5-tetrahydro-1,2,5-benzothiadiazepine-8-carboxylate 1,1-dioxide (Intermediate 105; 0.8 g, 1.38 mmol) in THF (10 mL), $LiALH_4$ (2M in THF, 0.69 mL, 1.38 mmol) was added dropwise at 0 °C and reaction mixture was stirred for 30 minutes at RT. After completion of the reaction (monitored by TLC), the reaction mixture was quenched with saturated ammonium chloride solution (25 mL) at 0 °C and the aqueous layer was extracted with EtOAc (2 x 25 mL). The organic layer was separated, dried over anhydrous $Na_2SO_4$, filtered and concentrated under vacuum. The resulting crude was forwarded to the next step without any further purification. **Yield:** 47% (0.6 g, brown gum).

**LCMS:** (Method B) 552.3 ($M^+$+H), Rt. 2.63 Min, 59.0% (Max).

**Intermediate 107**

**Ethyl 2-(((3-butyl-7-(dimethylamino)-3-ethyl-2-(4-methoxybenzyl)-1,1-dioxido-5-phenyl-2,3,4,5-tetrahydro-1,2,5-benzothiadiazepin-8-yl)methyl)thio)acetate**

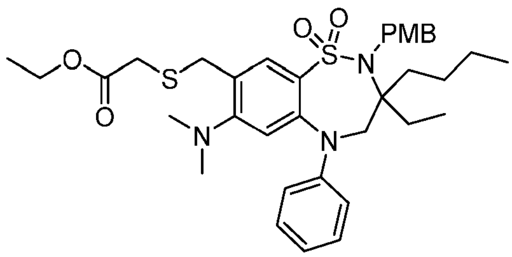

To a stirred solution of 3-butyl-7-(dimethylamino)-3-ethyl-8-(hydroxymethyl)-2-(4-methoxybenzyl)-5-phenyl-2,3,4,5-tetrahydro-1,2,5-benzothiadiazepine 1,1-dioxide (Intermediate 106; 0.4 g, 0.73 mmol) in DCM (5 mL) at 0 °C, ethyl 2-mercaptoacetate (0.09 g, 0.73 mmol) and zinc(II) iodide (0.23 g, 0.73 mmol) were added and the reaction mixture was stirred for 3 hours at RT. After completion of the reaction (monitored by TLC), the reaction mixture was concentrated under vacuum. The resulting solid was quenched with water (20 mL) and the aqueous layer was extracted with EtOAc (2 x 25 mL). The combined organic layer was separated, dried over anhydrous $Na_2SO_4$, filtered and concentrated under high vacuum. The resulting crude compound was forwarded as such to the next step without any further purification. **Yield:** 400 mg (crude, light brown solid).

**Intermediate 108**

**2-(((3-butyl-7-(dimethylamino)-3-ethyl-2-(4-methoxybenzyl)-1,1-dioxido-5-phenyl-2,3,4,5-tetrahydro-1,2,5-benzothiadiazepin-8-yl)methyl)thio)acetic acid**

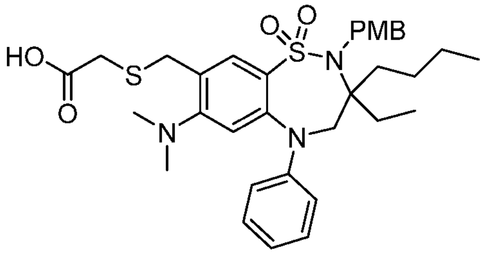

To a stirred solution of ethyl 2-(((3-butyl-7-(dimethylamino)-3-ethyl-2-(4-methoxybenzyl)-1,1-dioxido-5-phenyl-2,3,4,5-tetrahydro-1,2,5-benzothiadiazepin-8-yl)methyl)thio)acetate (Intermediate 107; 0.4 g, 0.61 mmol) in 1,4-dioxane (10 mL) at 0 °C, lithium hydroxide (0.03 g, 1.22 mmol) in water (2 mL) was added and the reaction mixture was stirred for 1 hour at RT. After completion of the reaction (monitored by TLC), the reaction mixture was quenched with dilute HCl solution (1.5 N, 10 mL) and the aqueous layer was extracted with EtOAc (2 x 25 mL). The combined organic layer was dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. Yield: 28% (0.3 g, off-white solid).

**LCMS:** (Method E) 626.2 ($M^+$+H), Rt 2.13 min, 35.5% (Max).

**Intermediate 109**

**3-Butyl-3-ethyl-8-hydroxy-2-(4-methoxybenzyl)-7-(methylsulfonyl)-5-phenyl-2,3,4,5-tetrahydro-1,2,5-benzothiadiazepine 1,1-dioxide**

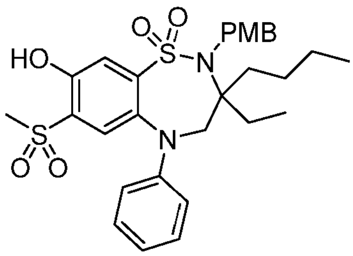

To a stirred solution of 3-butyl-3-ethyl-8-hydroxy-2-(4-methoxybenzyl)-7-(methylthio)-5-phenyl-2,3,4,5-tetrahydro-1,2,5-benzothiadiazepine 1,1-dioxide (3.2 g, 5.92 mmol) in THF (30 mL) and water (3 mL) at 0 °C, oxone (18.19 g, 59.2 mmol) was added and the reaction mixture was stirred for 16 hours at RT. After completion of the reaction (monitored by TLC), the reaction mixture was diluted with water (100 mL) and the aqueous layer was extracted with EtOAc (3 x 100 mL). The combined organic layer was washed with brine (100 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 63% (2.7 g, off-white solid).

**LCMS:** (Method E) 573.2 ($M^+$+H), Rt 2.44 min, 79.19% (Max).

**Intermediate 110**

**3-Butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl trifluoromethanesulfonate**

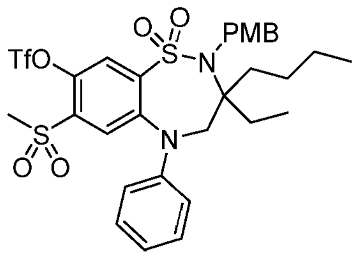

To a stirred solution of 3-butyl-3-ethyl-8-hydroxy-2-(4-methoxybenzyl)-7-(methylsulfonyl)-5-phenyl-2,3,4,5-tetrahydro-1,2,5-benzothiadiazepine 1,1-dioxide (Intermediate 109; 1.70 g, 2.97 mmol) in DCM (10 mL) at 0 °C, pyridine (0.48 mL, 5.94 mmol) and trifluoromethanesulfonic anhydride (0.5 mL, 2.97 mmol) were added dropwise and the reaction mixture was stirred for 30 minutes at RT. After completion of the reaction (monitored by TLC), the reaction mixture was quenched with water (20 mL) and the aqueous layer was extracted with DCM (2 x 25 mL). The organic layer was separated, dried over anhydrous $Na_2SO_4$, filtered and concentrated under vacuum. The resulting crude material was purified by Isolera column chromatography (eluent: 0-30% EtOAc/PE; silica gel: 230-400 mesh) to afford the title compound. **Yield:** 63% (1.5 g, off-white solid).

**Intermediate 111**

**Methyl 3-butyl-3-ethyl-2-(4-methoxybenzyl)-7-(methylsulfonyl)-5-phenyl-2,3,4,5-tetrahydro-1,2,5-benzothiadiazepine-8-carboxylate 1,1-dioxide**

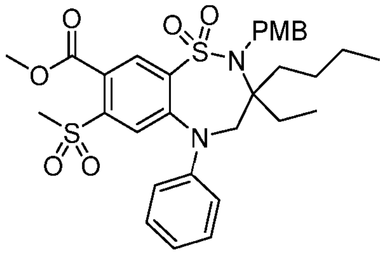

To a stirred solution of 3-butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl trifluoromethanesulfonate (Intermediate 110; 3.0 g, 4.26 mmol) in MeOH (20 mL) and DMF (15 mL) were added triethylamine (0.6 mL, 4.26 mmol), $Pd_2(dba)_3$ (0.19 g, 0.21 mmol) and dppf (0.28 g, 0.51 mmol) under nitrogen and the reaction mixture was stirred for 16 h at 75 °C in a tinyclave, under 5 kg carbon monoxide gas pressure. After completion of the reaction (monitored by TLC), the reaction mixture was concentrated under vacuum. The obtained crude was quenched with water (20 mL) and the aqueous layer was extracted with EtOAc (2 x 25 mL). The combined organic layer was separated, dried over anhydrous $Na_2SO_4$, filtered and concentrated under high vacuum. The resulting crude material was purified by Isolera column chromatography (eluent: 0-30% EtOAc/PE; silica gel: 230-400 mesh) to afford the title compound. **Yield:** 38% (1.8 g, brown solid).

**Intermediate 112**

**3-Butyl-3-ethyl-8-(hydroxymethyl)-2-(4-methoxybenzyl)-7-(methylsulfonyl)-5-phenyl-2,3,4,5-tetrahydro-1,2,5-benzothiadiazepine 1,1-dioxide**

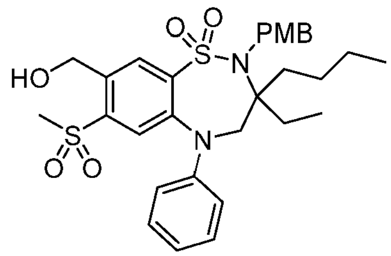

To a stirred solution of methyl 3-butyl-3-ethyl-2-(4-methoxybenzyl)-7-(methylsulfonyl)-5-phenyl-2,3,4,5-tetrahydro-1,2,5-benzothiadiazepine-8-carboxylate 1,1-dioxide (Intermediate 111; 1.8 g, 2.93 mmol) in THF (10 mL) at 0 °C, $LiAlH_4$ (2M in THF, 1.47 ml, 2.93 mmol) was added dropwise and the reaction mixture was stirred for 30 minutes at RT. After completion of the reaction (monitored by TLC), the reaction mixture was quenched with saturated ammonium chloride solution (15 mL) at 0 °C and the aqueous layer was extracted with EtOAc (2 x 20 mL). The combined organic layer was dried over anhydrous $Na_2SO_4$, filtered and concentrated under vacuum. The resulting crude was forwarded as such to the next step without any further purification. **Yield:** 1.8 g (crude, light brown liquid).

**Intermediate 113**

**Ethyl 2-(((3-butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl)methyl)thio)acetate**

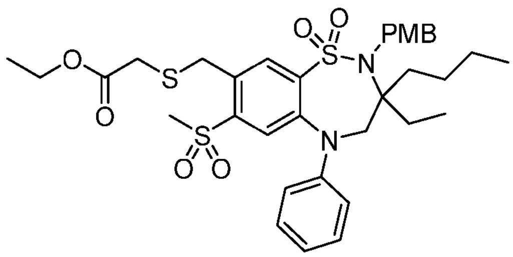

To a stirred solution of 3-butyl-3-ethyl-8-(hydroxymethyl)-2-(4-methoxybenzyl)-7-(methylsulfonyl)-5-phenyl-2,3,4,5-tetrahydro-1,2,5-benzothiadiazepine 1,1-dioxide (Intermediate 112; 0.5 g, 0.85 mmol) in DCM (10 mL) at 0 °C, ethyl 2-mercaptoacetate (0.10 g, 0.85 mmol) followed by zinc(II) iodide (0.27 g, 0.85 mmol) were added and the reaction mixture was stirred for 3 hours at RT. After completion of the reaction (monitored by TLC), the reaction mixture was concentrated under vacuum and the resulting crude was quenched with water (10 mL) and the aqueous layer was extracted with EtOAc (3 x 20 mL). The combined organic layer was separated, dried over anhydrous $Na_2SO_4$, filtered and concentrated under vacuum. The resulting crude was forwarded as such to the next step without any further purification. **Yield:** 500 mg (crude, light brown liquid).

**Intermediate 114**

**2-(((3-Butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl)methyl)thio)acetic acid**

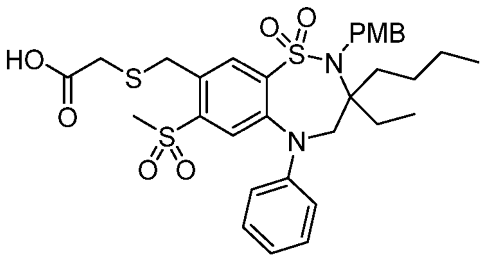

To a stirred solution of ethyl 2-(((3-butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl)methyl)thio)acetate (Intermediate 1113; 0.5 g, 0.73 mmol) in 1,4-dioxane (6 mL) at 0 °C, lithium hydroxide (0.04 g, 1.45 mmol) in water (4 mL) was added and the reaction mixture was stirred for 1 hour at RT. After completion of the reaction (monitored by TLC), the reaction mixture was quenched with dilute HCl (1.5 N, 15 mL) and the aqueous layer was extracted with EtOAc (2 x 25 mL). The combined organic layer was separated, dried over anhydrous $Na_2SO_4$, filtered and concentrated under vacuum. The resulting crude material was purified by Isolera column chromatography (eluent: 0-30% EtOAc/PE; silica gel: 230-400 mesh) to afford the title compound. **Yield:** 38% (0.3 g, off-white solid).

**Intermediate 115**

**(R)-7-bromo-3-butyl-8-methoxy-2-(4-methoxybenzyl)-5-phenyl-2,3,4,5-tetrahydro-1,2,5-benzothiadiazepine 1,1-dioxide**

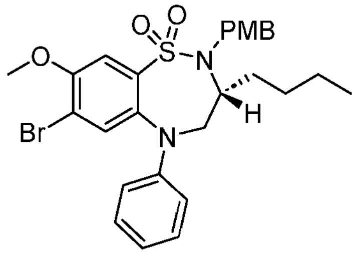

To a stirred solution of (R)-7-bromo-3-butyl-8-methoxy-5-phenyl-2,3,4,5-tetrahydro-1,2,5-benzothiadiazepine 1,1-dioxide (2.5 g, 5.69 mmol) in DMF (20 mL) at 0 °C, $Cs_2CO_3$ (3.71 g, 11.38 mmol) was added, then 1-(chloromethyl)-4-methoxybenzene (1.34 g, 8.53 mmol) was added dropwise and the reaction mixture was stirred for 16 hours at RT. After completion of the reaction (monitored by TLC), the reaction mixture was diluted with water (50 mL) and the aqueous layer was extracted with EtOAc (3 x 50 mL). The combined organic layer was washed with brine (50 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 69% (2.2 g, brown solid).

**LCMS:** (Method B) 558.8 ($M^+$), Rt.1.91 min, 56.52% (Max).

**Intermediate 116**

**(R)-3-butyl-7-(dimethylamino)-8-methoxy-2-(4-methoxybenzyl)-5-phenyl-2,3,4,5-tetrahydro-1,2,5-benzothiadiazepine 1,1-dioxide**

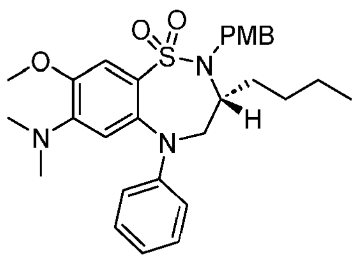

To a stirred solution of (R)-7-bromo-3-butyl-8-methoxy-2-(4-methoxybenzyl)-5-phenyl-2,3,4,5-tetrahydro-1,2,5-benzothiadiazepine 1,1-dioxide (Intermediate 115; 2 g, 3.57 mmol) in 1,4-dioxane (20 mL) were added dimethylamine (0.08 g, 1.70 mmol) and sodium *tert*-butoxide (0.49 g, 5.11 mmol) and the solution was purged with nitrogen gas for 10 minutes. $Pd_2(dba)_3$ (0.08 g, 0.09 mmol) and RuPhos (0.08 g, 0.17 mmol) were then added and the reaction mixture was heated for 2 hours at 80 °C. After completion of the reaction (monitored by LCMS), the reaction mixture was diluted with water (20 mL) and the aqueous layer was extracted with EtOAc (3 x 20 mL). The combined organic layer was washed with brine (20 mL), dried over anhydrous $Na_2SO_4$, filtered and concentrated under reduced pressure. The resulting crude was

purified by Isolera column chromatography (eluent: 20-30% EtOAc/PE; silica gel: 230-400 mesh) to afford the title compound. **Yield:** 52% (1.4 g, brown solid). **LCMS:** (Method B) 524.3 ($M^{+}$+H), Rt. 2.87 min, 43.87% (Max).

**Intermediate 117**

**(R)-3-butyl-7-(dimethylamino)-8-hydroxy-2-(4-methoxybenzyl)-5-phenyl-2,3,4,5-tetrahydro-1,2,5-benzothiadiazepine 1,1-dioxide**

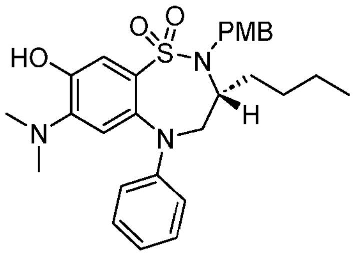

To a stirred solution of (R)-3-butyl-7-(dimethylamino)-8-methoxy-2-(4-methoxybenzyl)-5-phenyl-2,3,4,5-tetrahydro-1,2,5-benzothiadiazepine 1,1-dioxide (Intermediate 116; 2.0 g, 3.82 mmol) in DMF (10 mL), NaSMe (1.34 g, 19.10 mmol) was added and the reaction mixture was stirred for 12 hours at 70 °C. After completion of the reaction (monitored by TLC), the reaction mixture was quenched with water (20 mL) and the aqueous layer was extracted with EtOAc (3 x 50 mL). The combined organic layer was washed with brine (20 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 42% (1.2 g, off-white solid). **LCMS:** (Method E) 510.2 ($M^{+}$+H), Rt 1.97 min, 68.55% (Max).

**Intermediate 118**

**(R)-3-butyl-7-(dimethylamino)-2-(4-methoxybenzyl)-1,1-dioxido-5-phenyl-2,3,4,5-tetrahydro-1,2,5-benzothiadiazepin-8-yl trifluoromethanesulfonate**

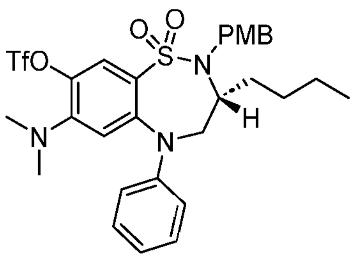

To a stirred solution of (R)-3-butyl-7-(dimethylamino)-8-hydroxy-2-(4-methoxybenzyl)-5-phenyl-2,3,4,5-tetrahydro-1,2,5-benzothiadiazepine 1,1-dioxide (Intermediate 117; 1.5 g, 2.94 mmol) in DCM (10 mL) at 0 °C, pyridine (0.47 mL, 5.89 mmol) and trifluoromethanesulfonic anhydride (0.5 mL, 2.94 mmol) were added dropwise and the reaction mixture was stirred for 30 minutes at RT. After completion of the reaction (monitored by TLC), the reaction mixture was quenched with water (20 mL) and the aqueous layer was extracted with DCM (2 x 20 mL). The combined organic layer was washed with brine (20 mL), dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 59% (1.4 g, brown solid).
**LCMS:** (Method E) 642.2 ($M^{+}$+H), Rt 2.80 min, 79.5% (Max).

**Intermediate 119**

**Methyl (R)-3-butyl-7-(dimethylamino)-2-(4-methoxybenzyl)-5-phenyl-2,3,4,5-tetrahydro-1,2,5-benzothiadiazepine-8-carboxylate 1,1-dioxide**

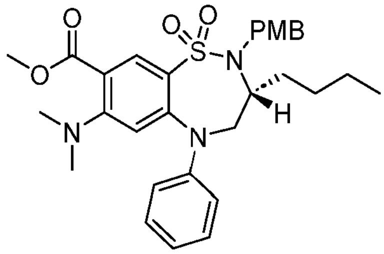

To a stirred solution of (R)-3-butyl-7-(dimethylamino)-2-(4-methoxybenzyl)-1,1-dioxido-5-phenyl-2,3,4,5-tetrahydro-1,2,5-benzothiadiazepin-8-yl trifluoromethanesulfonate (Intermediate 118; 2.8 g, 4.36 mmol) in MeOH (20 mL) and DMF (15 mL), triethylamine (0.61 mL, 4.36 mmol), $Pd_2(dba)_3$ (0.20 g, 0.22 mmol) and dppf (0.29 g, 0.52 mmol) were added under a nitrogen atmosphere and the reaction mixture was stirred for 16 h at 75 °C in a tinyclave, under 5 kg carbon monoxide gas pressure. After completion of the reaction (monitored by TLC) the reaction mixture was concentrated, and the resulting mixture was quenched with water (20 mL). The aqueous layer was extracted with EtOAc (2 x 15 mL) and the combined organic layer was separated and dried over anhydrous $Na_2SO_4$. The organic part was filtered, concentrated under vacuum and the resulting crude was purified by Isolera column chromatography (eluent: 0-30% EtOAc/PE; silica gel: 230-400 mesh) to afford the title compound. **Yield:** 54% (1.3 g, white solid).

**LCMS:** (Method E) 552.3 ($M^{+}$+H), Rt. 2.36 min, 62.07% (Max).

**Intermediate 120**

**(R)-3-butyl-7-(dimethylamino)-8-(hydroxymethyl)-2-(4-methoxybenzyl)-5-phenyl-2,3,4,5-tetrahydro-1,2,5-benzothiadiazepine 1,1-dioxide**

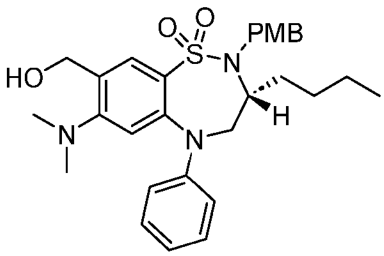

To a stirred solution of methyl (R)-3-butyl-7-(dimethylamino)-2-(4-methoxybenzyl)-5-phenyl-2,3,4,5-tetrahydro-1,2,5-benzothiadiazepine-8-carboxylate 1,1-dioxide (Intermediate 119; 600 mg, 1.09 mmol) in THF (10 mL) at 0 °C, $LiAlH_4$ (2M in THF, 0.54 mL, 1.09 mmol) was added dropwise and the reaction mixture was stirred for 30 minutes at RT. After completion of the reaction (monitored by TLC), the reaction mixture was quenched with saturated ammonium chloride solution (10 mL) at 0 °C, and the aqueous layer was extracted with EtOAc (2 x 20 mL). The combined organic layer was dried over anhydrous $Na_2SO_4$ and concentrated under vacuum. The resulting crude was forwarded as such to the next step without any further purification.

**$^1$H-NMR** (400 MHz, DMSO-ds): δ 7.96 (s, 1H), 7.24-7.21 (m, 5H), 6.89-6.81 (m, 5H), 5.37 (t, J = 5.6 Hz, 1H), 4.53 (d, J = 5.6 Hz, 2H), 4.15 (bs, 2H), 3.75 (s, 1H), 3.72 (s, 2H), 3.71 (s, 3H), 2.68 (s, 6H), 1.43-1.36 (m, 2H), 0.95-0.91 (m, 4H), 0.62 (t, J = 6.80 Hz, 3H). **LCMS:** (Method B) 524.2 ($M^{+}$+H), Rt 2.63 min, 54.42% (Max).

**Intermediate 121**

**Ethyl (R)-2-(((3-butyl-7-(dimethylamino)-2-(4-methoxybenzyl)-1,1-dioxido-5-phenyl-2,3,4,5-tetrahydro-1,2,5-benzothiadiazepin-8-yl)methyl)thio)acetate**

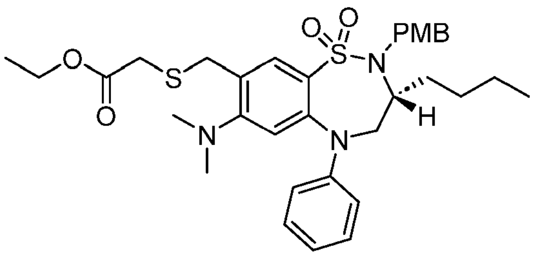

To a stirred solution of (R)-3-butyl-7-(dimethylamino)-8-(hydroxymethyl)-2-(4-methoxybenzyl)-5-phenyl-2,3,4,5-tetrahydro-1,2,5-benzothiadiazepine 1,1-dioxide (Intermediate 120; 300 mg, 0.57 mmol) in DCM (10 mL) at 0 °C, ethyl 2-mercaptoacetate (68.8 mg, 0.57 mmol) and zinc (II) iodide (183 mg, 0.57 mmol) were added and the reaction mixture was stirred for 3 hours at RT. After completion of the reaction (monitored by TLC), the reaction mixture was concentrated under vacuum and quenched with water (20 mL). The aqueous layer was extracted with EtOAc (2 x 25 mL), dried over anhydrous $Na_2SO_4$ and concentrated under vacuum. The resulting crude was forwarded as such to the next step without any further purification.

**Intermediate 122**

**(R)-2-(((3-butyl-7-(dimethylamino)-2-(4-methoxybenzyl)-1,1-dioxido-5-phenyl-2,3,4,5-tetrahydro-1,2,5-benzothiadiazepin-8-yl)methyl)thio)acetic acid**

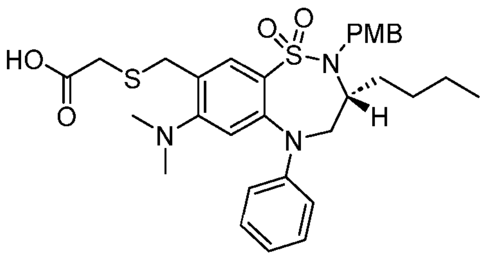

To a stirred solution of ethyl (R)-2-(((3-butyl-7-(dimethylamino)-2-(4-methoxybenzyl)-1,1-dioxido-5-phenyl-2,3,4,5-tetrahydro-1,2,5-benzothiadiazepin-8-yl)methyl)thio)acetate (Intermediate 121; 300 mg, 0.48 mmol) in 1,4-dioxane (6 mL) at 0 °C, lithium hydroxide (22.96 mg, 0.96 mmol) in water (1 mL) was added and the reaction mixture was stirred for 1 hour at RT. After completion of the reaction (monitored by TLC), the reaction mixture was quenched with dilute HCl (1.5 N, 15 mL) and the aqueous layer was extracted EtOAc (2 x 25 mL). The combined organic layer was dried over anhydrous $Na_2SO_4$ and concentrated under high vacuum. The resulting crude was purified by Isolera column chromatography (eluent: 0-50% EtOAc/PE; silica gel: 230-400 mesh) to afford the title compound. **Yield:** 47% (250 mg, white solid).

**LCMS:** (Method B) 598.2 ($M^+$+H), Rt 2.05 min, 54.26% (Max).

**Intermediate 123**

**(R)-3-butyl-8-hydroxy-2-(4-methoxybenzyl)-7-(methylthio)-5-phenyl-2,3,4,5-tetrahydro-1,2,5-benzothiadiazepine 1,1-dioxide**

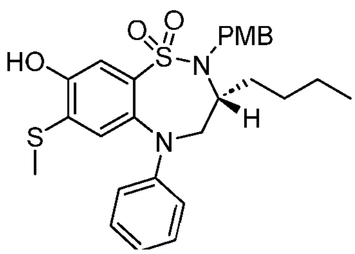

To a stirred solution of (R)-7-bromo-3-butyl-8-methoxy-2-(4-methoxybenzyl)-5-phenyl-2,3,4,5-tetrahydro-1,2,5-benzothiadiazepine 1,1-dioxide (Intermediate 115; 2.5 g, 4.47 mmol) in DMF (10 mL), NaSMe (1.57 g, 22.34 mmol) was added at RT and the reaction mixture was stirred for 12 hours at 90 °C. After completion of the reaction (monitored by TLC), the reaction mixture was quenched with water (25 mL) and the aqueous layer was extracted with EtOAc (2 x 25 mL). The organic layer was separated, dried over anhydrous $Na_2SO_4$ and concentrated under high vacuum. The resulting crude was purified by Isolera column chromatography (eluent: 0-30% EtOAc/PE; silica gel: 230-400 mesh) to afford the title compound. **Yield:** 57% (1.5 g, off-white solid).

**LCMS:** (Method E) 513.2 ($M^+$+H), Rt. 2.49 min, 87.85% (Max).

**Intermediate 124**

**(R)-3-butyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate**

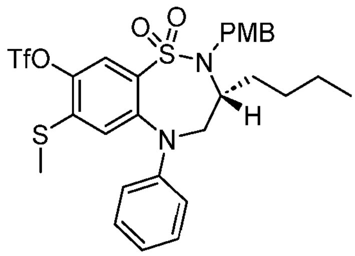

To a stirred solution of (R)-3-butyl-8-hydroxy-2-(4-methoxybenzyl)-7-(methylthio)-5-phenyl-2,3,4,5-tetrahydro-1,2,5-benzothiadiazepine 1,1-dioxide (Intermediate 123; 1.5 g, 2.93 mmol) in DCM (10 mL) at 0 °C, pyridine (0.47 mL, 5.85 mmol) and trifluoromethanesulfonic anhydride (0.5 mL, 2.93 mmol) were added dropwise and the reaction mixture was stirred for 30 minutes at RT. After completion of the reaction (monitored by TLC), the reaction mixture was quenched with water (20 mL) and the aqueous layer was extracted with DCM (2 x 20 mL). The organic part was dried over anhydrous $Na_2SO_4$ and concentrated under high vacuum. The resulting crude was purified by Isolera column chromatography (eluent: 0-30% EtOAc/PE; silica gel: 230-400 mesh) to afford the title compound. **Yield:** 28% (0.6 g, brown solid).

**Intermediate 125**

**Methyl (R)-3-butyl-2-(4-methoxybenzyl)-7-(methylthio)-5-phenyl-2,3,4,5-tetrahydro-1,2,5-benzothiadiazepine-8-carboxylate 1,1-dioxide**

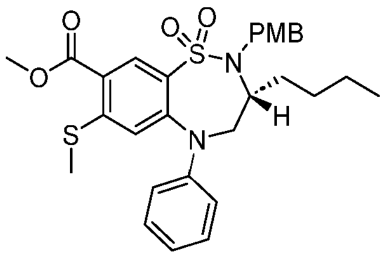

To a stirred solution of (R)-3-butyl-2-(4-methoxybenzyl)-7-(methylthio)-1,1-dioxido-5-phenyl-2,3,4,5-tetrahydro-1,2,5-benzothiadiazepin-8-yl trifluoromethanesulfonate (Intermediate 124; 1.2 g, 1.86 mmol) in methanol (20 mL) and DMF (15 mL) were added triethylamine (0.26 mL, 1.86 mmol), $Pd_2(dba)_3$ (0.09 g, 0.09 mmol) and dppf (0.12 g, 0.22 mmol) under nitrogen, and the reaction mixture was stirred for 16 hours at 75 °C in a tinyclave under 5 kg carbon monoxide gas pressure. After completion of the reaction (monitored by TLC), the reaction mixture was concentrated under vacuum and quenched with water (10 mL). The aqueous layer was extracted with EtOAc (2 x 15 mL), the organic layer was dried over anhydrous sodium sulphate and concentrated under high vacuum. The resulting crude was purified by Isolera column chromatography (eluent: 0-30% EtOAc/PE; silica gel: 230-400 mesh) to afford the title compound. **Yield:** 40% (0.6 g, off white solid).

**LCMS:** (Method B) 555.1($M^+$+H), Rt 2.83 min, 69.63% (Max).

**Intermediate 126**

**(R)-3-butyl-8-(hydroxymethyl)-2-(4-methoxybenzyl)-7-(methylthio)-5-phenyl-2,3,4,5-tetrahydro-1,2,5-benzothiadiazepine 1,1-dioxide**

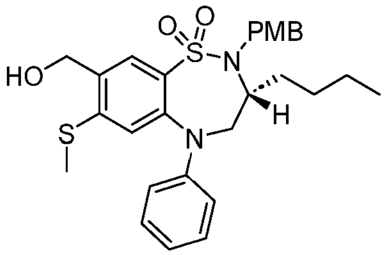

To a stirred solution of methyl (R)-3-butyl-2-(4-methoxybenzyl)-7-(methylthio)-5-phenyl-2,3,4,5-tetrahydro-1,2,5-benzothiadiazepine-8-carboxylate 1,1-dioxide (Intermediate 125; 0.6 g, 1.08 mmol) in THF (10 mL) at 0 °C, $LiAlH_4$ (2 M in THF, 0.54 mL, 1.08 mmol) was added dropwise and the reaction mixture was stirred for 30 minutes at RT. After completion of the reaction (monitored by TLC), the reaction mixture was quenched with saturated ammonium chloride solution (10 mL) at 0 °C, then extracted with ethyl acetate (2 x 20 mL). The combined organic extract was washed with brine (50 mL), dried over anhydrous $Na_2SO_4$ and concentrated under vacuum. The resulting crude was forwarded as such to the next step without any further purification.

**Intermediate 127**

**Ethyl (R)-2-(((3-butyl-2-(4-methoxybenzyl)-7-(methylthio)-1,1-dioxido-5-phenyl-2,3,4,5-tetrahydro-1,2,5-benzothiadiazepin-8-yl)methyl)thio)acetate**

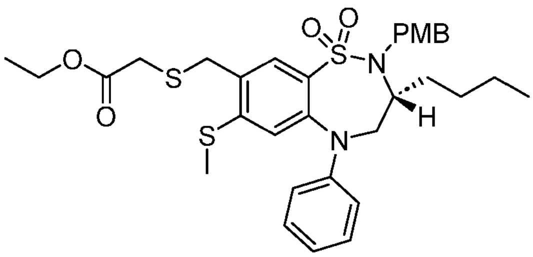

To a stirred solution of (R)-3-butyl-8-(hydroxymethyl)-2-(4-methoxybenzyl)-7-(methylthio)-5-phenyl-2,3,4,5-tetrahydro-1,2,5-benzothiadiazepine 1,1-dioxide (Intermediate 126; 600 mg, 1.14 mmol) in DCM (5 mL) at 0 °C, ethyl 2-mercaptoacetate (137 mg, 1.14 mmol) and zinc (II) iodide (364 mg, 1.14 mmol) were added and the reaction mixture was stirred for 3 hours at RT. After completion of the reaction (monitored by TLC), the reaction mixture was concentrated under vacuum and quenched with water (20 mL). The aqueous layer was extracted with EtOAc (2 x 20 mL), the combined organic layer was dried over anhydrous $Na_2SO_4$ and concentrated under vacuum to afford the crude. The resulting crude material was forwarded as such to the next step without any further purification.

**Intermediate 128**

**(R)-2-(((3-butyl-2-(4-methoxybenzyl)-7-(methylthio)-1,1-dioxido-5-phenyl-2,3,4,5-tetrahydro-1,2,5-benzothiadiazepin-8-yl)methyl)thio)acetic acid**

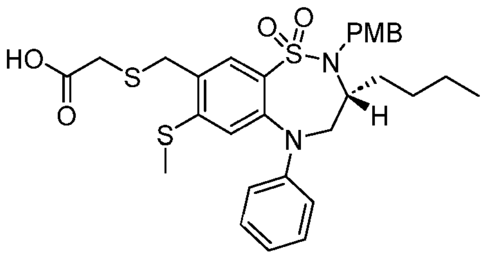

To a stirred solution of ethyl (R)-2-(((3-butyl-2-(4-methoxybenzyl)-7-(methylthio)-1,1-dioxido-5-phenyl-2,3,4,5-tetrahydro-1,2,5-benzothiadiazepin-8-yl)methyl)thio)acetate (Intermediate 127; 500 mg, 0.79 mmol) in 1,4-dioxane (10 mL), lithium hydroxide (38.1 mg, 1.59 mmol) in water (2 mL) was added at 0 °C and the reaction mixture was stirred for 1 hour at RT. After completion of the reaction (monitored by TLC), the reaction mixture was quenched with dilute HCl (15 mL, 1.5 N) and the aqueous layer was extracted with EtOAc (2 x 20 mL). The combined organic layer was separated, dried over anhydrous $Na_2SO_4$ and concentrated under vacuum. The resulting crude was purified by Isolera column chromatography (eluent: 0-50% EtOAc/PE; silica gel: 230-400 mesh) to afford the title compound. **Yield:** 50% (0.45 g, off-white solid).

**LCMS:** (Method B), 599.1 ($M^+$-H), Rt 2.0 min, 53.31% (Max).

**Intermediate 129**

**(R)-7-bromo-3-butyl-8-hydroxy-2-(4-methoxybenzyl)-5-phenyl-2,3,4,5-tetrahydro-1,2,5-benzothiadiazepine 1,1-dioxide**

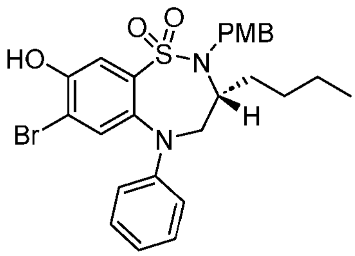

To a stirred solution of 7-bromo-3-butyl-8-methoxy-2-(4-methoxybenzyl)-5-phenyl-2,3,4,5-tetrahydro-1,2,5-benzothiadiazepine 1,1-dioxide (Intermediate 115; 1.5 g, 2.68 mmol) in DMSO (12 mL) at 0 °C, lithium chloride (1.14 g, 26.8 mmol) was added and the reaction mixture was stirred for 24 hours at 120 °C. After completion of the reaction (monitored by TLC), the reaction mixture was quenched with ice-cold water (20 mL). The obtained solid was collected by filtration and dried under vacuum. The resulting crude material was purified by Isolera column chromatography (eluent: 0-30% EtOAc/PE; silica gel: 230-400 mesh) to afford the title compound. **Yield:** 37% (1.0 g, off-white solid). **LCMS:** (Method A), 544.0 ($M^+$-H), Rt. 2.65 min, 54.3 % (Max).

**Intermediate 130**

**(R)-3-butyl-8-hydroxy-7-methoxy-2-(4-methoxybenzyl)-5-phenyl-2,3,4,5-tetrahydro-1,2,5-benzothiadiazepine 1,1-dioxide**

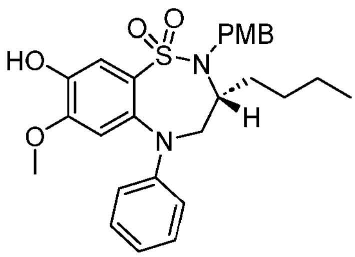

To a stirred solution of 7-bromo-3-butyl-8-hydroxy-2-(4-methoxybenzyl)-5-phenyl-2,3,4,5-tetrahydro-1,2,5-benzothiadiazepine 1,1-dioxide (Intermediate 129; 0.7 g, 1.28 mmol) in MeOH (10 mL) at 0 °C, sodium methoxide (25% in methanol, 5.5 mL, 6.42 mmol) and copper(I) bromide (0.18 g, 1.28 mmol) were added and the reaction mixture was stirred for 16 hours at 75 °C. After completion of the reaction (monitored by UPLC), the reaction mixture was diluted with water (50 mL) and the aqueous layer was extracted with EtOAc (3 x 100 mL). The combined organic layer was washed with brine (50 mL), dried over anhydrous $Na_2SO_4$, filtered and concentrated under vacuum. The resulting crude material was purified by Isolera column chromatography (eluent: 0-30% EtOAc/PE; silica gel: 230-400 mesh) to afford the title compound. **Yield:** 69% (0.5 g, off-white solid).

**LCMS:** (Method C), 497.3 ($M^+$+H), Rt. 2.23 min, 88.76% (Max).

**Intermediate 131**

**(R)-3-butyl-7-methoxy-2-(4-methoxybenzyl)-1,1-dioxido-5-phenyl-2,3,4,5-tetrahydro-1,2,5-benzothiadiazepin-8-yl trifluoromethanesulfonate**

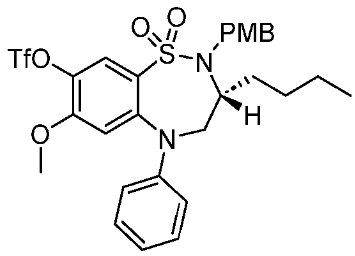

To a stirred solution of (R)-3-butyl-8-hydroxy-7-methoxy-2-(4-methoxybenzyl)-5-phenyl-2,3,4,5-tetrahydro-1,2,5-benzothiadiazepine 1,1-dioxide (Intermediate 130; 1 g, 2.01 mmol) in DCM (5 mL) at 0 °C, pyridine (0.32 mL, 4.03 mmol) and triflic anhydride (0.5 mL, 3.02 mmol) were added dropwise and the reaction mixture was stirred for 30 minutes at RT. After completion of the reaction (monitored by TLC), the reaction mixture was quenched with water (25 mL) and the organic layer was extracted with DCM (2 x 25 mL). The combined organic layer was separated, dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 41% (0.55 g, pale yellow gum).

**Intermediate 132**

**Methyl (R)-3-butyl-7-methoxy-2-(4-methoxybenzyl)-5-phenyl-2,3,4,5-tetrahydro-1,2,5-benzothiadiazepine-8-carboxylate 1,1-dioxide**

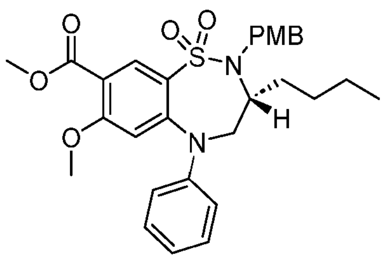

To a stirred solution of 3-butyl-7-methoxy-2-(4-methoxybenzyl)-1,1-dioxido-5-phenyl-2,3,4,5-tetrahydro-1,2,5-benzothiadiazepin-8-yl trifluoromethanesulfonate (Intermediate 131; 550 mg, 0.88 mmol) in MeOH (10 mL) and DMF (10 mL) were added triethylamine (0.24 mL, 1.75 mmol), $Pd_2(dba)_3$ (80 mg, 0.09 mmol) and dppf (48.5 mg, 0.09 mmol) under nitrogen and the reaction mixture was stirred for 16 h at 75 °C in a tinyclave, under 5 kg carbon monoxide gas pressure. After completion of the reaction (monitored by TLC), the reaction mixture was concentrated under vacuum and the resulting crude material was purified by Isolera column chromatography (eluent: 0-30% EtOAc/PE; silica gel: 230-400 mesh) to afford the title compound. **Yield:** 56% (0.35 g, off-white solid).

**LCMS:** (Method E), 539.1 ($M^{+}$+H), Rt. 1.89 Min, 75.4% (Max)

**Intermediate 133**

**(R)-3-butyl-8-(hydroxymethyl)-7-methoxy-2-(4-methoxybenzyl)-5-phenyl-2,3,4,5-tetrahydro-1,2,5-benzothiadiazepine 1,1-dioxide**

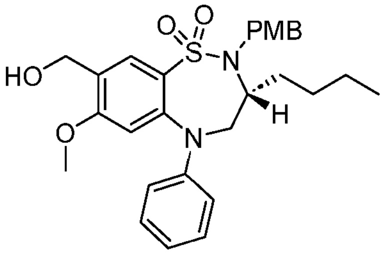

To a stirred solution of methyl (R)-3-butyl-7-methoxy-2-(4-methoxybenzyl)-5-phenyl-2,3,4,5-tetrahydro-1,2,5-benzothiadiazepine-8-carboxylate 1,1-dioxide (Intermediate 132; 350 mg, 0.65 mmol) in THF (4 mL), $LiAlH_4$ (2M in THF, 0.33 mL, 0.65 mmol) was added dropwise at 0 °C and the reaction mixture was stirred for 30 minutes at RT. After completion of the reaction (monitored by TLC), the reaction mixture was quenched with saturated ammonium chloride solution (10 mL) at 0 °C and the aqueous layer was extracted with EtOAc (2 x 20 mL). The organic layer was dried over anhydrous $Na_2SO_4$, filtered and concentrated under vacuum. The resulting crude was forwarded as such to the next step without any further purification. **Yield:** 86% (0.31 g, brown solid).

**LCMS:** (Method C), 511.4 ($M^{+}$+H), Rt. 2.26 Min, 92.18% (Max)

**Intermediate 134**

**Ethyl (R)-2-(((3-butyl-7-methoxy-1,1-dioxido-5-phenyl-2,3,4,5-tetrahydro-1,2,5-benzothiadiazepin-8-yl)methyl) thio)acetate**

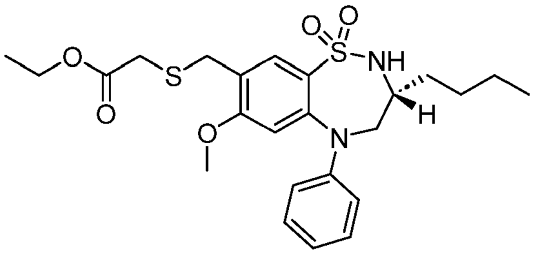

To a stirred solution of (R)-3-butyl-8-(hydroxymethyl)-7-methoxy-2-(4-methoxybenzyl)-5-phenyl-2,3,4,5-tetrahydro-1,2,5-benzothiadiazepine 1,1-dioxide (Intermediate 133; 300 mg, 0.59 mmol) in DCM (6 mL) at 0 °C, ethyl 2-mercaptoacetate (70.6 mg, 0.59 mmol) followed by zinc iodide (188 mg, 0.59 mmol) were added and the reaction mixture was stirred for 3 hours at RT. The reaction mixture was then quenched with water (10 mL) and the aqueous layer was extracted with EtOAc (2 x 20 mL). The combined organic layer was dried over anhydrous $Na_2SO_4$ and concentrated under vacuum to afford the title compound. The resulting compound was forwarded as such to the next step without any further purification. **Yield:** 55% (0.28 g, brown colour gum).

**Intermediate 135**

**Ethyl 2-aminohexanoate hydrochloride**

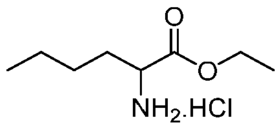

To a stirred solution of 2-aminohexanoic acid (12 g, 91 mmol) in ethanol (130 mL), thionyl chloride (33 mL, 457 mmol) was added at 0 °C and the reaction mixture was heated for 16 hours at 80° C. The reaction mixture was then concentrated under vacuum to afford the crude title compound which was used as such for next step without any further purification. **Yield:** 94% (18 g, white solid). **LCMS:** (Method B) 160.3 ($M^{+}$+H), Rt. 1.72 min, 93.76% (max).

**Intermediate 136**

**Ethyl (E)-2-(benzylideneamino)hexanoate**

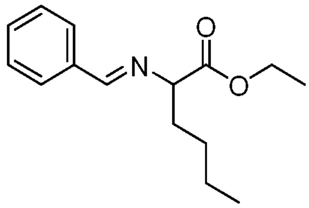

To a stirred solution of ethyl 2-aminohexanoate hydrochloride (Intermediate 135; 18 g, 92 mmol) in DCM (170 mL) at 0 °C, triethyl amine (25.6 mL, 184 mmol) was added over a period of 30 minutes. Magnesium sulfate (11.07 g, 92 mmol) was then added portionwise at 0° C. Benzaldehyde (9.37 mL, 92 mmol) was added to the reaction mixture at 0 °C over a period of 20 minutes and the reaction mixture was stirred for 16 hours at RT. After completion of the reaction (monitored by TLC), the reaction mixture was filtered through celite and the filtrate was concentrated under vacuum. The resulting crude was dissolved in petroleum ether (1000 mL), again filtered through celite and the filtrate was concentrated under vacuum to afford the title compound. The crude material was forwarded to the next step without any further purification. **Yield:** 73.5% (22 g, pale brown liquid). **LCMS:** (Method B) 248.2 ($M^{+}$+H), Rt. 2.48 min, 93.76% (max).

**Intermediate 137**

**Ethyl (E)-2-(benzylideneamino)-2-methylhexanoate**

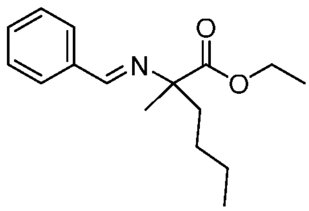

To a stirred solution of NaH (60%, 3.23 g, 81 mmol) in DMF (50 mL) at 0 °C was added a solution of ethyl (E)-2-(benzylideneamino)hexanoate (Intermediate 136; 20 g, 81 mmol) in DMF (150 mL) slowly over a period of 30 minutes, and the reaction mixture was stirred for 1.5 hours at RT. Methyl iodide (5 mL, 81 mmol) was then added at 0 °C and the reaction mixture was stirred for 1 hour at RT. The reaction mixture was then quenched with 2-propanol (10 mL) at 0 °C, and diluted with water (500 mL). The aqueous layer was extracted with petroleum ether (2 x 500 mL). The organic layer was washed with brine (200 mL) and dried over anhydrous $Na_2SO_4$. The organic part was concentrated under vacuum and the resulting crude material was forwarded to the next step without any further purification. **Yield:** 65.6% (21 g, yellow liquid).

**LCMS:** (Method B) 262.2 ($M^{+}$+H), Rt. 2.64 min, 66.47% (max).

**Intermediate 138**

**Ethyl 2-amino-2-methylhexanoate**

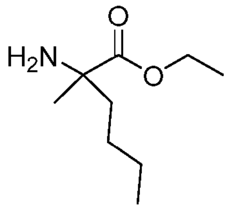

To a stirred solution of ethyl (E)-2-(benzylideneamino)-2-methylhexanoate (Intermediate 137; 30 g, 115 mmol) in petroleum ether (100 mL), dilute HCl (150 mL, 1.5 N) was added at 0 °C and the reaction mixture was stirred vigorously for 16 hours at RT. The organic layer was separated, and the aqueous layer was washed with EtOAc (2 x 100 mL). The aqueous layer was then basified (pH ~8.5) by using solid sodium bicarbonate (5 g) and extracted with EtOAc (2 x 100 mL). The organic layer was then washed with water (2 x 25 mL). The combined organic part was dried over anhydrous $Na_2SO_4$ and concentrated under vacuum to afford the title compound. The crude material was forwarded as such to the next step without any further purification. **Yield:** 39.8% (8 g, pale yellow liquid).

**LCMS:** (Method C) 174.3 ($M^{+}$+1), Rt. 1.1 min, 99.74% (max).

**Intermediate 139**

**2-Amino-2-methyl-N-phenylhexanamide**

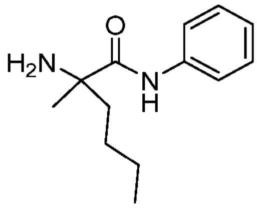

To a stirred solution of aniline (8.96 mL, 98 mmol) in THF (80 mL) at -78 °C, n-BuLi (2.5M in hexanes) (41.6 mL, 104 mmol) was added dropwise over a period of 30 minutes and the reaction mixture was stirred for 45 minutes at -25 °C to -30 °C. Ethyl 2-amino-2-methylhexanoate (Intermediate 138; 10 g, 57.7 mmol) in THF (80 mL) was then added at -78 °C and the reaction mixture was stirred for 2 hours at -78 °C. After completion of the reaction (monitored by TLC), the reaction mixture was quenched with water (200 mL) at -78 °C, and the reaction mixture was extracted with EtOAc (2 x 250 mL).The organic layer was washed with water (2 x 25 mL), dried over anhydrous $Na_2SO_4$ and concentrated under vacuum to afford the title compound as crude. The crude product was dissolved in petroleum ether (1000 mL), washed with 30% methanol in water (2 x 250 mL) and dried over anhydrous $Na_2SO_4$. The organic part was concentrated under vacuum and the resulting crude was forwarded as such to the next step without any further purification. **Yield:** 72% (15 g, yellow liquid).

**LCMS:** (Method E) 221.3 ($M^{+}$+1), Rt. 1.15 min, 61.25% (max).

**Intermediate 140**

**2-Methyl-N1-phenylhexane-1,2-diamine**

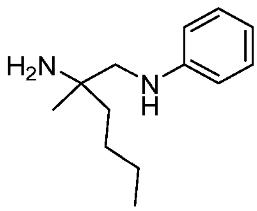

To a stirred solution of 2-amino-2-methyl-N-phenylhexanamide (Intermediate 139; 27 g, 123 mmol) in THF (250 mL), borane dimethyl sulphide complex (2M in THF, 61.3 mL, 123 mmol) was added at 0 °C and the reaction mixture was heated for 16 hours at 70 °C. After completion of the reaction (monitored by TLC), the reaction mixture was quenched with methanol (65 mL) at 0 °C and the reaction mixture was heated for 2 hours at 70 °C. The reaction mixture was then concentrated under vacuum and the obtained residue was dissolved in EtOAc (250 mL). The organic layer was washed with water (2 x 50 mL), dried over anhydrous $Na_2SO_4$ and concentrated under vacuum. The resulting crude was purified by Isolera column chromatography (eluent: 40% EtOAc in hexane; silica gel: 230-400 mesh) to afford the title compound. **Yield:** 31.3% (8 g, yellow liquid).

**$^1$H NMR** (400 MHz, DMSO-$d_6$): δ 7.07-7.03 (m, 2H), 6.62 (dd, $J$ = 1.2, 8.6 Hz, 2H), 6.52-6.52 (m, 1H), 5.30 (t, $J$ = 5.6 Hz, 1H), 2.85 (d, $J$ = 6.0 Hz, 2H), 2.26 (bs, 2H), 1.36-1.32 (m, 3H), 1.30-1.24 (m, 3H), 1.02 (s, 3H), 0.88 (t, $J$ = 6.8 Hz, 3H). **LCMS:** (Method C) 207.3 ($M^+$+H), Rt. 1.44 min, 99.59% (max).

**Intermediate 141**

**1,2-bis(2,4-dibromo-5-methoxyphenyl)disulfane**

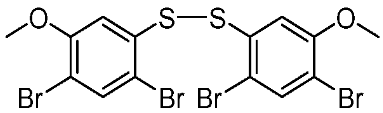

To a stirred solution of 3-methoxybenzenethiol (100 g, 0.7 mol) in methanol (1000 mL), bromine (73 mL, 1.4 mol) was added dropwise at 0 °C and the reaction mixture was stirred for 24 hours at RT. The reaction mixture was evaporated under vacuum and the obtained crude was diluted with EtOAc (2000 mL) and washed with water (2 x 500 mL). The organic layer was dried over anhydrous $Na_2SO_4$ and concentrated under vacuum. The resulting crude was dissolved in glacial acetic acid (600 mL), bromine (20 mL) was added dropwise at RTand the reaction mixture was stirred for 2 hours at RT. The obtained solid was filtered off, triturated with DCM and dried under vacuum to afford the pure title compound. **Yield:** 37% (78 g, white solid).

**$^1$H NMR** (400 MHz, DMSO-$d_6$): δ 7.69 (s, 2H), 7.17 (s, 2H), 3.84 (s, 6H).

**Intermediate 142**

**2,4-Dibromo-5-methoxybenzenesulfonyl chloride**

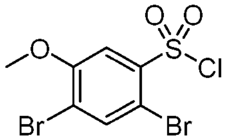

To a stirred suspension of 1,2-bis(2,4-dibromo-5-methoxyphenyl)disulfane (Intermediate 141; 20.0 g, 33.67 mmol) and potassium nitrate (17.02 g, 168.35 mmol) in acetonitrile (200 mL), sulfuryl chloride (13.6 mL, 168.35 mmol) was dropwise added at 0 °C and the reaction mixture was stirred for 24 hours at RT. After completion of the reaction (monitored by TLC), the reaction mixture was poured onto crushed ice and the solid obtained was filtered off. The obtained solid was washed with water and dried under vacuum to afford the pure title compound. **Yield:** 91% (22.5 g, white solid).

**$^1$H NMR** (400 MHz, DMSO-$d_6$): δ 8.05 (s, 1H), 7.66 (s, 1H), 4.01 (s, 3H).

**Intermediate 143**

**2,4-Dibromo-5-methoxy-N-(2-methyl-1-(phenylamino)hexan-2-yl)benzenesulfonamide**

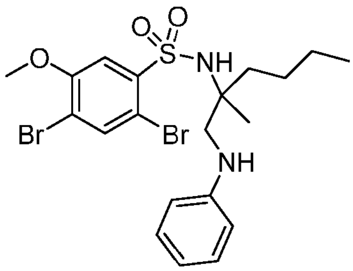

To a stirred solution of 2-methyl-N1-phenylhexane-1,2-diamine (Intermediate 140; 10 g, 48.5 mmol) in THF (100 mL), 2,4-dibromo-5-methoxybenzenesulfonyl chloride (Intermediate 142; 21.2 g, 58.2 mmol) and triethyl amine (20.3 mL, 145 mmol) were added at 0 °C and the reaction mixture was stirred for 16 hours at RT. After completion of the reaction (monitored by TLC), the reaction mixture was diluted with EtOAc (250 mL)- The organic layer was washed with water (2 x 50 mL) and dried over anhydrous $Na_2SO_4$. The organic part was concentrated under vacuum and the resulting crude was purified by Isolera column chromatography (eluent: 10% EtOAc/PE; silica gel: 230-400 mesh) to afford the title compound. **Yield:** 60.4% (23 g, white solid).

**LCMS:** (Method C) 533.1 ($M^+$+H), Rt. 2.71 min, 79.63% (max).

**Intermediate 144**

**7-Bromo-3-butyl-8-methoxy-3-methyl-5-phenyl-2,3,4,5-tetrahydro-1,2,5-benzothiadiazepine 1,1-dioxide**

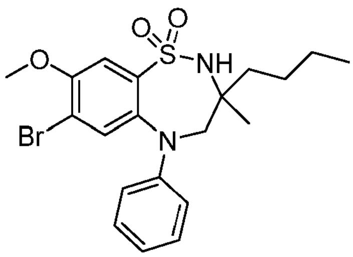

To a stirred solution of 2,4-dibromo-5-methoxy-N-(2-methyl-1-(phenylamino)hexan-2-yl)benzenesulfonamide (Intermediate 143; 15 g, 28.1 mmol) in DMF (100 mL), potassium carbonate (11.6 g, 84.3 mmol) and copper powder (885 mg, 14.05 mmol) were added and the reaction mixture was heated for 24 hours at 150 °C. The reaction mixture was then filtered through celite and washed with EtOAc (250 mL). The filtrate part was concentrated under vacuum and the resulting crude was purified by Isolera column chromatography (eluent: 20% EtOAc/PE; silica gel: 230-400 mesh) to afford the title compound. **Yield:** 66.5% (9 g, white solid).

**$^1$H NMR** (400 MHz, DMSO-$d_6$): δ 7.40-7.36 (m, 2H), 7.27 (t, $J$ = 8.0 Hz, 2H), 7.16 (bs, 1H), 7.10-7.05 (m, 2H), 6.94-6.92 (m, 1H), 3.90 (s, 3H), 3.31 (s, 2H), 1.71-1.61 (m, 1H), 1.35-1.30 (m, 2H), 1.18-1.10 (m, 6H), 0.78 (t, $J$ = 6.8 Hz, 3H). **LCMS:** (Method E) 455.1 ($M^+$+2), Rt. 2.54 min, 94.22% (max).

**Intermediate 145**

**7-Bromo-3-butyl-8-methoxy-2-(4-methoxybenzyl)-3-methyl-5-phenyl-2,3,4,5-tetrahydro-1,2,5-benzothiadiazepine 1,1-dioxide**

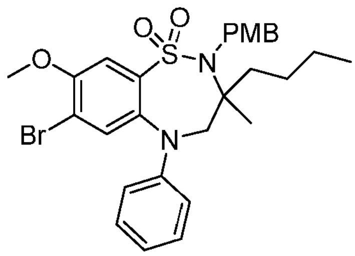

To a stirred solution of 7-bromo-3-butyl-8-methoxy-3-methyl-5-phenyl-2,3,4,5-tetrahydro-1,2,5-benzothiadiazepine 1,1-dioxide (Intermediate 144; 2.5 g, 5.51 mmol) in DMF (25 mL) at 0 °C, $Cs_2CO_3$ (3.59 g, 11.03 mmol) was added. 1-(Chloromethyl)-4-methoxybenzene (1.30 g, 8.27 mmol) was then added dropwise and the reaction mixture was stirred for 16 hours at RT. After completion of the reaction (monitored by TLC), the reaction mixture was diluted with water (50 mL) and the aqueous layer was extracted with EtOAc (3 x 50 mL). The combined organic layer was washed with brine (50 mL), dried over anhydrous $Na_2SO_4$, filtered and concentrated under reduced pressure. The resulting crude was purified by Isolera column chromatography (eluent: 20% EtOAc/PE; silica gel: 230-400 mesh) to afford the title compound. **Yield:** 70% (2.25 g, brown solid).

**LCMS:** (Method C) 575.3 ($M^{+}$+2), Rt.2.59 min, 98.8% (Max).

**Intermediate 146**

**3-Butyl-7-(dimethylamino)-8-methoxy-2-(4-methoxybenzyl)-3-methyl-5-phenyl-2,3,4,5-tetrahydro-1,2,5-benzothiadiazepine 1,1-dioxide**

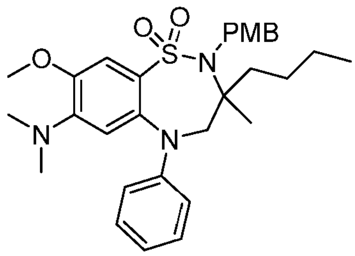

To a stirred solution of 7-bromo-3-butyl-8-methoxy-2-(4-methoxybenzyl)-3-methyl-5-phenyl-2,3,4,5-tetrahydro-1,2,5-benzothiadiazepine 1,1-dioxide (Intermediate 145; 1g, 1.74 mmol) in 1,4-dioxane (10 ml) were added dimethylamine (40% in aqueous solution, 0.4 mL, 3.49 mmol), sodium *tert*-butoxide (1.01 g, 10.46 mmol), $Pd_2(dba)_3$ (0.16 g, 0.17 mmol) and RuPhos (0.16 g, 0.35 mmol) and the reaction mixture was stirred for 2 hours at 100 °C. After completion of the reaction (monitored by LCMS), the reaction mixture was diluted with water (50 mL) and the aqueous layer was extracted with EtOAc (3 x 100 mL). The combined organic layer was washed with brine (50 mL), dried over anhydrous $Na_2SO_4$, filtered and concentrated under reduced pressure. The resulting crude was purified by Isolera column chromatography (eluent: 0-30 % EtOAc/PE; silica gel: 230-400 mesh) to afford the title compound. **Yield:** 16% (0.6 g, brown solid).

**LCMS:** (Method E) 538.2 ($M^{+}$+H), Rt.1.78 min, 24.7% (Max).

**Intermediate 147**

**3-Butyl-7-(dimethylamino)-8-hydroxy-2-(4-methoxybenzyl)-3-methyl-5-phenyl-2,3,4,5-tetrahydro-1,2,5-benzothiadiazepine 1,1-dioxide**

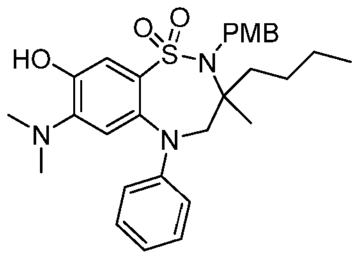

To a stirred solution of 3-butyl-7-(dimethylamino)-8-methoxy-2-(4-methoxybenzyl)-3-methyl-5-phenyl-2,3,4,5-tetrahydro-1,2,5-benzothiadiazepine 1,1-dioxide (Intermediate 146; 1.2 g, 2.23 mmol) in DMF (20 mL) at 0 °C, sodium thiomethoxide (0.78 g, 11.16 mmol) was added portionwise and the reaction mixture was stirred for 16 hours at RT. After completion of the reaction (monitored by UPLC), the reaction mixture was diluted with water (50 mL) and the aqueous layer was extracted with EtOAc (3 x 50 mL). The combined organic layer was washed with brine (50 mL), dried over anhydrous $Na_2SO_4$, filtered and concentrated under reduced pressure. The resulting crude was purified by Isolera column chromatography (eluent: 0-30 % EtOAc/PE; silica gel: 230-400 mesh) to afford the title compound. **Yield:** 75% (1.1 g, off-white solid).

**LCMS:** (Method E) 524.3 ($M^+$+H), Rt 2.02 min, 79.23 % (Max).

**Intermediate 148**

**3-Butyl-7-(dimethylamino)-2-(4-methoxybenzyl)-3-methyl-1,1-dioxido-5-phenyl-2,3,4,5-tetrahydro-1,2,5-benzothiadiazepin-8-yl trifluoromethanesulfonate**

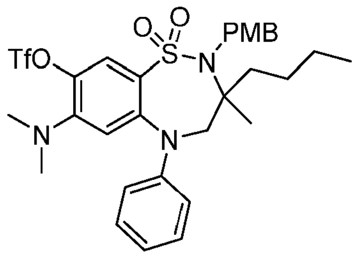

To a stirred solution of 3-butyl-7-(dimethylamino)-8-hydroxy-2-(4-methoxybenzyl)-3-methyl-5-phenyl-2,3,4,5-tetrahydro-1,2,5-benzothiadiazepine 1,1-dioxide (Intermediate 147; 1.8 g, 3.44 mmol) in DCM (10 mL) at 0 °C, pyridine (0.56 mL, 6.87 mmol) and triflic anhydride (0.87 mL, 5.16 mmol) were added dropwise and the reaction mixture was stirred for 1 hour at RT. After completion of the reaction (monitored by TLC), the reaction mixture was diluted with water (50 mL) and the aqueous layer was extracted with EtOAc (3 x 100 mL). The combined organic layer was washed with brine (50 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 45% (2.0 g, off-white solid).

**LCMS:** (Method E) 656.1 ($M^+$+H), Rt 2.09 min, 50.82% (Max).

**Intermediate 149**

**Methyl 3-butyl-7-(dimethylamino)-2-(4-methoxybenzyl)-3-methyl-5-phenyl-2,3,4,5-tetrahydro-1,2,5-benzothiadiazepine-8-carboxylate 1,1-dioxide**

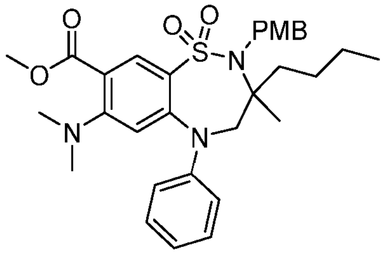

To a stirred solution of 3-butyl-7-(dimethylamino)-2-(4-methoxybenzyl)-3-methyl-1,1-dioxido-5-phenyl-2,3,4,5-tetrahydro-1,2,5-benzothiadiazepin-8-yl trifluoromethanesulfonate (Intermediate 148; 2 g, 3.05 mmol) in MeOH (10 mL) and DMF (5 mL), triethylamine (0.85 mL, 6.10 mmol), $Pd_2(dba)_3$ (0.28 g, 0.31 mmol) and dppf (0.17 g, 0.31 mmol) were added under nitrogen and the reaction mixture was stirred for 16 hours at 75 °C in a tinyclave, under 5 kg carbon monoxide gas pressure. After completion of the reaction (monitored by TLC), the reaction mixture was filtered through a celite bed and the filtrate was concentrated under vacuum. The resulting crude was purified by Isolera column chromatography (eluent: 0-30% EtOAc/PE; silica gel: 230-400 mesh) to afford the title compound. **Yield:** 21% (2.0 g, brown solid).

**LCMS:** (Method E) 566.2 ($M^+$+H), Rt 2.35 min, 22.25% (Max).

**Intermediate 150**

**3-Butyl-7-(dimethylamino)-8-(hydroxymethyl)-2-(4-methoxybenzyl)-3-methyl-5-phenyl-2,3,4,5-tetrahydro-1,2,5-benzothiadiazepine 1,1-dioxide**

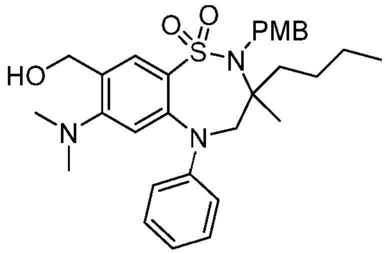

To a stirred solution of methyl 3-butyl-7-(dimethylamino)-2-(4-methoxybenzyl)-3-methyl-5-phenyl-2,3,4,5-tetrahydro-1,2,5-benzothiadiazepine-8-carboxylate 1,1-dioxide (Intermediate 149; 1.5 g, 2.65 mmol) in THF (10 mL), $LiAlH_4$ (2M in THF, 1.33 mL, 2.65 mmol) was added dropwise at 0 °C and the reaction mixture was stirred for 30 minutes at RT. After completion of the reaction (monitored by TLC), the reaction mixture was quenched with saturated ammonium chloride solution (10 mL) at 0 °C and the aqueous layer was extracted with EtOAc (2 x 20 mL). The organic layer was dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 52% (1 g, off-white solid).

**LCMS:** (Method E) 538.3 ($M^+$+H), Rt 2.02 min, 74.56% (Max).

**Intermediate 151**

**Ethyl 2-(((3-butyl-7-(dimethylamino)-2-(4-methoxybenzyl)-3-methyl-1,1-dioxido-5-phenyl-2,3,4,5-tetrahydro-1,2,5-benzothiadiazepin-8-yl)methyl)thio)acetate**

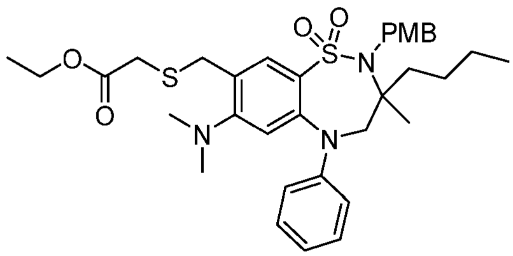

To a stirred solution of 3-butyl-7-(dimethylamino)-8-(hydroxymethyl)-2-(4-methoxybenzyl)-3-methyl-5-phenyl-2,3,4,5-tetrahydro-1,2,5-benzothiadiazepine 1,1-dioxide (Intermediate 150; 1.0 g, 1.86 mmol) in DCM (10 mL) at 0 °C, ethyl 2-mercaptoacetate (0.22 g, 1.86 mmol) and zinc(II) iodide (0.59 g, 1.86 mmol) were added and the reaction mixture was stirred for 3 hours at RT. After completion of the reaction (monitored by TLC), the reaction mixture was concentrated under vacuum and the resulting crude was quenched with water (25 mL). The aqueous layer was extracted with EtOAc (2 x 25 mL), and the combined organic layer was dried over anhydrous $Na_2SO_4$, filtered and concentrated under vacuum. The resulting crude was forwarded to the next step without any further purification.

**Intermediate 152**

**2-(((3-Butyl-7-(dimethylamino)-2-(4-methoxybenzyl)-3-methyl-1,1-dioxido-5-phenyl-2,3,4,5-tetrahydro-1,2,5-benzothiadiazepin-8-yl)methyl)thio)acetic acid**

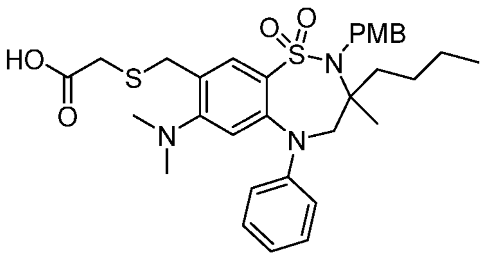

To a stirred solution of ethyl 2-(((3-butyl-7-(dimethylamino)-2-(4-methoxybenzyl)-3-methyl-1,1-dioxido-5-phenyl-2,3,4,5-tetrahydro-1,2,5-benzothiadiazepin-8-yl)methyl)thio)acetate (Intermediate 151; 1 g, 1.56 mmol) in 1,4-dioxane (5 mL), LiOH (0.13 g, 3.13 mmol) in water (5 mL) was added and the reaction mixture was stirred for 1 hour at RT. After completion of the reaction (monitored by TLC), the reaction mixture was quenched with dilute HCl (1.5 N, 15 mL) and the aqueous layer was extracted with EtOAc (2 x 25 mL). The combined organic layer was separated, dried over anhydrous $Na_2SO_4$, filtered and concentrated under vacuum. The resulting crude was purified by Isolera reverse phase column chromatography (eluent: 0-30% ACN: 5 mM ammonium bicarbonate in $H_2O$; C-18 column) to afford the title compound. **Yield:** 10% (95 mg, off-white solid).

**LCMS:** (Method B) 612.2 ($M^+$-H), Rt 1.53 min, 95.4% (Max).

**Intermediate 153**

**7-Bromo-3-butyl-8-hydroxy-2-(4-methoxybenzyl)-3-methyl-5-phenyl-2,3,4,5-tetrahydro-1,2,5-benzothiadiazepine 1,1-dioxide**

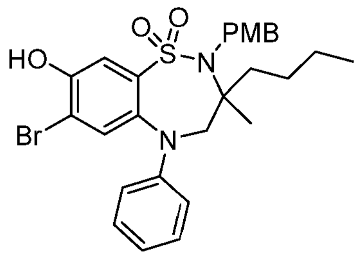

To a stirred solution of 7-bromo-3-butyl-8-methoxy-2-(4-methoxybenzyl)-3-methyl-5-phenyl-2,3,4,5-tetrahydro-1,2,5-benzothiadiazepine 1,1-dioxide (Intermediate 145; 3 g, 5.23 mmol) in DMSO (12 mL) at 0 °C, lithium chloride (2.22 g, 52.3 mmol) was added and the reaction mixture was stirred for 24 hours at 120 °C. After completion of the reaction (monitored by UPLC), the reaction mixture was quenched with ice-cold water (25 mL) and the obtained solid was collected by filtration and dried under vacuum. The resulting crude was purified by Isolera column chromatography (eluent: 0-30% EtOAc/PE; silica gel: 230-400 mesh) to afford the title compound. **Yield:** 42% (1.6 g, off-white solid). **LCMS:** (Method C), 558.2 ($M^+$), Rt. 2.44 min, 49.23% (Max).

**Intermediate 154**

**3-Butyl-8-hydroxy-7-methoxy-2-(4-methoxybenzyl)-3-methyl-5-phenyl-2,3,4,5-tetrahydro-1,2,5-benzothiadiazepine 1,1-dioxide**

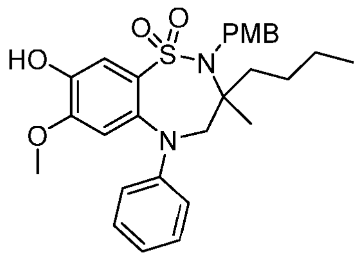

To a stirred solution of 7-bromo-3-butyl-8-hydroxy-2-(4-methoxybenzyl)-3-methyl-5-phenyl-2,3,4,5-tetrahydro-1,2,5-benzothiadiazepine 1,1-dioxide (Intermediate 153; 1 g, 1.79 mmol) in MeOH (10 mL) at 0 °C, sodium methoxide (25% in methanol, 7.70 mL, 8.94 mmol) and copper(I) bromide (0.26 g, 1.79 mmol) were added and the reaction mixture was stirred for 16 hours at 75 °C. After completion of the reaction (monitored by UPLC), the reaction mixture was diluted with water (50 mL) and the aqueous layer was extracted with EtOAc (3 x 100 mL). The combined organic layer was washed with brine (50 mL), dried over anhydrous $Na_2SO_4$, filtered and concentrated under reduced pressure. The resulting crude was purified by Isolera column chromatography (eluent: 0-30% EtOAc/PE; silica gel: 230-400 mesh) to afford the title compound. **Yield:** 54% (0.51 g, off-white solid). **LCMS:** (Method C), 511.2 ($M^+$+H), Rt. 2.30 min, 96.28% (Max).

**Intermediate 155**

**3-Butyl-7-methoxy-2-(4-methoxybenzyl)-3-methyl-1,1-dioxido-5-phenyl-2,3,4,5-tetrahydro-1,2,5-benzothiadiazepin-8-yl trifluoromethanesulfonate**

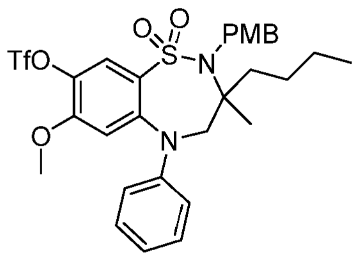

To a stirred solution of 3-butyl-8-hydroxy-7-methoxy-2-(4-methoxybenzyl)-3-methyl-5-phenyl-2,3,4,5-tetrahydro-1,2,5-benzothiadiazepine 1,1-dioxide (Intermediate 154; 0.8 g, 1.57 mmol) in DCM (10 mL) at 0 °C, pyridine (0.25 mL, 3.13 mmol) and triflic anhydride (0.32 mL, 1.88 mmol) were added dropwise and the reaction mixture was stirred for 30 minutes at RT. After completion of the reaction (monitored by TLC), the reaction mixture was quenched with water (25 mL) and the aqueous layer was extracted with DCM (2 x 25 mL). The combined organic layer was dried over anhydrous $Na_2SO_4$, filtered and concentrated under vacuum. The resulting crude material was purified by Isolera column chromatography (eluent: 0-30% EtOAc/PE; silica gel: 230-400 mesh) to afford the title compound. **Yield:** 53% (0.55 g, light yellow gum).

**Intermediate 156**

**Methyl 3-butyl-7-methoxy-2-(4-methoxybenzyl)-3-methyl-5-phenyl-2,3,4,5-tetrahydro-1,2,5-benzothiadiazepine-8-carboxylate 1,1-dioxide**

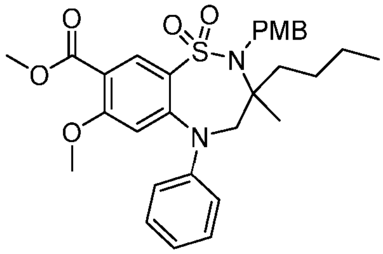

To a stirred solution of 3-butyl-7-methoxy-2-(4-methoxybenzyl)-3-methyl-1,1-dioxido-5-phenyl-2,3,4,5-tetrahydro-1,2,5-benzothiadiazepin-8-yl trifluoromethanesulfonate (Intermediate 155; 450 mg, 0.700 mmol) in MeOH (5 mL) and DMF (5 mL), triethylamine (0.09 mL, 0.70 mmol), $Pd_2(dba)_3$ (641 mg, 0.70 mmol) and dppf (388 mg, 0.70 mmol) were added under nitrogen and the reaction mixture was stirred for 16 hours at 70 °C in a tinyclave, under 5 kg carbon monoxide gas pressure. After completion of the reaction (monitored by TLC), the reaction mixture was concentrated under reduced pressure. The resulting crude was purified by Isolera column chromatography (eluent: 0-30% EtOAc/PE; silica gel: 230-400 mesh) to afford the title compound. **Yield:** 27% (0.23 g, off-white solid). **LCMS:** (Method B), 554.2 ($M^+$+2), Rt.1.8 Min, 14.1% (Max)

**Intermediate 157**

**3-Butyl-8-(hydroxymethyl)-7-methoxy-2-(4-methoxybenzyl)-3-methyl-5-phenyl-2,3,4,5-tetrahydro-1,2,5-benzothiadiazepine 1,1-dioxide**

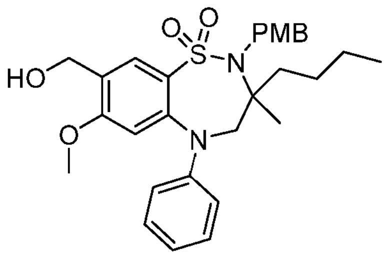

To a stirred solution of methyl 3-butyl-7-methoxy-2-(4-methoxybenzyl)-3-methyl-5-phenyl-2,3,4,5-tetrahydro-1,2,5-benzothiadiazepine-8-carboxylate 1,1-dioxide (Intermediate 156; 250 mg, 0.45 mmol) in dry THF (10 mL) at 0 °C, lithium aluminium hydride (2M in THF, 0.23 mL, 0.45 mmol) was added dropwise and the reaction mixture was stirred for 30 minutes at RT. After completion of the reaction (monitored by TLC), the reaction mixture was quenched with saturated ammonium chloride solution (10 mL) and the aqueous layer was extracted with EtOAc (2 x 15 mL). The combined organic layer was washed with water (15 mL), brine (15 mL) and dried over anhydrous $Na_2SO_4$. The organic part was filtered, concentrated under vacuum and the resulting crude was forwarded as such to the next step without any further purification. **Yield:** 55% (0.23 g, off-white solid).

**Intermediate 158**

**Ethyl 2-(((3-butyl-7-methoxy-3-methyl-1,1-dioxido-5-phenyl-2,3,4,5-tetrahydro-1,2,5-benzothiadiazepin-8-yl) methyl)thio)acetate**

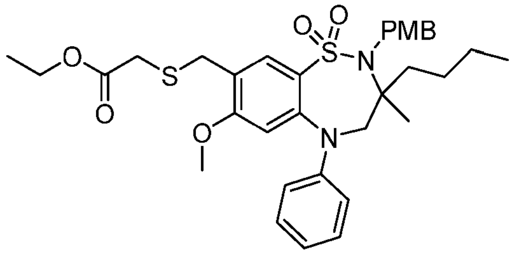

To a stirred solution of 3-butyl-8-(hydroxymethyl)-7-methoxy-2-(4-methoxybenzyl)-3-methyl-5-phenyl-2,3,4,5-tetrahydro-1,2,5-benzothiadiazepine 1,1-dioxide (Intermediate 157; 230 mg, 0.44 mmol) in DCM (6 mL) at 0 °C, zinc iodide (140 mg, 0.44 mmol) and then ethyl 2-mercaptoacetate (52.7 mg, 0.44 mmol) were added and the reaction mixture was stirred for 3 hours at RT. After completion of the reaction (monitored by TLC), the reaction mixture was quenched with water (10 mL) and the aqueous layer was extracted with DCM (2 x 20 mL). The combined organic layer was washed with water (20 mL), brine (20 mL) and dried over anhydrous $Na_2SO_4$. The organic part was filtered, concentrated under vacuum and the resulting crude material was forwarded as such to the next step without any further purification. **Yield:** 230 mg (crude, brown solid)

**Intermediate 159**

**3-Butyl-8-hydroxy-2-(4-methoxybenzyl)-3-methyl-7-(methylthio)-5-phenyl-2,3,4,5-tetrahydro-1,2,5-benzothiadiazepine 1,1-dioxide**

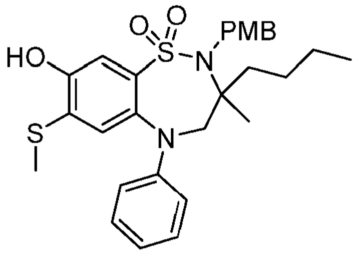

To stirred solution of 3-butyl-8-methoxy-2-(4-methoxybenzyl)-3-methyl-7-(methylthio)-5-phenyl-2,3,4,5-tetrahydro-1,2,5-benzothiadiazepine 1,1-dioxide (2 g, 3.70 mmol) in DMF (20 mL), sodium methanethiolate (1.29 g, 18.49 mmol) was added at RT and the reaction mixture was stirred for 16 hours at 90 °C. After completion of the reaction (monitored by TLC), the reaction mixture was diluted with water (100 mL) and the aqueous layer was extracted with EtOAc (3 x 150 mL). The combined organic layer was washed with brine (100 mL), dried over anhydrous $Na_2SO_4$, filtered and concentrated under reduced pressure. The resulting crude was purified by Isolera column chromatography (eluent: 0-20% EtOAc/PE; silica gel: 230-400 mesh) to afford the title compound. **Yield:** 46% (1.5 g, white solid).

**LCMS:** (Method C) 527.3 ($M^{+}$+1), Rt.2.38 min, 60.06% (Max).

**Intermediate 160**

**3-Butyl-2-(4-methoxybenzyl)-3-methyl-7-(methylthio)-1,1-dioxido-5-phenyl-2,3,4,5-tetrahydro-1,2,5-benzothiadiazepin-8-yl trifluoromethanesulfonate**

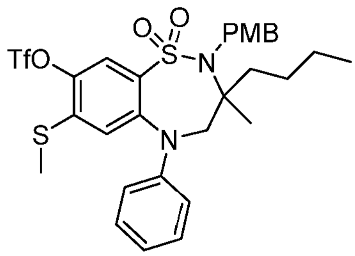

To a stirred solution of 3-butyl-8-hydroxy-2-(4-methoxybenzyl)-3-methyl-7-(methylthio)-5-phenyl-2,3,4,5-tetrahydro-1,2,5-benzothiadiazepine 1,1-dioxide (Intermediate 159; 1.5 g, 2.85 mmol) in DCM (10 mL) at 0 °C, pyridine (0.46 mL, 5.70 mmol) and trifluoromethanesulfonic anhydride (0.5 mL, 2.85 mmol) were added dropwise and the reaction mixture was stirred for 30 minutes at RT. After completion of the reaction (monitored by TLC), the reaction mixture was quenched with water (25 mL) and the aqueous layer was extracted with DCM (2 x 30 mL). The combined organic layer was separated, dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 13% (0.6 g, brown solid).

**Intermediate 161**

**Methyl 3-butyl-2-(4-methoxybenzyl)-3-methyl-7-(methylthio)-5-phenyl-2,3,4,5-tetrahydro-1,2,5-benzothiadiazepine-8-carboxylate 1,1-dioxide**

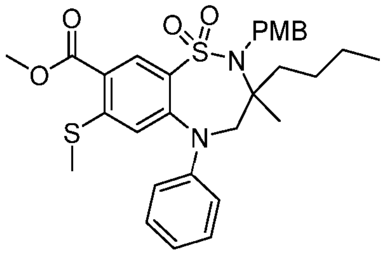

To a stirred solution of 3-butyl-2-(4-methoxybenzyl)-3-methyl-7-(methylthio)-1,1-dioxido-5-phenyl-2,3,4,5-tetrahydro-1,2,5-benzothiadiazepin-8-yl trifluoromethanesulfonate (Intermediate 160; 1.1 g, 1.67 mmol) in methanol (20 mL) and DMF (15 mL), triethylamine (0.23 mL, 1.67 mmol), $Pd_2(dba)_3$ (0.08 g, 0.08 mmol) and dppf (0.11 g, 0.20 mmol) were added under nitrogen atmosphere and the reaction mixture was stirred for 16 hours at 75 °C in a tinyclave, under 5 kg carbon monoxide gas pressure. After completion of the reaction (monitored by TLC), the reaction mixture was concentrated under vacuum and the obtained crude diluted with water (25 mL). The aqueous layer was extracted with EtOAc (3 x 25 mL), and the combined organic layer was dried over anhydrous $Na_2SO_4$, filtered and concentrated under vacuum. The resulting crude was purified by Isolera column chromatography (eluent: 0-30% EtOAc/PE; silica gel: 230-400 mesh) to afford the title compound. **Yield:** 8% (0.15 g, off-white solid).

**Intermediate 162**

**3-Butyl-8-(hydroxymethyl)-2-(4-methoxybenzyl)-3-methyl-7-(methylthio)-5-phenyl-2,3,4,5-tetrahydro-1,2,5-benzothiadiazepine 1,1-dioxide**

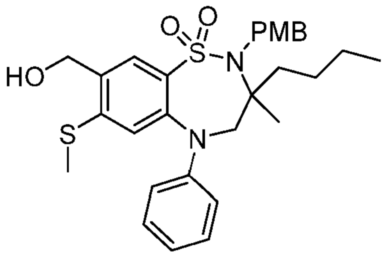

To a stirred solution of methyl 3-butyl-2-(4-methoxybenzyl)-3-methyl-7-(methylthio)-5-phenyl-2,3,4,5-tetrahydro-1,2,5-benzothiadiazepine-8-carboxylate 1,1-dioxide (Intermediate 161; 0.15 g, 0.264 mmol) in THF (10 mL), $LiAlH_4$ (2M in THF, 1.32 mL, 0.26 mmol) was added dropwise at 0 °C and the reaction mixture was stirred for 30 minutes at RT. After completion of the reaction (monitored by TLC), the reaction mixture was quenched with saturated ammonium chloride solution (10 mL) at 0 °C and the aqueous layer was extracted with EtOAc (2 x 20 mL). The organic layer was dried over anhydrous $Na_2SO_4$, filtered and concentrated under vacuum. The resulting crude was forwarded as such to the next step without any further purification. **Yield:** 150 mg (crude, light brown liquid).

**Intermediate 163**

**Ethyl 2-(((3-butyl-2-(4-methoxybenzyl)-3-methyl-7-(methylthio)-1,1-dioxido-5-phenyl-2,3,4,5-tetrahydro-1,2,5-benzothiadiazepin-8-yl)methyl)thio)acetate**

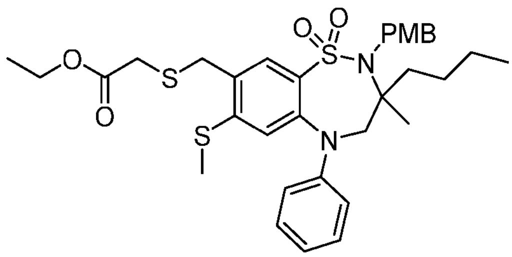

To a stirred solution of 3-butyl-8-(hydroxymethyl)-2-(4-methoxybenzyl)-3-methyl-7-(methylthio)-5-phenyl-2,3,4,5-tetrahydro-1,2,5-benzothiadiazepine 1,1-dioxide (Intermediate 162; 0.15 g, 0.28 mmol) in DCM (10 mL) at 0 °C, ethyl 2-mercaptoacetate (0.0.3 g, 0.28 mmol) and zinc(II) iodide (0.09 g, 0.28 mmol) were added and the reaction mixture was stirred for 3 hours at RT. After completion of the reaction (monitored by TLC), the reaction mixture was concentrated under vacuum and the resulting crude was quenched with water (25 mL). The aqueous layer was extracted with EtOAc (2 x 25 mL), and the combined organic layer was dried over anhydrous $Na_2SO_4$, filtered and concentrated under vacuum. The resulting crude was forwarded as such to the next step without any further purification. **Yield:** 150 mg (crude, light brown liquid).

**Example 1**

**2-(((3-butyl-3-ethyl-7-(methylthio)-1,1-dioxido-5-phenyl-2,3,4,5-tetrahydro-1,5-benzothiazepin-8-yl)methyl)thio) acetic acid**

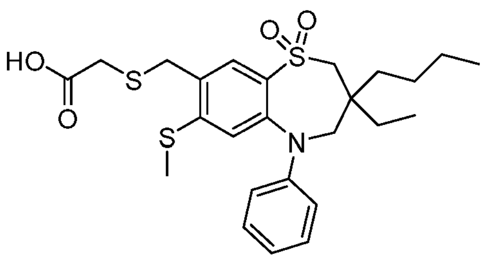

To a stirred solution of ethyl-2-(((3-butyl-3-ethyl-7-(methylthio)-1,1-dioxido-5-phenyl-2,3,4,5-tetrahydro-1,5-benzothiazepin-8-yl)methyl)thio)acetate (Intermediate 4; 70 mg, 0.13 mmol) in a mixture of 1,4-dioxane and water (2:1, 9 mL) at 0 °C, lithium hydroxide (11 mg, 0.26 mmol) was added and the reaction mixture was stirred for 1 hour at RT. After completion of the reaction (monitored by TLC), the reaction mixture was acidified with dilute HCl (1.5 N, 5 mL) and the aqueous layer was extracted with EtOAc (2 x 10 mL). The combined organic layer was washed with water (10 mL) and with brine (10 mL) and then dried over anhydrous $Na_2SO_4$. The organic part was filtered and concentrated under vacuum and the obtained material was triturated with pentane to afford the title compound. **Yield:** 58% (38 mg, off-white solid).

**$^1$H NMR** (400 MHz, DMSO-ds): δ 7.74 (s, 1H), 7.33 (t, J = 8.0 Hz, 2H), 7.22 (d, J = 7.6 Hz, 2H), 7.04 (t, J = 7.2 Hz, 1H), 6.58 (s, 1H), 3.87 (s, 2H), 3.82-3.65 (m, 2H), 3.38-3.34 (m, 2H), 3.26-3.22 (m, 2H), 2.15 (s, 3H), 1.60-1.54 (m, 1H), 1.48-1.31 (m, 3H), 1.12-0.98 (m, 4H), 0.75-0.70 (m, 6H). **LCMS:** (Method H) 506.1 ($M^+$-H) Rt. 2.15 min, 95.71% (Max). **HPLC:** (Method E) Rt. 5.90 min, 97.42% (Max).

**Example 2**

**(S)-2-(((3-butyl-3-ethyl-7-(methylthio)-1,1-dioxido-5-phenyl-2,3,4,5-tetrahydro-1,5-benzothiazepin-8-yl)methyl)thio)acetic acid and (R)-2-(((3-butyl-3-ethyl-7-(methylthio)-1,1-dioxido-5-phenyl-2,3,4,5-tetrahydro-1,5-benzothiazepin-8-yl)methyl)thio)acetic acid**

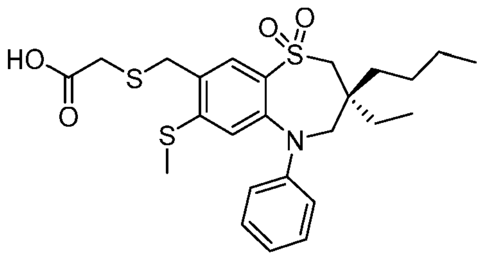

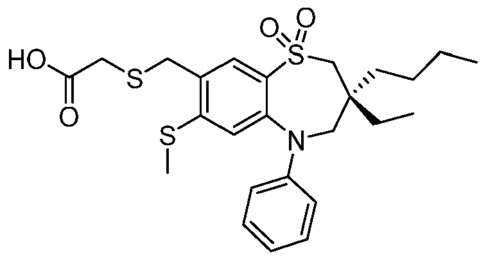

The two enantiomers of racemic 2-(((3-butyl-3-ethyl-7-(methylthio)-1,1-dioxido-5-phenyl-2,3,4,5-tetrahydro-1,5-benzothiazepin-8-yl)methyl)thio)acetic acid (Example 1; 180 mg, 0.33 mmol) were separated by chiral SFC (Method N). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 17% (30 mg, off-white solid). **$^1$H NMR** (400 MHz, DMSO-$d_6$): δ 7.74 (s, 1H), 7.32 (t, *J* = 8.4 Hz, 2H), 7.20 (d, *J* = 7.2 Hz, 2H), 7.03 (t, *J* = 7.2 Hz, 1H), 6.58 (s, 1H), 3.85 (s, 2H), 3.82-3.61 (m, 2H), 3.35-3.34 (m, 2H), 3.08 (s, 2H), 2.14 (s, 3H), 1.55-1.42 (m, 1H), 1.38-.30 (m, 3H), 1.16-0.96 (m, 4H), 0.78-0.65 (m, 6H). **LCMS:** (Method H) 506.1 ($M^+$-H), Rt. 2.15 min, 98.52% (Max). **HPLC:** (Method E) Rt. 5.88 min, 98.28% (Max). **Chiral SFC:** (Method O) Rt. 3.53 min, 98.38% (Max). Enantiomer 2: **Yield:** 16% (28 mg, off-white solid). **$^1$H NMR** (400 MHz, DMSO-$d_6$): δ 7.74 (s, 1H), 7.32 (t, *J* = 8.4 Hz, 2H), 7.20 (d, *J* = 7.2 Hz, 2H), 7.02 (t, *J* = 7.6 Hz, 1H), 6.58 (s, 1H), 3.80 (s, 2H), 3.78-3.57 (m, 2H), 3.35-3.34 (m, 2H), 3.02 (s, 2H), 2.14 (s, 3H), 1.55-1.51 (m, 1H), 1.42-1.31 (m, 3H), 1.24-0.98 (m, 4H), 0.79-0.74 (m, 6H). **LCMS:** (Method H) 506.1 ($M^+$-H), Rt. 2.14 min, 93.81% (Max). **HPLC:** (Method E) Rt. 5.88 min, 93.92% (Max). **Chiral SFC:** (Method O) Rt. 4.87 min, 94.89% (Max).

**Example 3**

**2-(((3,3-dibutyl-7-(methylthio)-1,1-dioxido-5-phenyl-2,3,4,5-tetrahydro-1,5-benzothiazepin-8-yl)methyl)thio)acetic acid**

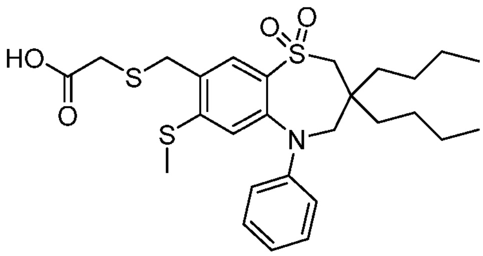

To a solution of ethyl 2-(((3,3-dibutyl-7-(methylthio)-1,1-dioxido-5-phenyl-2,3,4,5-tetrahydro-1,5-benzothiazepin-8-yl)methyl)thio)acetate (Intermediate 8; 90 mg, 0.16 mmol) in a mixture of 1,4-dioxane and water (3:1, 4 mL) at 0 °C, lithium hydroxide (13 mg, 0.32 mmol) was added and the reaction mixture was stirred for 16 hours at RT. After completion of the reaction (monitored by TLC and LCMS), the reaction mixture was quenched with dilute HCl (1.5 N, 5 mL) and the aqueous layer was extracted with EtOAc (2 x 20 mL). The combined organic layer was washed with water (5 mL) and with brine (5 mL) and then dried over anhydrous $Na_2SO_4$. The organic part was filtered, concentrated under vacuum and the resulting crude was purified by Prep HPLC purification (Method D) to afford the title compound. **Yield:** 20.46% (18 mg, white solid).

**$^1$H NMR** (400 MHz, DMSO-ds): δ 12.71 (s, 1H), 7.73 (s, 1H), 7.33 (t, *J* = 8.0 Hz, 2H), 7.25-7.23 (m, 2H), 7.05 (t, *J* = 7.2 Hz, 1H), 6.56 (s, 1H), 4.05-3.65 (m, 4H), 3.36-3.33 (m, 2H), 3.20 (s, 2H), 2.14 (s, 3H), 1.50-1.20 (m, 4H), 1.20-0.90 (m, 8H), 0.80-0.70 (m, 6H). **LCMS:** (Method B) 534.0 ($M^+$-H), Rt. 2.14 min, 97.58% (Max). **HPLC:** (Method D) Rt. 6.00 min, 97.21% (Max).

**Example 4**

**2-(((3-butyl-3-methyl-7-(methylthio)-1,1-dioxido-5-phenyl-2,3,4,5-tetrahydro-1,5-benzothiazepin-8-yl)methyl)thio)acetic acid**

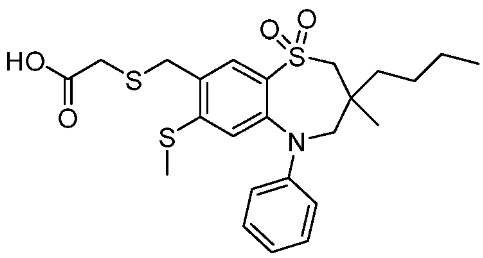

To a solution of ethyl 2-(((3-butyl-3-methyl-7-(methylthio)-1,1-dioxido-5-phenyl-2,3,4,5-tetrahydro-1,5-benzothiazepin-8-yl)methyl)thio)acetate (Intermediate 12; 0.14 g, 0.27 mmol) in a mixture of 1,4-dioxane and water (3:1, 4 mL) at 0 °C, lithium hydroxide (22 mg, 0.54 mmol) was added and the reaction mixture was stirred for 3 hours at RT. After completion of the reaction (monitored by TLC), the reaction mixture was acidified with dilute HCl (1.5 N, 5 mL) and the aqueous layer was extracted with EtOAc (2 x 20 mL). The combined organic layer was washed with water (5 mL) and with brine (10 mL) and then dried over anhydrous $Na_2SO_4$. The organic part was filtered and concentrated under vacuum to afford the title compound which was triturated with pentane. **Yield:** 91% (120 mg, white solid).

**$^1$H NMR** (400 MHz, DMSO-ds): δ 12.79 (s, 1H), 7.82 (s, 1H), 7.34 (t, *J* = 8.0 Hz, 2H), 7.19 (d, *J* = 7.2 Hz, 2H), 7.02 (t, *J* = 7.6 Hz, 1H), 6.77 (s, 1H), 3.94 (s, 2H), 3.90-3.70 (m, 2H), 3.48-3.44 (m, 2H), 3.28 (s, 2H), 2.26 (s, 3H), 1.60 - 1.45 (m, 1H), 1.42-1.35 (m, 1H), 1.30-1.06 (m, 7H), 0.81 (t, *J* = 6.8 Hz, 3H). **LCMS:** (Method B) 492.0 ($M^+$-H), Rt. 1.94 min, 99.23% (Max). **HPLC:** (Method E) Rt. 3.57 min, 99.71% (Max).

**Example 5**

**(S)-2-(((3-butyl-3-methyl-7-(methylthio)-1,1-dioxido-5-phenyl-2,3,4,5-tetrahydro-1,5-benzothiazepin-8-yl)methyl)thio)acetic acid and (R)-2-(((3-butyl-3-methyl-7-(methylthio)-1,1-dioxido-5-phenyl-2,3,4,5-tetrahydro-1,5-benzothiazepin-8-yl)methyl)thio)acetic acid**

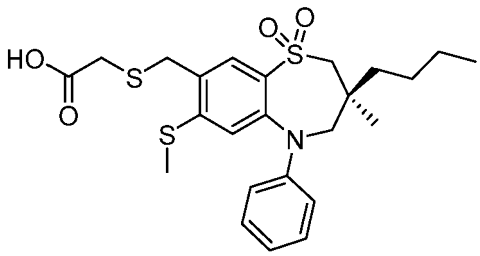
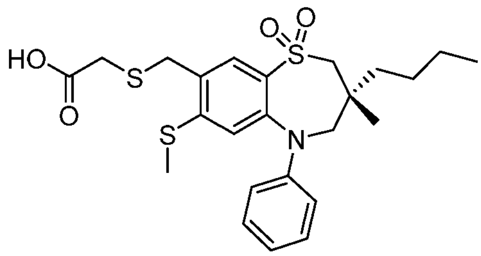

The two enantiomers of racemic 2-(((3-butyl-3-methyl-7-(methylthio)-1,1-dioxido-5-phenyl-2,3,4,5-tetrahydro-1,5-benzothiazepin-8-yl)methyl)thio)acetic acid (Example 4; 110 mg, 0.22 mmol) were separated by chiral SFC (Method M). The material was concentrated under vacuum at 45 °C. The first eluting fraction corresponded to enantiomer 1 and the second eluting fraction corresponded to enantiomer 2. The absolute configuration of the two enantiomers is not known.

Enantiomer 1: **Yield:** 20% (23 mg, off-white solid). **$^1$H NMR** (400 MHz, DMSO-$d_6$): δ 12.69 (s, 1H), 7.76 (s, 1H), 7.29 (t, *J* = 8.0 Hz, 2H), 7.13 (d, *J* = 7.6 Hz, 2H), 6.97 (t, *J* = 7.6 Hz, 1H), 6.71 (s, 1H), 3.89 (s, 2H), 3.85-3.50 (m, 2H), 3.46-3.38 (m, 2H), 3.22 (s, 2H), 2.20 (s, 3H), 1.58-1.42 (m, 1H), 1.35-1.28 (m, 1H), 1.22-1.08 (m, 4H), 1.01 (s, 3H), 0.8 (t, *J* = 6.8 Hz, 3H). **LCMS:** (Method B) 492.0 ($M^+$-H), Rt. 1.94 min, 89.98% (Max). **HPLC:** (Method E) Rt. 5.94 min, 96.82% (Max). **Chiral HPLC:** (Method P) Rt. 4.56 min, 99.26% (Max).

Enantiomer 2: **Yield:** 20% (22 mg, off-white solid). **$^1$H NMR** (400 MHz, DMSO-$d_6$): δ 12.65 (s, 1H), 7.76 (s, 1H), 7.28 (t, *J* = 7.6 Hz, 2H), 7.13 (d, *J* = 7.2 Hz, 2H), 6.97 (t, *J* = 7.2 Hz, 1H), 6.71 (s, 1H), 3.89 (s, 2H), 3.85-3.55 (m, 2H), 3.48-3.38 (m, 2H), 3.23 (s, 2H), 2.20 (s, 3H), 1.55-1.40 (m, 1H), 1.40-1.30 (m, 1H), 1.25-1.10 (m, 3H), 1.10-0.95 (m, 4H), 0.80 (t, *J* = 6.8 Hz, 3H). **LCMS:** (Method B) 492.0 ($M^+$-H), Rt. 1.948 min, 97.37% (Max). **HPLC:** (Method E) Rt. 5.94 min, 97.63% (Max). **Chiral HPLC:** (Method P) Rt. 5.83 min, 98.10% (Max).

**Example 6**

**2-(((3-butyl-3-ethyl-5-(4-fluorophenyl)-7-(methylthio)-1,1-dioxido-2,3,4,5-tetrahydro-1,5-benzothiazepin-8-yl) methyl)thio)acetic acid**

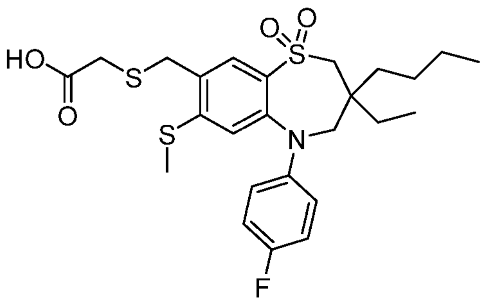

To a solution of ethyl 2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetate (Intermediate 16; 0.37 g, 0.66 mmol) in a mixture of 1,4-dioxane and water (3:1, 8 mL) at 0 °C, lithium hydroxide (56 mg, 1.33 mmol) was added and the reaction mixture was stirred for 16 hours at 80 °C. After completion of the reaction (monitored by TLC and LCMS), the reaction mixture was acidified with dilute HCl (1.5 N, 5 mL) and the aqueous layer was extracted with EtOAc (2 x 20 mL). The combined organic layer was washed with water (5 mL) and with brine (10 mL) and then dried over anhydrous $Na_2SO_4$. The organic part was filtered, concentrated under vacuum and the crude was purified by Prep HPLC (Method B) to afford the title compound. **Yield:** 14% (50 mg, off-white solid).

**$^1$H NMR** (400 MHz, DMSO-$d_6$): δ 12.65 (s, 1H), 7.72 (s, 1H), 7.31-7.30 (m, 2H), 7.19 (t, *J* = 8.8 Hz, 2H), 6.48 (s, 1H), 3.85 (s, 2H), 3.79-3.74 (m, 2H), 3.39-3.34 (m, 2H), 3.20 (s, 2H), 2.16 (s, 3H), 1.55-1.50 (m, 1H), 1.38-1.30 (m, 3H), 1.23-1.10 (m, 2H), 1.08-0.98 (m, 2H), 0.75-0.65 (m, 6H). **LCMS:** (Method H) 524.0 ($M^+$-H), Rt. 2.17 min, 94.57% (Max). **HPLC:** (Method E) Rt. 5.79 min, 95.80% (Max).

**Example 7**

**(S)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetic acid and (R)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetic acid**

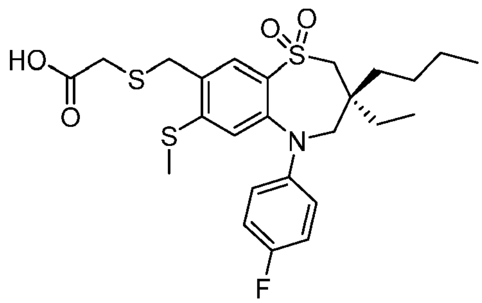

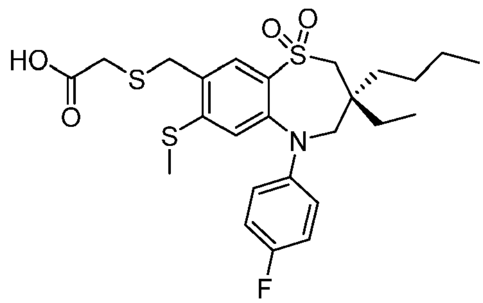

The two enantiomers of racemic 2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetic acid (Example 6; 45 mg, 0.09 mmol) were separated by chiral SFC (Method M). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 40.2% (140 mg, off-white solid).**$^1$H NMR** (400 MHz, $CD_3OD$): δ 7.84 (s, 1H), 7.31-7.28 (m, 2H), 7.14-7.10 (m, 2H), 6.59 (s, 1H), 3.90 (s, 2H), 3.86-3.79 (m, 2H), 3.38-3.36 (m, 2H), 3.27 (s, 2H), 2.18 (s, 3H), 1.63-1.60 (m, 1H), 1.53-1.50 (m, 3H), 1.48-1.46 (m, 2H), 1.30-1.24 (m, 2H), 0.89-0.70 (m, 6H). **LCMS:** (Method A) 524.0 (M+-H), Rt. 2.69 min, 90.53% (Max). **HPLC:** (Method E) Rt. 5.75 min, 99.35% (Max). **Chiral HPLC:** (Method P) Rt. 2.2 min, 99.21% (Max).

Enantiomer 2: **Yield:** 40% (140 mg, off-white solid).**$^1$H NMR** (400 MHz, DMSO-ds): 7.72 (s, 1H), 7.40-7.25 (m, 2H), 7.20-7.15 (m, 2H), 6.48 (s, 1H), 3.85 (s, 2H), 3.82-3.65 (m, 2H), 3.38-3.34 (m, 2H), 3.13 (s, 2H), 2.16 (s, 3H), 1.55-1.50 (m, 1H), 1.47-1.31 (m, 3H), 1.24-1.08 (m, 2H), 0.99-0.90 (m, 2H), 0.81-0.73 (m, 6H). **LCMS:** (Method A) 524.0 ($M^+$-H), Rt. 2.68 min, 93.62% (Max). **HPLC:** (Method E) Rt. 5.78 min, 99.92% (Max). **Chiral HPLC:** (Method P) Rt. 2.69 min, 99.64% (Max).

**Example 8**

**2-(((3,3-dibutyl-7-(methylthio)-1,1-dioxido-5-phenyl-2,3,4,5-tetrahydro-1,2,5-benzothiadiazepin-8-yl)methyl)thio)acetic acid**

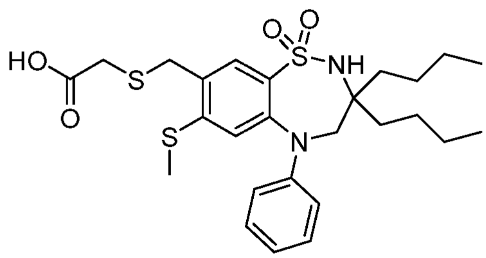

To a stirred solution of ethyl 2-(((3,3-dibutyl-7-(methylthio)-1,1-dioxido-5-phenyl-2,3,4,5-tetrahydro-1,2,5-benzothiadiazepin-8-yl)methyl)thio)acetate (Intermediate 21; 85 mg, 0.15 mmol) in a mixture of 1,4-dioxane and water (4:1, 2.5 mL) at 0 °C, lithium hydroxide (7.21 mg, 0.30 mmol) was added and the resulting reaction mixture was stirred for 3 hours at RT. After completion of the reaction (monitored by TLC), the reaction mixture was quenched with dilute HCl (1.5 N, 2 mL) and the aqueous layer was extracted with EtOAc (2 x 10 mL). The combined organic layer was washed with water (5 mL) and with brine (5 mL) and then dried over anhydrous $Na_2SO_4$. The organic part was filtered, concentrated under vacuum and the resulting crude material was purified by prep HPLC (Method E) to afford the title compound. **Yield:** 18% (15 mg, light yellow solid).

**$^1$H NMR** (400 MHz, DMSO-$d_6$): δ 12.59 (s, 1H), 7.58 (s, 1H), 7.49 (s, 1H), 7.39 (t, $J$ = 7.6 Hz, 2H), 7.31 (d, $J$ = 7.2

Hz, 2H), 7.13 (t, *J* = 6.8 Hz, 1H), 6.44 (s, 1H), 4.15 - 3.90 (m, 2H), 3.82 (s, 2H), 3.18 (s, 2H), 2.08 (s, 3H), 1.52-1.45 (m, 2H), 1.41-1.38 (m, 2H), 1.24-1.20 (m, 2H), 1.08-1.01 (m, 2H), 0.93-0.86 (m, 4H), 0.7 (t, *J* = 6.4 Hz, 6H). **LCMS:** (Method A) 535.1 ($M^+$-H), Rt. 2.96 min, 93.70% (Max). **HPLC:** (Method E) Rt. 6.18 min, 95.01% (Max).

**Example 9**

**2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid**

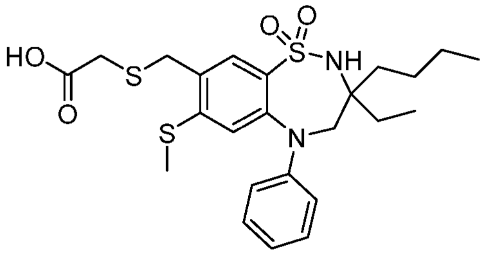

To a stirred solution of ethyl 2-(((3-butyl-3-ethyl-7-(methylthio)-1,1-dioxido-5-phenyl-2,3,4,5-tetrahydro-1,2,5-benzothiadiazepin-8-yl)methyl)thio)acetate (Intermediate 26; 100 mg, 0.19 mmol) in a mixture of 1,4-dioxane and water (5 mL, 2:1) at 0 °C, lithium hydroxide (7.82 mg, 0.19 mmol) was added and the reaction mixture was stirred for 2 hours at RT. After completion of the reaction (monitored by TLC), the reaction mixture was quenched with dilute HCl (1.5 N, 5 mL) and the aqueous layer was extracted with EtOAc (2 x 10 mL). The combined organic layer was washed with water (10 mL) and with brine (10 mL) and dried over anhydrous $Na_2SO_4$. The organic part was concentrated under vacuum and the resulting crude material was purified by Isolera column chromatography (eluent: 30-35% EtOAc/PE; silica gel: 230-400 mesh) to afford the title compound. **Yield:** 37% (36 mg, off-white solid).

**$^1$H NMR** (400 MHz, DMSO-$d_6$): δ 12.67 (s, 1H), 7.58 (s, 1H), 7.48 (s, 1H), 7.39 (t, *J* = 7.60 Hz, 2H), 7.29 (d, *J* = 6.80 Hz, 2H), 7.13 (t, *J* = 7.20 Hz, 1H), 6.44 (s, 1H), 4.01-3.98 (m, 2H), 3.82 (s, 2H), 3.18 (s, 2H), 2.08 (s, 3H), 1.65-1.51 (m, 1H), 1.45-1.35 (m, 3H), 1.24-1.05 (m, 2H), 0.89-0.71 (m, 2H), 0.70-0.68 (m, 6H). **LCMS:** (Method C) 506.9 ($M^+$-H), Rt. 2.93 min, 98.51% (Max). **HPLC:** (Method E) Rt. 5.42 min, 96.61% (Max).

**Example 10**

**(S)-2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid and (R)-2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid**

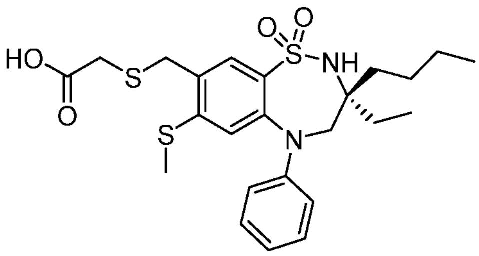

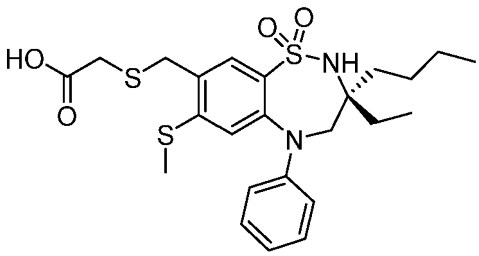

The two enantiomers of racemic compound 2-(((3-butyl-3-ethyl-7-(methylthio)-1,1-dioxido-5-phenyl-2,3,4,5-tetrahydro-1,2,5-benzothiadiazepin-8-yl)methyl)thio)acetic acid (Example 9; 30 mg, 0.06 mmol) were separated by chiral SFC (Method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 32.2% (10 mg, off-white solid). **$^1$H NMR** (400 MHz, DMSO-$d_6$): δ 12.73 (bs, 1H), 7.58 (s, 1H), 7.47 (s, 1H), 7.38 (t, *J* = 7.60 Hz, 2H), 7.29 (d, *J* = 7.60 Hz, 2H), 7.12 (t, *J* = 7.20 Hz, 1H), 6.44 (s, 1H), 4.10-3.99 (m, 2H), 3.80 (s, 2H), 3.14 (s, 2H), 2.08 (s, 3H), 1.61-1.49 (m, 1H), 1.48-1.35 (m, 3H), 1.25-1.05 (m, 2H), 0.89- 0.86 (m, 2H), 0.72-0.68 (m,

6H). **LCMS:** (Method A) 507.1 ($M^+$-H), Rt. 2.70 min, 94.57% (Max). **HPLC:** (Method E) Rt. 5.60 min, 96.63% (Max). **Chiral HPLC:** (Method P) Rt. 2.19 min, 100% (Max).

Enantiomer 2: **Yield:** 30% (9 mg, off-white solid). **$^1$H NMR** (400 MHz, DMSO-$d_6$): δ 12.63 (bs, 1H), 7.58 (s, 1H), 7.49 (s, 1H), 7.39 (t, *J* = 7.60 Hz, 2H), 7.29 (d, *J* = 7.20 Hz, 2H), 7.12 (t, *J* = 7.60 Hz, 1H), 6.44 (s, 1H), 4.02-3.98 (m, 2H), 3.81 (s, 2H), 3.16 (s, 2H), 2.08 (s, 3H), 1.62-1.52 (m, 1H), 1.48-1.35 (m, 3H), 1.24-1.05 (m, 2H), 0.89-0.86 (m, 2H), 0.71-0.68 (m, 6H). **LCMS:** (Method A) 507.0 ($M^+$-H), Rt. 2.71 min, 99.26% (Max). **HPLC:** (Method E) Rt. 5.60 min, 98.54% (Max). **Chiral HPLC:** (Method P) Rt. 2.77 min, 99.77% (Max).

**Example 11**

**2-(((3-butyl-2-methyl-7-(methylthio)-1,1-dioxido-5-phenyl-2,3,4,5-tetrahydro-1,2,5-benzothiadiazepin-8-yl) methyl)thio)acetic acid**

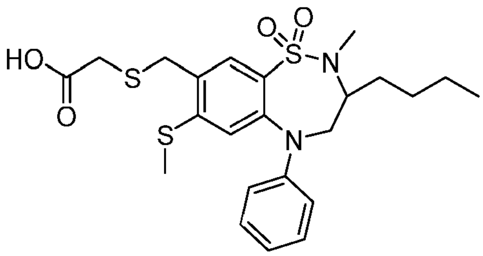

To a stirred solution of ethyl 2-(((3-butyl-2-methyl-7-(methylthio)-1,1-dioxido-5-phenyl-2,3,4,5-tetrahydro-1,2,5-benzothiadiazepin-8-yl)methyl)thio)acetate (Intermediate 30; 0.2 g, 0.38 mmol) in a mixture of 1,4-dioxane and water (6 mL, 2:1) at 0 °C, lithium hydroxide (0.02 g, 0.76 mmol) was added and the reaction mixture was stirred for 2 hours at RT. After completion of the reaction (monitored by TLC), the reaction mixture quenched with dilute HCl (1.5 N, 5 mL) and the aqueous layer was extracted with EtOAc (2 x 10 mL). The combined organic layer was washed with water (10 mL) and with brine (10 mL) and then dried over anhydrous $Na_2SO_4$. The organic part was concentrated, the resulting crude material was purified by Isolera column chromatography (eluent: 30-35% EtOAc/PE; silica gel: 230-400 mesh) to afford the title compound. **Yield:** 94% (0.18 g, off-white solid).

**$^1$H NMR** (400 MHz, DMSO-$d_6$): δ 12.68 (bs, 1H), 7.76 (s, 1H), 7.21-7.16 (m, 3H), 6.76 (t, *J* = 7.2 Hz, 1H), 6.69 (d, *J* = 8.0 Hz, 2H), 4.09-4.05 (m, 1H), 3.94 (s, 2H), 3.84-3.82 (m, 1H), 3.32-3.31 (m, 1H), 3.23 (s, 2H), 2.48 (s, 3H), 2.43 (s, 3H), 1.64-1.50 (m, 2H), 1.37-1.24 (m, 4H), 0.91 (t, *J* = 6.80 Hz, 3H). **LCMS:** (Method B) 493.0 ($M^+$-H), Rt. 2.00 min, 99.84% (Max). **HPLC:** (Method E) Rt. 5.37 min, 99.45% (Max).

**Example 12**

**(S)-2-(((3-butyl-2-methyl-7-(methylthio)-1,1-dioxido-5-phenyl-2,3,4,5-tetrahydro-1,2,5-benzothiadiazepin-8-yl) methyl)thio)acetic acid and (R)-2-(((3-butyl-2-methyl-7-(methylthio)-1,1-dioxido-5-phenyl-2,3,4,5-tetrahydro-1,2,5-benzothiadiazepin-8-yl)methyl)thio)acetic acid**

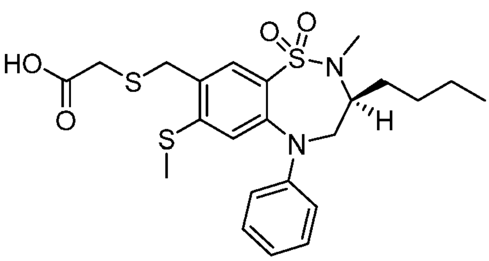

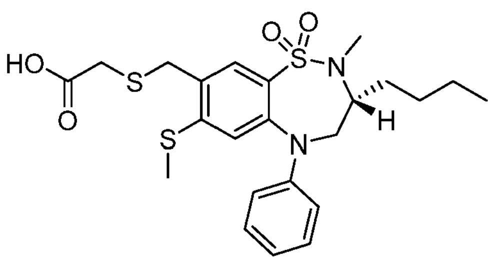

The two enantiomers of racemic 2-(((3-butyl-2-methyl-7-(methylthio)-1,1-dioxido-5-phenyl-2,3,4,5-tetrahydro-1,2,5-benzothiadiazepin-8-yl)methyl)thio)acetic acid (Example 11; 0.15 g, 0.30 mmol) were separated by chiral SFC (Method S). The material was concentrated under vacuum at 40 °C. The first eluting fraction corresponded to enantiomer 1 and the second eluting fraction corresponded to enantiomer 2. The absolute configuration of the two

enantiomers is not known.

Enantiomer 1: **Yield:** 32.2% (50 mg, white solid). **$^1$H NMR** (400 MHz, DMSO-$d_6$): δ 12.75 (bs, 1H), 7.76 (s, 1H), 7.21 - 7.16 (m, 3H), 6.76 (t, *J* = 7.2 Hz, 1H), 6.69 (d, *J* = 8.0 Hz, 2H), 4.09-4.05 (m, 1H), 3.94 (s, 2H), 3.84-3.82 (m, 1H), 3.32-3.31 (m, 1H), 3.23 (s, 2H), 2.48 (s, 3H), 2.43 (s, 3H), 1.62-1.50 (m, 2H), 1.37-1.34 (m, 4H), 0.91 (t, *J* = 6.80 Hz, 3H). **LCMS:** (Method A) 493.1 ($M^+$-H), Rt. 3.01 min, 97.16% (Max). **HPLC:** (Method E) Rt. 5.57min, 96.51% (Max). **Chiral HPLC:** (Method S) Rt. 3.66 min, 100% (Max).

Enantiomer 2: **Yield:** 32.6% (50 mg, white solid). **$^1$H NMR** (400 MHz, DMSO-$d_6$): δ 12.68 (bs, 1H), 7.76 (s, 1H), 7.21-7.15 (m, 3H), 6.76 (t, *J* = 7.2 Hz, 1H), 6.69 (d, *J* = 8.0 Hz, 2H), 4.08-4.04 (m, 1H), 3.93 (s, 2H), 3.84-3.82 (m, 1H), 3.32-3.31 (m, 1H), 3.23 (s, 2H), 2.48 (s, 3H), 2.43(s, 3H), 1.62-1.51 (m, 2H), 1.37-1.31 (m, 4H), 0.91 (t, *J* = 6.80 Hz, 3H). **LCMS:** (Method A) 493.1($M^+$-H), Rt. 2.62 min, 98.86%

(Max). **HPLC:** (Method E) Rt. 5.57 min, 97.93% (Max). **Chiral HPLC:** (Method S) Rt.4.84 min, 98.38% (Max).

**Example 13**

**2-(((3-Butyl-3-ethyl-7-methoxy-1,1-dioxido-5-phenyl-2,3,4,5-tetrahydro-1,5-benzothiazepin-8-yl)methyl)thio) acetic acid**

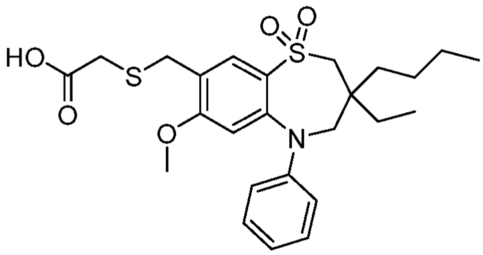

To a stirred solution of ethyl 2-(((3-butyl-3-ethyl-7-methoxy-1,1-dioxido-5-phenyl-2,3,4,5-tetrahydro-1,5-benzothiazepin-8-yl)methyl)thio)acetate (Intermediate 36; 0.15 g, 0.29 mmol) in a mixture of 1,4-dioxane and water (3:1, 8 mL) at 0 °C, lithium hydroxide (0.014 g, 0.58 mmol) was added and the reaction mixture was stirred for 1 hour at RT. After completion of the reaction (monitored by TLC), the reaction mixture quenched with dilute HCl (1.5 N, 5 mL) and the aqueous layer was extracted with EtOAc (2 x 10 mL). The combined organic layer was washed with water (10 mL) and with brine (10 mL) and dried over anhydrous $Na_2SO_4$. The organic layer was filtered, concentrated under vacuum and the resulting crude was purified by Prep-HPLC (Method D) to afford the title compound. **Yield:** 70% (0.15 g, white solid).

**$^1$H NMR** (400 MHz, DMSO-$d_6$): 12.61 (s, 1H), 7.75 (s, 1H), 7.30 (t, *J* = 7.6 Hz, 2H), 7.18 (d, *J* = 7.6 Hz, 2H), 7.00 (t, *J* = 7.2 Hz, 1H), 6.35 (s, 1H), 3.81 (s, 2H), 3.79 - 3.77 (m, 2H), 3.56 (s, 3H), 3.29 (s, 2H), 3.20 (s, 2H), 1.56-1.40 (m, 1H), 1.37-1.30 (m, 3H), 1.10-0.96 (m, 4H), 0.89-0.76 (m, 6H). **LCMS:** (Method A) 490.0 ($M^+$-H) Rt. 2.66 min, 98.37% (Max). **HPLC:** (Method B) Rt. 5.68 min, 99.00% (Max).

**Example 14**

**(S)-2-(((3-butyl-3-ethyl-7-methoxy-1,1-dioxido-5-phenyl-2,3,4,5-tetrahydro-1,5-benzothiazepin-8-yl)methyl)thio) acetic acid and (R)-2-(((3-butyl-3-ethyl-7-methoxy-1,1-dioxido-5-phenyl-2,3,4,5-tetrahydro-1,5-benzothiazepin-8-yl)methyl)thio)acetic acid**

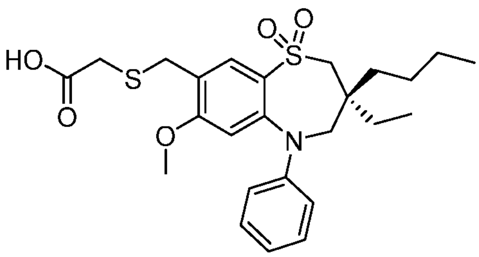

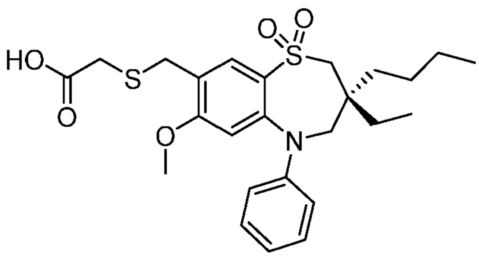

The two enantiomers of racemic 2-(((3-butyl-3-ethyl-7-methoxy-1,1-dioxido-5-phenyl-2,3,4,5-tetrahydro-1,5-benzothiazepin-8-yl)methyl)thio)acetic acid (Example 13; 0.09 g, 0.183 mmol) were separated by chiral SFC (Method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 21.82% (0.02 g, white solid). **$^1$H NMR** (400 MHz, DMSO-*d$_6$*): δ 12.61 (bs, 1H), 7.76 (s, 1H), 7.30 (t, *J* = 8.4 Hz, 2H), 7.18 (d, *J* = 7.2 Hz, 2H), 7.00 (t, *J* = 7.2 Hz, 1H), 6.36 (s, 1H), 3.95 - 3.81 (m, 1H), 3.77 (s, 3H), 3.57 (s, 3H), 3.32-3.30 (m, 2H), 3.20 (s, 2H), 1.66-1.50 (m, 1H), 1.41-1.30 (m, 3H), 1.18-1.07 (m, 4H), 0.71 (t, *J* = 6.80 Hz, 6H). **LCMS:** (Method A) 490.0 ($M^+$-H), Rt. 2.63 min, 97.91% (Max). **HPLC:** (Method E) Rt. 5.67min, 98.21% (Max). **Chiral HPLC:** (Method P) Rt. 2.62 min,100% (Max).

Enantiomer 2: **Yield:** 22.01% (20 mg, white solid). **$^1$H NMR** (400 MHz, DMSO-*d$_6$*): δ 12.61 (bs, 1H), 7.76 (s, 1H), 7.31 (t, *J* = 8.4 Hz, 2H), 7.18 (d, *J* = 8.0 Hz, 2H), 7.00 (t, *J* = 7.2 Hz, 1H), 6.36 (s, 1H), 3.95 - 3.81 (m, 1H), 3.77 (s, 3H), 3.57 (s, 3H), 3.34-3.29 (m, 2H), 3.20 (s, 2H), 1.52-1.40 (m, 1H), 1.37-1.30 (m, 3H), 1.18-1.06 (m, 4H), 0.74 (t, *J* = 6.80 Hz, 6H). **LCMS:** (Method A) 490.0 ($M^+$-H), Rt. 2.63 min, 99.01% (Max). **HPLC:** (Method E) Rt. 5.67 min, 99.09% (Max). **Chiral HPLC:** (Method P) Rt.3.37 min, 99.74% (Max).

## Example 15

**2-(((3-Butyl-3-ethyl-5-(4-fluorophenyl)-7-(methylthio)-1,1-dioxido-2,3,4,5-tetrahydro-1,5-benzothiazepin-8-yl) methyl)thio)-2-methylpropanoic acid**

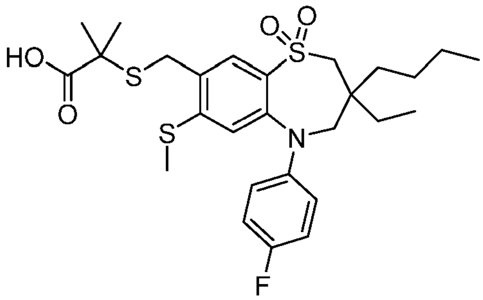

To a stirred solution of methyl 2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoate (Intermediate 37; 230 mg, 0.42 mmol) in a mixture of 1,4-dioxane and water (3:2, 5 mL) at 0 °C, a solution of lithium hydroxide (32.4 mg, 0.81 mmol) in water (1 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 15 mL), and the combined organic layer was washed with water (10 mL) and brine (10 mL). The organic part was dried over anhydrous $Na_2SO_4$, filtered and concentrated under vacuum. The resulting crude material was purified by Isolera column chromatography (eluent: 15-20% EtOAc/PE; silica gel: 230-400 mesh). **Yield:** 43% (100 mg, white solid).

**$^1$H NMR** (400 MHz, DMSO-ds): δ 12.70 (s, 1H), 7.71 (s, 1H), 7.30 (s, 2H), 7.18 (t, *J* = 8.80 Hz, 2H), 6.44 (s, 1H), 3.89-3.70 (m, 2H), 3.48-3.45 (m, 4H), 2.15 (s, 3H), 1.51-1.46 (m, 10H), 1.24-0.98 (m, 4H), 0.75-0.68 (m, 6H). **LCMS:** (Method A) 552.0 ($M^+$-H) Rt.2.12 min, 92.97% (Max). **HPLC:** (Method B) Rt. 3.90 min, 92.32% (Max).

## Example 16

**(S)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid and (R)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid**

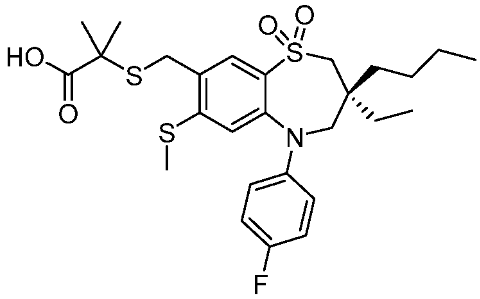 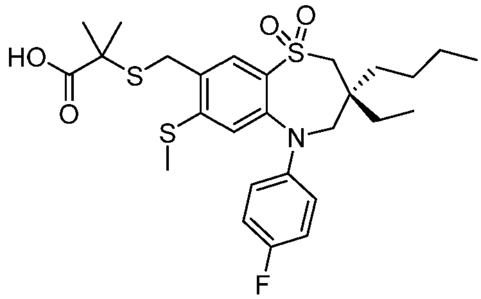

The two enantiomers of racemic 2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid (Example 15; 0.09 g, 0.16 mmol) were separated by chiral SFC (Method S). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 22% (0.02 g, white solid). **$^1$H NMR** (400 MHz, DMSO-*d$_6$*): δ 12.71 (s, 1H), 7.72 (s, 1H), 7.29 (s, 2H), 7.20-7.16 (m, 2H), 6.45 (s, 1H), 3.89 (s, 2H), 3.78-3.73 (m, 2H), 3.37 (s, 2H), 2.15 (s, 3H), 1.52-1.44 (m, 10H), 1.33-1.31 (m, 2H), 0.99-1.10 (m, 2H), 0.75-0.69 (m, 6H). **LCMS:** (Method E) 552.1 ($M^+$-H), Rt. 2.92 min, 96.30% (Max). **HPLC:** (Method B) Rt. 6.09 min, 98.09% (Max). **Chiral HPLC** (Method S): Rt. 2.65 min, 100% (Max).

Enantiomer 2: **Yield:** 28% (0.03 g, off-white solid). **$^1$H NMR** (400 MHz, DMSO-*d$_6$*): δ 12.74 (s, 1H), 7.72 (s, 1H), 7.30 (s, 2H), 7.20-7.16 (m, 2H), 6.45 (s, 1H), 3.89 (s, 2H), 3.82-3.73 (m, 2H), 3.33-3.32 (s, 2H), 2.15 (s, 3H), 1.49-1.31 (m, 10H), 1.24-0.86 (m, 4H), 0.75-0.68 (m, 6H). **LCMS:** (Method H) 552.0 ($M^+$-H), Rt. 2.27 min, 98.73% (Max). **HPLC:** (Method B) Rt. 6.16 min, 98.14% (Max). **Chiral HPLC** (Method S): Rt. 3.88 min,100% (Max).

## Example 17

**2-(((3,3-Dibutyl-7-(methylthio)-1,1-dioxido-5-phenyl-2,3,4,5-tetrahydro-1,5-benzothiazepin-8-yl)methyl)thio)-2-methylpropanoic acid**

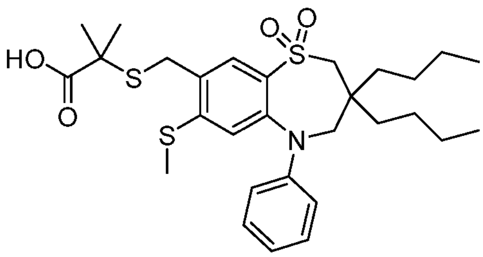

To a solution of methyl 2-(((3,3-dibutyl-7-(methylthio)-1,1-dioxido-5-phenyl-2,3,4,5-tetrahydro-1,5-benzothiazepin-8-yl)methyl)thio)-2-methylpropanoate (Intermediate 38; 0.09 g, 0.16 mmol) in a mixture of 1,4-dioxane and water (7:3,10 mL) at 0 °C, a solution of lithium hydroxide (7.46 mg, 0.31 mmol) in water (2 mL) was added and the reaction mixture was stirred for 1 hour at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 10 mL), and the combined organic layer was washed with water (10 mL) and brine (10 mL).The organic part was dried over anhydrous $Na_2SO_4$, filtered and concentrated under vacuum. The resulting crude material was purified by Isolera column chromatography (eluent: 40-60% EtOAc/PE; silica gel: 230-400 mesh) to afford the compound with 80% HPLC purity. The obtained material was re-purified by Prep HPLC (Method A) to afford the title compound. **Yield:** 23.34% (20.7 mg, off-white solid).

**$^1$H NMR** (400 MHz, DMSO-*d$_6$*): δ 12.67 (s, 1H), 7.72 (s, 1H), 7.35-7.31 (m, 2H), 7.24-7.22 (m, 2H), 7.07-7.04 (m, 1H), 6.52 (s, 1H), 3.90 (s, 2H), 3.80-3.78 (m, 2H), 2.53 (s, 2H), 2.13 (s, 3H), 1.46 (s, 8H), 1.43-1.32 (m, 2H), 1.16-0.98 (m, 8H), 0.76-0.74 (m, 6H). **LCMS:** (Method B) 562.1 ($M^+$-H) Rt. 2.25 min, 99.26% (Max). **HPLC:** (Method B) Rt. 6.67 min, 99.43% (Max).

## Example 18

**2-(((3-Butyl-3-methyl-7-(methylthio)-1,1-dioxido-5-phenyl-2,3,4,5-tetrahydro-1,5-benzothiazepin-8-yl)methyl) thio)-2-methylpropanoic acid**

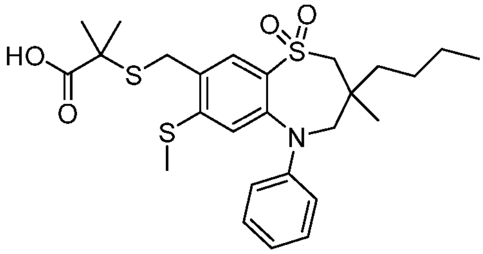

To a stirred solution of methyl 2-(((3-butyl-3-methyl-7-(methylthio)-1,1-dioxido-5-phenyl-2,3,4,5-tetrahydro-1,5-benzothiazepin-8-yl)methyl)thio)-2-methylpropanoate (Intermediate 39; 0.05 g, 0.09 mmol) in a mixture of 1,4-dioxane and water (3:2, 5 mL) at 0 °C, lithium hydroxide (4.46 mg, 0.19 mmol) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL). The organic part was dried over anhydrous $Na_2SO_4$, filtered and concentrated under vacuum. The resulting crude material was purified by Isolera column chromatography (eluent: 9% MeOH/DCM; silica gel: 230-400 mesh) to afford the title compound. **Yield:** 91% (0.04 g, white solid).

**$^1$H NMR** (400 MHz, DMSO-*d*$_6$): δ 12.70 (s, 1H), 7.75 (s, 1H), 7.28 (t, *J* = 7.60 Hz, 2H), 7.12 (d, *J* = 7.20 Hz, 2H), 6.98 (t, *J* = 7.60 Hz, 1H), 6.68 (s, 1H), 3.92 (s, 2H), 3.78-3.60 (m, 2H), 3.32 (s, 2H), 2.19 (s, 3H), 1.47 (s, 7H), 1.31-1.24 (m, 1H), 1.21-1.12 (m, 3H), 1.00 (s, 4H), 0.75 (t, *J* = 6.40 Hz, 3H). **LCMS:** (Method B) 520.0 ($M^+$-H), Rt. 2.21 min, 98.47% (Max). **HPLC:** (Method B) Rt. 5.96 min, 98.19% (Max).

**Example 19**

**(S)-2-(((3-butyl-3-methyl-7-(methylthio)-1,1-dioxido-5-phenyl-2,3,4,5-tetrahydro-1,5-benzothiazepin-8-yl) methyl)thio)-2-methylpropanoic acid and (R)-2-(((3-butyl-3-methyl-7-(methylthio)-1,1-dioxido-5-phenyl-2,3,4,5-tetrahydro-1,5-benzothiazepin-8-yl)methyl)thio)-2-methylpropanoic acid**

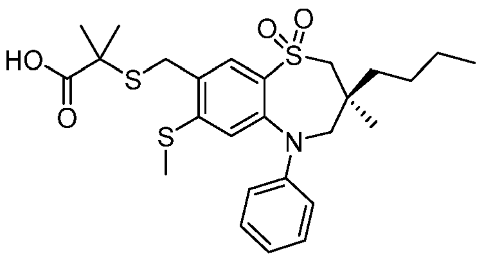

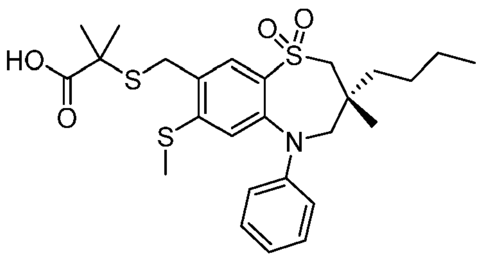

The two enantiomers of racemic 2-(((3-butyl-3-methyl-7-(methylthio)-1,1-dioxido-5-phenyl-2,3,4,5-tetrahydro-1,5-benzothiazepin-8-yl)methyl)thio)-2-methylpropanoic acid (Example 18; 0.035 g, 0.07 mmol) were separated by chiral SFC (Method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 28.2% (0.01 g, white solid). $^1$H **NMR** (400 MHz, DMSO-*d*$_6$): δ 12.70 (s, 1H), 7.75 (s, 1H), 7.28 (t, *J* = 8.00 Hz, 2H), 7.12 (d, *J* = 7.20 Hz, 2H), 6.97 (t, *J* = 7.20 Hz, 1H), 6.68 (s, 1H), 3.92 (s, 2H), 3.78-3.71 (m, 2H), 3.34 (s, 2H), 2.19 (s, 3H), 1.46 (s, 6H), 1.31-1.24 (m, 2H), 1.15-1.11 (m, 4H), 1.00 (s, 3H), 0.76 (t, *J* = 6.80 Hz, 3H). **LCMS:** (Method B) 520.1($M^+$-H) Rt. 2.01 min, 97.05% (Max). **HPLC:** (Method B) Rt. 6.00 min, 98.69% (Max). **Chiral HPLC** (Method P) Rt. 2.26 min, 99.85% (Max). Enantiomer 2: **Yield:** 28.6% (0.02 g, white solid). **$^1$H NMR** (400 MHz, DMSO-*d*$_6$): δ 12.70 (s, 1H), 7.76 (s, 1H), 7.28 (t, *J* = 8.00 Hz, 2H), 7.12 (d, *J* = 7.20 Hz, 2H), 6.96 (t, *J* = 7.60 Hz, 1H), 6.68 (s, 1H), 3.92 (s, 2H), 3.42 (s, 2H), 3.38-3.28 (m, 2H), 2.19 (s, 3H), 1.46 (s, 7H), 1.37-1.34 (m, 1H), 1.30-1.29 (m, 1H), 1.24-1.11 (m, 3H), 1.06 (s, 3H), 0.76 (t, *J* = 6.40 Hz, 3H). **LCMS:** (Method B) 520.1($M^+$-H) Rt. 2.00 min, 89.89% (Max). **HPLC:** (Method B) Rt. 5.99 min, 91.84% (Max). **Chiral HPLC** (Method P) Rt.2.75 min, 99.66% (Max).

**Example 20**

**2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid**

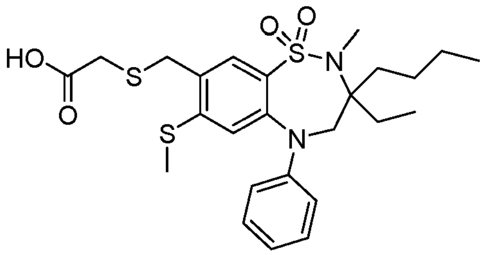

To a stirred solution of ethyl 2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetate (Intermediate 43; 35 mg, 0.06 mmol) in a mixture of 1,4-dioxane and water (3:2, 5 mL) at 0 °C, lithium hydroxide (13.33 mg, 0.32 mmol) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 30-35% EtOAc/PE; silica gel: 230-400 mesh) to afford the title compound. **Yield:** 43.9% (0.015 g, off-white solid).

**$^1$H NMR** (400 MHz, DMSO-$d_6$): δ 7.60 (s, 1H), 7.40-7.34 (m, 4H), 7.16-7.14 (m, 1H), 6.42 (s, 1H), 4.17 (s, 2H), 3.82 (s, 2H), 3.20 (s, 2H), 2.89 (s, 3H), 2.08 (s, 3H), 1.92-1.80 (m, 2H), 1.52-1.50 (m, 2H), 1.15-1.12 (m, 2H), 1.08-0.93 (m, 2H), 0.73-0.71 (m, 6H). **LCMS:** (Method A) 521.0 ($M^+$-H) Rt. 3.02 min, 98.00% (Max). **HPLC:** (Method B) Rt. 5.92 min, 97.20% (Max).

**Example 21**

**(S)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid and (R)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid**

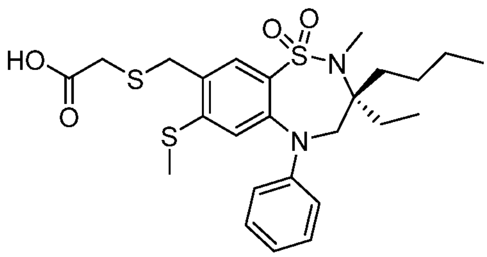

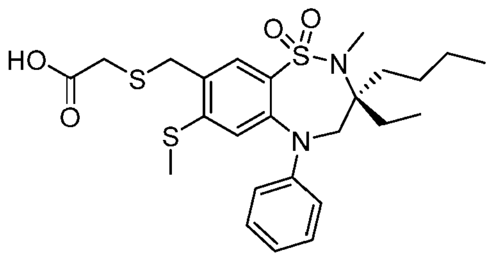

Enantiomers 1 and 2 of the title compound were obtained following the same procedure as described for Example 20, but starting from enantiomers 1 (45 mg) and 2 (47 mg) of Intermediate 44, respectively.The absolute configuration of the two enantiomers is not known.

Enantiomer 1: **Yield:** 45% (20 mg, white solid). **$^1$H NMR** (400 MHz, DMSO-$d_6$): δ 12.65 (s, 1H), 7.59 (s, 1H), 7.42-7.40 (m, 2H), 7.38-7.31 (m, 2H), 7.17-7.14 (m, 1H), 6.41 (s, 1H), 4.38-4.10 (m, 2H), 3.81 (s, 2H), 3.19 (s, 2H), 2.90 (s, 3H), 2.08 (s, 3H), 1.99-1.90 (m, 2H), 1.62-1.58 (m, 2H), 1.24-0.86 (m, 4H), 0.74-0.69 (m, 6H). **LCMS:** (Method A) 521.2 ($M^+$-H) Rt. 2.80 min, 97.05% (Max). **HPLC:** (Method B) Rt. 5.90 min, 95.94% (Max). **Chiral HPLC** (Method S) Rt. 9.04 min, 99.50% (Max).

Enantiomer 2: **Yield:** 42% (19 mg, white solid). **$^1$H NMR** (400 MHz, DMSO-$d_6$): δ 12.65 (s, 1H), 7.59 (s, 1H), 7.42-7.40 (m, 2H), 7.38-7.31 (m, 2H), 7.17-7.14 (m, 1H), 6.41 (s, 1H), 4.38-4.16 (m, 2H), 3.82 (s, 2H), 3.19 (s, 2H), 2.90 (s, 3H), 2.08 (s, 3H), 1.93-1.90 (m, 2H), 1.79-1.46 (m, 2H), 1.24-0.86 (m, 4H), 0.74-0.69 (m, 6H). **LCMS:** (Method A) 521.2 ($M^+$-H) Rt. 2.81 min, 97.87% (Max). **HPLC:** (Method B) Rt. 5.90 min, 98.43% (Max). **Chiral HPLC** (Method S) Rt. 7.18 min,

99.07%

**Example 22**

**2-(((3,3-dibutyl-2-methyl-7-(methylthio)-1,1-dioxido-5-phenyl-2,3,4,5-tetrahydro-1,2,5-benzothiadiazepin-8-yl) methyl)thio)acetic acid**

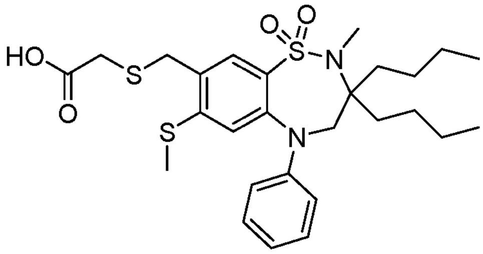

To a stirred solution of ethyl 2-(((3,3-dibutyl-2-methyl-7-(methylthio)-1,1-dioxido-5-phenyl-2,3,4,5-tetrahydro-1,2,5-benzothiadiazepin-8-yl)methyl)thio)acetate (Intermediate 50; 0.11 g, 0.19 mmol) in a mixture of 1,4-dioxane and water (3:2, 5 mL) at 0 °C, lithium hydroxide (9.10 mg, 0.38 mmol) was added and the reaction mixture was stirred for 3 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL). The organic part was dried over anhydrous $Na_2SO_4$, filtered and concentrated under vacuum. The resulting crude material was purified by Isolera column chromatography (eluent: 35% EtOAc/PE; silica gel: 230-400 mesh). **Yield**: 56.3% (0.06 g, white solid).

$^1$**H NMR** (400 MHz, DMSO-*d*$_6$): δ 12.64 (s, 1H), 7.59 (s, 1H), 7.42-7.38 (m, 2H), 7.35-7.33 (m, 2H), 7.16 (t, J = 7.20 Hz, 1H), 6.42 (s, 1H), 4.20 (s, 2H), 3.82 (s, 2H), 3.20 (s, 2H), 2.90 (s, 3H), 2.08 (s, 3H), 1.82-1.79 (m, 2H), 1.50-1.44 (m, 2H), 1.24-1.16 (m, 4H), 1.13-0.96 (m, 4H), 0.75-0.73 (m, 6H). **LCMS:** (Method A) 549.2 ($M^+$-1) Rt. 3.02 min, 97.49% (Max). **HPLC:** (Method B) Rt. 6.45 min, 98.20% (Max).

**Example 23**

**2-(((3,3-dibutyl-2-methyl-7-(methylthio)-1,1-dioxido-5-phenyl-2,3,4,5-tetrahydro-1,2,5-benzothiadiazepin-8-yl) methyl)thio)-2-methylpropanoic acid**

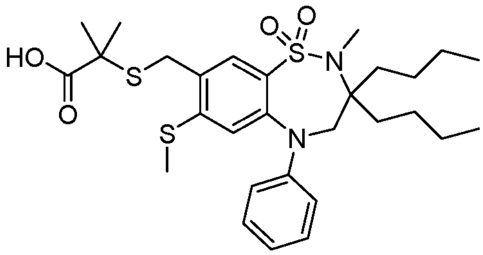

To a stirred solution of methyl 2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoate (Intermediate 51; 0.40 g, 0.68 mmol) in a mixture of 1,4-dioxane and water (3:1, 8 mL) at 0 °C, lithium hydroxide (0.03 g, 1.35 mmol) was added and the reaction mixture was stirred for 16 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 20 mL), then the combined organic layer was washed with water (10 mL) and brine (10 mL). The organic part was dried over anhydrous $Na_2SO_4$, filtered and concentrated under vacuum. The resulting crude material was purified by Isolera column chromatography (eluent: 25-30% EtOAc/PE; silica gel: 230-400 mesh) to afford the title compound. **Yield:** 66.5% (0.26 g, off-white solid).

$^1$**H NMR** (400 MHz, DMSO-*d*$_6$): δ 12.68 (s, 1H), 7.57 (s, 1H), 7.42-7.38 (m, 2H), 7.34-7.32 (m, 2H), 7.18-7.15 (m, 1H), 6.38 (s, 1H), 4.25-4.19 (m, 2H), 3.85 (s, 2H), 2.90 (s, 3H), 2.07 (s, 3H), 1.85-1.79 (m, 2H), 1.46 (s, 8H), 1.24-0.97 (m,

8H), 0.75-0.72 (m, 6H). **LCMS:** (Method A) 577.2 ($M^+$-H) Rt.3.75 min, 98.24 % (Max). **HPLC:** (Method B) Rt. 6.73 min, 97.99 % (Max).

**Example 24**

**2-(((3,3-dibutyl-2-methyl-7-(methylthio)-1,1-dioxido-5-phenyl-2,3,4,5-tetrahydro-1,2,5-benzothiadiazepin-8-yl) methyl)thio)-2-methylpropanoic acid**

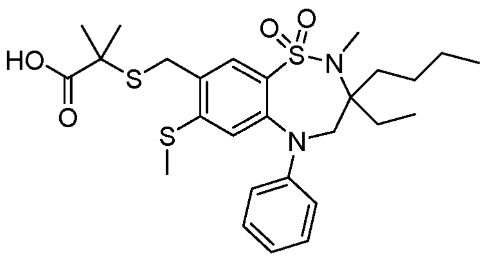

To a stirred solution of methyl 2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)-2-methylpropanoate (Intermediate 52; 0.42 g, 0.744 mmol) in a mixture of 1,4-dioxane and water (3:1, 8 mL) at 0 °C, lithium hydroxide (0.04 g, 1.49 mmol) was added and the reaction mixture was stirred for 16 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 20 mL) and the combined organic layer was washed with water (10 mL) and brine (10 mL). The organic part was dried over anhydrous $Na_2SO_4$, filtered and concentrated under vacuum. The resulting crude material was purified by Isolera column chromatography (eluent: 25-30% EtOAc/PE; silica gel: 230-400 mesh) to afford the title compound. **Yield:** 53.4% (0.22 g, off-white solid).

**$^1$H NMR** (400 MHz, DMSO-$d_6$): δ 12.69 (s, 1H), 7.58 (s, 1H), 7.40 (t, *J* = 7.20 Hz, 2H), 7.32 (d, *J* = 7.60 Hz, 2H), 7.16 (t, *J* = 7.20 Hz, 1H), 6.37 (s,1H), 4.17 (s, 2H), 3.86 (s, 2H), 2.89 (s, 3H), 2.07 (s, 3H), 1.93-1.88 (m, 2H), 1.55-1.46 (m, 8H), 1.18-1.13 (m, 2H), 1.12-1.08 (m, 2H), 0.74-0.69 (m, 6H). **LCMS:** (Method A) 549.2 ($M^+$-H) Rt. 3.52 min, 98.58% (Max). **HPLC:** (Method B) Rt. 6.43 min, 99.34% (Max).

**Example 25**

**2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid**

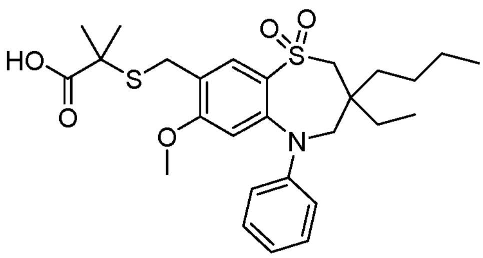

To a stirred solution of methyl 2-(((3-butyl-3-ethyl-7-methoxy-1,1-dioxido-5-phenyl-2,3,4,5-tetrahydro-1,5-benzothiazepin-8-yl)methyl)thio)-2-methylpropanoate (Intermediate 53; 0.30 g, 0.56 mmol) in a mixture of 1,4-dioxane and water (3:2, 5 mL) at 0 °C, lithium hydroxide (0.03 g, 1.12 mmol) was added and the reaction mixture was stirred for 2 hour at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL). The organic part was dried over anhydrous $Na_2SO_4$, filtered and concentrated under vacuum. The resulting crude material was purified by Isolera column chromatography (eluent: 9% MeOH/DCM; silica gel: 230-400 mesh) to afford the title compound. **Yield:** 40.7% (0.12 g, white solid).

**$^1$H NMR** (400 MHz, DMSO-ds) : δ 12.66 (s, 1H), 7.73 (s, 1H), 7.30 (t, *J* = 8.00 Hz, 2H), 7.17-7.16 (m, 2H), 7.00 (t, *J*

= 6.80 Hz, 1H), 6.34 (s, 1H), 3.81 (s, 2H), 3.75 (s, 1H), 3.57 (s, 3H), 3.29 (s, 3H), 1.51 (s, 1H), 1.50-1.37 (m, 6H), 1.35 -1.31 (m, 3H), 1.07-0.98 (m, 4H), 0.74-0.72 (m, 6H). **LCMS:** (Method A) 518.1 ($M^+$-H), Rt. 3.27 min, 97.67% (Max). **HPLC:** (Method B) Rt. 5.82 min, 99.04% (Max).

**Example 26**

**(S)-2-(((3-butyl-3-ethyl-7-methoxy-1,1-dioxido-5-phenyl-2,3,4,5-tetrahydro-1,5-benzothiazepin-8-yl)methyl) thio)-2-methylpropanoic acid and (R)-2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid**

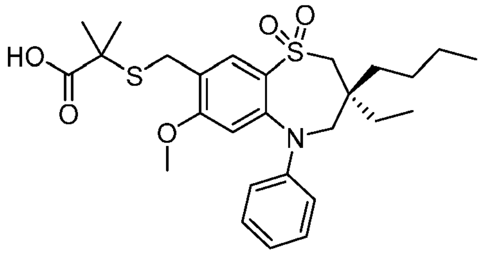

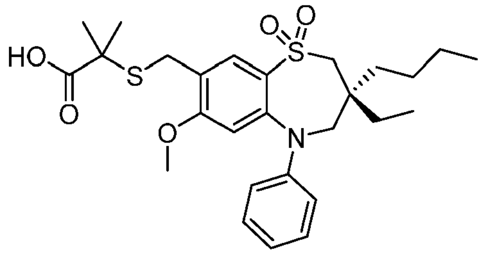

The two enantiomers of racemic 2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid (Example 25; 90 mg, 0.17 mmol) were separated by chiral SFC (method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 33% (0.03 g, white solid). **$^1$H NMR** (400 MHz, DMSO-*d*$_6$): $\delta$ 12.63 (s, 1H), 7.73 (s, 1H), 7.30 (t, *J* = 7.60 Hz, 2H), 7.17-7.16 (m, 2H), 7.01-6.98 (m, 1H), 6.33 (s, 1H), 3.81 (s, 2H), 3.78-3.74( m, 2H), 3.56 (s, 3H), 3.28 (s, 2H), 1.51-1.50 (m, 1H), 1.49-1.33 (m, 6H), 1.31-1.16 (m, 3H), 1.08-1.05 (m, 4H), 0.75-0.70 (m, 6H). **LCMS:** (Method A) 518.1 ($M^+$-H), Rt. 2.89 min, 95.55% (Max). **HPLC:** (Method B) Rt. 6.06 min, 99.00% (Max). **Chiral HPLC** (Method P): Rt. 2.39 min,100% (Max). Enantiomer 2: **Yield:** 27.3% (0.025 g, white solid). **$^1$H NMR** (400 MHz, DMSO-*d*$_6$): $\delta$ 12.59 (s, 1H), 7.73 (s, 1H), 7.30 (t, *J* = 7.60 Hz, 2H), 7.17-7.15 (m, 2H), 7.02-6.98 (m, 1H), 6.33 (s, 1H), 3.81 (s, 2H), 3.78-3.70 (m, 2H), 3.56 (s, 3H), 3.28 (s, 2H), 1.52-1.51 (m, 1H), 1.44 (s, 6H), 1.41-1.35 (m, 3H), 1.18-1.02 (m, 4H), 0.75-0.65 (m, 6H). **LCMS:** (Method A) 518.1 ($M^+$-H), Rt. 2.89 min, 98.40% (Max). **HPLC:** (Method B) Rt. 6.06 min, 98.34% (Max). **Chiral HPLC** (Method P): Rt.3.20 min,99.57% (Max).

**Example 27**

**2-(((3-butyl-7-methoxy-3-methyl-1,1-dioxido-5-phenyl-2,3,4,5-tetrahydro-1,5-benzothiazepin-8-yl)methyl)thio) acetic acid**

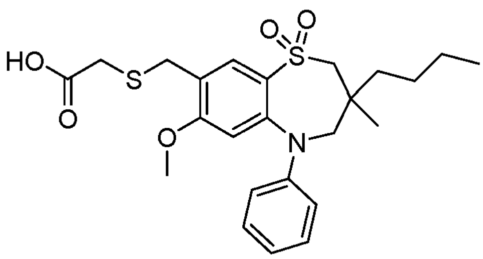

To a stirred solution of ethyl 2-(((3-butyl-7-methoxy-3-methyl-1,1-dioxido-5-phenyl-2,3,4,5-tetrahydro-1,5-benzothiazepin-8-yl)methyl)thio)acetate (Intermediate 59; 0.25 g, 0.49 mmol) in a mixture of 1,4-dioxane and water (3:2, 5 mL) at 0 °C, a solution of lithium hydroxide (0.02 g, 0.99 mmol) in water (2 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL and the combined organic layer was washed with water (5 mL) and brine (5 mL). The organic part was dried over anhydrous $Na_2SO_4$, filtered

and concentrated under vacuum. The resulting crude material was purified by Isolera column chromatography (eluent: 9% MeOH/DCM; silica gel: 230-400 mesh) to afford the title compound. **Yield:** 50.07% (0.12 g, white solid).

**$^1$H NMR** (400 MHz, DMSO-*d*$_6$): δ 12.61 (s, 1H), 7.77 (s, 1H), 7.26 (t, *J* = 7.60 Hz, 2H), 7.10 (d, *J* = 7.20 Hz, 2H), 6.95-6.91 (m, 1H), 6.49 (s, 1H), 3.79 (s, 2H), 3.62 (s, 3H), 3.37 (s, 3H), 3.33 (s, 1H), 3.21 (s, 2H), 1.45 (s, 1H), 1.33-1.27 (m, 1H), 1.24-1.11 (m, 4H), 1.00 (s, 3H), 0.78-0.75 (m, 3H). **LCMS:** (Method A) 476.0 ($M^+$-H), Rt. 2.55 min, 99.42% (Max). **HPLC:** (Method B) Rt. 5.43 min, 99.82% (Max).

**Example 28**

**(S)-2-(((3-butyl-7-methoxy-3-methyl-1,1-dioxido-5-phenyl-2,3,4,5-tetrahydro-1,5-benzothiazepin-8-yl)methyl) thio)acetic acid and (R)-2-(((3-butyl-7-methoxy-3-methyl-1,1-dioxido-5-phenyl-2,3,4,5-tetrahydro-1,5-benzothiazepin-8-yl)methyl)thio)acetic acid**

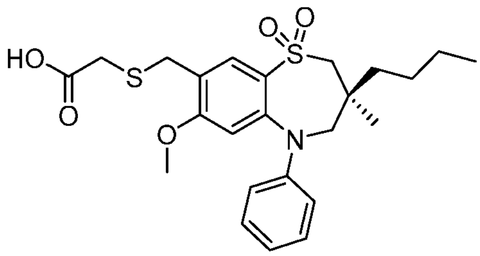

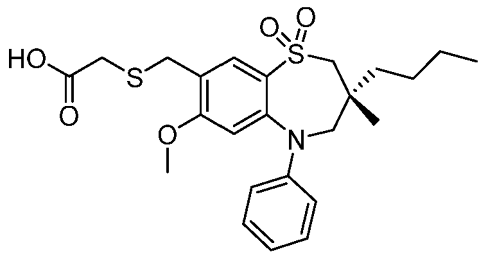

The two enantiomers of racemic 2-(((3-butyl-7-methoxy-3-methyl-1,1-dioxido-5-phenyl-2,3,4,5-tetrahydro-1,5-benzothiazepin-8-yl)methyl)thio)acetic acid (Example 27; 0.1 g, 0.21 mmol) were separated by chiral SFC (method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 29.9% (30 mg, white solid). **$^1$H NMR** (400 MHz, DMSO-*d*$_6$): δ 12.55 (s, 1H), 7.77 (s, 1H), 7.28-7.24 (m, 2H), 7.10-7.08 (m, 2H), 6.95-6.91 (m, 1H), 6.49 (s, 1H), 3.79 (s, 2H), 3.61 (s, 3H), 3.30 (s, 2H), 3.26 (s, 1H), 3.22 (s, 1H), 3.20 (s, 2H), 1.51-1.40 (m, 1H), 1.30-1.10 (m, 5H), 1.09-0.99 (m, 3H), 0.78-0.75 (m, 3H). **LCMS:** (Method A) 476.0 ($M^+$-H), Rt. 2.93 min, 98.50% (Max). **HPLC:** (Method B) Rt. 5.30 min, 99.60% (Max). **Chiral HPLC** (Method P): Rt. 2.84 min, 99.38% (Max).

Enantiomer 2: **Yield:** 25.8% (25 mg, white solid). **$^1$H NMR** (400 MHz, DMSO-*d*$_6$): δ 12.55 (s, 1H), 7.77 (s, 1H), 7.28-7.24 (m, 2H), 7.10-7.08 (m, 2H), 6.95-6.91 (m, 1H), 6.49 (s, 1H), 3.79 (s, 2H), 3.61 (s, 3H), 3.30 (s, 2H), 3.26 (s, 1H), 3.22 (s, 1H), 3.21 (s, 2H), 1.51-1.45 (m, 1H), 1.33-1.18 (m, 5H), 1.16-1.04 (m, 3H), 0.78-0.73 (m, 3H). **LCMS:** (Method A) 476.0 ($M^+$-H), Rt. 2.93 min, 97.88% (Max). **HPLC:** (Method B) Rt. 5.43 min, 98.70% (Max). **Chiral HPLC** (Method P) : Rt. 3.33 min, 99.85% (Max).

**Example 29**

**2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid**

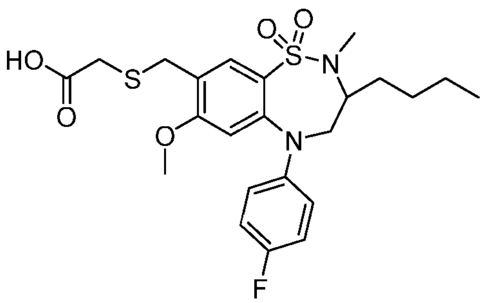

To a stirred solution of ethyl 2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetate (Intermediate 65; 0.37 g, 0.71 mmol) in a mixture of 1,4-dioxane and water (3:2, 10 mL) at 0 °C, a solution of lithium hydroxide (0.02 g, 0.99 mmol) in water (2 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 5 mL), and the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 40% EtOAc/PE; silica gel: 230-400 mesh) to afford the title compound. **Yield:** 71% (0.25 g, off-white solid).

**$^1$H NMR** (400 MHz, DMSO-$d_6$): δ 12.60 (s, 1H), 7.75 (s, 1H), 7.03 (t, *J* = 8.40 Hz, 2H), 6.88 (s, 1H), 6.75-6.71 (m, 2H), 4.03-4.00 (m, 1H), 3.77 (s, 6H), 3.37-3.33 (m, 1H), 3.18 (s, 2H), 2.47 (s, 3H), 1.57-1.50 (m, 2H), 1.34-1.29 (m, 4H), 0.92-0.89 (m, 3H). **LCMS:** (Method A) 495.0 ($M^+$-H), Rt. 2.83 min, 98.68% (Max). **HPLC:** (Method B) Rt. 5.40 min, 99.01% (Max).

**Example 30**

**(S)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid and (R)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid**

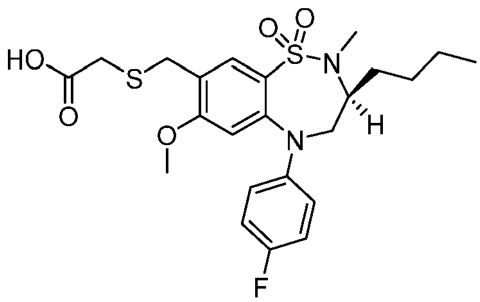

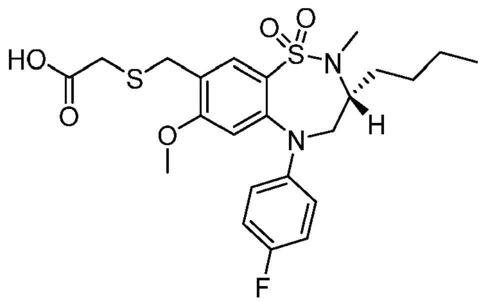

The two enantiomers of racemic 2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid (Example 29; 0.24 g, 0.48 mmol) were separated by chiral SFC (Method S). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 40% (97 mg, white solid). **$^1$H NMR** (400 MHz, DMSO-$d_6$): δ 12.73 (s, 1H), 7.75 (s, 1H), 7.03 (t, *J* = 8.80 Hz, 2H), 6.88 (s, 1H), 6.74-6.71 (m, 2H), 4.03-3.99 (m, 1H), 3.81 (s, 2H), 3.77 (m, 4H), 3.37-3.33 (m, 1H), 3.18 (s, 2H), 2.47 (s, 3H), 1.57-1.56 (m, 2H), 1.34-1.24 (m, 4H), 0.92-0.87 (m, 3H). **LCMS:** (Method A) 495.0 ($M^+$-H) Rt. 2.30 min, 97.46% (Max). **HPLC:** (Method B) Rt. 5.23 min, 98.18% (Max). **Chiral HPLC** (Method S): Rt. 4.86 min,100% (Max).

Enantiomer 2: **Yield:** 28% (20 mg, white solid). **$^1$H NMR** (400 MHz, DMSO-$d_6$): δ 12.63 (s, 1H), 7.75 (s, 1H), 7.04 (t, *J* = 8.40 Hz, 2H), 6.89 (s, 1H), 6.75-6.72 (m, 2H), 4.05-4.00 (m, 1H), 3.83 (s, 2H), 3.78 (s, 4H), 3.37-3.32 (m, 1H), 3.22 (s, 2H), 2.48 (s, 3H), 1.58-1.52 (m, 2H),1.50-1.35 (m, 4H), 0.93-0.91 (m, 3H). **LCMS:** (Method A) 495.0 ($M^+$-H), Rt. 2.30 min, 94.25% (Max). **HPLC:** (Method B) Rt. 5.23 min, 95.84% (Max). **Chiral HPLC** (Method S): Rt. 7.01 min,100% (Max).

**Example 31**

**2-(((3,3-dibutyl-7-methoxy-1,1-dioxido-5-phenyl-2,3,4,5-tetrahydro-1,5-benzothiazepin-8-yl)methyl)thio)acetic acid**

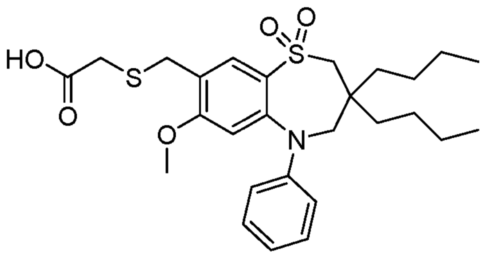

To a stirred solution of ethyl 2-(((3,3-dibutyl-7-methoxy-1,1-dioxido-5-phenyl-2,3,4,5-tetrahydro-1,5-benzothiazepin-8-yl)methyl)thio)acetate (Intermediate 71; 0.15 g, 0.27 mmol) in a mixture of 1,4-dioxane and water (3:2, 5 mL) at 0 °C, a solution of lithium hydroxide (0.013 g, 0.55 mmol) in water (2 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 5 mL), and the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 9% MeOH/DCM; silica gel: 230-400 mesh) to afford the title compound. **Yield:** 34.4% (50 mg, white solid).

**$^1$H NMR** (400 MHz, DMSO-*d*$_6$): δ 12.60 (s, 1H), 7.74 (s, 1H), 7.31 (*J* = 7.2 Hz, 2H), 7.2 (d, *J* = 7.6 Hz, 2H), 7.02 (t, *J* = 7.2 Hz, 1H), 6.32 (s, 1H), 4.1-4.08 (m, 1H), 3.76 (s, 3H), 3.55 (s, 3H), 3.30 (s, 1H), 3.19 (s, 2H), 3.18-3.16 (m, 1H), 1.42-1.28 (m, 4H), 1.16-1.00 (m, 8H), 0.78-0.70 (m, 6H). **LCMS:** (Method A) 518.2 (M$^+$-H), Rt. 3.34 min, 98.64% (Max). **HPLC:** (Method B) Rt. 6.21 min, 98.60% (Max).

### Example 32

**2-(((3,3-dibutyl-5-(4-fluorophenyl)-7-methoxy-1,1-dioxido-2,3,4,5-tetrahydro-1,5-benzothiazepin-8-yl)methyl)thio)acetic acid**

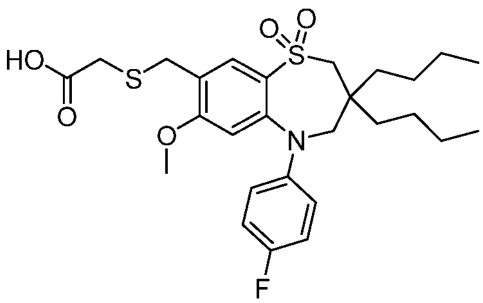

To a stirred solution of ethyl 2-(((3,3-dibutyl-5-(4-fluorophenyl)-7-methoxy-1,1-dioxido-2,3,4,5-tetrahydro-1,5-benzothiazepin-8-yl)methyl)thio)acetate (Intermediate 77; 100 mg, 0.18 mmol) in a mixture of 1,4-dioxane and water (3:1, 8 mL) at 0 °C, lithium hydroxide (16.93 mg, 0.71 mmol) was added and the reaction mixture was stirred for 1 hour at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 20 mL), and the combined organic layer was washed with water (10 mL) and brine (10 mL).The organic part was dried over anhydrous $Na_2SO_4$, filtered and concentrated under vacuum. The resulting crude material was purified by Isolera column chromatography (eluent: 15-20% EtOAc/PE; silica gel: 230-400 mesh) to afford the title compound. **Yield:** 98.7% (0.094 g, off-white solid).

**$^1$H NMR** (400 MHz, DMSO-*d*$_6$): δ 7.87 (s, 1H), 7.21-7.17 (m, 2H), 7.07 (t, *J* = 8.80 Hz, 2H), 6.14 (s, 1H), 3.83-3.80 (m, 4H), 3.61 (s, 3H), 3.25-3.23 (m, 4H), 1.51-1.48 (m, 2H), 1.44-1.38 (m, 2H), 1.20-1.09 (m, 8H), 0.83 (s, 6H). **LCMS:** (Method A) 536.1 (M$^+$-H) Rt. 3.29 min, 97.91% (Max). **HPLC:** (Method B) Rt. 6.19 min, 98.28% (Max).

### Example 33

**2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid**

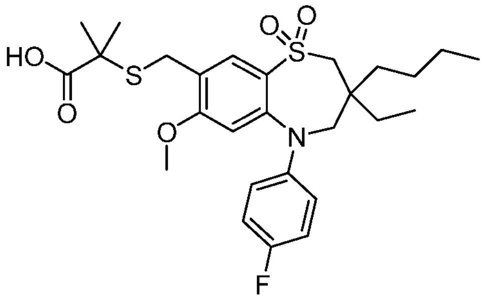

To a stirred solution of methyl 2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoate (Intermediate 83; 0.5 g, 0.91 mmol) in a mixture of 1,4-dioxane and water (3:2, 10 mL) at 0 °C, a solution of lithium hydroxide (0.02 g, 0.91 mmol) in water (5 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL). The organic part was dried over anhydrous $Na_2SO_4$, filtered and concentrated under vacuum. The resulting crude material was purified by Isolera column chromatography (eluent: 9% MeOH/DCM; silica gel: 230-400 mesh) to afford the title compound. **Yield:** 60.1% (0.3 g, white solid).

**$^1$H NMR** (400 MHz, DMSO-$d_6$): δ 12.62 (s, 1H), 7.71 (s, 1H), 7.25 (s, 2H), 7.16 (t, *J* = 9.20 Hz, 2H), 6.24 (s, 1H), 3.80 (s, 2H), 3.57 (s, 3H), 3.33 (s, 4H), 1.50-1.41 (m, 6H), 1.37-1.32 (m, 4H), 1.14-1.11 (m, 2H), 1.09-0.98 (m, 2H), 0.75-0.68 (m, 6H). **LCMS:** (Method A) 536.2 ($M^+$-H), Rt. 2.83 min, 96.97% (Max). **HPLC:** (Method B) Rt. 6.05 min, 97.72% (Max).

**Example 34**

**(S)-2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid and (R)-2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid**

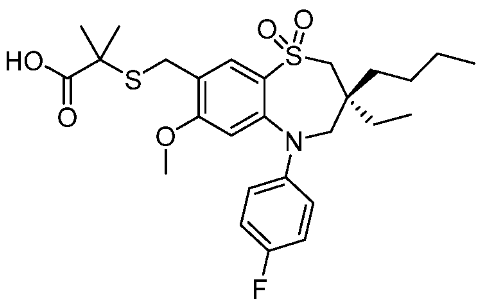

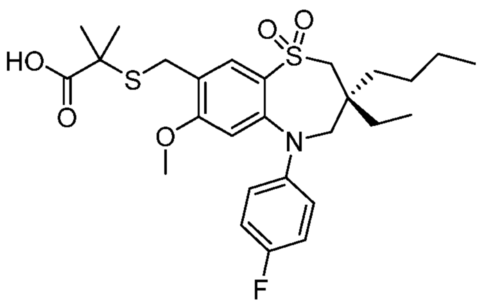

The two enantiomers of racemic 2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid (Example 33; 0.27 g, 0.50 mmol) were separated by chiral SFC (Method P). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 43.6% (0.12 g, white solid). **$^1$H NMR** (400 MHz, DMSO-$d_6$): δ 12.61 (s, 1H), 7.71 (s, 1H), 7.27-7.25 (m, 2H), 7.18-7.14 (m, 2H), 6.23 (s, 1H), 3.80 (s, 2H), 3.75-3.70 (m, 1H), 3.57 (s, 3H), 3.32 (s, 3H), 1.59-1.51 (m,1H), 1.44 (s, 6H), 1.37-1.35 (m, 3H), 1.11-0.98 (m, 4H), 0.75-0.68 (m, 6H). **LCMS:** (Method A) 536.1 ($M^+$-H), Rt. 3.22 min, 97.33% (Max). **HPLC:** (Method B) Rt. 6.05 min, 98.15% (Max). **Chiral HPLC** (Method P): Rt.1.63 min,100% (Max).

Enantiomer 2: **Yield:** 44.4% (0.12 g, white solid). **$^1$H NMR** (400 MHz, DMSO-$d_6$): δ 12.61 (s, 1H), 7.71 (s, 1H), 7.26-7.24 (m, 2H), 7.18-7.14 (m, 2H), 6.24 (s, 1H), 3.80 (s, 2H), 3.75-3.70 (m, 1H), 3.57 (s, 3H), 3.33 (s, 3H), 1.46 (s, 7H), 1.37-1.32 (m, 3H), 1.11-0.98 (m, 4H), 0.75-0.68 (m, 6H). **LCMS:** (Method A) 536.2 ($M^+$-H), Rt. 2.83 min, 99.23 % (Max). **HPLC:** (Method B) Rt. 5.83 min, 99.92% (Max). **Chiral HPLC** (Method P): Rt. 2.16 min,100% (Max).

**Example 35**

**2-(((3-butyl-7-methoxy-2-methyl-1,1-dioxido-5-phenyl-2,3,4,5-tetrahydro-1,2,5-benzothiadiazepin-8-yl)methyl) thio)acetic acid**

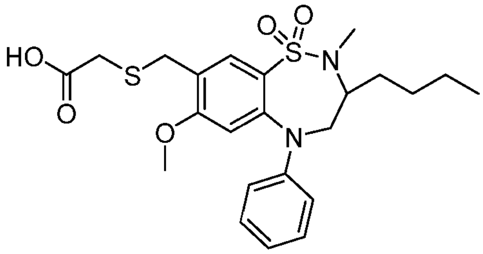

To a stirred solution of ethyl 2-(((3-butyl-7-methoxy-2-methyl-1,1-dioxido-5-phenyl-2,3,4,5-tetrahydro-1,2,5-benzothiadiazepin-8-yl)methyl)thio)acetate (Intermediate 89; 0.35 g, 0.69 mmol) in a mixture of 1,4-dioxane and water (3:2, 10 mL) at 0 °C, a solution of lithium hydroxide (0.02 g, 0.91 mmol) in water (3 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 5 mL), and the combined organic layer was washed with water (5 mL) and brine (5 mL). The organic part was dried over anhydrous $Na_2SO_4$, filtered and concentrated under vacuum. The resulting crude material was purified by Isolera column chromatography (eluent: 9% MeOH/ DCM; silica gel: 230-400 mesh) to afford the title compound. **Yield:** 26.8% (90 mg, white solid).

**$^1$H NMR** (400 MHz, DMSO-*d$_6$*): δ 12.62 (s, 1H), 7.76 (s, 1H), 7.18 (t, *J* = 7.60 Hz, 2H), 6.95 (s, 1H), 6.77-6.69 (m, 3H), 4.07-4.03 (m, 1H), 3.84 (s, 3H), 3.79 (s, 3H), 3.36 (s,1H), 3.22 (s, 2H), 2.46 (s, 3H), 1.61-1.58 (m, 2H), 1.36-1.30 (m, 4H), 0.91 (t, *J* = 6.80 Hz, 3H). **LCMS:** (Method A) 477.1 ($M^+$-H), Rt. 2.51 min, 98.20% (Max). **HPLC:** (Method B) Rt. 5.71 min, 98.46% (Max).

**Example 36**

**(S)-2-(((3-butyl-7-methoxy-2-methyl-1,1-dioxido-5-phenyl-2,3,4,5-tetrahydro-1,2,5-benzothiadiazepin-8-yl) methyl)thio)acetic acid and (R)-2-(((3-butyl-7-methoxy-2-methyl-1,1-dioxido-5-phenyl-2,3,4,5-tetrahydro-1,2,5-benzothiadiazepin-8-yl)methyl)thio)acetic acid**

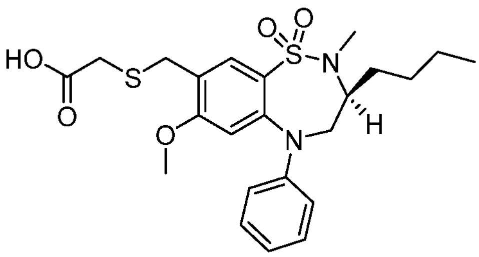

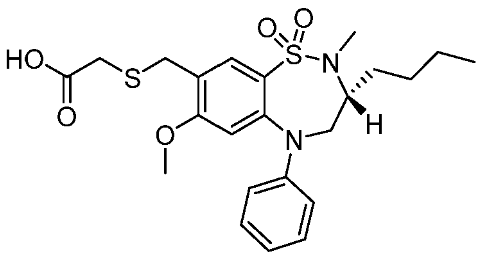

The two enantiomers of racemic 2-(((3-butyl-7-methoxy-2-methyl-1,1-dioxido-5-phenyl-2,3,4,5-tetrahydro-1,2,5-benzothiadiazepin-8-yl)methyl)thio)acetic acid (Example 35; 0.08 g, 0.17 mmol) were separated by chiral SFC (Method S). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 24.22% (20 mg, white solid). **$^1$H NMR** (400 MHz, DMSO-*d$_6$*): δ 12.67 (s, 1H), 7.82 (s, 1H), 7.26-7.23 (m, 2H), 7.01 (s, 1H), 6.81-6.78 (m, 1H), 6.76-6.74 (m, 2H), 4.22-4.11(m, 1H), 3.90 (s, 2H), 3.85 (s, 4H), 3.28 (s, 3H), 2.56 (s, 3H), 1.70-1.58 (m, 2H),1.42-1.41 (m, 4H), 0.99-0.97 (m, 3H). **LCMS:** (Method A) 477.0 ($M^+$-H), Rt. 2.88 min, 95.02% (Max). **HPLC:** (Method B) Rt. 5.18 min, 96.81% (Max). **Chiral HPLC** (Method S): Rt.3.33 min, 99.66% (Max).

Enantiomer 2: **Yield:** 18.73% (15 mg, white solid). **$^1$H NMR** (400 MHz, DMSO-*d$_6$*): δ 12.62 (s, 1H), 7.76 (s, 1H), 7.19 (t, *J* = 8.40 Hz, 2H), 6.95 (s, 1H), 6.77-6.69 (m, 3H), 4.07-4.03 (m, 1H), 3.84 (s, 2H), 3.79 (s, 4H), 3.27 (s, 1H), 3.22 (s, 2H), 2.46 (s, 3H), 1.61-1.58 (m, 1H), 1.52-1.51 (m, 1H), 1.36-1.24 (m, 4H), 0.91 (t, *J* = 7.20 Hz, 3H). **LCMS:** (Method A) 477.1 ($M^+$-H), Rt. 2.51 min, 99.60% (Max). **HPLC:** (Method B) Rt. 5.36min, 99.89% (Max). **Chiral HPLC** (Method H): Rt.

3.05 min, 99.68% (Max).

**Example 37**

**2-(((3-butyl-7-methoxy-2-methyl-1,1-dioxido-5-phenyl-2,3,4,5-tetrahydro-1,2,5-benzothiadiazepin-8-yl)methyl) thio)-2-methylpropanoic acid**

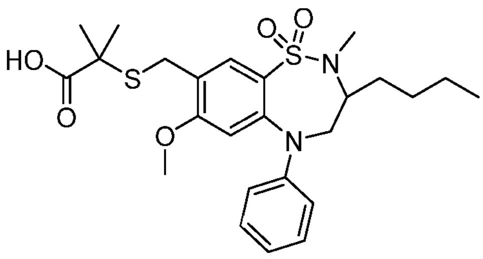

To a stirred solution of methyl 2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoate (Intermediate 90; 0.3 g, 0.58 mmol) in 1,4-dioxane (7 mL), a solution of lithium hydroxide (0.03 g, 1.15 mmol) in water (3 mL) was added and the reaction mixture was stirred for 2 hours at RT. After completion of the reaction (monitored by TLC), the reaction mixture was acidified with dilute HCl (1.5 N, pH~4) and ice-cold water (2 mL). The aqueous layer was extracted with EtOAc (2 x 5 mL), and the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 9% MeOH/DCM; silica gel: 230-400 mesh) **Yield:** 65.1% (0.2 g, white solid).

**$^1$H NMR** (400 MHz, DMSO-$d_6$): δ 12.66 (s, 1H), 7.75 (s, 1H), 7.18 (t, *J* = 8.40 Hz, 2H), 6.92 (s, 1H), 6.77-6.67 (m, 3H), 4.06-4.02 (m, 1H), 3.88 (s, 2H), 3.79 (s, 4H), 3.25 (s, 1H), 2.45 (s, 3H), 1.61-1.56 (m, 1H), 1.46 (s, 6H), 1.41 (s, 1H), 1.36-1.30 (m, 4H), 0.91 (t, *J* = 6.80 Hz, 3H). **LCMS:** (Method A) 505.1 ($M^+$-H), Rt. 2.68 min, 94.51% (Max). **HPLC:** (Method B) Rt. 5.54 min, 95.09% (Max).

**Example 38**

**(S)-2-(((3-butyl-7-methoxy-2-methyl-1,1-dioxido-5-phenyl-2,3,4,5-tetrahydro-1,2,5-benzothiadiazepin-8-yl) methyl)thio)-2-methylpropanoic acid and (R)-2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoic acid**

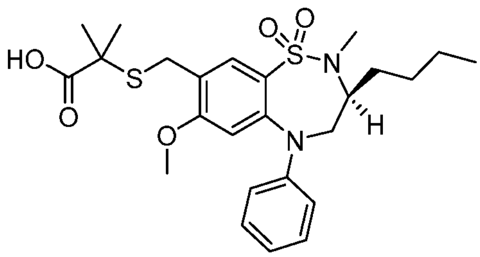

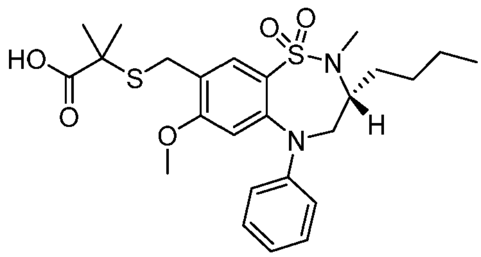

The two enantiomers of racemic 2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoic acid (Example 37; 0.17 g, 0.34 mmol) were separated by chiral SFC (Method C). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 11.65% (20 mg, white solid). **$^1$H NMR** (400 MHz, DMSO-$d_6$): δ 12.65 (s, 1H), 7.75 (s, 1H), 7.20-7.16 (m, 2H), 6.92 (s, 1H), 6.77-6.73 (m, 1H), 6.68 (d, *J* = 8.4 Hz, 2H), 4.07-4.03 (m, 1H), 3.88 (s, 2H), 3.82-3.81 (m, 1H), 3.78 (s, 3H), 3.28 (s, 1H), 2.45 (s, 3H), 1.61-1.56 (m, 1H), 1.50-1.49 (m, 1H), 1.46 (s, 6H), 1.36-1.24 (m, 4H), 0.91 (t, *J* = 6.40 Hz, 3H). **LCMS:** (Method A) 505.1 ($M^+$-H), Rt. 2.67 min, 98.62% (Max). **HPLC:** (Method B) Rt. 5.75 min, 99.10% (Max). **Chiral HPLC** (Method C): Rt.1.87 min,100% (Max).

Enantiomer 2: **Yield:** 14.66% (25 mg, white solid). **$^1$H NMR** (400 MHz, DMSO-$d_6$): δ 12.65 (s,1H), 7.76 (s, 1H), 7.16 (m, 2H), 6.92 (s, 1H), 6.77-6.75 (m, 1H), 6.68 (d, *J* = 8.4 Hz, 2H), 4.06-4.02 (m, 1H), 3.88 (s, 2H), 3.82-3.81 (m, 1H), 3.78 (s, 3H), 3.26 (s, 1H), 2.45 (s, 3H), 1.63-1.58 (m, 1H), 1.56-1.52 (m, 1H), 1.45 (s, 6H), 1.35-1.24 (m, 4H), 0.91 (t, *J* = 6.80 Hz, 3H). **LCMS:** (Method A) 505.1 (M$^+$-H), Rt. 3.07 min, 99.56% (Max). **HPLC:** (Method B) Rt. 5.55 min, 99.71% (Max). **Chiral HPLC** (Method H): Rt. 2.52 min, 99.11% (Max).

**Example 39**

**2-(((3-butyl-3-ethyl-5-(4-fluorophenyl)-7-methoxy-1,1-dioxido-2,3,4,5-tetrahydro-1,5-benzothiazepin-8-yl) methyl)thio)acetic acid**

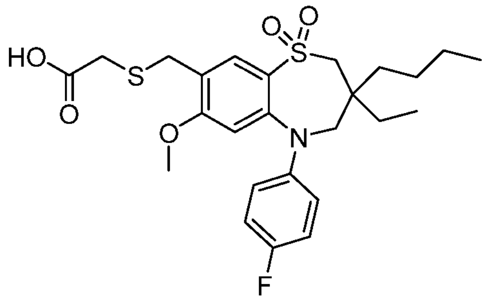

To a stirred solution of ethyl 2-(((3-butyl-3-ethyl-5-(4-fluorophenyl)-7-methoxy-1,1-dioxido-2,3,4,5-tetrahydro-1,5-benzothiazepin-8-yl)methyl)thio)acetate (Intermediate 91; 450 mg, 0.84 mmol) in 1,4-dioxane (7 mL) at 0 °C, a solution of lithium hydroxide (20.04 mg, 0.84 mmol) in water (3 mL) was added and the reaction mixture was stirred for 2 hours at RT. After completion of the reaction (monitored by TLC), the reaction mixture was acidified with dilute HCl (1.5 N, pH~4) and diluted with ice-cold water (2 mL).The aqueous layer was extracted with EtOAc (2 x 15 mL), and the combined organic layer was washed with water (15 mL) and brine (15 mL). The organic part was dried over anhydrous $Na_2SO_4$, filtered and concentrated under vacuum. The resulting crude material was purified by Isolera column chromatography (eluent: 9% MeOH/DCM; silica gel: 230-400 mesh) **Yield:** 58.1% (250 mg, white solid).

**$^1$H NMR** (400 MHz, DMSO-$d_6$): δ 12.65 (s, 1H), 7.73 (s, 1H), 7.27 (s, 2H), 7.16 (t, *J* = 8.80 Hz, 2H), 6.26 (s, 1H), 3.76 (s, 4H), 3.57 (s, 3H), 3.33 (s, 2H), 3.19 (s, 2H), 1.52-1.49 (m, 1H), 1.38-1.30 (m, 3H), 1.11-0.98 (m, 4H), 0.76-0.69 (m, 6H). **LCMS:** (Method A) 508.1 (M$^+$-H), Rt. 2.67 min, 98.37% (Max). **HPLC:** (Method B) Rt. 5.49 min, 99.13%(Max).

**Example 40**

**(S)-2-(((3-butyl-3-ethyl-5-(4-fluorophenyl)-7-methoxy-1,1-dioxido-2,3,4,5-tetrahydro-1,5-benzothiazepin-8-yl) methyl)thio)acetic acid and (R)-2-(((3-butyl-3-ethyl-5-(4-fluorophenyl)-7-methoxy-1,1-dioxido-2,3,4,5-tetrahydro-1,5-benzothiazepin-8-yl)methyl)thio)acetic acid**

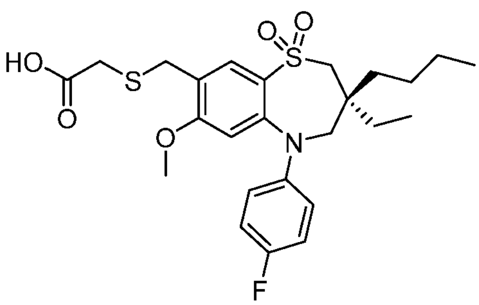

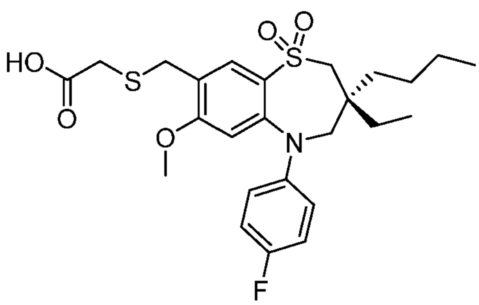

The two enantiomers of racemic 2-(((3-butyl-3-ethyl-5-(4-fluorophenyl)-7-methoxy-1,1-dioxido-2,3,4,5-tetrahydro-1,5-benzothiazepin-8-yl)methyl)thio)acetic acid (Example 39; 0.2 g, 0.39 mmol) were separated by chiral SFC (Method P). The material was concentrated under vacuum at 40 °C. The first eluting fraction corresponded to enantiomer 1 and the second eluting fraction corresponded to enantiomer 2. The absolute configuration of the two enantiomers is not

known.

Enantiomer 1: **Yield:** 29.6% (60 mg, white solid). **$^1$H NMR** (400 MHz, DMSO-*d*$_6$): δ 12.65 (s, 1H), 7.74 (s, 1H), 7.35-7.27 (m, 2H), 7.16 (t, *J* = 8.80 Hz, 2H), 6.25 (s, 1H), 3.80 (s, 3H), 3.78-3.76 (m, 1H), 3.56 (s, 3H), 3.34 (s, 2H), 3.19 (s, 2H), 1.51-1.48 (m, 1H), 1.38-1.30 (m, 3H), 1.11-0.98 (m, 4H), 0.76-0.69 (m, 6H). **LCMS:** (Method A) 508.1 ($M^+$-H), Rt. 3.06 min, 99.03% (Max). **HPLC:** (Method B) Rt. 5.50 min, 98.81% (Max). **Chiral HPLC** (Method P): Rt.1.87 min,100% (Max).

Enantiomer 2: **Yield:** 32.2% (65 mg, white solid). **$^1$H NMR** (400 MHz, DMSO-*d*$_6$): δ 12.63 (s, 1H), 7.74 (s, 1H), 7.30-7.26 (m, 2H), 7.16 (t, *J* = 8.80 Hz, 2H), 6.25 (s, 1H), 3.80 (s, 3H), 3.78-3.76 (m, 1H), 3.57 (s, 3H), 3.34 (s, 2H), 3.19 (s, 2H), 1.52-1.49 (m, 1H), 1.38-1.30 (m, 3H), 1.11-0.98 (m, 4H), 0.76-0.69 (m, 6H). **LCMS:** (Method A) 508.1 ($M^+$-H), Rt. 3.07 min, 98.70% (Max). **HPLC:** (Method B) Rt. 5.49 min, 99.21% (Max). **Chiral HPLC** (Method P): Rt. 2.33 min,100% (Max).

**Example 41**

**2-(((3-butyl-7-methoxy-3-methyl-1,1-dioxido-5-phenyl-2,3,4,5-tetrahydro-1,5-benzothiazepin-8-yl)methyl) thio)-2-methylpropanoic acid**

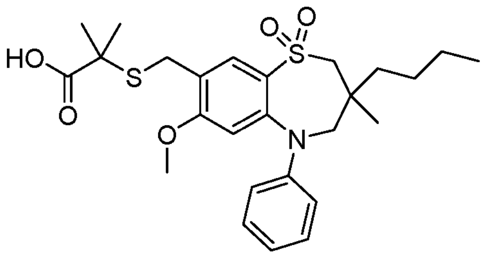

To a stirred solution of 2-(((3-butyl-7-methoxy-3-methyl-1,1-dioxido-5-phenyl-2,3,4,5-tetrahydro-1,5-benzothiazepin-8-yl)methyl)thio)-2-methylpropanoate (Intermediate 92; 200 mg, 0.39 mmol) in a mixture of 1,4-dioxane and water (7:3,10 mL) at 0 °C, lithium hydroxide (18 mg, 0.77 mmol) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 10 mL), amd the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 9% MeOH/DCM; silica gel: 230-400 mesh) **Yield:** 97.89% (0.19 g, white solid).

**$^1$H NMR** (400 MHz, DMSO-*d*$_6$): δ 12.63 (s, 1H), 7.76 (s, 1H), 7.28-7.23 (m, 2H), 7.08 (d, *J* = 7.20 Hz, 2H), 6.93 (t, *J* = 7.20 Hz, 1H), 6.48 (s, 1H), 3.83 (s, 2H), 3.61 (s, 3H), 3.33 (s, 2H), 3.25-3.22 (m, 2H), 1.45 (s, 6H), 1.33-1.26 (m, 2H), 1.20-1.14 (m, 4H), 0.99 (s, 3H), 0.76 (t, *J* = 6.80 Hz, 3H). **LCMS:** (Method A) 504.1 ($M^+$-H), Rt. 2.76 min, 98.77% (Max). **HPLC:** (Method B) Rt. 5.82 min, 99.10% (Max).

**Example 42**

**(S)-2-(((3-butyl-7-methoxy-3-methyl-1,1-dioxido-5-phenyl-2,3,4,5-tetrahydro-1,5-benzothiazepin-8-yl)methyl) thio)-2-methylpropanoic acid and (R)-2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid**

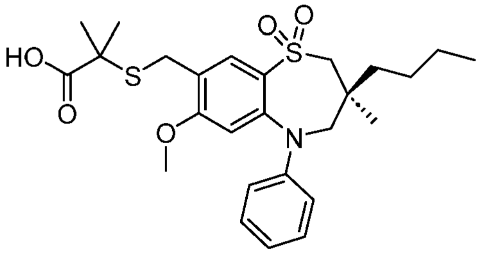

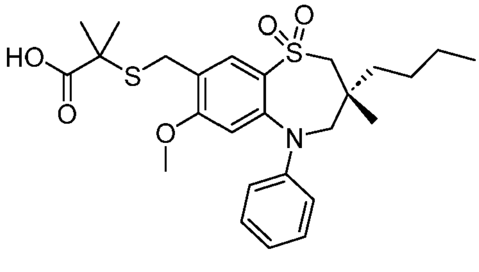

The two enantiomers of racemic 2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid (Example 41; 0.17 g, 0.33 mmol) were separated by chiral SFC

(Method S). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 40.8% (70 mg, white solid). **$^1$H NMR** (400 MHz, DMSO-$d_6$): δ 12.63 (s, 1H), 7.76 (s, 1H), 7.28-7.23 (m, 2H), 7.08 (d, *J* = 7.20 Hz, 2H), 6.93 (t, *J* = 7.20 Hz, 1H), 6.48 (s, 1H), 3.83 (s, 2H), 3.61 (s, 3H), 3.23-3.26 (m, 4H), 1.45 (s, 6H), 1.33-1.20 (m, 1H), 1.10-1.04 (m, 5H), 0.99 (s, 3H), 0.76 (t, *J = 6.80* Hz, 3H). **LCMS:** (Method A) 504.1 ($M^+$-H), Rt. 2.76 min, 97.61% (Max). **HPLC:** (Method B) Rt. 5.61 min, 99.00 % (Max). **Chiral HPLC** (Method S): Rt. 2.17 min,100% (Max).

Enantiomer 2: **Yield:** 29% (50 mg, white solid). **$^1$H NMR** (400 MHz, DMSO-$d_6$): δ 12.63 (s, 1H), 7.76 (s, 1H), 7.26 (t, *J* = 8.00 Hz, 2H), 7.08 (d, *J* = 7.60 Hz, 2H), 6.93 (t, *J* = 7.20 Hz, 1H), 6.48 (s, 1H), 3.83 (s, 2H), 3.61 (s, 3H), 3.26-3.22 (m, 4H), 1.45 (s, 6H), 1.32-1.21 (m, 1H), 1.10-1.04 (m, 5H), 0.99 (s, 3H), 0.76 (t, *J* = 6.80 Hz, 3H). **LCMS:** (Method A) 504.1 ($M^+$-H), Rt. 2.76 min, 97.33% (Max). **HPLC:** (Method B) Rt. 5.83 min, 98.34% (Max). **Chiral HPLC** (Method S): Rt. 2.67 min,99.74% (Max).

## Example 43

**2-(((3,3-dibutyl-7-methoxy-1,1-dioxido-5-phenyl-2,3,4,5-tetrahydro-1,5-benzothiazepin-8-yl)methyl)thio)-2-methylpropanoic acid**

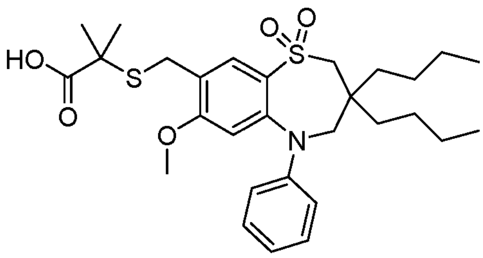

To a stirred solution of methyl 2-(((3,3-dibutyl-7-methoxy-1,1-dioxido-5-phenyl-2,3,4,5-tetrahydro-1,5-benzothiazepin-8-yl)methyl)thio)-2-methylpropanoate (Intermediate 93; 0.2 g, 0.36 mmol) in a mixture of 1,4-dioxane and water (7:3,10 mL) at 0 °C, lithium hydroxide (8.53 mg, 0.36 mmol) was added and the reaction mixture was stirred for 2 hours at RT. After completion of the reaction (monitored by TLC), the reaction mixture was acidified with dilute HCl (1.5 N, pH~4) and diluted with ice-cold water (2 mL). The aqueous layer was extracted with EtOAc (2 x 5 mL), and the combined organic layer was washed with water (5 mL) and brine (5 mL).The organic part was dried over anhydrous $Na_2SO_4$, filtered and concentrated under vacuum. The resulting crude material was purified by Isolera column chromatography (eluent: 9% MeOH/DCM; silica gel: 230-400 mesh). **Yield:** 76.9% (0.15 g, white solid).

**$^1$H NMR** (400 MHz, DMSO-$d_6$): δ 12.60 (S, 1H), 7.72 (s, 1H), 7.31 (t, *J* = 7.20 Hz, 2H), 7.19 (d, *J* = 7.60 Hz, 2H), 7.01 (t, *J* = 7.60 Hz, 1H), 6.31 (s, 1H), 3.81 (s, 3H), 3.78-3.76 (m,1H), 3.56 (s, 3H), 3.31-3.29 (m, 2H), 1.45 (s, 6H), 1.42-1.34 (m, 2H), 1.34-1.28 (m, 2H), 1.16-1.01 (m, 8H), 0.74 (t, *J* = 6.80 Hz, 6H). **LCMS:** (Method A) 546.2 ($M^+$-H), Rt. 3.07 min, 99.66% (Max). **HPLC:** (Method B) Rt. 6.55 min, 99.75% (Max).

## Example 44

**2-(((3-butyl-3-ethyl-7-methoxy-1,1-dioxido-5-phenyl-2,3,4,5-tetrahydro-1,2,5-benzothiadiazepin-8-yl)methyl) thio)acetic acid**

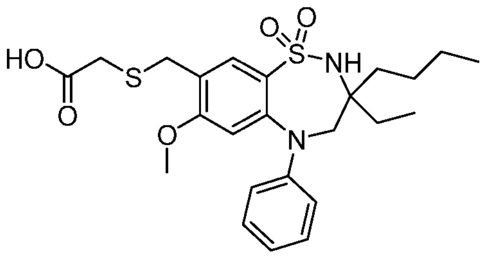

To a stirred solution of 2-(((3-butyl-3-ethyl-7-methoxy-2-(4-methoxybenzyl)-1,1-dioxido-5-phenyl-2,3,4,5-tetrahydro-1,2,5-benzothiadiazepin-8-yl)methyl)thio)acetic acid (Intermediate 101; 170 mg, 0.28 mmol) in DCM (10 mL) at 0 °C, zinc iodide (89 mg, 0.28 mmol) was added and the reaction mixture was stirred for 3 hours at RT. After completion of the reaction (monitored by LCMS), the reaction mixture was concentrated under vacuum and the reaction mixture was quenched with water (15 mL). The aqueous layer was extracted with EtOAc (2 x 15 mL), the organic layer was separated and dried over anhydrous $Na_2SO_4$. The organic part was concentrated under vacuum and the resulting crude was purified by Prep-HPLC (Method D) to afford the title compound. **Yield:** 3% (4 mg, off-white solid).

**$^1$H-NMR** (400 MHz, DMSO-*d*$_6$): δ 12.75 (s, 1H), 7.58 (s, 1H), 7.36 (t, *J* = 8.0 Hz, 3H), 7.28-7.05 (m, 2H), 7.09 (t, *J* = 7.2 Hz, 1H), 6.18 (s, 1H), 3.97-3.91 (m, 2H), 3.74 (s, 2H), 3.48 (s, 3H), 3.13 (s, 2H), 1.60-1.43 (m, 4H), 1.35-0.90 (m, 4H), 0.72-0.66 (m, 6H). **LCMS:** (Method B) 491.1 ($M^+$-H), Rt. 1.86 Min, 98.70% (Max), **HPLC:** (Method B) Rt. 5.23 min, 99.87% (Max).

**Example 45**

**(R)-2-(((3-butyl-3-ethyl-7-methoxy-1,1-dioxido-5-phenyl-2,3,4,5-tetrahydro-1,2,5-benzothiadiazepin-8-yl)methyl)thio)acetic acid and (S)-2-(((3-butyl-3-ethyl-7-methoxy-1,1-dioxido-5-phenyl-2,3,4,5-tetrahydro-1,2,5-benzothiadiazepin-8-yl)methyl)thio)acetic acid**

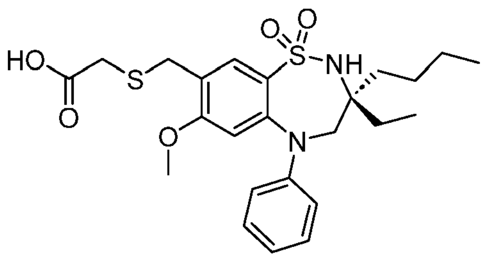
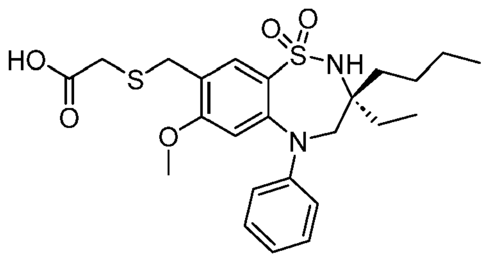

The two enantiomers of racemic 2-(((3-butyl-3-ethyl-7-methoxy-1,1-dioxido-5-phenyl-2,3,4,5-tetrahydro-1,2,5-benzothiadiazepin-8-yl)methyl)thio)acetic acid (Example 44; 0.07 g, 0.14 mmol ) were separated by chiral SFC (Method U). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 28.5% (0.020 g, white solid) **$^1$H NMR** (400 MHz, DMSO-*d*$_6$): δ 12.61 (bs, 1H), 7.59 (s, 1H), 7.37 (t, *J* = 8.0 Hz, 3H), 7.28 (d, *J* = 7.2 Hz, 2H), 7.10 (t, *J* = 7.2 Hz, 1H), 6.17 (s, 1H), 3.97-3.94 (m, 2H), 3.72 (s, 2H), 3.48 (s, 3H), 3.16 (s, 2H), 1.62-1.43 (m, 4H), 1.24-0.90 (m, 4H), 0.72-0.66 (m, 6H). **LCMS:** (Method B) 491.1 ($M^+$-H), Rt. 1.58 min, 96.38% (Max). **HPLC:** (Method B) Rt. 5.64 min, 98.20% (Max). **Chiral HPLC:** (Method U) Rt.4.38 min, 100% (Max).

Enantiomer 2: **Yield:** 30% (0.021 g, white solid). **$^1$H NMR** (400 MHz, DMSO-*d*$_6$): δ 12.61 (bs, 1H), 7.59 (s, 1H), 7.37 (t, *J* = 8.0 Hz, 3H), 7.28 (d, *J* = 6.8 Hz, 2H), 7.10 (t, *J* = 7.2 Hz, 1H), 6.17 (s, 1H), 3.96-3.94 (m, 2H), 3.72 (s, 2H), 3.48 (s, 3H), 3.16 (s, 2H), 1.64-1.43 (m, 4H), 1.24-0.90 (m, 4H), 0.72-0.65 (m, 6H). **LCMS:** (Method B) 491.1 ($M^+$-H), Rt. 1.70 min, 95.03% (Max). **HPLC:** (Method B) Rt. 5.64 min, 99.30% (Max). **Chiral HPLC:** (Method U) Rt. 5.34 min,100% (Max).

**Example 46**

**2-(((3-butyl-7-(dimethylamino)-3-ethyl-1,1-dioxido-5-phenyl-2,3,4,5-tetrahydro-1,2,5-benzothiadiazepin-8-yl)methyl)thio)acetic acid**

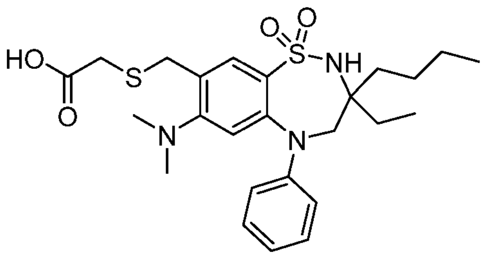

To a stirred solution of 2-(((3-butyl-7-(dimethylamino)-3-ethyl-2-(4-methoxybenzyl)-1,1-dioxido-5-phenyl-2,3,4,5-tetrahydro-1,2,5-benzothiadiazepin-8-yl)methyl)thio)acetic acid (Intermediate 108; 0.3 g, 0.48 mmol) in toluene (15 mL) at 0 °C, triphenylamine (0.12 g, 0.48 mmol) and TFA (0.27 g, 2.39 mmol) were added and the reaction mixture was stirred for 16 hours at RT. After completion of the reaction (monitored by LCMS), the reaction mixture was concentrated under vacuum and the obtained material was diluted with water (20 mL). The aqueous layer was extracted with EtOAc (2 x 25 mL) and the combined organic layer was washed with brine (20 mL). The organic part was dried over anhydrous $Na_2SO_4$, filtered and concentrated under reduced pressure. The resulting crude was purified by Prep-HPLC (Method A) to afford the title compound. **Yield:** 8% (0.02 g, off-white solid). **$^1$H-NMR** (400 MHz, DMSO-$d_6$): δ 12.64 (bs, 1H), 7.65 (s, 1H), 7.36 (t, $J$ = 8.0 Hz, 2H), 7.30-7.25 (m, 3H), 7.08 (t, $J$ = 6.8 Hz, 1H), 6.23 (s, 1H), 3.96-3.94 (m, 2H), 3.83 (s, 2H), 3.27 (s, 2H), 2.48 (s, 6H), 1.61-1.36 (m, 4H), 1.24-0.89 (m, 4H), 0.68-0.66 (m, 6H). **LCMS:** (Method B) 506.2 ($M^+$+H), Rt. 1.92 Min, 98.31% (Max). **HPLC:** (Method B) Rt. 4.64 min, 98.85% (Max).

**Example 47**

**2-(((3-butyl-3-ethyl-7-(methylsulfonyl)-1,1-dioxido-5-phenyl-2,3,4,5-tetrahydro-1,2,5-benzothiadiazepin-8-yl) methyl)thio)acetic acid**

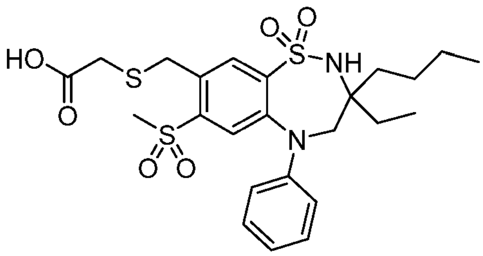

To a stirred solution of 2-(((3-butyl-3-ethyl-2-(4-methoxybenzyl)-7-(methylsulfonyl)-1,1-dioxido-5-phenyl-2,3,4,5-tetrahydro-1,2,5-benzothiadiazepin-8-yl)methyl)thio)acetic acid (Intermediate 114; 0.3 g, 0.45 mmol) in toluene (15 mL) at 0 °C, triphenylamine (0.11 g, 0.45 mmol) and TFA (0.26 g, 2.27 mmol) were added and the reaction mixture was stirred for 2 hours at RT. After completion of the reaction (monitored by TLC), the reaction mixture was concentrated under vacuum and the resulting mixture was diluted with water (10 mL). The aqueous layer was extracted with EtOAc (3 x 10 mL). The combined organic layer was washed with brine (5 mL), dried over anhydrous $Na_2SO_4$, filtered and concentrated under reduced pressure. The resulting crude was purified by Prep-HPLC (Method C) to afford the title compound. **Yield:** 25% (0.06 g, white solid).

**$^1$H NMR** (400 MHz, DMSO-$d_6$): δ 12.72 (bs, 1H), 7.90 (s, 1H), 7.86 (bs, 1H), 7.45 (t, $J$ = 8.0 Hz, 2H), 7.37 (d, $J$ = 7.6 Hz, 2H), 7.22 (t, $J$ = 7.6 Hz, 2H), 4.21 (s, 2H), 4.09-3.99 (m, 2H), 3.32 (s, 2H), 3.24 (s, 3H), 1.62-1.40 (m, 4H), 1.24-0.86 (m, 4H), 0.69-0.59 (m, 6H). **LCMS:** (Method B) 539.0 ($M^+$-H) Rt. 1.58 min, 99.21% (Max). **HPLC:** (Method B) Rt. 5.43 min, 99.29% (Max).

**Example 48**

**(S)-2-(((3-Butyl-3-ethyl-7-(methylsulfonyl)-1,1-dioxido-5-phenyl-2,3,4,5-tetrahydro-1,2,5-benzothiadiazepin-8-yl)methyl)thio)acetic acid and (R)-2-(((3-butyl-3-ethyl-7-(methylsulfonyl)-1,1-dioxido-5-phenyl-2,3,4,5-tetrahydro-1,2,5-benzothiadiazepin-8-yl)methyl)thio)acetic acid**

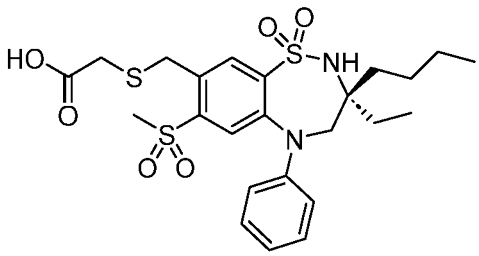 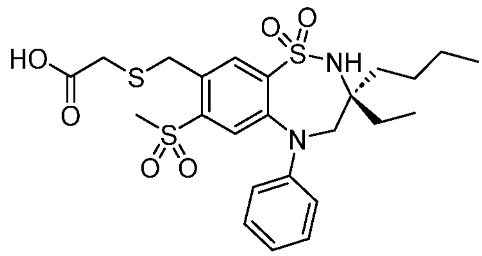

The two enantiomers of racemic 2-(((3-butyl-3-ethyl-7-(methylsulfonyl)-1,1-dioxido-5-phenyl-2,3,4,5-tetrahydro-1,2,5-benzothiadiazepin-8-yl)methyl)thio)acetic acid (Example 47; 0.05 g 0.09 mmol ) were separated by chiral SFC (Method T). The material was concentrated under vacuum at 40 °C. The first eluting fraction corresponded to enantiomer 1 and the second eluting fraction corresponded to enantiomer 2. The absolute configuration of the two enantiomers is not known.

Enantiomer 1: **Yield:** 29% (0.015 g, white solid). **$^1$H NMR** (400 MHz, DMSO-*d*$_6$): δ 12.72 (bs, 1H), 7.90 (s, 1H), 7.86 (bs, 1H), 7.45 (t, *J* = 8.0 Hz, 2H), 7.37 (d, *J* = 6.8 Hz, 2H), 7.24-7.22 (m, 2H), 4.21 (s, 2H), 4.10-4.09 (m, 2H), 3.31 (s, 2H), 3.24 (s, 3H), 1.61-1.43 (m, 4H), 1.12-0.83 (m, 4H), 0.68-0.55 (m, 6H). **LCMS:** (Method B) 539.1 ($M^+$-H), Rt. 1.71 min, 96.79% (Max). **HPLC:** (Method B) Rt. 5.60 min, 97.60% (Max). **Chiral HPLC:** (Method T) Rt. 6.12 min,100% (Max).

Enantiomer 2: **Yield:** 35% (0.018 g, white solid). **$^1$H NMR** (400 MHz, DMSO-*d*$_6$): δ 12.72 (bs, 1H), 7.90 (s, 1H), 7.86 (bs, 1H), 7.45 (t, *J* = 7.6 Hz, 2H), 7.37 (d, *J* = 7.2 Hz, 2H), 7.22 (t, *J* = 7.6 Hz, 2H), 4.21 (s, 2H), 4.09-4.05 (m, 2H), 3.24 (s, 2H), 3.22 (s, 3H), 1.61-1.42 (m, 4H), 1.12-0.83 (m, 4H), 0.68-0.55 (m, 6H). **LCMS:** (Method B) 539.1 ($M^+$-H), Rt. 1.7 min, 96.11% (Max). **HPLC:** (Method B) Rt. 5.66 min, 97.35% (Max). **Chiral HPLC:** (Method T) Rt.7.68 min, 100% (Max).

**Example 49**

**(R)-2-(((3-butyl-7-(dimethylamino)-1,1-dioxido-5-phenyl-2,3,4,5-tetrahydro-1,2,5-benzothiadiazepin-8-yl) methyl)thio)acetic acid**

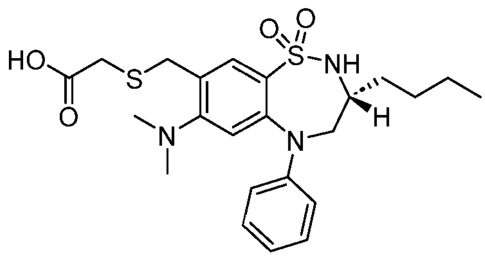

To a stirred solution of (R)-2-(((3-butyl-7-(dimethylamino)-2-(4-methoxybenzyl)-1,1-dioxido-5-phenyl-2,3,4,5-tetrahydro-1,2,5-benzothiadiazepin-8-yl)methyl)thio)acetic acid (Intermediate 122; 250 mg, 0.42 mmol) in toluene (10 mL) at 0 °C, triphenylamine (205 mg, 0.836 mmol) and TFA (0.64 mL, 8.36 mmol) were added and the reaction mixture was stirred for 3 hours at RT. After completion of the reaction (monitored by TLC), the reaction mixture was concentrated under high vacuum and the resulting mixture was diluted with water (20 mL). The aqueous layer was extracted with EtOAc (3 x 20 mL) and the combined organic layer was washed with brine (10 mL). The organic part was dried over anhydrous $Na_2SO_4$, filtered and concentrated under reduced pressure. The resulting crude was purified by Prep-HPLC (Method C) to afford the title compound. **Yield:** 5% (10 mg, off-white solid). **$^1$H NMR** (400 MHz, DMSO-*d*$_6$): δ 7.86 (s, 1H), 7.16 (q, *J* = 7.2 Hz, 3H), 6.89 (s, 1H), 6.72 (t, *J* = 7.2 Hz, 1H), 6.66 (d, *J* = 8.0 Hz, 2H), 4.35-4.31 (m, 1H), 3.95 (s, 2H), 3.35-3.34 (m, 1H), 3.26 (s, 2H), 2.94-2.91 (m, 1H), 2.70 (s, 6H), 1.51-1.25 (m, 6H), 0.87 (t, *J* = 7.20 Hz, 3H). **LCMS:** (Method B) 478.3 ($M^+$+H), Rt. 1.51 min, 99.52% (Max). **HPLC:** (Method B) Rt.4.85 min, 99.6 % (Max). **SFC:** (method K) Rt. 2.17 min, 99.92 %.

**Example 50**

**(R)-2-(((3-butyl-7-(methylthio)-1,1-dioxido-5-phenyl-2,3,4,5-tetrahydro-1,2,5-benzothiadiazepin-8-yl)methyl) thio)acetic acid**

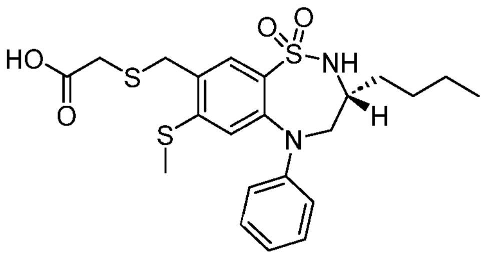

To a stirred solution of (R)-2-(((3-butyl-2-(4-methoxybenzyl)-7-(methylthio)-1,1-dioxido-5-phenyl-2,3,4,5-tetrahydro-1,2,5-benzothiadiazepin-8-yl)methyl)thio)acetic acid (Intermediate 128; 450 mg, 0.75 mmol) in toluene (10 mL) at 0 $^0$C, triphenylamine (367 mg, 1.49 mmol) and 2,2,2-trifluoroacetic acid (1.15 mL, 14.98 mmol) were added and the reaction mixture was stirred 16 h at RT. After completion of the reaction (monitored by TLC), the reaction mixture was concentrated under high vacuum, the resulting reaction mixture was diluted with water (10 mL). The aqueous layer was extracted with ethyl acetate (3 X 10 mL) and the combined organic layer was washed with brine solution (10 mL) and dried over anhydrous $Na_2SO_4$. The organic part was filtered and concentrated under reduced pressure. The resulting crude was purified by prep-HPLC (Method C) to afford the title compound. **Yield:** 14% (0.05 g, off-white solid).

**$^1$H-NMR** (400 MHz, DMSO-*d*$_6$): δ 7.80 (s, 1H), 7.30 (bs, 1H), 7.18 (t, *J* = 8.4 Hz, 2H), 7.14 (s, 1H), 6.74 (t, *J* = 7.2 Hz, 1H), 6.67 (d, *J* = 8.4 Hz, 2H), 4.37-4.34 (m, 1H), 3.91 (s, 2H), 3.37-3.35 (m, 1H), 3.15 (s, 2H), 2.95-2.92 (m, 1H), 2.42 (s, 3H), 1.59-1.27 (m, 6H), 0.90 (t, *J* = 7.20 Hz, 3H). **LCMS:** (Method B) 479.1 ($M^+$-H), Rt. 1.83 min, 98.96% (Max). **HPLC:** (Method B) Rt. 5.11 min, 99.45% (Max). **SFC:** (Method S) Rt. 3.06 min, 98.36%.

**Example 51**

**(R)-2-(((3-butyl-7-methoxy-1,1-dioxido-5-phenyl-2,3,4,5-tetrahydro-1,2,5-benzothiadiazepin-8-yl)methyl)thio) acetic acid**

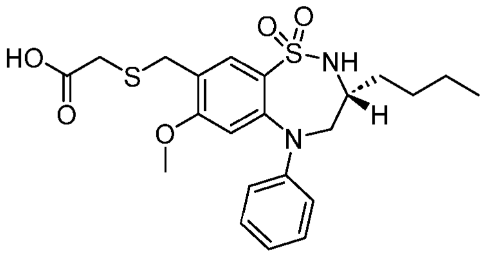

To a stirred solution of ethyl (R)-2-(((3-butyl-7-methoxy-1,1-dioxido-5-phenyl-2,3,4,5-tetrahydro-1,2,5-benzothiadiazepin-8-yl)methyl)thio)acetate (Intermediate 134; 270 mg, 0.55 mmol) in 1,4-dioxane (3 mL), LiOH (39.4 mg, 1.64 mmol) in water (3 mL) was added and the reaction mixture was stirred for 1 hour at RT. After completion of the reaction (monitored by TLC), the reaction mixture was quenched with dilute HCl (1.5 N, 15 mL) and the aqueous layer was extracted with EtOAc (2 x 15 mL). The combined organic layer was separated, dried over anhydrous $Na_2SO_4$, filtered and concentrated under vacuum. The resulting crude was purified by Prep-HPLC (Method C) to afford the title compound. **Yield:** 55% (0.14 g, white solid).

**$^1$H NMR** (400 MHz, DMSO-*d*$_6$) : δ 12.70 (bs, 1H), 7.80 (s, 1H), 7.20-7.16 (m, 3H), 6.92 (s, 1H), 6.75 (d, *J* = 7.2 Hz, 1H), 6.72-6.67 (m, 2H), 4.37-4.33 (m, 1H), 3.86-3.82 (m, 2H), 3.78 (s, 3H), 3.77-3.67 (m, 1H), 3.40-3.32 (m, 2H), 2.94-2.85 (m, 1H), 1.59-1.54 (m, 4H), 1.48-1.36 (m, 2H), 0.90 (t, *J* = 7.20 Hz, 3H). **LCMS:** (Method B) 463.1($M^+$-H) Rt. 1.46 min, 98.71% (Max). **HPLC:** (Method B) Rt. 5.21 min, 98.98% (Max).

**Example 52**

**2-(((3-Butyl-7-(dimethylamino)-3-methyl-1,1-dioxido-5-phenyl-2,3,4,5-tetrahydro-1,2,5-benzothiadiazepin-8-yl) methyl)thio)acetic acid**

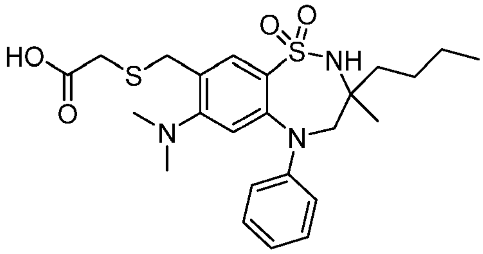

To a stirred solution of 2-(((3-butyl-7-(dimethylamino)-2-(4-methoxybenzyl)-3-methyl-1,1-dioxido-5-phenyl-2,3,4,5-tetrahydro-1,2,5-benzothiadiazepin-8-yl)methyl)thio)acetic acid (Intermediate 152; 95 mg, 0.16 mmol) in toluene (2 mL) at 0 °C, triphenylamine (72.7 mg, 0.31 mmol) and TFA (0.24 mL, 3.11 mmol) were added and the reaction mixture was stirred for 1 hour at RT. After completion of the reaction (monitored by LCMS), the reaction mixture was concentrated under vacuum and the resulting crude was diluted with water (50 mL). The aqueous layer was extracted with EtOAc (3 x 50 mL). The combined organic layer was washed with brine (50 mL), dried over anhydrous $Na_2SO_4$, filtered and concentrated under reduced pressure. The resulting crude was purified by Prep-HPLC (Method C) to afford the title compound. **Yield:** 54% (0.04 g, off-white solid).

**$^1$H-NMR** (400 MHz, MeOD): δ 7.84 (s, 1H), 7.37-7.33 (m, 2H), 7.24-7.22 (m, 2H), 7.07 (t, J = 7.2 Hz, 1H), 6.46 (s, 1H), 4.12-3.98 (m, 2H), 3.92 (s, 2H), 3.24 (s, 2H), 2.58 (s, 6H), 1.73-1.42 (m, 3H), 1.25-1.21 (m, 3H), 1.20 (s, 3H), 0.83 (t, *J* = 7.20 Hz, 3H). **LCMS:** (Method B) 492.2 ($M^+$+H), Rt. 1.56 min, 99.19% (Max). **HPLC:** (Method B) Rt.4.68 min, 99.78% (Max).

**Example 53**

**2-(((3-butyl-7-methoxy-3-methyl-1,1-dioxido-5-phenyl-2,3,4,5-tetrahydro-1,2,5-benzothiadiazepin-8-yl)methyl)thio)acetic acid**

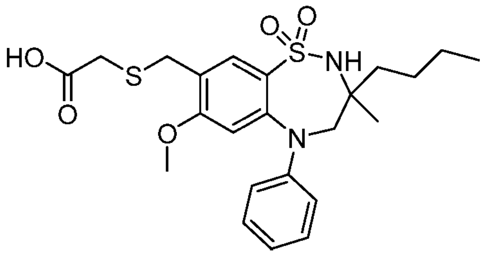

To a stirred solution of ethyl 2-(((3-butyl-7-methoxy-3-methyl-1,1-dioxido-5-phenyl-2,3,4,5-tetrahydro-1,2,5-benzothiadiazepin-8-yl)methyl)thio)acetate (Intermediate 158; 230 mg, 0.454 mmol) in 1,4-dioxane (2 mL), LiOH (43.5 mg, 1.816 mmol) in water (0.5 mL) was added. The reaction mixture was stirred at RT for 1 hour. The reaction mixture was then quenched with 1.5 N HCl solution, and the aqueous layer was extracted with EtOAc. The organic layer was dried over anhydrous $Na_2SO_4$, filtered and concentrated under high vacuum. The crude compound was purified by Prep-HPLC (Method C) to afford the title compound. **Yield:** 16% (0.035 g, off-white solid).

**$^1$H NMR** (400 MHz, DMSO-$d_6$): δ 7.64 (s, 1H), 7.33-7.29 (m, 3H), 7.18-7.11 (m, 2H), 7.10-6.91 (m, 1H), 6.34-6.28 (m, 1H), 3.97-3.91 (m, 2H), 3.76 (s, 2H), 3.72 (s, 3H), 3.12 (s, 2H), 1.62-1.31 (m, 3H), 1.30-1.24 (m, 3H), 1.09 (s, 3H), 0.77 (t, *J* = 6.80 Hz, 3H), **LCMS:** (Method B) 477.1($M^+$-H) Rt. 1.52 min, 96.0% (Max). **HPLC:** (Method B) Rt. 5.41 min, 99.48% (Max).

**Example 54**

**2-(((3-Butyl-3-methyl-7-(methylthio)-1,1-dioxido-5-phenyl-2,3,4,5-tetrahydro-1,2,5-benzothiadiazepin-8-yl)methyl)thio)acetic acid**

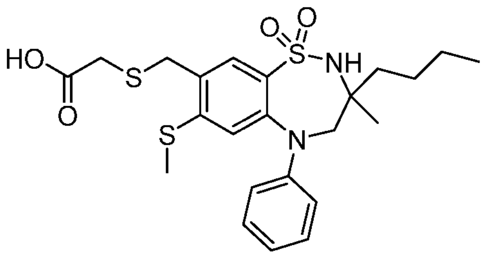

To a stirred solution of ethyl 2-(((3-butyl-2-(4-methoxybenzyl)-3-methyl-7-(methylthio)-1,1-dioxido-5-phenyl-2,3,4,5-tetrahydro-1,2,5-benzothiadiazepin-8-yl)methyl)thio)acetate (Intermediate 163; 0.15 g, 0.23 mmol) in 1,4-dioxane (10 mL) at 0 °C, lithium hydroxide (0.01 g, 0.47 mmol) in water (2 mL) was added and the reaction mixture was stirred for 1 hour at RT. After completion of the reaction (monitored by TLC), the reaction mixture was quenched with dilute HCl (1.5 N, 15 mL) and the aqueous layer was extracted with EtOAc (2 x 25 mL). The combined organic layer was dried over anhydrous $Na_2SO_4$, filtered and concentrated under vacuum. The resulting crude was purified by Prep-HPLC (Method C) to afford the title compound. **Yield:** 13% (0.015 g, white solid).

**$^1$H-NMR** (400 MHz, DMSO-*d*$_6$): δ 12.75 (s, 1H), 7.63 (s, 1H), 7.50 (s, 1H), 7.34 (t, *J* = 8.0 Hz, 2H), 7.21-7.19 (m, 2H), 7.11-7.05 (m, 1H), 6.58-6.51 (m, 1H), 4.11-4.01 (m, 2H), 3.84 (s, 2H), 3.16 (s, 2H), 2.14 (s, 3H), 1.62-1.36 (m, 3H), 1.24-1.15 (m, 3H), 1.10 (s, 3H), 0.76 (t, *J* = 6.80 Hz, 3H). **LCMS:** (Method B) 493.1 ($M^+$-H), Rt 1.73 min, 98.65% (Max). **HPLC:** (Method B) Rt. 5.92 min, 99.93% (Max). BIOLOGICAL ASSAYS

*IBAT (h/m) assay protocol*

10,000 cells (Human or Mouse IBAT-overexpressing cells) were seeded in 96-wells plate (Corning CLS3809) in 200 μL MEM-alpha medium (Gibco 12571-063) supplemented with 10% FBS (Gibco 10438026) containing Puromycin (Gibco A1113803) (10 μg/mL) and incubated at 37 °C in 5% $CO_2$ for 48 hours. After incubation, media was decanted from the wells and cells were washed two times with 300 μL of basal MEM-alpha medium (FBS-free). After decanting basal MEM-alpha medium each time, plates were tapped against paper towel to ensure maximum removal of residual media. Test inhibitor dilutions (highest test concentration being 10 μM, 3-fold serial dilution, 10 points) prepared in DMSO (Sigma D2650) were added in incubation mix (maintaining 0.2% final DMSO concentration) containing 0.25 μM 3H-taurocholic acid (ARC ART-1368) and 5 μM of cold taurocholic acid (Sigma T4009). 50 μL of incubation mix containing test inhibitors was then added to the wells (in duplicate) and the plates were incubated for 20 minutes in a $CO_2$ incubator at 37 °C. After incubation, the reaction was stopped by keeping the plates on ice water mix for 2-3 minutes and then the incubation mix was aspirated completely from the wells. The wells were washed two times with 250 μL of chilled unlabelled 1 mM taurocholic acid dissolved in HEPES (Gibco 15630080)-buffered (10 mM) HBSS (Gibco 14175079) (pH 7.4). The plates were tapped against a paper towel after every wash to ensure maximum removal of blocking buffer.

100 μL of MicroScint-20 (PerkinElmer 6013621) was added to the wells and kept overnight at RT before reading the plates in TopCount NXT™ Microplate Scintillation and Luminescence Counter from PerkinElmer under 3H Test protocol (set at 120 seconds reading time per well).

*LBAT (h/m) assay protocol*

20,000 cells (Human or Mouse LBAT-overexpressing cells) were seeded in 96-wells plate (Corning CLS3809) in 100 μL MEM-alpha medium (Gibco 12571-063) supplemented with 10% FBS (Gibco 10438026) containing Geneticin (Gibco 10131-027) (1 mg/mL) and incubated at 37 °C in 5% $CO_2$ for 24 hours. After incubation, media was decanted from the wells and cells were washed two times with 300 μL of basal MEM-alpha medium (FBS-free). After decanting basal MEM-alpha medium each time, plates were tapped against paper towel to ensure maximum removal of residual media. For human LBAT, incubation mix was prepared by adding test inhibitor dilutions (3-fold serial dilution in DMSO (Sigma D2650), 10 points) in MEM-alpha (without FBS) containing 0.3 μM 3H-taurocholic acid (ARC ART-1368) and 7.5 μM cold taurocholic acid (Sigma T4009) (maintaining 0.2% final DMSO concentration). For mouse LBAT, incubation mix was prepared by adding test inhibitor dilutions (3-fold serial dilution in DMSO, 10 points) in MEM-alpha (without FBS) containing 0.3 μM 3H-taurocholic acid and 25 μM cold taurocholic acid maintaining 0.2% final DMSO concentration). 50 μL of incubation mix containing test inhibitors was then added to the wells (in duplicate) and the plates were incubated for 20 minutes in a $CO_2$ incubator at 37 °C. After incubation, the reaction was stopped by keeping the plates on ice water mix for 2-3 minutes and then the incubation mix was aspirated completely from the wells. The wells were washed two times with 250 μL of chilled unlabelled 1 mM taurocholic acid dissolved in HEPES (Gibco 15630080)-buffered (10 mM) HBSS (Gibco 14175079) (pH 7.4). The plates were tapped against a paper towel after every wash to ensure maximum removal of blocking buffer.

100 μL of MicroScint-20 (PerkinElmer 6013621) was added to the wells and kept overnight at RT before reading the plates in TopCount NXT™ Microplate Scintillation and Luminescence Counter from PerkinElmer under 3H Test protocol (set at 120 seconds reading time per well, with normal plate orientation).

*Bidirectional permeability assay (Caco-2 cells)*

Caco-2 cells (Evotec) were seeded at a density of 70,000 cells/well in Millicell® 24-well cell culture insert plates and maintained in an incubator (37 °C, 5% $CO_2$, 95% RH) for 21 days with media change on alternate days.

Stock solutions (10 mM) of the test compounds, atenolol (low permeability marker), propranolol (high permeability marker) and digoxin (substrate for P-gp transport pathway) were prepared in dimethylsulfoxide (DMSO). An intermediate stock solution (1 mM) was prepared by diluting 10 μL of 10 mM master stock solution with 90 μL of neat DMSO. A working stock solution (10 μM) was prepared by diluting 50 μL of 1 mM with 4950 μL of FaSSIF buffer. Post addition of compounds to the FaSSIF, samples were subjected to sonication for 2 hours, and centrifuged at 4000 RPM for 30 minutes at 37 °C. The 4 mL of resultant supernatant was directly used in the assay. The final DMSO concentration in the transport experiments was 1%.

On the day of assay, Caco-2 monolayers were washed twice with transport buffer (HBSS, pH 7.4) and pre-incubated for 30 min (37 °C, 5% $CO_2$, 95% RH) in an incubator. The electrical resistance of the monolayers was measured with a Millicell® - ERS system. Monolayers with trans-epithelial electrical resistance (TEER) values greater than 350 ohm.$cm^2$ were selected for the assay.

The assay was conducted in absorptive direction (A2B) and secretory (B2A) directions. Transport experiments were initiated by addition of transport assay buffer (FaSSIF buffer prepared in HBSS) consisting of compounds to the donor compartment (apical chamber A-B; basolateral chamber B-A) in duplicate (n=2) wells. Drug free HBSS buffer (pH 7.4) containing 1% bovine serum albumin (BSA) was introduced to the receiver (A-B-basolateral; B-A- Apical) compartments. The volumes of apical and basolateral compartments were 0.4 and 0.8 mL, respectively. After adding dosing solution, plates were incubated in an incubator for 120 minutes at 37 °C. After 120 minutes, donor and receiver samples were collected and matrix matched (1:1, 30 μL study sample + 30 μL blank buffer) with the opposite buffer. Dosing samples matrix matched (1:1, 30 μL study sample + 30 μL blank buffer) with the opposite buffer. Samples were processed by adding acetonitrile containing internal standard (60 μL study sample + 200 μL acetonitrile containing internal standard -Tolbutamide, 500 ng/mL).

Samples were vortexed and centrifuged at 4000 rpm for 10 minutes. The obtained supernatant (100 μL) was diluted with 100 μL of water and transferred to fresh 96 well plates. The concentration of compounds in the samples was analyzed by liquid chromatography tandem mass spectrometry (LC-MS/MS) method using discovery grade bio-analytical method, as applicable.

The mean apparent permeability ($P_{app}$, x $10^{-6}$ cm/sec) of the test compounds, atenolol, propranolol and digoxin were calculated as follows:

$$\text{Papp} = \frac{dq}{dt} X \frac{1}{Co} X \frac{1}{A}$$

where dq/dt = rate of transport (rate of transport of compound in the receiver compartment), $C_0$ = initial concentration in the donor compartment, A = surface area of the effective filter membrane.

*HepaRG-based assay protocol*

A cryopreserved vial of differentiated HepaRG cells (Biopredic International HPR116080) is thawed in HepaRG Thawing/Plating/General Purpose Medium (Biopredic International ADD670C) supplemented with 200 mM Glutamax (Gibco 35050061) following the protocol provided by Biopredic International. 70,000 cells per well are seeded in 96-wells plate (Corning CLS3809) in 100 μL of HepaRG Thawing/Plating/General Purpose Medium supplemented with 200 mM Glutamax and incubated at 37 °C in 5% $CO_2$ for 24 hours. Post incubation, the seeding media is replaced by HepaRG Maintenance/Metabolism Medium (Biopredic International ADD620C) and incubated for 6 days, with fresh HepaRG Maintenance/Metabolism Medium replenishment every 48 hours. After 7 days incubation post seeding, incubation media is decanted from the wells and cells are washed two times with 250 μL of William's E Basal Media (Gibco 12551032). After decanting William's E Basal Media each time, plates are tapped against paper towel to ensure maximum removal of residual media. Incubation mix is prepared by adding test inhibitor dilutions (3-fold serial dilution in DMSO (Sigma D2650)) in William's E media (basal) containing 0.3 μM 3H-taurocholic acid (ARC ART-1368) and 7.5 μM cold taurocholic acid (Sigma T4009) (maintaining 0.2% final DMSO concentration). 50 μl of incubation mix containing test inhibitors is then added to the wells (in duplicate) and the plates are incubated for 30 minutes in 5% $CO_2$ incubator at 37 °C. After incubation,

the reaction is stopped by keeping the plates on ice water mix for 2-3 minutes and the incubation mix is then aspirated completely from the wells. The wells are washed two times with 250 μL of chilled unlabelled 1 mM taurocholic acid dissolved in HEPES (Gibco 15630080)-buffered (10mM) HBSS (Gibco 14175079) (pH 7.4). The plates are tapped against a paper towel after every wash to ensure maximum removal of blocking buffer.

100 μL of MicroScint-20 (PerkinElmer 6013621) is added to the wells and kept overnight at RT before reading the plates in TopCount NXT™ Microplate Scintillation and Luminescence Counter from PerkinElmer under 3H Test protocol (set at 120 seconds reading time per well, with normal plate orientation).

*Preparation of test compound dilutions*

All test compounds were provided in powder form at RT. 10 mM DMSO stocks of the test compounds were prepared, aliquoted and stored at -20 °C. From the 10 mM DMSO stock of the compounds, a 3-fold serial dilution in DMSO was prepared to get a total of 10 dilutions of the test compounds. 0.5 μL of this dilution in DMSO was added to 250 μL of FBS-free basal media containing 3H-taurocholic acid and cold taurocholic acid to prepare the incubation mixture.

*Bioavailability studies*

Male mice (C57BL/6 or CD1) or Wistar rats of 8-9 weeks old were used. For each test compound, two groups of 3 animals each were used. One group was administered a single intravenous dose of 1 mg/kg (vehicle 100% DMSO) through the tail vein and the other group was administered a single oral dose of 10 mg/kg through gavage needle. The group that was administered an oral dose was fasted overnight. Blood samples were collected after 0.083, 0.25, 0.5, 1, 2, 4, 6, 8 and 24 hours following intravenous administration, and after 0.25, 0.5, 1, 2, 4, 6, 8 and 24 hours following oral administration. Blood samples were taken from saphenous vein. 0.2% EDTA was used as the anticoagulant. The samples were analyzed by a discovery grade bioanalytical method developed for the estimation of test compound in plasma, using an LC-MS/MS system.

*Results*

Biological data for the compounds of the examples is shown in Table 8 below.

**Table 8**

| Example | hLBAT $IC_{50}$ (nM) | hIBAT $IC_{50}$ (nM) | Permeability (Caco-2) | |
|---|---|---|---|---|
| | | | $P_{app}$ A2B (x $10^{-6}$ cm/sec) | $P_{app}$ B2A (x $10^{-6}$ cm/sec) |
| 1 | 3824 | | | |
| 2, *enantiomer 1* | 2019 | 0.2 | 7.5 | 9.3 |
| 2, *enantiomer 2* | 3148 | 2.8 | | |
| 3 | 3366 | 1.7 | | |
| 4 | 7261 | 0.8 | | |
| 5, *enantiomer 1* | 5996 | 0.7 | 29.3 | 25.3 |
| 5, *enantiomer 2* | 4400 | 44 | | |
| 6 | >2200 | 0.6 | | |
| 7, *enantiomer 1* | 5045 | 0.4 | 24.3 | 21.3 |
| 7, *enantiomer 2* | 4843 | 2.8 | | |
| 8 | >3333 | 0.8 | | |
| 9 | >3333 | 0.5 | | |
| 10, *enantiomer 1* | >3333 | 0.3 | | |
| 10, *enantiomer 2* | >3333 | 1.7 | | |
| 11 | 34 | 70 | | |
| 12, *enantiomer 1* | >10000 | 115 | | |

(continued)

| Example | hLBAT $IC_{50}$ (nM) | hIBAT $IC_{50}$ (nM) | Permeability (Caco-2) | |
|---|---|---|---|---|
| | | | $P_{app}$ A2B (x $10^{-6}$ cm/sec) | $P_{app}$ B2A (x $10^{-6}$ cm/sec) |
| 12, *enantiomer 2* | 34 | 116 | | |
| 13 | | 2.3 | | |
| 14, *enantiomer 1* | >1000 | 1.1 | | |
| 14, *enantiomer 2* | >1000 | 64 | | |
| 15 | 176 | 60 | | |
| 16, *enantiomer 1* | 164 | 10 | | |
| 16, *enantiomer 2* | 220 | 659 | | |
| 17 | 216 | 88 | | |
| 18 | 135 | 404 | | |
| 19, *enantiomer 1* | 194 | 192 | | |
| 19, *enantiomer 2* | 176 | 1872 | | |
| 20 | 2514 | 522 | | |
| 21, *enantiomer 1* | 3310 | >243 | | |
| 21, *enantiomer 2* | 1899 | >554 | | |
| 22 | >3333 | 900 | | |
| 23 | 145 | >3333 | | |
| 24 | 85 | >3333 | | |
| 25 | 135 | 217 | | |
| 26, *enantiomer 1* | 104 | 195 | | |
| 26, *enantiomer 2* | 245 | 5106 | | |
| 27 | >10000 | 14 | | |
| 28, *enantiomer 1* | >10000 | 7.7 | | |
| 28, *enantiomer 2* | >10000 | 532 | | |
| 29 | 107 | 558 | | |
| 30, *enantiomer 1* | >10000 | >10000 | | |
| 30, *enantiomer 2* | 200 | 349 | | |
| 31 | >3333 | 1.3 | | |
| 32 | >3333 | 0.6 | | |
| 33 | 150 | 295 | | |
| 34, *enantiomer 1* | 110 | 161 | | |
| 34, *enantiomer 2* | 155 | 1963 | | |
| 35 | 362 | 3113 | | |
| 36, *enantiomer 1* | >10000 | >10000 | | |
| 36, *enantiomer 2* | 155 | 2755 | | |
| 37 | 21 | >10000 | | |
| 38, *enantiomer 1* | 1132 | >10000 | | |
| 38, *enantiomer 2* | 6.6 | 1602 | | |
| 39 | >3333 | 2.5 | | |

(continued)

| Example | hLBAT $IC_{50}$ (nM) | hIBAT $IC_{50}$ (nM) | Permeability (Caco-2) | |
|---|---|---|---|---|
| | | | $P_{app}$ A2B (x $10^{-6}$ cm/sec) | $P_{app}$ B2A (x $10^{-6}$ cm/sec) |
| 40, *enantiomer 1* | >10000 | 1.4 | | |
| 40, *enantiomer 2* | >10000 | 36 | | |
| 41 | 134 | >1111 | | |
| 42, *enantiomer 1* | 223 | 704 | | |
| 42, *enantiomer 2* | 241 | >10000 | | |
| 43 | 251 | 861 | | |
| 44 | >1000 | 5.2 | | |
| 45, enantiomer 1 | >1000 | 28 | | |
| 45, enantiomer 2 | >1000 | 3.2 | | |
| 46 | >1000 | 4.8 | | |
| 47 | >1000 | 14 | | |
| 48, enantiomer 1 | >1000 | 231 | | |
| 48, enantiomer 2 | >1000 | 5.8 | | |
| 49 | 248 | 2325 | | |
| 50 | 50 | 558 | | |
| 51 | 142 | 2610 | | |
| 52 | >1000 | 7.4 | | |
| 53 | >1000 | 14 | | |
| 54 | >1000 | 0.1 | | |

*PD model: Evaluation of test compound on total bile acids levels in male C57BL6 mice.*

C57BL/6N Tac mice of 8-9 weeks old are used to study the effect of bile acid modulators on bile acid levels. After completion of quarantine and acclimatization period, animals are randomized based on bodyweight into x experimental groups: (i) vehicle control, and (ii) test compound y mg/kg po once daily. Animals are treated with test compound for 7 days. On day 5 of the study, animals are individually housed in fresh cages. On day 7, feces are collected from each cage, followed by blood withdrawal from each animal through retro-orbital route. Animals are euthanized to collect liver and terminal ileum from each animal for further analysis. Bodyweight and food consumption are measured twice weekly. Serum lipid profiles are analyzed in serum samples of day 7. Total bile acids in serum is measured in the serum samples of day 7. Fecal bile excretion is measured in the fecal sample of day 7. Hepatic expression of CYP7A1 and SHP are quantified in the liver samples of day 7. Liver triglycerides and total cholesterol are analyzed in the liver samples of day 7.

*Urine bile acid model: Evaluation of test compounds on urine bile acid levels in male C57BL/6N mice.*

C57BL/6N Tac mice of 8-9 weeks old are used to study the effect of bile acid modulators on bile acid levels. After completion of quarantine and acclimatization period, animals are randomized based on bodyweight into x experimental groups: (i) vehicle control, and (ii) test compound y mg/kg po once daily. Animals are treated with test compound for 7 days. On day 6 of the study, animals are transferred to a metabolic cage. On day 7, feces and urine are collected from each metabolic cage, followed by blood withdrawal from each animal through retro-orbital route. Animals are euthanized to collect kidney from each animal for further analysis. Bodyweight is measured twice weekly. Total bile acids in serum is measured in serum samples of day 7. Fecal bile acid excretion is measured in the fecal sample of day 7. Urine excretion of bile acids is measured in the sample of day 7. Kidney expression of ASBT, OSTa, OSTAb and MRP2 is quantified in the samples of day 7.

## Claims

1. A compound of formula (I)

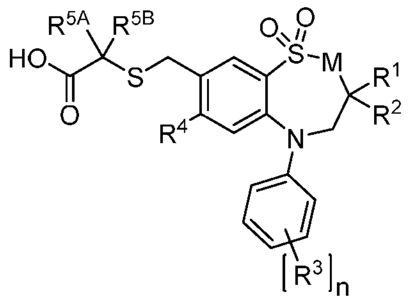

(I)

wherein

M is $-CH_2-$ or $-NR^6-$;
$R^1$ is $C_{1-4}$ alkyl;
$R^2$ is selected from the group consisting of hydrogen and $C_{1-4}$ alkyl;
$R^3$ is independently selected from the group consisting of hydrogen, halogen, hydroxy, $C_{1-4}$ alkyl, $C_{1-4}$ haloalkyl, $C_{1-4}$ alkoxy, $C_{1-4}$ haloalkoxy, cyano, nitro, amino, *N*-($C_{1-4}$ alkyl)amino and *N,N*-di($C_{1-4}$ alkyl)amino;
n is an integer 1, 2 or 3;
$R^4$ is selected from the group consisting of hydrogen, halogen, cyano, $C_{1-4}$ alkyl, $C_{3-6}$ cycloalkyl, $C_{1-4}$ alkoxy, $C_{3-6}$ cycloalkyloxy, $C_{1-4}$ alkylthio, $C_{3-6}$ cycloalkylthio, $C_{1-4}$ alkylsulfonyl, $C_{3-6}$ cycloalkylsulfonyl, amino, *N*-($C_{1-4}$ alkyl) amino and *N,N*-di($C_{1-4}$ alkyl)amino;
$R^{5A}$ and $R^{5B}$ are each independently selected from the group consisting of hydrogen, halogen, hydroxy, $C_{1-4}$ alkyl and $C_{1-4}$ alkoxy; or $R^{5A}$ and $R^{5B}$, together with the carbon atom to which they are attached, form a 3- to 5-membered saturated carbocyclic ring;
$R^6$ is selected from the group consisting of hydrogen and $C_{1-4}$ alkyl;

or a pharmaceutically acceptable salt thereof.

2. A compound according to claim 1, wherein $R^1$ is *n*-butyl.

3. A compound according to claim 1 or 2, wherein $R^2$ is hydrogen.

4. A compound according to claim 1 or 2, wherein $R^2$ is methyl, ethyl or n-butyl.

5. A compound according to any one of claims 1 to 4, wherein $R^3$ is independently selected from the group consisting of hydrogen, fluoro, chloro, bromo, hydroxy, methoxy, amino, methylamino and dimethylamino.

6. A compound according to any one of claims 1 to 5, wherein $R^4$ is selected from the group consisting of fluoro, chloro, bromo, methyl, ethyl, cyclopropyl, methoxy, ethoxy, methylthio, ethylthio, methylsulfonyl, amino, methylamino and dimethylamino.

7. A compound according to any one of claims 1 to 6, wherein $R^{5A}$ and $R^{5B}$ are each independently hydrogen or $C_{1-4}$ alkyl, or together with the carbon atom to which they are attached, form a cyclopropyl ring.

8. A compound according to any one of claims 1 to 7, wherein $R^6$ is hydrogen or methyl.

9. A compound according to claim 1, selected from the group consisting of:

2-(((3-butyl-3-ethyl-7-(methylthio)-1,1-dioxido-5-phenyl-2,3,4,5-tetrahydro-1,5-benzothiazepin-8-yl)methyl) thio)acetic acid;
(S)-2-(((3-butyl-3-ethyl-7-(methylthio)-1,1-dioxido-5-phenyl-2,3,4,5-tetrahydro-1,5-benzothiazepin-8-yl) methyl)thio)acetic acid;
(R)-2-(((3-butyl-3-ethyl-7-(methylthio)-1,1-dioxido-5-phenyl-2,3,4,5-tetrahydro-1,5-benzothiazepin-8-yl)methyl)thio)acetic acid;
2-(((3,3-dibutyl-7-(methylthio)-1,1-dioxido-5-phenyl-2,3,4,5-tetrahydro-1,5-benzothiazepin-8-yl)methyl)thio) acetic acid;
2-(((3-butyl-3-methyl-7-(methylthio)-1,1-dioxido-5-phenyl-2,3,4,5-tetrahydro-1,5-benzothiazepin-8-yl)methyl) thio)acetic acid;
(S)-2-(((3-butyl-3-methyl-7-(methylthio)-1,1-dioxido-5-phenyl-2,3,4,5-tetrahydro-1,5-benzothiazepin-8-yl) methyl)thio)acetic acid;
(R)-2-(((3-butyl-3-methyl-7-(methylthio)-1,1-dioxido-5-phenyl-2,3,4,5-tetrahydro-1,5-benzothiazepin-8-yl) methyl)thio)acetic acid;
2-(((3-butyl-3-ethyl-5-(4-fluorophenyl)-7-(methylthio)-1,1-dioxido-2,3,4,5-tetrahydro-1,5-benzothiazepin-8-yl) methyl)thio)acetic acid;
(S)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetic acid;
(R)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)acetic acid;
2-(((3,3-dibutyl-7-(methylthio)-1,1-dioxido-5-phenyl-2,3,4,5-tetrahydro-1,2,5-benzothiadiazepin-8-yl)methyl) thio)acetic acid;
2-(((3-butyl-3-ethyl-7-(methylthio)-1,1-dioxido-5-phenyl-2,3,4,5-tetrahydro-1,2,5-benzothiadiazepin-8-yl) methyl)thio)acetic acid;
(S)-2-(((3-butyl-3-ethyl-7-(methylthio)-1,1-dioxido-5-phenyl-2,3,4,5-tetrahydro-1,2,5-benzothiadiazepin-8-yl) methyl)thio)acetic acid;
(R)-2-(((3-butyl-3-ethyl-7-(methylthio)-1,1-dioxido-5-phenyl-2,3,4,5-tetrahydro-1,2,5-benzothiadiazepin-8-yl) methyl)thio)acetic acid;
2-(((3-butyl-2-methyl-7-(methylthio)-1,1-dioxido-5-phenyl-2,3,4,5-tetrahydro-1,2,5-benzothiadiazepin-8-yl) methyl)thio)acetic acid;
(S)-2-(((3-butyl-2-methyl-7-(methylthio)-1,1-dioxido-5-phenyl-2,3,4,5-tetrahydro-1,2,5-benzothiadiazepin-8-yl) methyl)thio)acetic acid;
(R)-2-(((3-butyl-2-methyl-7-(methylthio)-1,1-dioxido-5-phenyl-2,3,4,5-tetrahydro-1,2,5-benzothiadiazepin-8-yl) methyl)thio)acetic acid;
2-(((3-Butyl-3-ethyl-7-methoxy-1,1-dioxido-5-phenyl-2,3,4,5-tetrahydro-1,5-benzothiazepin-8-yl)methyl)thio) acetic acid;
(S)-2-(((3-butyl-3-ethyl-7-methoxy-1,1-dioxido-5-phenyl-2,3,4,5-tetrahydro-1,5-benzothiazepin-8-yl)methyl) thio)acetic acid;
(R)-2-(((3-butyl-3-ethyl-7-methoxy-1,1-dioxido-5-phenyl-2,3,4,5-tetrahydro-1,5-benzothiazepin-8-yl)methyl) thio)acetic acid;
2-(((3-Butyl-3-ethyl-5-(4-fluorophenyl)-7-(methylthio)-1,1-dioxido-2,3,4,5-tetrahydro-1,5-benzothiazepin-8-yl) methyl)thio)-2-methylpropanoic acid;
(S)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid;
(R)-2-(((3-butyl-3-ethyl-5-(4-fluorophenyl)-7-(methylthio)-1,1-dioxido-2,3,4,5-tetrahydro-1,5-benzothiazepin-8-yl)methyl)thio)-2-methylpropanoic acid;
2-(((3,3-Dibutyl-7-(methylthio)-1,1-dioxido-5-phenyl-2,3,4,5-tetrahydro-1,5-benzothiazepin-8-yl)methyl) thio)-2-methylpropanoic acid;
2-(((3-Butyl-3-methyl-7-(methylthio)-1,1-dioxido-5-phenyl-2,3,4,5-tetrahydro-1,5-benzothiazepin-8-yl)methyl) thio)-2-methylpropanoic acid;
(S)-2-(((3-butyl-3-methyl-7-(methylthio)-1,1-dioxido-5-phenyl-2,3,4,5-tetrahydro-1,5-benzothiazepin-8-yl) methyl)thio)-2-methylpropanoic acid;
(R)-2-(((3-butyl-3-methyl-7-(methylthio)-1,1-dioxido-5-phenyl-2,3,4,5-tetrahydro-1,5-benzothiazepin-8-yl) methyl)thio)-2-methylpropanoic acid;
2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
(S)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
(R)-2-(((3-butyl-3-ethyl-2-methyl-7-(methylthio)-1,1-dioxido-5-phenyl-2,3,4,5-tetrahydro-1,2,5-benzothiadiazepin-8-yl)methyl)thio)acetic acid;
2-(((3,3-dibutyl-2-methyl-7-(methylthio)-1,1-dioxido-5-phenyl-2,3,4,5-tetrahydro-1,2,5-benzothiadiazepin-8-yl) methyl)thio)acetic acid;
2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoic acid;
2-(((3,3-dibutyl-2-methyl-7-(methylthio)-1,1-dioxido-5-phenyl-2,3,4,5-tetrahydro-1,2,5-benzothiadiazepin-8-yl)methyl)thio)-2-methylpropanoic acid;
2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
(S)-2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
(R)-2-(((3-butyl-3-ethyl-7-methoxy-1,1-dioxido-5-phenyl-2,3,4,5-tetrahydro-1,5-benzothiazepin-8-yl)methyl)thio)-2-methylpropanoic acid;
2-(((3-butyl-7-methoxy-3-methyl-1,1-dioxido-5-phenyl-2,3,4,5-tetrahydro-1,5-benzothiazepin-8-yl)methyl)thio)acetic acid;
(S)-2-(((3-butyl-7-methoxy-3-methyl-1,1-dioxido-5-phenyl-2,3,4,5-tetrahydro-1,5-benzothiazepin-8-yl)methyl)thio)acetic acid;
(R)-2-(((3-butyl-7-methoxy-3-methyl-1,1-dioxido-5-phenyl-2,3,4,5-tetrahydro-1,5-benzothiazepin-8-yl)methyl)thio)acetic acid;
2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;
(S)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;
(R)-2-(((3-butyl-5-(4-fluorophenyl)-7-methoxy-2-methyl-1,1-dioxido-2,3,4,5-tetrahydro-1,2,5-benzothiadiazepin-8-yl)methyl)thio)acetic acid;
2-(((3,3-dibutyl-7-methoxy-1,1-dioxido-5-phenyl-2,3,4,5-tetrahydro-1,5-benzothiazepin-8-yl)methyl)thio)acetic acid;
2-(((3,3-dibutyl-5-(4-fluorophenyl)-7-methoxy-1,1-dioxido-2,3,4,5-tetrahydro-1,5-benzothiazepin-8-yl)methyl)thio)acetic acid;
2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid;
(S)-2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid;
(R)-2-(((3-butyl-3-ethyl-5-(4-fluorophenyl)-7-methoxy-1,1-dioxido-2,3,4,5-tetrahydro-1,5-benzothiazepin-8-yl)methyl)thio)-2-methylpropanoic acid;
2-(((3-butyl-7-methoxy-2-methyl-1,1-dioxido-5-phenyl-2,3,4,5-tetrahydro-1,2,5-benzothiadiazepin-8-yl)methyl)thio)acetic acid;
(S)-2-(((3-butyl-7-methoxy-2-methyl-1,1-dioxido-5-phenyl-2,3,4,5-tetrahydro-1,2,5-benzothiadiazepin-8-yl)methyl)thio)acetic acid;
(R)-2-(((3-butyl-7-methoxy-2-methyl-1,1-dioxido-5-phenyl-2,3,4,5-tetrahydro-1,2,5-benzothiadiazepin-8-yl)methyl)thio)acetic acid;
2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoic acid;
(S)-2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoic acid;
(R)-2-(((3-butyl-7-methoxy-2-methyl-1,1-dioxido-5-phenyl-2,3,4,5-tetrahydro-1,2,5-benzothiadiazepin-8-yl)methyl)thio)-2-methylpropanoic acid;
2-(((3-butyl-3-ethyl-5-(4-fluorophenyl)-7-methoxy-1,1-dioxido-2,3,4,5-tetrahydro-1,5-benzothiazepin-8-yl)methyl)thio)acetic acid;
(S)-2-(((3-butyl-3-ethyl-5-(4-fluorophenyl)-7-methoxy-1,1-dioxido-2,3,4,5-tetrahydro-1,5-benzothiazepin-8-yl)methyl)thio)acetic acid;
(R)-2-(((3-butyl-3-ethyl-5-(4-fluorophenyl)-7-methoxy-1,1-dioxido-2,3,4,5-tetrahydro-1,5-benzothiazepin-8-yl)methyl)thio)acetic acid;
2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid;
(S)-2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid;
(R)-2-(((3-butyl-7-methoxy-3-methyl-1,1-dioxido-5-phenyl-2,3,4,5-tetrahydro-1,5-benzothiazepin-8-yl)methyl)thio)-2-methylpropanoic acid;
2-(((3,3-dibutyl-7-methoxy-1,1-dioxido-5-phenyl-2,3,4,5-tetrahydro-1,5-benzothiazepin-8-yl)methyl)thio)-2-methylpropanoic acid;
2-(((3-butyl-3-ethyl-7-methoxy-1,1-dioxido-5-phenyl-2,3,4,5-tetrahydro-1,2,5-benzothiadiazepin-8-yl)methyl)thio)acetic acid;
(R)-2-(((3-butyl-3-ethyl-7-methoxy-1,1-dioxido-5-phenyl-2,3,4,5-tetrahydro-1,2,5-benzothiadiazepin-8-yl)methyl)thio)acetic acid;
(S)-2-(((3-butyl-3-ethyl-7-methoxy-1,1-dioxido-5-phenyl-2,3,4,5-tetrahydro-1,2,5-benzothiadiazepin-8-yl)methyl)thio)acetic acid;
2-(((3-butyl-7-(dimethylamino)-3-ethyl-1,1-dioxido-5-phenyl-2,3,4,5-tetrahydro-1,2,5-benzothiadiazepin-8-yl)methyl)thio)acetic acid;
2-(((3-butyl-3-ethyl-7-(methylsulfonyl)-1,1-dioxido-5-phenyl-2,3,4,5-tetrahydro-1,2,5-benzothiadiazepin-8-yl)methyl)thio)acetic acid;
(S)-2-(((3-Butyl-3-ethyl-7-(methylsulfonyl)-1,1-dioxido-5-phenyl-2,3,4,5-tetrahydro-1,2,5-benzothiadiazepin-8-yl)methyl)thio)acetic acid;
(R)-2-(((3-butyl-3-ethyl-7-(methylsulfonyl)-1,1-dioxido-5-phenyl-2,3,4,5-tetrahydro-1,2,5-benzothiadiazepin-8-yl)methyl)thio)acetic acid;
(R)-2-(((3-butyl-7-(dimethylamino)-1,1-dioxido-5-phenyl-2,3,4,5-tetrahydro-1,2,5-benzothiadiazepin-8-yl)methyl)thio)acetic acid;
(R)-2-(((3-butyl-7-(methylthio)-1,1-dioxido-5-phenyl-2,3,4,5-tetrahydro-1,2,5-benzothiadiazepin-8-yl)methyl)thio)acetic acid;
(R)-2-(((3-butyl-7-methoxy-1,1-dioxido-5-phenyl-2,3,4,5-tetrahydro-1,2,5-benzothiadiazepin-8-yl)methyl)thio)acetic acid;
2-(((3-Butyl-7-(dimethylamino)-3-methyl-1,1-dioxido-5-phenyl-2,3,4,5-tetrahydro-1,2,5-benzothiadiazepin-8-yl)methyl)thio)acetic acid;
2-(((3-butyl-7-methoxy-3-methyl-1,1-dioxido-5-phenyl-2,3,4,5-tetrahydro-1,2,5-benzothiadiazepin-8-yl)methyl)thio)acetic acid; and
2-(((3-Butyl-3-methyl-7-(methylthio)-1,1-dioxido-5-phenyl-2,3,4,5-tetrahydro-1,2,5-benzothiadiazepin-8-yl)methyl)thio)acetic acid;

or a pharmaceutically acceptable salt thereof.

**10.** A pharmaceutical composition comprising a therapeutically effective amount of a compound according to any one of claims 1 to 9, and one or more pharmaceutically acceptable excipients.

**11.** The compound according to any one of claims 1 to 9, for use as a medicament.

**12.** The compound according to any one of claims 1 to 9, for use in the treatment or prevention of a cardiovascular disease or a disorder of fatty acid metabolism or a glucose utilization disorder, including hypercholesterolemia; type 1 and type 2 diabetes mellitus; complications of diabetes, including cataracts, micro- and macrovascular diseases, retinopathy, neuropathy, nephropathy and delayed wound healing, tissue ischaemia, diabetic foot, arteriosclerosis, myocardial infarction, acute coronary syndrome, unstable angina pectoris, stable angina pectoris, stroke, peripheral arterial occlusive disease, cardiomyopathy, heart failure, heart rhythm disorders and vascular restenosis; diabetes-related diseases including insulin resistance (impaired glucose homeostasis), hyperglycemia, hyperinsulinemia, elevated blood levels of fatty acids or glycerol, obesity, dyslipidemia, hyperlipidemia including hypertriglyceridemia, metabolic syndrome (syndrome X), atherosclerosis and hypertension; and for increasing high density lipoprotein levels.

**13.** The compound according to any one of claims 1 to 9, for use in the treatment or prevention of a gastrointestinal disease or disorder, including constipation (including chronic constipation, functional constipation, chronic idiopathic constipation (CIC), intermittent/sporadic constipation, constipation secondary to diabetes mellitus, constipation secondary to stroke, constipation secondary to chronic kidney disease, constipation secondary to multiple sclerosis, constipation secondary to Parkinson’s disease, constipation secondary to systemic sclerosis, drug induced constipation, irritable bowel syndrome with constipation (IBS-C), irritable bowel syndrome mixed (IBS-M), pediatric functional constipation and opioid induced constipation); Crohn’s disease; primary bile acid malabsorption; irritable bowel syndrome (IBS); inflammatory bowel disease (IBD); ileal inflammation; and reflux disease and complications thereof, including Barrett’s esophagus, bile reflux esophagitis and bile reflux gastritis.

**14.** The compound according to any one of claims 1 to 9, for use in the treatment or prevention of a liver disease or disorder, including an inherited metabolic disorder of the liver; inborn errors of bile acid synthesis; congenital bile duct anomalies; biliary atresia; post-Kasai biliary atresia; post-liver transplantation biliary atresia; neonatal hepatitis; neonatal cholestasis; hereditary forms of cholestasis; cerebrotendinous xanthomatosis; a secondary defect of BA synthesis; Zellweger’s syndrome; cystic fibrosis-associated liver disease; alpha1-antitrypsin deficiency; Alagilles

syndrome (ALGS); Byler syndrome; a primary defect of bile acid (BA) synthesis; progressive familial intrahepatic cholestasis (PFIC) including PFIC-1, PFIC-2, PFIC-3 and non-specified PFIC, post-biliary diversion PFIC and post-liver transplant PFIC; benign recurrent intrahepatic cholestasis (BRIC) including BRIC1, BRIC2 and non-specified BRIC, post-biliary diversion BRIC and post-liver transplant BRIC; autoimmune hepatitis; primary biliary cirrhosis (PBC); liver fibrosis; non-alcoholic fatty liver disease (NAFLD); non-alcoholic steatohepatitis (NASH); portal hypertension; cholestasis; Down syndrome cholestasis; drug-induced cholestasis; intrahepatic cholestasis of pregnancy (jaundice during pregnancy); intrahepatic cholestasis; extrahepatic cholestasis; parenteral nutrition associated cholestasis (PNAC); low phospholipid-associated cholestasis; lymphedema cholestasis syndrome 1 (LCS1); primary sclerosing cholangitis (PSC); immunoglobulin G4 associated cholangitis; primary biliary cholangitis; cholelithiasis (gallstones); biliary lithiasis; choledocholithiasis; gallstone pancreatitis; Caroli disease; malignancy of bile ducts; malignancy causing obstruction of the biliary tree; biliary strictures; AIDS cholangiopathy; ischemic cholangiopathy; pruritus due to cholestasis or jaundice; pancreatitis; chronic autoimmune liver disease leading to progressive cholestasis; hepatic steatosis; alcoholic hepatitis; acute fatty liver; fatty liver of pregnancy; drug-induced hepatitis; iron overload disorders; congenital bile acid synthesis defect type 1 (BAS defect type 1); drug-induced liver injury (DILI); hepatic fibrosis; congenital hepatic fibrosis; hepatic cirrhosis; Langerhans cell histiocytosis (LCH); neonatal ichthyosis sclerosing cholangitis (NISCH); erythropoietic protoporphyria (EPP); idiopathic adulthood ductopenia (IAD); idiopathic neonatal hepatitis (INH); non syndromic paucity of interlobular bile ducts (NS PILBD); North American Indian childhood cirrhosis (NAIC); hepatic sarcoidosis; amyloidosis; necrotizing enterocolitis; serum bile acid-caused toxicities, including cardiac rhythm disturbances (including atrial fibrillation) in setting of abnormal serum bile acid profile, cardiomyopathy associated with liver cirrhosis ("cholecardia"), and skeletal muscle wasting associated with cholestatic liver disease; polycystic liver disease; viral hepatitis (including hepatitis A, hepatitis B, hepatitis C, hepatitis D and hepatitis E); hepatocellular carcinoma (hepatoma); cholangiocarcinoma; bile acid-related gastrointestinal cancers; and cholestasis caused by tumours and neoplasms of the liver, of the biliary tract and of the pancreas; or for use in the enhancement of corticosteroid therapy in liver disease.

**15.** The compound according to any one of claims 1 to 9, for use in the treatment or prevention of hyperabsorption syndromes (including abetalipoproteinemia, familial hypobetalipo-proteinemia (FHBL), chylomicron retention disease (CRD) and sitosterolemia); hypervitaminosis and osteopetrosis; hypertension; glomerular hyperfiltration; polycystic kidney disease (PKD), including autosomal dominant polycystic kidney disease (ADPKD) and autosomal recessive polycystic kidney disease (ARPKD); and pruritus of renal failure; or for use in the protection against liver- or metabolic disease-associated kidney injury.

## Patentansprüche

**1.** Verbindung der Formel (I)

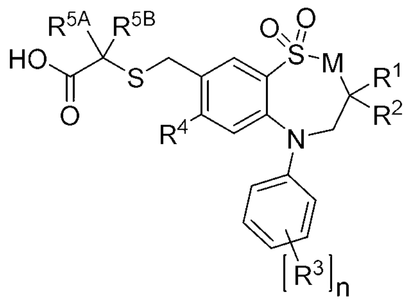

(I)

wobei

M -$CH_2$- or -$NR^6$- ist;
$R^1$ $C_{1-4}$-Alkyl ist;
$R^2$ ausgewählt ist aus der Gruppe bestehend aus Wasserstoff und $C_{1-4}$-Alkyl;
$R^3$ unabhängig ausgewählt ist aus der Gruppe bestehend aus Wasserstoff, Halogen, Hydroxy, $C_{1-4}$-Alkyl, $C_{1-4}$-Halogenalkyl, $C_{1-4}$-Alkoxy, $C_{1-4}$-Halogenalkoxy, Cyano, Nitro, Amino, *N*-($C_{1-4}$-Alkyl)amino und *N,N*-Di($C_{1-4}$-alkyl)amino;
n eine ganze Zahl 1, 2 oder 3 ist;
$R^4$ ausgewählt ist aus der Gruppe bestehend aus Wasserstoff, Halogen, Cyano, $C_{1-4}$-Alkyl, $C_{3-6}$-Cycloalkyl,

$C_{1-4}$-Alkoxy, $C_{3-6}$-Cycloalkyloxy, $C_{1-4}$-Alkylthio, $C_{3-6}$-Cycloalkylthio, $C_{1-4}$-Alkylsulfonyl, $C_{3-6}$-Cycloalkylsulfonyl, Amino, N-($C_{1-4}$-Alkyl)amino und *N,N*-Di($C_{1-4}$-alkyl)amino;
$R^{5A}$ und $R^{5B}$ jeweils unabhängig voneinander ausgewählt sind aus der Gruppe bestehend aus Wasserstoff, Halogen, Hydroxy, $C_{1-4}$-Alkyl und $C_{1-4}$-Alkoxy; oder $^{5A}$ und $R^{5B}$ zusammen mit dem Kohlenstoffatom, an das sie gebunden sind, einen 3- bis 5-gliedrigen gesättigten carbocyclischen Ring bilden;
$R^6$ ausgewählt ist aus der Gruppe bestehend aus Wasserstoff und $C_{1-4}$-Alkyl;

oder ein pharmazeutisch akzeptables Salz davon.

**2.** Verbindung nach Anspruch 1, wobei $R^1$ *n*-Butyl ist.

**3.** Verbindung nach Anspruch 1 oder 2, wobei $R^2$ Wasserstoff ist.

**4.** Verbindung nach Anspruch 1 oder 2, wobei $R^2$ Methyl, Ethyl oder n-Butyl ist.

**5.** Verbindung nach einem der Ansprüche 1 bis 4, wobei $R^3$ unabhängig ausgewählt ist aus der Gruppe bestehend aus Wasserstoff, Fluor, Chlor, Brom, Hydroxy, Methoxy, Amino, Methylamino und Dimethylamino.

**6.** Verbindung nach einem der Ansprüche 1 bis 5, wobei $R^4$ ausgewählt ist aus der Gruppe bestehend aus Fluor, Chlor, Brom, Methyl, Ethyl, Cyclopropyl, Methoxy, Ethoxy, Methylthio, Ethylthio, Methylsulfonyl, Amino, Methylamino und Dimethylamino.

**7.** Verbindung nach einem der Ansprüche 1 bis 6, wobei $R^{5A}$ und $R^{5B}$ jeweils unabhängig voneinander Wasserstoff oder $C_{1-4}$-Alkyl sind oder zusammen mit dem Kohlenstoffatom, an das sie gebunden sind, einen Cyclopropylring bilden.

**8.** Verbindung nach einem der Ansprüche 1 bis 7, wobei $R^6$ Wasserstoff oder Methyl ist.

**9.** Verbindung nach Anspruch 1, ausgewählt aus der Gruppe bestehend aus:

2-(((3-Butyl-3-ethyl-7-(methylthio)-1,1-dioxido-5-phenyl-2,3,4,5-tetrahydro-1,5-benzothiazepin-8-yl)methyl) thio)essigsäure;
(S)-2-(((3-Butyl-3-ethyl-7-(methylthio)-1,1-dioxido-5-phenyl-2,3,4,5-tetrahydro-1,5-benzothiazepin-8-yl)methyl)thio)essigsäure;
(R)-2-(((3-Butyl-3-ethyl-7-(methylthio)-1,1-dioxido-5-phenyl-2,3,4,5-tetrahydro-1,5-benzothiazepin-8-yl)methyl)thio)essigsäure;
2-(((3,3-Dibutyl-7-(methylthio)-1,1-dioxido-5-phenyl-2,3,4,5-tetrahydro-1,5-benzothiazepin-8-yl)methyl)thio) essigsäure;
2-(((3-Butyl-3-methyl-7-(methylthio)-1,1-dioxido-5-phenyl-2,3,4,5-tetrahydro-1,5-benzothiazepin-8-yl)methyl) thio)essigsäure;
(S)-2-(((3-Butyl-3-methyl-7-(methylthio)-1,1-dioxido-5-phenyl-2,3,4,5-tetrahydro-1,5-benzothiazepin-8-yl)methyl)thio)essigsäure;
(R)-2-(((3-Butyl-3-methyl-7-(methylthio)-1,1-dioxido-5-phenyl-2,3,4,5-tetrahydro-1,5-benzothiazepin-8-yl)methyl)thio)essigsäure;
2-(((3-Butyl-3-ethyl-5-(4-fluorphenyl)-7-(methylthio)-1,1-dioxido-2,3,4,5-tetrahydro-1,5-benzothiazepin-8-yl) methyl)thio)essigsäure;
(S)-2-(((3-Butyl-3-ethyl-5-(4-fluorphenyl)-7-(methylthio)-1,1-dioxido-2,3,4,5-tetrahydro-1,5-benzothiazepin-8-yl)methyl)thio)essigsäure;
(R)-2-(((3-Butyl-3-ethyl-5-(4-fluorphenyl)-7-(methylthio)-1,1-dioxido-2,3,4,5-tetrahydro-1,5-benzothiazepin-8-yl)methyl)thio)essigsäure;
2- (((3,3-Dibutyl-7-(methylthio)-1,1-dioxido-5-phenyl-2,3,4,5-tetrahydro-1,2,5-benzothiadiazepin-8-yl)methyl) thio)essigsäure;
2-(((3-Butyl-3-ethyl-7-(methylthio)-1,1-dioxido-5-phenyl-2,3,4,5-tetrahydro-1,2,5-benzothiadiazepin-8-yl)methyl)thio)essigsäure;
(S)-2-(((3-Butyl-3-ethyl -7-(methylthio)-1,1-dioxido-5-phenyl-2,3,4,5-tetrahydro-1,2,5-benzothiadiazepin-8-yl) methyl)thio)essigsäure;
(R)-2-(((3-Butyl-3-ethyl-7-(methylthio)-1,1-dioxido-5-phenyl-2,3,4,5-tetrahydro-1,2,5-benzothiadiazepin-8-yl) methyl)thio)essigsäure;
2-(((3-Butyl-2-methyl-7-(methylthio)-1,1-dioxido-5-phenyl-2,3,4,5-tetrahydro-1,2,5-benzothiadiazepin-8-yl)methyl)thio)essigsäure;
(S)-2-(((3-Butyl-2-methyl-7-(methylthio)-1,1-dioxido-5-phenyl-2,3,4,5-tetrahydro-1,2,5-benzothiadiazepin-8-yl)methyl)thio)essigsäure;
(R)-2-(((3-Butyl-2-methyl-7-(methylthio)-1,1-dioxido-5-phenyl-2,3,4,5-tetrahydro-1,2,5-benzothiadiazepin-8-yl)methyl)thio)essigsäure;
2-(((3-Butyl-3-ethyl-7-methoxy-1,1-dioxido-5-phenyl-2,3,4,5-tetrahydro-1,5-benzothiazepin-8-yl)methyl)thio)essigsäure;
(S)-2-(((3-Butyl-3-ethyl-7-methoxy-1,1-dioxido-5-phenyl-2,3,4,5-tetrahydro-1,5-benzothiazepin-8-yl)methyl)thio)essigsäure;
(R)-2-(((3-Butyl-3-ethyl-7-methoxy-1,1-dioxido-5-phenyl-2,3,4,5-tetrahydro-1,5-benzothiazepin-8-yl)methyl)thio)essigsäure;
2-(((3-Butyl-3-ethyl-5-(4-fluorphenyl)-7-(methylthio)-1,1-dioxido-2,3,4,5-tetrahydro-1,5-benzothiazepin-8-yl)methyl)thio)-2-methylpropansäure;
(S)-2-(((3-Butyl-3-ethyl-5-(4-fluorphenyl)-7-(methylthio)-1,1-dioxido-2,3,4,5-tetrahydro-1,5-benzothiazepin-8-yl)methyl)thio)-2-methylpropansäure;
(R)-2-(((3-Butyl-3-ethyl-5-(4-fluorphenyl)-7-(methylthio)-1,1-dioxido-2,3,4,5-tetrahydro-1,5-benzothiazepin-8-yl)methyl)thio)-2-methylpropansäure;
2-(((3,3-Dibutyl-7-(methylthio)-1,1-dioxido-5-phenyl-2,3,4,5-tetrahydro-1,5-benzothiazepin-8-yl)methyl)thio)-2-methylpropansäure;
2-(((3-Butyl-3-methyl-7-(methylthio)-1,1-dioxido-5-phenyl-2,3,4,5-tetrahydro-1,5-benzothiazepin-8-yl)methyl)thio)-2-methylpropansäure;
(S)-2-(((3-Butyl-3-methyl-7-(methylthio)-1,1-dioxido-5-phenyl-2,3,4,5-tetrahydro-1,5-benzothiazepin-8-yl)methyl)thio)-2-methylpropansäure;
(R)-2-(((3-Butyl-3-methyl-7-(methylthio)-1,1-dioxido-5-phenyl-2,3,4,5-tetrahydro-1,5-benzothiazepin-8-yl)methyl)thio)-2-methylpropansäure;
2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)essigsäure;
(S)-2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)essigsäure;
(R)-2-(((3-Butyl-3-ethyl-2-methyl-7-(methylthio)-1,1-dioxido-5-phenyl-2,3,4,5-tetrahydro-1,2,5-benzothiadiazepin-8-yl)methyl)thio)essigsäure;
2-(((3,3-Dibutyl-2-methyl-7-(methylthio)-1,1-dioxido-5-phenyl-2,3,4,5-tetrahydro-1,2,5-benzothiadiazepin-8-yl)methyl)thio)essigsäure;
2-(((3,3-Dibutyl-2-methyl-7-(methylthio)-1,1-dioxido-5-phenyl-2,3,4,5-tetrahydro-1,2,5-benzothiadiazepin-8-yl)methyl)thio)-2-methylpropansäure;
2-(((3,3-Dibutyl-2-methyl-7-(methylthio)-1,1-dioxido-5-phenyl-2,3,4,5-tetrahydro-1,2,5-benzothiadiazepin-8-yl)methyl)thio)-2-methylpropansäure;
2-(((3-Butyl-3-ethyl-7-methoxy-1,1-dioxido-5-phenyl-2,3,4,5-tetrahydro-1,5-benzothiazepin-8-yl)methyl)thio)-2-methylpropansäure;
(S)-2-(((3-Butyl-3-ethyl-7-methoxy-1,1-dioxido-5-phenyl-2,3,4,5-tetrahydro-1,5-benzothiazepin-8-yl)methyl)thio)-2-methylpropansäure;
(R)-2-(((3-Butyl-3-ethyl-7-methoxy-1,1-dioxido-5-phenyl-2,3,4,5-tetrahydro-1,5-benzothiazepin-8-yl)methyl)thio)-2-methylpropansäure;
2-(((3-Butyl-7-methoxy-3-methyl-1,1-dioxido-5-phenyl-2,3,4,5-tetrahydro-1,5-benzothiazepin-8-yl)methyl)thio)essigsäure;
(S)-2-(((3-Butyl-7-methoxy-3-methyl-1,1-dioxido-5-phenyl-2,3,4,5-tetrahydro-1,5-benzothiazepin-8-yl)methyl)thio)essigsäure;
(R)-2-(((3-Butyl-7-methoxy-3-methyl-1,1-dioxido-5-phenyl-2,3,4,5-tetrahydro-1,5-benzothiazepin-8-yl)methyl)thio)essigsäure;
2-(((3-Butyl-5-(4-fluorphenyl)-7-methoxy-2-methyl-1,1-dioxido-2,3,4,5-tetrahydro-1,2,5-benzothiadiazepin-8-yl)methyl)thio)essigsäure;
(S)-2-(((3-Butyl-5-(4-fluorphenyl)-7-methoxy-2-methyl-1,1-dioxido-2,3,4,5-tetrahydro-1,2,5-benzothiadiazepin-8-yl)methyl)thio)essigsäure;
(R)-2-(((3-Butyl-5-(4-fluorphenyl)-7-methoxy-2-methyl-1,1-dioxido-2,3,4,5-tetrahydro-1,2,5-benzothiadiazepin-8-yl)methyl)thio)essigsäure;
2-(((3,3-Dibutyl-7-methoxy-1,1-dioxido-5-phenyl-2,3,4,5-tetrahydro-1,5-benzothiazepin-8-yl)methyl)thio)essigsäure;
2-(((3,3-Dibutyl-5-(4-fluorphenyl)-7-methoxy-1,1-dioxido-2,3,4,5-tetrahydro-1,5-benzothiazepin-8-yl)methyl)thio)essigsäure;
2-(((3-Butyl-3-ethyl-5-(4-fluorphenyl)-7-methoxy-1,1-dioxido-2,3,4,5-tetrahydro-1,5-benzothiazepin-8-yl)methyl)thio)-2-methylpropansäure;
(S)-2-(((3-Butyl-3-ethyl-5-(4-fluorphenyl)-7-methoxy-1,1-dioxido-2,3,4,5-tetrahydro-1,5-benzothiazepin-8-yl)methyl)thio)-2-methylpropansäure;
(R)-2-(((3-Butyl-3-ethyl-5-(4-fluorphenyl)-7-methoxy-1,1-dioxido-2,3,4,5-tetrahydro-1,5-benzothiazepin-8-yl)methyl)thio)-2-methylpropansäure;
2-(((3-Butyl-7-methoxy-2-methyl-1,1-dioxido-5-phenyl-2,3,4,5-tetrahydro-1,2,5-benzothiadiazepin-8-yl)methyl)thio)essigsäure;
(S)-2-(((3-Butyl-7-methoxy-2-methyl-1,1-dioxido-5-phenyl-2,3,4,5-tetrahydro-1,2,5-benzothiadiazepin-8-yl)methyl)thio)essigsäure;
(R)-2-(((3-Butyl-7-methoxy-2-methyl-1,1-dioxido-5-phenyl-2,3,4,5-tetrahydro-1,2,5-benzothiadiazepin-8-yl)methyl)thio)essigsäure;
2-(((3-Butyl-7-methoxy-2-methyl-1,1-dioxido-5-phenyl-2,3,4,5-tetrahydro-1,2,5-benzothiadiazepin-8-yl)methyl)thio)-2-methylpropansäure;
(S)-2-(((3-Butyl-7-methoxy-2-methyl-1,1-dioxido-5-phenyl-2,3,4,5-tetrahydro-1,2,5-benzothiadiazepin-8-yl)methyl)thio)-2-methylpropansäure;
(R)-2-(((3-Butyl-7-methoxy-2-methyl-1,1-dioxido-5-phenyl-2,3,4,5-tetrahydro-1,2,5-benzothiadiazepin-8-yl)methyl)thio)-2-methylpropansäure;
2-(((3-Butyl-3-ethyl-5-(4-fluorphenyl)-7-methoxy-1,1-dioxido-2,3,4,5-tetrahydro-1,5-benzothiazepin-8-yl)methyl)thio)essigsäure;
(S)-2-(((3-Butyl-3-ethyl-5-(4-fluorphenyl)-7-methoxy-1,1-dioxido-2,3,4,5-tetrahydro-1,5-benzothiazepin-8-yl)methyl)thio)essigsäure;
(R)-2-(((3-Butyl-3-ethyl-5-(4-fluorphenyl)-7-methoxy-1,1-dioxido-2,3,4,5-tetrahydro-1,5-benzothiazepin-8-yl)methyl)thio)essigsäure;
2-(((3-Butyl-7-methoxy-3-methyl-1,1-dioxido-5-phenyl-2,3,4,5-tetrahydro-1,5-benzothiazepin-8-yl)methyl)thio)-2-methylpropansäure;
(S)-2-(((3-Butyl-7-methoxy-3-methyl-1,1-dioxido-5-phenyl-2,3,4,5-tetrahydro-1,5-benzothiazepin-8-yl)methyl)thio)-2-methylpropansäure;
(R)-2-(((3-Butyl-7-methoxy-3-methyl-1,1-dioxido-5-phenyl-2,3,4,5-tetrahydro-1,5-benzothiazepin-8-yl)methyl)thio)-2-methylpropansäure;
2-(((3,3-Dibutyl-7-methoxy-1,1-dioxido-5-phenyl-2,3,4,5-tetrahydro-1,5-benzothiazepin-8-yl)methyl)thio)-2-methylpropansäure;
2-(((3-Butyl-3-ethyl-7-methoxy-1,1-dioxido-5-phenyl-2,3,4,5-tetrahydro-1,2,5-benzothiadiazepin-8-yl)methyl)thio)essigsäure;
(R)-2-(((3-Butyl-3-ethyl-7-methoxy-1,1-dioxido-5-phenyl-2,3,4,5-tetrahydro-1,2,5-benzothiadiazepin-8-yl)methyl)thio)essigsäure;
(S)-2-(((3-Butyl-3-ethyl-7-methoxy-1,1-dioxido-5-phenyl-2,3,4,5-tetrahydro-1,2,5-benzothiadiazepin-8-yl)methyl)thio)essigsäure;
2-(((3-Butyl-7-(dimethylamino)-3-ethyl-1,1-dioxido-5-phenyl-2,3,4,5-tetrahydro-1,2,5-benzothiadiazepin-8-yl)methyl)thio)essigsäure;
2-(((3-Butyl-3-ethyl-7-(methylsulfonyl)-1,1-dioxido-5-phenyl-2,3,4,5-tetrahydro-1,2,5-benzothiadiazepin-8-yl)methyl)thio)essigsäure;
(S)-2-(((3-Butyl-3-ethyl-7-(methylsulfonyl)-1,1-dioxido-5-phenyl-2,3,4,5-tetrahydro-1,2,5-benzothiadiazepin-8-yl)methyl)thio)essigsäure;
(R)-2-(((3-Butyl-3-ethyl-7-(methylsulfonyl)-1,1-dioxido-5-phenyl-2,3,4,5-tetrahydro-1,2,5-benzothiadiazepin-8-yl)methyl)thio)essigsäure;
(R)-2-(((3-Butyl-7-(dimethylamino)-1,1-dioxido-5-phenyl-2,3,4,5-tetrahydro-1,2,5-benzothiadiazepin-8-yl)methyl)thio)essigsäure;
(R)-2-(((3-Butyl-7-(methylthio)-1,1-dioxido-5-phenyl-2,3,4,5-tetrahydro-1,2,5-benzothiadiazepin-8-yl)methyl)thio)essigsäure;
(R)-2-(((3-Butyl-7-methoxy-1,1-dioxido-5-phenyl-2,3,4,5-tetrahydro-1,2,5-benzothiadiazepin-8-yl)methyl)thio)essigsäure;
2-(((3-Butyl-7-(dimethylamino)-3-methyl-1,1-dioxido-5-phenyl-2,3,4,5-tetrahydro-1,2,5-benzothiadiazepin-8-yl)methyl)thio)essigsäure;
2-(((3-Butyl-7-methoxy-3-methyl-1,1-dioxido-5-phenyl-2,3,4,5-tetrahydro-1,2,5-benzothiadiazepin-8-yl)methyl)thio)essigsäure; und
2-(((3-Butyl-3-methyl-7-(methylthio)-1,1-dioxido-5-phenyl-2,3,4,5-tetrahydro-1,2,5-benzothiadiazepin-8-yl)methyl)thio)essigsäure;

oder ein pharmazeutisch akzeptables Salz davon.

**10.** Pharmazeutische Zusammensetzung, umfassend eine therapeutisch wirksame Menge einer Verbindung nach einem der Ansprüche 1 bis 9 und einen oder mehrere pharmazeutisch akzeptable Hilfsstoffe.

**11.** Verbindung nach einem der Ansprüche 1 bis 9 zur Verwendung als Arzneimittel.

**12.** Verbindung nach einem der Ansprüche 1 bis 9 zur Verwendung bei der Behandlung oder Prävention einer Herz-Kreislauf-Erkrankung oder einer Störung des Fettsäurestoffwechsels oder einer Störung der Glukoseverwertung, einschließlich Hypercholesterinämie; Diabetes mellitus Typ 1 und Typ 2; Komplikationen des Diabetes, einschließlich Katarakt, mikro- und makrovaskulärer Erkrankungen, Retinopathie, Neuropathie, Nephropathie und verzögerter Wundheilung, Gewebeischämie, diabetischer Fuß, Arteriosklerose, Myokardinfarkt, akutes Koronarsyndrom, instabile Angina pectoris, stabile Angina pectoris, Schlaganfall, periphere arterielle Verschlusskrankheit, Kardiomyopathie, Herzinsuffizienz, Herzrhythmusstörungen und vaskuläre Restenose; diabetesbedingte Erkrankungen einschließlich Insulinresistenz (gestörte Glukosehomöostase), Hyperglykämie, Hyperinsulinämie, erhöhte Blutspiegel von Fettsäuren oder Glycerol, Adipositas, Dyslipidämie, Hyperlipidämie einschließlich Hypertriglyzeridämie, metabolisches Syndrom (Syndrom X), Atherosklerose und Hypertonie; sowie zur Erhöhung der High-Density-Lipoprotein-Spiegel.

**13.** Verbindung nach einem der Ansprüche 1 bis 9 zur Verwendung bei der Behandlung oder Prävention einer gastrointestinalen Krankheit oder Störung, einschließlich Verstopfung (einschließlich chronischer Verstopfung, funktioneller Verstopfung, chronischer idiopathischer Verstopfung (CIC), intermittierender/sporadischer Verstopfung, Verstopfung als Folge von Diabetes mellitus, Verstopfung als Folge eines Schlaganfalls, Verstopfung als Folge einer chronischen Nierenerkrankung, Verstopfung als Folge von Multipler Sklerose, Verstopfung infolge von Parkinson, Verstopfung infolge von systemischer Sklerose, medikamenteninduzierte Verstopfung, Reizdarmsyndrom mit Verstopfung (IBS-C), gemischtes Reizdarmsyndrom (IBS-M), funktionelle Verstopfung bei Kindern und opioidinduzierte Verstopfung); Morbus Crohn; primäre Gallensäuremalabsorption; Reizdarmsyndrom (IBS); entzündliche Darmerkrankung (IBD); Ileumentzündung; sowie Refluxerkrankungen und deren Komplikationen, einschließlich Barrett-Ösophagus, Gallenrefluxösophagitis und Gallenrefluxgastritis.

**14.** Verbindung nach einem der Ansprüche 1 bis 9 zur Verwendung bei der Behandlung oder Prävention einer Lebererkrankung oder -störung, einschließlich einer erblichen Stoffwechselstörung der Leber; angeborener Störungen der Gallensäuresynthese; angeborener Gallengangsanomalien; Gallengangatresie; Gallengangatresie nach Kasai-Operation; Gallengangatresie nach Lebertransplantation; neonataler Hepatitis; neonataler Cholestase; erblicher Formen der Cholestase; zerebrotendinöse Xanthomatose; Byler-Syndrom; eines primären Defekts der Gallensäuresynthese; einer progressiven familiären intrahepatischen Cholestase (PFIC), einschließlich PFIC-1, PFIC-2, PFIC-3 und nicht näher bezeichneter PFIC, PFIC nach Gallengangumleitung und PFIC nach Lebertransplantation; benigne rezidivierende intrahepatische Cholestase (BRIC), einschließlich BRIC1, BRIC2 und nicht näher spezifizierter BRIC, BRIC nach Gallengangumleitung und BRIC nach Lebertransplantation; Autoimmunhepatitis; primäre biliäre Zirrhose (PBC); Leberfibrose; nichtalkoholische Fettlebererkrankung (NAFLD); nichtalkoholischer Steatohepatitis (NASH); portaler Hypertonie; Cholestase; Down-Syndrom-Cholestase; arzneimittelinduzierte Cholestase; intrahepatische Cholestase der Schwangerschaft (Gelbsucht während der Schwangerschaft); intrahepatische Cholestase; extrahepatische Cholestase; parenteraler Ernährung assoziierte Cholestase (PNAC); Low-Phospholipid-assoziierte Cholestase; Lymphödem-Cholestase-Syndrom 1 (LCS1); primär sklerosierende Cholangitis (PSC); Immunglobulin-G4-assoziierte Cholangitis; primärer biliärer Cholangitis; Cholelithiasis (Gallensteine); Gallengangslithiasis; Choledocholithiasis; Gallenstein-Pankreatitis; Caroli-Krankheit; bösartiger Tumor der Gallengänge; bösartiger Tumor, der eine Obstruktion des Gallengangsystems verursacht; Gallengangsstrikturen; AIDS-Cholangiopathie; ischämische Cholangiopathie; Pruritus aufgrund von Cholestase oder Gelbsucht; Pankreatitis; chronische autoimmune Lebererkrankung, die zu einer progressiven Cholestase führt; Lebersteatose; alkoholischer Hepatitis; akuter Fettleber; Schwangerschaftsfettleber; arzneimittelinduzierter Hepatitis; Eisenüberladungserkrankungen; angeborenem Gallensäuresynthesedefekt Typ 1 (BAS-Defekt Typ 1); arzneimittelinduzierter Leberschädigung (DILI); Leberfibrose; angeborener Leberfibrose; Leberzirrhose; Langerhans-Zell-Histiozytose (LCH); neonatale Ichthyose mit sklerosierender Cholangitis (NISCH); erythropoetischer Protoporphyrie (EPP); idiopathischer Ductopenie im Erwachsenenalter (IAD); idiopathischer neonataler Hepatitis (INH); nicht-syndromaler Interlobulär-Galleweg-Mangel (NS PILBD); Nordamerikanisch-indianische Kinderzirrhose (NAIC); hepatische Sarkoidose; Amyloidose; nekrotisierende Enterokolitis; durch Serum-Gallensäuren verursachter Toxizitäten, einschließlich Herzrhythmusstörungen

(einschließlich Vorhofflimmern) im Zusammenhang mit einem abnormalen Serum-Gallensäureprofil, einer mit Leberzirrhose assoziierten Kardiomyopathie ("Cholecardie") und einem mit cholestatischer Lebererkrankung assoziierten Skelettmuskelschwund; viraler Hepatitis (einschließlich Hepatitis A, Hepatitis B, Hepatitis C, Hepatitis D und Hepatitis E); Leberzellkarzinom (Hepatom); Cholangiokarzinom; gallensäurebedingte Magen-Darm-Krebserkrankungen; und Cholestase, verursacht durch Tumoren und Neoplasien der Leber, der Gallenwege und der Bauchspeicheldrüse; oder zur Verwendung bei der Unterstützung einer Kortikosteroidtherapie bei Lebererkrankungen.

**15.** Verbindung nach einem der Ansprüche 1 bis 9 zur Verwendung bei der Behandlung oder Prävention von Hyperabsorptionssyndromen (einschließlich Abetalipoproteinämie, familiärer Hypobetalipoproteinämie (FHBL), Chylomikron-Retentionssyndrom (CRD) und Sitosterinämie); Hypervitaminose und Osteopetrose; Bluthochdruck; glomeruläre Hyperfiltration; polyzystische Nierenerkrankung (PKD), einschließlich autosomal-dominanter polyzystischer Nierenerkrankung (ADPKD) und autosomal-rezessiver polyzystischer Nierenerkrankung (ARPKD); sowie Juckreiz bei Nierenversagen; oder zur Verwendung beim Schutz vor Nierenschäden im Zusammenhang mit Leber- oder Stoffwechselerkrankungen.

## Revendications

**1.** Composé de la formule (I)

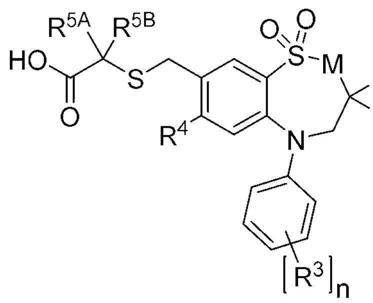

(I)

dans lequel

M est $-CH_2-$ ou $-NR^6-$;
$R^1$ est un alkyle en $C_{1-4}$ ;
$R^2$ est choisi dans le groupe constitué par l'hydrogène et l'alkyle en $C_{1-4}$ ;
$R^3$ est indépendamment choisi dans le groupe constitué par l'hydrogène, l'halogène, l'hydroxy, l'alkyle en $C_{1-4}$, l'haloalkyle en $C_{1-4}$, l'alcoxy en $C_{1-4}$, l'haloalcoxy en $C_{1-4}$, le cyano, le nitro, l'amino, le N-(alkyle en $C_{1-4}$ )amino et le N,N-di(alkyle en $C_{1-4}$ )amino ;
n est un nombre entier 1, 2 ou 3 ;
$R^4$ est choisi dans le groupe constitué par l'hydrogène, l'halogène, le cyano, l'alkyle en $C_{1-4}$, le cycloalkyle en $C_{3-6}$, l'alcoxy en $C_{1-4}$, le cycloalkyloxy en $C_{3-6}$, l'alkylthio en $C_{1-4}$, le cycloalkylthio en $C_{3-6}$, l'alkylsulfonyl en $C_{1-4}$, le cycloalkylsulfonyl en $C_{3-6}$, l'amino, le N-(alkyl en $C_{1-4}$ )amino et le N,N-di(alkyl en $C_{1-4}$ )amino ;
$R^{5A}$ et $R^{5B}$ sont chacun indépendamment choisis dans le groupe constitué par l'hydrogène, l'halogène, l'hydroxy, l'alkyle en $C_{1-4}$ et l'alcoxy en $C_{1-4}$; ou $R^{5A}$ et $R^{5B}$ forment, avec l'atome de carbone auquel ils sont attachés, un cycle carbocyclique saturé à 3 à 5 chaînons ;
$R^6$ est choisi dans le groupe constitué par l'hydrogène et l'alkyle en $C_{1-4}$ ;

ou un sel pharmaceutiquement acceptable de ceux-ci.

**2.** Composé selon la revendication 1, dans lequel $R^1$ est un n-butyle.

**3.** Composé selon la revendication 1 ou 2, dans lequel $R^2$ est un hydrogène.

**4.** Composé selon la revendication 1 ou 2, dans lequel $R^2$ est un méthyle, un éthyle ou un *n*-butyle.

**5.** Composé selon l'une quelconque des revendications 1 à 4, dans lequel $R^3$ est indépendamment choisi dans le groupe constitué par l'hydrogène, le fluoro, le chloro, le bromo, l'hydroxy, le méthoxy, l'amino, le méthylamino et le

diméthylamino.

6. Composé selon l'une quelconque des revendications 1 à 5, dans lequel $R^4$ est choisi dans le groupe constitué par le fluoro, le chloro, le bromo, le méthyl, l'éthyl, le cyclopropyl, le méthoxy, l'éthoxy, le méthylthio, l'éthylthio, le méthylsulfonyl, l'amino, le méthylamino et le diméthylamino.

7. Composé selon l'une quelconque des revendications 1 à 6, dans lequel $R^{5A}$ et $R^{5B}$ sont chacun indépendamment un hydrogène ou un alkyle en $C_{1-4}$, ou forment avec l'atome de carbone auquel ils sont attachés un cycle cyclopropyle.

8. Composé selon l'une quelconque des revendications 1 à 7, dans lequel $R^6$ est un hydrogène ou un méthyle.

9. Composé selon la revendication 1, choisi dans le groupe constitué de :

acide 2-(((3-butyl-3-éthyl-7-(méthylthio)-1,1-dioxido-5-phényl-2,3,4,5-tétrahydro-1,5-benzothiazépine-8-yl)méthyl)thio)acétique ;
acide (S)-2-(((3-butyl-3-éthyl-7-(méthylthio)-1,1-dioxido-5-phényl-2,3,4,5-tétrahydro-1,5-benzothiazépine-8-yl)méthyl)thio)acétique ;
acide (R)-2-(((3-butyl-3-éthyl-7-(méthylthio)-1,1-dioxido-5-phényl-2,3,4,5-tétrahydro-1,5-benzothiazépine-8-yl)méthyl)thio)acétique ;
acide 2-(((3,3-dibutyl-7-(méthylthio)-1,1-dioxido-5-phényl-2,3,4,5-tétrahydro-1,5-benzothiazépine-8-yl)méthyl)thio)acétique ;
acide 2-(((3-butyl-3-méthyl-7-(méthylthio)-1,1-dioxido-5-phényl-2,3,4,5-tétrahydro-1,5-benzothiazépine-8-yl)méthyl)thio)acétique ;
acide (S)-2-(((3-butyl-3-méthyl-7-(méthylthio)-1,1-dioxido-5-phényl-2,3,4,5-tétrahydro-1,5-benzothiazépine-8-yl)méthyl)thio)acétique ;
acide (R)-2-(((3-butyl-3-méthyl-7-(méthylthio)-1,1-dioxido-5-phényl-2,3,4,5-tétrahydro-1,5-benzothiazépine-8-yl)méthyl)thio)acétique ;
acide 2-(((3-butyl-3-éthyl-5-(4-fluorophényl)-7-(méthylthio)-1,1-dioxido-2,3,4,5-tétrahydro-1,5-benzothiazépine-8-yl)méthyl)thio)acétique ;
acide (S)-2-(((3-butyl-3-éthyl-5-(4-fluorophényl)-7-(méthylthio)-1,1-dioxido-2,3,4,5-tétrahydro-1,5-benzothiazépine-8-yl)méthyl)thio)acétique ;
acide (R)-2-(((3-butyl-3-éthyl-5-(4-fluorophényl)-7-(méthylthio)-1,1-dioxido-2,3,4,5-tétrahydro-1,5-benzothiazépine-8-yl)méthyl)thio)acétique ;
acide 2-(((3,3-dibutyl-7-(méthylthio)-1,1-dioxido-5-phényl-2,3,4,5-tétrahydro-1,2,5-benzothiazépine-8-yl)méthyl)thio)acétique ;
acide 2-(((3-butyl-3-éthyl-7-(méthylthio)-1,1-dioxido-5-phényl-2,3,4,5-tétrahydro-1,2,5-benzothiadiazépine-8-yl)méthyl)thio)acétique ;
acide (S)-2-(((3-butyl-3-éthyl-7-(méthylthio)-1,1-dioxido-5-phényl-2,3,4,5-tétrahydro-1,2,5-benzothiadiazépine-8-yl)méthyl)thio)acétique ;
acide (R)-2-(((3-butyl-3-éthyl-7-(méthylthio)-1,1-dioxido-5-phényl-2,3,4,5-tétrahydro-1,2,5-benzothiadiazépine-8-yl)méthyl)thio)acétique ;
acide 2-(((3-butyl-2-méthyl-7-(méthylthio)-1,1-dioxido-5-phényl-2,3,4,5-tétrahydro-1,2,5-benzothiadiazépine-8-yl)méthyl)thio)acétique ;
acide (S)-2-(((3-butyl-2-méthyl-7-(méthylthio)-1,1-dioxido-5-phényl-2,3,4,5-tétrahydro-1,2,5-benzothiadiazépine-8-yl)méthyl)thio)acétique ;
acide (R)-2-(((3-butyl-2-méthyl-7-(méthylthio)-1,1-dioxido-5-phényl-2,3,4,5-tétrahydro-1,2,5-benzothiadiazépine-8-yl)méthyl)thio)acétique ;
acide 2-(((3-Butyl-3-éthyl-7-méthoxy-1,1-dioxido-5-phényl-2,3,4,5-tétrahydro-1,5-benzothiazépine-8-yl)méthyl)thio)acétique ;
acide (S)-2-(((3-butyl-3-éthyl-7-méthoxy-1,1-dioxido-5-phényl-2,3,4,5-tétrahydro-1,5-benzothiazépine-8-yl)méthyl)thio)acétique ;
acide (R)-2-(((3-butyl-3-éthyl-7-méthoxy-1,1-dioxido-5-phényl-2,3,4,5-tétrahydro-1,5-benzothiazépine-8-yl)méthyl)thio)acétique ;
acide 2-(((3-Butyl-3-éthyl-5-(4-fluorophényl)-7-(méthylthio)-1,1-dioxido-2,3,4,5-tétrahydro-1,5-benzothiazépine-8-yl)méthyl)thio)-2-méthylpropanoïque ;
acide (S)-2-(((3-butyl-3-éthyl-5-(4-fluorophényl)-7-(méthylthio)-1,1-dioxido-2,3,4,5-tétrahydro-1,5-benzothiazépine-8-yl)méthyl)thio)-2-méthylpropanoïque ;
acide (R)-2-(((3-butyl-3-éthyl-5-(4-fluorophényl)-7-(méthylthio)-1,1-dioxido-2,3,4,5-tétrahydro-1,5-benzothiazépine-8-yl)méthyl)thio)-2-méthylpropanoïque ;

acide 2-(((3,3-Dibutyl-7-(méthylthio)-1,1-dioxido-5-phényl-2,3,4,5-tétrahydro-1,5-benzothiazépine-8-yl)méthyl)thio)-2-méthylpropanoïque ;

acide 2-(((3-Butyl-3-méthyl-7-(méthylthio)-1,1-dioxido-5-phényl-2,3,4,5-tétrahydro-1,5-benzothiazépine-8-yl)méthyl)thio)-2-méthylpropanoïque ;

acide (S)-2-(((3-butyl-3-méthyl-7-(méthylthio)-1,1-dioxido-5-phényl-2,3,4,5-tétrahydro-1,5-benzothiazépine-8-yl)méthyl)thio)-2-méthylpropanoïque ;

acide (R)-2-(((3-butyl-3-méthyl-7-(méthylthio)-1,1-dioxido-5-phényl-2,3,4,5-tétrahydro-1,5-benzothiazépine-8-yl)méthyl)thio)-2-méthylpropanoïque ;

acide 2-(((3-Butyl-3-éthyl-2-méthyl-7-(méthylthio)-1,1-dioxido-5-phényl-2,3,4,5-tétrahydro-1,2,5-benzothiadiazépine-8-yl)méthyl)thio)acétique ;

acide (S)-2-(((3-butyl-3-éthyl-2-méthyl-7-(méthylthio)-1,1-dioxido-5-phényl-2,3,4,5-tétrahydro-1,2,5-benzothiadiazépine-8-yl)méthyl)thio)acétique ;

acide (R)-2-(((3-butyl-3-éthyl-2-méthyl-7-(méthylthio)-1,1-dioxido-5-phényl-2,3,4,5-tétrahydro-1,2,5-benzothiadiazépine-8-yl)méthyl)thio)acétique ;

acide 2-(((3,3-dibutyl-2-méthyl-7-(méthylthio)-1,1-dioxido-5-phényl-2,3,4,5-tétrahydro-1,2,5-benzothiadiazépine-8-yl)méthyl)thio)acétique ;

acide 2-(((3,3-dibutyl-2-méthyl-7-(méthylthio)-1,1-dioxido-5-phényl-2,3,4,5-tétrahydro-1,2,5-benzothiadiazépine-8-yl)méthyl)thio)-2-méthylpropanoïque ;

acide 2-(((3,3-dibutyl-2-méthyl-7-(méthylthio)-1,1-dioxido-5-phényl-2,3,4,5-tétrahydro-1,2,5-benzothiadiazépine-8-yl)méthyl)thio)-2-méthylpropanoïque ;

acide 2-(((3-butyl-3-éthyl-7-méthoxy-1,1-dioxido-5-phényl-2,3,4,5-tétrahydro-1,5-benzothiazépine-8-yl)méthyl)thio)-2-méthylpropanoïque ;

acide (S)-2-(((3-butyl-3-éthyl-7-méthoxy-1,1-dioxido-5-phényl-2,3,4,5-tétrahydro-1,5-benzothiazépine-8-yl)méthyl)thio)-2-méthylpropanoïque ;

acide (R)-2-(((3-butyl-3-éthyl-7-méthoxy-1,1-dioxido-5-phényl-2,3,4,5-tétrahydro-1,5-benzothiazépine-8-yl)méthyl)thio)-2-méthylpropanoïque ;

acide 2-(((3-butyl-7-méthoxy-3-méthyl-1,1-dioxido-5-phényl-2,3,4,5-tétrahydro-1,5-benzothiazépine-8-yl)méthyl)thio)acétique ;

acide (S)-2-(((3-butyl-7-méthoxy-3-méthyl-1,1-dioxido-5-phényl-2,3,4,5-tétrahydro-1,5-benzothiazépine-8-yl)méthyl)thio)acétique ;

acide (R)-2-(((3-butyl-7-méthoxy-3-méthyl-1,1-dioxido-5-phényl-2,3,4,5-tétrahydro-1,5-benzothiazépine-8-yl)méthyl)thio)acétique ;

acide 2-(((3-butyl-5-(4-fluorophényl)-7-méthoxy-2-méthyl-1,1-dioxido-2,3,4,5-tétrahydro-1,2,5-benzothiadiazépine-8-yl)méthyl)thio)acétique ;

acide (S)-2-(((3-butyl-5-(4-fluorophényl)-7-méthoxy-2-méthyl-1,1-dioxido-2,3,4,5-tétrahydro-1,2,5-benzothiadiazépine-8-yl)méthyl)thio)acétique ;

acide (R)-2-(((3-butyl-5-(4-fluorophényl)-7-méthoxy-2-méthyl-1,1-dioxido-2,3,4,5-tétrahydro-1,2,5-benzothiadiazépine-8-yl)méthyl)thio)acétique ;

acide 2-(((3,3-dibutyl-7-méthoxy-1,1-dioxido-5-phényl-2,3,4,5-tétrahydro-1,5-benzothiazépine-8-yl)méthyl)thio)acétique ;

acide 2-(((3,3-dibutyl-5-(4-fluorophényl)-7-méthoxy-1,1-dioxido-2,3,4,5-tétrahydro-1,5-benzothiazépine-8-yl)méthyl)thio)acétique ;

acide 2-(((3-butyl-3-éthyl-5-(4-fluorophényl)-7-méthoxy-1,1-dioxido-2,3,4,5-tétrahydro-1,5-benzothiazépine-8-yl)méthyl)thio)-2-méthylpropanoïque ;

acide (S)-2-(((3-butyl-3-éthyl-5-(4-fluorophényl)-7-méthoxy-1,1-dioxido-2,3,4,5-tétrahydro-1,5-benzothiazépine-8-yl)méthyl)thio)-2-méthylpropanoïque ;

acide (R)-2-(((3-butyl-3-éthyl-5-(4-fluorophényl)-7-méthoxy-1,1-dioxido-2,3,4,5-tétrahydro-1,5-benzothiazépine-8-yl)méthyl)thio)-2-méthylpropanoïque ;

acide 2-(((3-butyl-7-méthoxy-2-méthyl-1,1-dioxido-5-phényl-2,3,4,5-tétrahydro-1,2,5-benzothiadiazépine-8-yl)méthyl)thio)acétique ;

acide (S)-2-(((3-butyl-7-méthoxy-2-méthyl-1,1-dioxido-5-phényl-2,3,4,5-tétrahydro-1,2,5-benzothiadiazépine-8-yl)méthyl)thio)acétique ;

acide (R)-2-(((3-butyl-7-méthoxy-2-méthyl-1,1-dioxido-5-phényl-2,3,4,5-tétrahydro-1,2,5-benzothiadiazépine-8-yl)méthyl)thio)acétique ;

acide 2-(((3-butyl-7-méthoxy-2-méthyl-1,1-dioxido-5-phényl-2,3,4,5-tétrahydro-1,2,5-benzothiadiazépine-8-yl)méthyl)thio)-2-méthylpropanoïque ;

acide (S)-2-(((3-butyl-7-méthoxy-2-méthyl-1,1-dioxido-5-phényl-2,3,4,5-tétrahydro-1,2,5-benzothiadiazépine-8-yl)méthyl)thio)-2-méthylpropanoïque ;
acide (R)-2-(((3-butyl-7-méthoxy-2-méthyl-1,1-dioxido-5-phényl-2,3,4,5-tétrahydro-1,2,5-benzothiadiazépine-8-yl)méthyl)thio)-2-méthylpropanoïque ;
acide 2-(((3-butyl-3-éthyl-5-(4-fluorophényl)-7-méthoxy-1,1-dioxido-2,3,4,5-tétrahydro-1,5-benzothiazépine-8-yl)méthyl)thio)acétique ;
acide (S)-2-(((3-butyl-3-éthyl-5-(4-fluorophényl)-7-méthoxy-1,1-dioxido-2,3,4,5-tétrahydro-1,5-benzothiazépine-8-yl)méthyl)thio)acétique ;
acide (R)-2-(((3-butyl-3-éthyl-5-(4-fluorophényl)-7-méthoxy-1,1-dioxido-2,3,4,5-tétrahydro-1,5-benzothiazépine-8-yl)méthyl)thio)acétique ;
acide 2-(((3-butyl-7-méthoxy-3-méthyl-1,1-dioxido-5-phényl-2,3,4,5-tétrahydro-1,5-benzothiazépine-8-yl)méthyl)thio)-2-méthylpropanoïque ;
acide (S)-2-(((3-butyl-7-méthoxy-3-méthyl-1,1-dioxido-5-phényl-2,3,4,5-tétrahydro-1,5-benzothiazépine-8-yl)méthyl)thio)-2-méthylpropanoïque ;
acide (R)-2-(((3-butyl-7-méthoxy-3-méthyl-1,1-dioxido-5-phényl-2,3,4,5-tétrahydro-1,5-benzothiazépine-8-yl)méthyl)thio)-2-méthylpropanoïque ;
acide 2-(((3,3-dibutyl-7-méthoxy-1,1-dioxido-5-phényl-2,3,4,5-tétrahydro-1,5-benzothiazépine-8-yl)méthyl)thio)-2-méthylpropanoïque ;
acide 2-(((3-butyl-3-éthyl-7-méthoxy-1,1-dioxido-5-phényl-2,3,4,5-tétrahydro-1,2,5-benzothiadiazépine-8-yl)méthyl)thio)acétique ;
acide (R)-2-(((3-butyl-3-éthyl-7-méthoxy-1,1-dioxido-5-phényl-2,3,4,5-tétrahydro-1,2,5-benzothiadiazépine-8-yl)méthyl)thio)acétique ;
acide (S)-2-(((3-butyl-3-éthyl-7-méthoxy-1,1-dioxido-5-phényl-2,3,4,5-tétrahydro-1,2,5-benzothiadiazépine-8-yl)méthyl)thio)acétique ;
acide 2-(((3-butyl-7-(diméthylamino)-3-éthyl-1,1-dioxido-5-phényl-2,3,4,5-tétrahydro-1,2,5-benzothiadiazépine-8-yl)méthyl)thio)acétique ;
acide 2-(((3-butyl-3-éthyl-7-(méthylsulfonyl)-1,1-dioxido-5-phényl-2,3,4,5-tétrahydro-1,2,5-benzothiadiazépine-8-yl)méthyl)thio)acétique ;
acide (S)-2-(((3-Butyl-3-éthyl-7-(méthylsulfonyl)-1,1-dioxido-5-phényl-2,3,4,5-tétrahydro-1,2,5-benzothiadiazépine-8-yl)méthyl)thio)acétique ;
acide (R)-2-(((3-butyl-3-éthyl-7-(méthylsulfonyl)-1,1-dioxido-5-phényl-2,3,4,5-tétrahydro-1,2,5-benzothiadiazépine-8-yl)méthyl)thio)acétique ;
acide (R)-2-(((3-butyl-7-(diméthylamino)-1,1-dioxido-5-phényl-2,3,4,5-tétrahydro-1,2,5-benzothiadiazépine-8-yl)méthyl)thio)acétique ;
acide (R)-2-(((3-butyl-7-(méthylthio)-1,1-dioxido-5-phényl-2,3,4,5-tétrahydro-1,2,5-benzothiadiazépine-8-yl)méthyl)thio)acétique ;
acide (R)-2-(((3-butyl-7-méthoxy-1,1-dioxido-5-phényl-2,3,4,5-tétrahydro-1,2,5-benzothiadiazépine-8-yl)méthyl)thio)acétique ;
acide 2-(((3-Butyl-7-(diméthylamino)-3-méthyl-1,1-dioxido-5-phényl-2,3,4,5-tétrahydro-1,2,5-benzothiadiazépine-8-yl)méthyl)thio)acétique ;
acide 2-(((3-butyl-7-méthoxy-3-méthyl-1,1-dioxido-5-phényl-2,3,4,5-tétrahydro-1,2,5-benzothiadiazépine-8-yl)méthyl)thio)acétique ; et
acide 2-(((3-Butyl-3-méthyl-7-(méthylthio)-1,1-dioxido-5-phényl-2,3,4,5-tétrahydro-1,2,5-benzothiadiazépine-8-yl)méthyl)thio)acétique ;

ou un sel pharmaceutiquement acceptable de ceux-ci.

**10.** Composition pharmaceutique comprenant une quantité thérapeutiquement efficace d'un composé selon l'une quelconque des revendications 1 à 9, et un ou plusieurs excipients pharmaceutiquement acceptables.

**11.** Composé selon l'une quelconque des revendications 1 à 9, pour une utilisation comme médicament.

**12.** Composé selon l'une quelconque des revendications 1 à 9, pour une utilisation dans le traitement ou la prévention d'une maladie cardiovasculaire ou d'un trouble du métabolisme des acides gras ou d'un trouble de l'utilisation du glucose, y compris l'hypercholestérolémie, le diabète sucré de type 1 et de type 2 ; les complications du diabète, y compris la cataracte, les maladies micro- et macrovasculaires, la rétinopathie, la neuropathie, la néphropathie et le retard de cicatrisation, l'ischémie tissulaire, le pied diabétique, l'artériosclérose, l'infarctus du myocarde, le syndrome coronarien aigu, l'angine de poitrine instable, l'angine de poitrine stable, l'accident vasculaire cérébral, l'artériopathie oblitérante périphérique, la cardiomyopathie, l'insuffisance cardiaque, les troubles du rythme cardiaque et la

resténose vasculaire ; les maladies liées au diabète, notamment la résistance à l'insuline (altération de l'homéostasie du glucose), l'hyperglycémie, l'hyperinsulinémie, les taux sanguins élevés d'acides gras ou de glycérol, l'obésité, la dyslipidémie, l'hyperlipidémie, y compris l'hypertriglycéridémie, le syndrome métabolique (syndrome X), l'athérosclérose et l'hypertension ; et l'augmentation des taux de lipoprotéines de haute densité.

**13.** Composé selon l'une quelconque des revendications 1 à 9, pour utilisation dans le traitement ou la prévention d'une maladie ou d'un trouble gastro-intestinal, y compris la constipation (y compris la constipation chronique, la constipation fonctionnelle, la constipation idiopathique chronique (CIC), la constipation intermittente/sporadique, la constipation secondaire au diabète sucré, la constipation secondaire à l'accident vasculaire cérébral, la constipation secondaire à une maladie rénale chronique, la constipation secondaire à la sclérose en plaques, la constipation secondaire à la maladie de Parkinson, la constipation secondaire à la sclérose systémique, la constipation médicamenteuse, le syndrome du côlon irritable avec constipation (IBS-C), le syndrome du côlon irritable mixte (IBS-M), la constipation fonctionnelle pédiatrique et la constipation induite par les opioïdes) ; la maladie de Crohn, la malabsorption primaire des acides biliaires, le syndrome du côlon irritable, les maladies inflammatoires de l'intestin, l'inflammation iléale et la maladie de reflux et ses complications, y compris l'œsophage de Barrett, l'œsophagite par reflux biliaire et la gastrite par reflux biliaire.

**14.** Composé selon l'une quelconque des revendications 1 à 9, pour une utilisation dans le traitement ou la prévention d'une maladie ou d'un trouble du foie, y compris un trouble métabolique héréditaire du foie ; les erreurs innées de la synthèse des acides biliaires ; les anomalies congénitales des voies biliaires ; l'atrésie des voies biliaires ; l'atrésie des voies biliaires post-Kasai ; l'atrésie des voies biliaires post-greffe de foie ; l'hépatite néonatale ; la cholestase néonatale ; les formes héréditaires de la cholestase ; la xanthomatose cérébrotendineuse ; une anomalie secondaire de la synthèse des acides biliaires ; le syndrome de Zellweger ; la maladie hépatique associée à la mucoviscidose ; le déficit en alpha1-antitrypsine ; le syndrome d'Alagilles (ALGS) ; le syndrome de Byler ; l'anomalie primaire de la synthèse des acides biliaires ; la cholestase intrahépatique familiale progressive (CIFP), y compris CIFP-1, CIFP-2, CIFP-3 et CIFP non spécifiée, la CIFP post-dérivation biliaire et la CIFP post-greffe hépatique ; la cholestase intrahépatique récurrente bénigne (BRIC), y compris BRIC1, BRIC2 et BRIC non spécifiée, la BRIC après dérivation biliaire et BRIC post-greffe hépatique ; l'hépatite auto-immune ; la cirrhose biliaire primitive (CBP) ; la fibrose hépatique ; la stéatose hépatique non alcoolique (NAFLD) ; la stéatohépatite non alcoolique (NASH) ; l'hypertension portale ; la cholestase ; la cholestase due au syndrome de Down ; la cholestase induite par des médicaments ; la cholestase intrahépatique de la grossesse (jaunisse pendant la grossesse) ; la cholestase intrahépatique ; la cholestase extrahépatique ; la cholestase associée à la nutrition parentérale (PNAC) ; la cholestase associée à une faible teneur en phospholipides ; le syndrome de cholestase par lymphoedème 1 (LCS1) ; la cholangite sclérosante primitive (CSP) ; la cholangite associée à l'immunoglobuline G4 ; la cholangite biliaire primitive ; la cholélithiase (calculs biliaires) ; la lithiase biliaire ; la cholédocholithiase ; la pancréatite biliaire ; la maladie de Caroli ; la tumeur maligne des voies biliaires ; la tumeur maligne entraînant une obstruction de l'arbre biliaire ; la sténoses biliaires ; la cholangiopathie du SIDA ; la cholangiopathie ischémique ; le prurit dû à la cholestase ou à la jaunisse ; la pancréatite ; la maladie hépatique auto-immune chronique entraînant une cholestase progressive ; la stéatose hépatique ; l'hépatite alcoolique ; la stéatose hépatique aiguë ; la stéatose hépatique de la grossesse ; l'hépatite médicamenteuse ; les troubles de la surcharge en fer ; l'anomalie congénitale de la synthèse des acides biliaires de type 1 (anomalie de type 1) ; les lésions hépatiques médicamenteuses ; la fibrose hépatique ; la fibrose hépatique congénitale ; la cirrhose hépatique ; l'Histiocytose à cellules de Langerhans (LCH) ; la cholangite sclérosante de l'ichtyose néonatale (NISCH) ; la protoporphyrie érythropoïétique (EPP) ; la ductopénie idiopathique à l'âge adulte (IAD) ; l'hépatite néonatale idiopathique (INH) ; la paucité non syndromique des voies biliaires interlobulaires (NS PILBD) ; la cirrhose infantile des Indiens d'Amérique du Nord (NAIC) ; la sarcoïdose hépatique ; l'amyloïdose ; l'entérocolite nécrosante ; les toxicités causées par les acides biliaires sériques, y compris les troubles du rythme cardiaque (y compris la fibrillation auriculaire) en cas de profil anormal des acides biliaires sériques, la cardiomyopathie associée à la cirrhose du foie ("cholécardie") et l'atrophie des muscles squelettiques associée à la maladie cholestatique du foie ; la maladie polykystique du foie ; l'hépatite virale (y compris l'hépatite A, l'hépatite B, l'hépatite C, l'hépatite D et l'hépatite E) ; le carcinome hépatocellulaire (hépatome) ; le cholangiocarcinome ; les cancers gastro-intestinaux liés aux acides biliaires ; et la cholestase causée par des tumeurs et des néoplasmes du foie, des voies biliaires et du pancréas ; ou pour l'utilisation dans l'amélioration de la thérapie corticostéroïde dans les maladies du foie.

**15.** Composé selon l'une quelconque des revendications 1 à 9, pour une utilisation dans le traitement ou la prévention des syndromes d'hyperabsorption (y compris l'abétalipoprotéinémie, l'hypobétalipoprotéinémie familiale (FHBL), la maladie de rétention des chylomicrons (CRD) et la sitostérolémie) ; l'hypervitaminose et l'ostéopétrose ; l'hypertension artérielle ; l'hyperfiltration glomérulaire ; la polykystose rénale, y compris la polykystose rénale autosomique

dominante (ADPKD) et la polykystose récessive autosomique (ARPKD) ; et le prurit d’insuffisance rénale ; ou pour une utilisation dans la protection contre les lésions rénales associées à une maladie hépatique ou métabolique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9316055 A
- WO 9418183 A
- WO 9418184 A
- WO 9605188 A
- WO 9608484 A
- WO 9616051 A
- WO 9733882 A
- WO 9803818 A
- WO 9807449 A
- WO 9840375 A
- WO 9935135 A
- WO 9964409 A
- WO 9964410 A
- WO 0047568 A
- WO 0061568 A
- WO 0038725 A
- WO 0038726 A
- WO 0038727 A
- WO 0038728 A
- WO 0038729 A
- WO 0166533 A
- WO 0168096 A
- WO 0232428 A
- WO 0250051 A
- WO 03020710 A
- WO 03022286 A
- WO 03022825 A
- WO 03022830 A
- WO 03061663 A
- WO 03091232 A
- WO 03106482 A
- WO 2004006899 A
- WO 2004076430 A
- WO 2007009655 A
- WO 2007009656 A
- WO 2011137135 A
- WO 2019234077 A
- WO 2020161216 A
- WO 2020161217 A
- WO 2021110883 A
- WO 2021110884 A
- WO 2021110885 A
- WO 2021110886 A
- WO 2021110887 A
- WO 2022029101 A
- DE 19825804
- EP 864582 A
- EP 489423 A
- EP 549967 A
- EP 573848 A
- EP 624593 A
- EP 624594 A
- EP 624595 A
- EP 624596 A
- EP 0864582 A
- EP 1173205 A
- EP 1535913 A
- EP 3210977 A
- US 9295677 B
- US 20180140219
- US 2016146715
- US 20050215882
- US 9872844 B
- WO 2017138877 A
- WO 2017138878 A
- WO 2019032026 A
- WO 2019032027 A


### Non-patent literature cited in the description


- **KOSTERS** ; **KARPEN**. *Xenobiotica*, 2008, vol. 38, 1043-1071
- **CHIANG**. *J. Lipid Res.*, 2009, vol. 50, 1955-1966
- **DAWSON**. *Handb. Exp. Pharmacol.*, 2011, vol. 201, 169-203
- **DONG et al.** *Mol. Pharm*, 2013, vol. 10, 1008-1019
- **VAZ et al.** *Hepatology*, 2015, vol. 61, 260-267
- **KARPEN** ; **DAWSON**. *Hepatology*, 2015, vol. 61, 24-27
- **LIU et al.** *Scientific Reports*, 2017, vol. 7 (9214), 1-7
- **DANESE et al.** *PLoS One*, 2017, vol. 12 (6), e0179200
- **KOOISTRA et al.** KLIFS: A structural kinase-ligand interaction database. *Nucleic Acids Res.*, 2016, vol. 44 (D1), D365-D371
- **FOLMER et al.** *Hepatology*, 2009, vol. 50 (5), 1597-1605
- **HSU et al.** *Hepatol Res.*, 2009, vol. 39 (6), 625-631

- **ALVAREZ et al.** *Hum Mol Genet.*, 2004, vol. 13 (20), 2451-2460
- **DAVIT-SPRAUL et al.** *Hepatology*, 2010, vol. 51 (5), 1645-1655
- **VITALE et al.** *J Gastroenterol.*, 2018, vol. 53 (8), 945-958
- **KLOMP et al.** *Hepatology*, 2004, vol. 40 (1), 27-38
- **ZARENEZHAD et al.** *Hepatitis Monthly*, 2017, vol. 17 (2), e43500
- **DIXON et al.** *Scientific Reports*, 2017, vol. 7, 11823
- **PAINTER et al.** *Eur J Hum Genet.*, 2005, vol. 13 (4), 435-439
- **DENG et al.** *World J Gastroenterol.*, 2012, vol. 18 (44), 6504-6509
- **GIOVANNONI et al.** *PLoS One*, 2015, vol. 10 (12), e0145021
- **LI et al.** *Hepatology International*, 2017, vol. 11 (1), S180
- **TOGAWA et al.** *Journal of Pediatric Gastroenterology and Nutrition*, 2018, vol. 67 (1), S363
- **MILOH et al.** *Gastroenterology*, 2006, vol. 130 (4), A759-A760
- **DRÖGE et al.** *Zeitschrift fur Gastroenterologie*, 2015, vol. 53 (12)
- **MIZUOCHI et al.** *Clin Chim Acta*, 2012, vol. 413 (15-16), 1301-1304
- **LIU et al.** *Hepatology International*, 2009, vol. 3 (1), 184-185
- **MCKAY et al.** *Version 2. F1000Res*, 2013, vol. 2, 32
- **HASEGAWA et al.** *Orphanet J Rare Dis.*, 2014, vol. 9, 89
- **STONE et al.** *J Biol Chem.*, 2012, vol. 287 (49), 41139-51
- **KANG et al.** *J Pathol Transl Med*, 16 May 2019
- **SHARMA et al.** *BMC Gastroenterol*, 2018, vol. 18 (1), 107
- **UEGAKI et al.** *Intern Med.*, 2008, vol. 47 (7), 599-602
- **GOLDSCHMIDT et al.** *Hepatol Res.*, 2016, vol. 46 (4), 306-311
- **LIU et al.** *J Pediatr Gastroenterol Nutr.*, 2010, vol. 50 (2), 179-183
- **JUNG et al.** *J Pediatr Gastroenterol Nutr.*, 2007, vol. 44 (4), 453-458
- **BOUNFORD**. Dissertation Abstracts International. University of Birmingham, 2016, vol. 75
- **STOLZ et al.** *Aliment Pharmacol Ther.*, 2019, vol. 49 (9), 1195-1204
- **IVASHKIN et al.** *Hepatology International*, 2016, vol. 10 (1), S461
- **BLACKMORE et al.** *J Clin Exp Hepatol.*, 2013, vol. 3 (2), 159-161
- **MATTE et al.** *J Pediatr Gastroenterol Nutr.*, 2010, vol. 51 (4), 488-493
- **SQUIRES et al.** *J Pediatr Gastroenterol Nutr.*, 2017, vol. 64 (3), 425-430
- **HAYSHI et al.** *EBioMedicine.*, 2018, vol. 27, 187-199
- **NAGASAKA et al.** *J Pediatr Gastroenterol Nutr.*, 2007, vol. 45 (1), 96-105
- **WANG et al.** *PLoS One*, 2016, vol. 11 (4), e0153114
- **NARCHI et al.** *Saudi J Gastroenterol.*, 2017, vol. 23 (5), 303-305
- **ALASHKAR et al.** Meeting Info.: 57th Annual Meeting of the American-Society-of-Hematology. *Blood*, 05 December 2015, vol. 126 (23)
- **FERREIRA et al.** Meeting Info: IPTA 7th Congress on Pediatric Transplantation. *Pediatric Transplantation*, 13 July 2013, vol. 17 (1), 99
- **PAULI-MAGNUS et al.** *J Hepatol.*, 2005, vol. 43 (2), 342-357
- **JERICHO et al.** *Journal of Pediatric Gastroenterology and Nutrition*, 2015, vol. 60 (3), 368-374
- **VAN DER WOERD et al.** *PLoS One*, 2013, vol. 8 (11), e80553
- **COPELAND et al.** *J Gastroenterol Hepatol.*, 2013, vol. 28 (3), 560-564
- **DRÖGE et al.** *J Hepatol.*, 2017, vol. 67 (6), 1253-1264
- **CHEN et al.** *Journal of Pediatrics*, 2002, vol. 140 (1), 119-124
- **JIRSA et al.** *Hepatol Res.*, 2004, vol. 30 (1), 1-3
- **VAN DER WOERD et al.** *Hepatology*, 2015, vol. 61 (4), 1382-1391
- **NOE et al.** *J Hepatol.*, 2005, vol. 43 (3), 536-543
- **LAM et al.** *Am J Physiol Cell Physiol.*, 2007, vol. 293 (5), C1709-16
- **STINDT et al.** *Liver Int.*, 2013, vol. 33 (10), 1527-1735
- **GAO et al.** *Shandong Yiyao*, 2012, vol. 52 (10), 14-16
- **STRAUTNIEKS et al.** *Gastroenterology.*, 2008, vol. 134 (4), 1203-1214
- **KAGAWA et al.** *Am J Physiol Gastrointest Liver Physiol*, 2008, vol. 294 (1), G58-67
- **BYRNE et al.** *Hepatology.*, 2009, vol. 49 (2), 553-567
- **CHEN et al.** *J Pediatr.*, 2008, vol. 153 (6), 825-832
- **DRÖGE et al.** *Sci Rep*, 2016, vol. 6, 24827
- **LANG et al.** *Pharmacogenet Genomics.*, 2007, vol. 17 (1), 47-60
- **ELLINGER et al.** *World J Gastroenterol*, 2017, vol. 23 (29), 5295-5303
- **KNISELY et al.** *Hepatology.*, 2006, vol. 44 (2), 478-86
- **ELLIS et al.** *Hepatology.*, 2018, vol. 67 (4), 1531-1545

- **LAM et al.** *J Hepatol.*, 2006, vol. 44 (1), 240-242
- **VARMA et al.** *Hepatology*, 2015, vol. 62 (1), 198-206
- **TREEPONGKARUNA et al.** *World J Gastroenterol.*, 2009, vol. 15 (34), 4339-4342
- **HAYASHI et al.** *Hepatol Res.*, 2016, vol. 46 (2), 192-200
- **GUORUI et al.** *Linchuang Erke Zazhi*, 2013, vol. 31 (10), 905-909
- **VAN MIL et al.** *Gastroenterology.*, 2004, vol. 127 (2), 379-384
- **ANZIVINO et al.** *Dig Liver Dis*, 2013, vol. 45 (3), 226-232
- **PARK et al.** *World J Gastroenterol.*, 2016, vol. 22 (20), 4901-4907
- **IMAGAWA et al.** *J Hum Genet.*, 2018, vol. 63 (5), 569-577
- **HU et al.** *Mol Med Rep.*, 2014, vol. 10 (3), 1264-1274
- **LANG et al.** *Drug Metab Dispos.*, 2006, vol. 34 (9), 1582-1599
- **MASAHATA et al.** *Transplant Proc.*, 2016, vol. 48 (9), 3156-3162
- **HOLZ et al.** *Hepatol Commun.*, 2018, vol. 2 (2), 152-154
- **FRANCALANCI et al.** *Laboratory Investigation*, 2011, vol. 91 (1), 360A
- **FRANCALANCI et al.** *Digestive and Liver Disease*, 2010, vol. 42 (1), S16
- **SHAH et al.** *J Pediatr Genet.*, 2017, vol. 6 (2), 126-127
- **GAO et al.** *Hepatitis Monthly*, 2017, vol. 17 (10), e55087, 1-e55087, 6
- **EVASON et al.** *Am J Surg Pathol.*, 2011, vol. 35 (5), 687-696
- **DAVIT-SPRAUL et al.** *Mol Genet Metab.*, 2014, vol. 113 (3), 225-229
- **MAGGIORE et al.** *J Hepatol.*, 2010, vol. 53 (5), 981-6
- **LIU et al.** *Pediatr Int.*, 2013, vol. 55 (2), 138-144
- **WAISBOURD-ZINMAN et al.** *Ann Hepatol.*, 2017, vol. 16 (3), 465-468
- **GRIFFIN et al.** Meeting Info: 2016 Canadian Digestive Diseases Week, CDDW 2016. *Canadian Journal of Gastroenterology and Hepatology 2016*, 26 February 2016, vol. 2016
- **QIU et al.** *Hepatology*, 2017, vol. 65 (5), 1655-1669
- **IMAGAWA et al.** *Sci Rep*, 2017, vol. 7, 41806
- **KANG et al.** *J Pathol Transl Med.*, 16 May 2019
- **TAKAHASHI et al.** *Eur J Gastroenterol Hepatol.*, 2007, vol. 19 (11), 942-6
- **SHIMIZU et al.** *Am J Transplant.*, 2011, vol. 11 (2), 394-398
- **KRAWCZYK et al.** *Ann Hepatol.*, 2012, vol. 11 (5), 710-744
- **SATTLER et al.** International Liver Congress / 52nd Annual Meeting of the European-Association-for-the-Study-of-the-Liver.. *Journal of Hepatology 2017*, 19 April 2017, vol. 66 (1), S177
- 17th National Congress SIGENP. *Sciveres. Digestive and Liver Disease 2010*, 07 October 2010, vol. 42 (5), S329
- **SOHN et al.** *Pediatr Gastroenterol Hepatol Nutr.*, 2019, vol. 22 (2), 201-206
- **HO et al.** *Pharmacogenet Genomics.*, 2010, vol. 20 (1), 45-57
- **WANG et al.** *Hepatol Res.*, 2018, vol. 48 (7), 574-584
- **SHAPRIO et al.** *J Hum Genet.*, 2010, vol. 55 (5), 308-313
- ProQuest Dissertations & Theses. Dissertation Abstracts International. Bounford. University of Birmingham, 2016, vol. 75
- **JANKOWSKA et al.** *J Pediatr Gastroenterol Nutr.*, 2014, vol. 58 (1), 92-95
- **KIM**. 49th Annual Meeting of the European Society for Paediatric Gastroenterology. *Journal of Pediatric Gastroenterology and Nutrition 2016*, 25 May 2016, vol. 62 (1), 620
- **LI et al.** 26th Annual Conference of the Asian Pacific Association for the Study of the Liver, APASL 2017. *Hepatology International 2017*, 15 February 2017, vol. 11 (1), S362
- **RUMBO et al.** 27th International Congress of The Transplantation Society, TTS 2018. *Transplantation 2018*, 30 June 2018, vol. 102 (7), S848
- **LEE et al.** *Pediatr Gastroenterol Hepatol Nutr.*, 2017, vol. 20 (2), 114-123
- **SHERRIF et al.** *Liver international: official journal of the International Association for the Study of the Liver*, 2013, vol. 33 (8), 1266-1270
- **LIN et al.** *Zhongguo Dang Dai Er Ke Za Zhi.*, 2018, vol. 20 (9), 758-764
- **HARMANCI et al.** 1st Congress of the Turkic World Transplantation Society. Astana, Kazakhstan. *Experimental and Clinical Transplantation 2015*, 20 May 2015, vol. 13 (2), 76
- **HERBST et al.** *Mol Cell Probes.*, 2015, vol. 29 (5), 291-298
- **MOGHADAMRAD et al.** *Hepatology.*, 2013, vol. 57 (6), 2539-2541
- **HOLZ et al.** Viszeralmedizin 2016, 71. Jahrestagung der Deutschen Gesellschaft fur Gastroenterologie, Verdauungs- und Stoffwechselkrankheiten mit Sektion Endoskopie - 10. Herbsttagung der Deutschen Gesellschaft fur Allgemein- und Viszeralchirurgie. *Zeitschrift fur Gastroenterologie 2016*, 21 September 2016, vol. 54 (8)
- **HAO et al.** *International Journal of Clinical and Experimental Pathology*, 2017, vol. 10 (3), 3480-3487

- **ARNELL et al.** *J Pediatr Gastroenterol Nutr.*, 2010, vol. 51 (4), 494-499
- **SHARMA et al.** 58th Annual Conference of the Indian Society of Gastroenterology, ISGCON 2017. *Indian Journal of Gastroenterology 2017*, 14 December 2017, vol. 36 (1), A99
- **BEAUSÉJOUR et al.** *Can J Gastroenterol.*, 2011, vol. 25 (6), 311-314
- **IMAGAWA et al.** World Congress of Pediatric Gastroenterology, Hepatology and Nutrition 2016. *Journal of Pediatric Gastroenterology and Nutrition 2016*, 05 October 2016, vol. 63 (2), S51
- **PENG et al.** *Zhonghua er ke za zhi (Chinese journal of pediatrics)*, 2018, vol. 56 (6), 440-444
- **TIBESAR et al.** *Case Rep Pediatr*, 2014, vol. 2014, 185923
- **NG et al.** 51st Annual Meeting European Society for Paediatric Gastroenterology, Hepatology and Nutrition, ESPGHAN 2018. *Journal of Pediatric Gastroenterology and Nutrition 2018*, 09 May 2018, vol. 66 (2), 860
- **WONG et al.** *Clin Chem.*, 2008, vol. 54 (7), 1141-1148
- **JERICHO et al.** *Journal of Pediatric Gastroenterology and Nutrition. 60*, vol. 3, 368-374
- **SCHEIMANN et al.** Digestive Disease Week Meeting/108th Annual Meeting of the American-Gastroenterological-Association. *Gastroenterology 2007*, 19 May 2007, vol. 132 (4), A452
- **JAQUOTOT-HAERRANZ et al.** *Rev Esp Enferm Dig.*, 2013, vol. 105 (1), 52-54
- **KHOSLA et al.** 80th Annual Scientific Meeting of the American-College-of-Gastroenterology. *American Journal of Gastroenterology 2015*, 16 October 2015, vol. 110 (1), S397
- **LIU et al.** *Liver International*, 2010, vol. 30 (6), 809-815
- **GUNAYDIN, M. et al.** *Hepat Med.*, 2018, vol. 10, 95-104
- **FERSLEW et al.** *Dig Dis Sci.*, 2015, vol. 60, 3318-3328
- **CHALASANI et al.** *Hepatology*, 2018, vol. 67 (1), 328-357
- **KLEINER et al.** *Hepatology.*, 2005, vol. 41 (6), 1313-1321
- **DI LASCIO et al.** *Ultrasound Med Biol.*, 2018, vol. 44 (8), 1585-1596
- **LV et al.** *J Clin Transl Hepatol.*, 2018, vol. 6 (2), 217-221
- **REEDER et al.** *J Magn Reson Imaging*, 2011, vol. 34 (4)
- **LÉDINGHEN V et al.** *J Gastroenterol Hepatol.*, 2016, vol. 31 (4), 848-855
- **KLEINER et al.** *Hepatology*, 2005, vol. 41 (6), 1313-1321
- **BRUNT et al.** *Am J Gastroenterol*, 1999, vol. 94, 2467-2474
- **ANGULO et al.** *Hepatology*, 2007, vol. 45 (4), 846-54
- **BRUNT et al.** *Am. J. Gastroenterol.*, 1999, vol. 94, 2467-2474
- **ISHAK et al.** *J. Hepatol.*, 1995, vol. 22, 696-699
- **MCPHERSON et al.** *Gut*, 2010, vol. 59 (9), 1265-9
- **ADAMS et al.** *Clin. Chem.*, 2005, vol. 51 (10), 1867-1873
- **LICHTINGHAGEN R et al.** *J Hepatol*, August 2013, vol. 59 (2), 236-42
- **NEUMAN et al.** *Can. J. Gastroenterol. Hepatol.*, 2014, vol. 28 (11), 607-618
- **DANESE et al.** *PLoS One.*, 2017, vol. 12 (6), e0179200
- **PEREZ MJ** ; **BRIZ O** ; **WORLD J**. *Gastroenterol*, 2009, vol. 15 (14), 1677-1689
- **SORRENTINO P et al.** *Dig. Dis. Sci.*, 2005, vol. 50 (6), 1130-1135
- **SATAPATHY SK** ; **SANYAL AJ**. *Semin. Liver Dis.*, 2015, vol. 35 (3), 221-235
- **MCPHERSON et al.** *Gut.*, 2010, vol. 59 (9), 1265-1269
- **ADAMS et al.** *Clin Chem.*, 2005, vol. 51 (10), 1867-1873
- **LICHTINGHAGEN et al.** *J Hepatol.*, 2013, vol. 59 (2), 236-242
- **NEUMAN et al.** *Can J Gastroenterol Hepatol.*, 2014, vol. 28 (11), 607-618
- **P.G.M WUTZ** ; **T.W. GREENE**. Greene's Protective Groups in Organic Synthesis. John Wiley & Sons, 2006